(19) Europäisches Patentamt
European Patent Office
Office européen des brevets

(11) EP 2 036 887 A1

(12) EUROPEAN PATENT APPLICATION
published in accordance with Art. 153(4) EPC

(43) Date of publication:
**18.03.2009 Bulletin 2009/12**

(21) Application number: **07768337.3**

(22) Date of filing: **27.06.2007**

(51) Int Cl.:
***C07D 209/08*** *(2006.01)* ***A61K 31/404*** *(2006.01)*
***A61K 31/416*** *(2006.01)* ***A61P 3/04*** *(2006.01)*
***A61P 3/06*** *(2006.01)* ***A61P 3/10*** *(2006.01)*
***A61P 5/14*** *(2006.01)* ***A61P 5/16*** *(2006.01)*
***A61P 9/04*** *(2006.01)* ***A61P 9/06*** *(2006.01)*
***A61P 17/00*** *(2006.01)* ***A61P 17/02*** *(2006.01)*
***A61P 17/14*** *(2006.01)* ***A61P 19/10*** *(2006.01)*
***A61P 25/24*** *(2006.01)* ***A61P 27/06*** *(2006.01)*
***A61P 35/00*** *(2006.01)* ***A61P 43/00*** *(2006.01)*
***C07D 209/12*** *(2006.01)* ***C07D 231/56*** *(2006.01)*

(86) International application number:
**PCT/JP2007/063612**

(87) International publication number:
**WO 2008/001959 (03.01.2008 Gazette 2008/01)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **28.06.2006 JP 2006178548**

(71) Applicant: **Sanwa Kagaku Kenkyusho Co., Ltd**
**Nagoya-shi**
**Aichi 461-8631 (JP)**

(72) Inventors:
- **ASANO, Yukiyasu**
**Nagoya-shi, Aichi 461-8631 (JP)**
- **MAEDA, Koji**
**Nagoya-shi, Aichi 461-8631 (JP)**
- **TSURUTA, Nobuaki**
**Nagoya-shi, Aichi 461-8631 (JP)**
- **MURASE, Toru**
**Nagoya-shi, Aichi 461-8631 (JP)**

(74) Representative: **Thomas, Simon**
**Urquhart-Dykes & Lord LLP**
**30 Welbeck Street**
**London W1G 8ER (GB)**

# (54) NOVEL 6-5 BICYCIC HETEROCYCLIC DERIVATIVE AND MEDICAL USE THEREOF

(57) An object of the present invention is to provide a medicament as a thyroid hormone receptor ligand which is sufficient in drug efficacy and safety, and has the excellent action as a drug. The present invention provides a compound represented by the following general formula (I) or a pharmaceutically acceptable salt thereof:

[Chemical Formula 1]

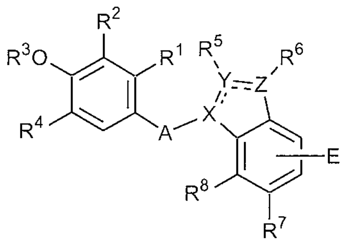

( I )

[wherein

[Chemical Formula 2] is a single bond or a double bond; A is $-CH_2-$ or -CO-; X, Y, and Z are each independently a nitrogen atom or a carbon atom; $R^1$ is a hydrogen atom or an aralkyl group; $R^2$ is an alkyl group or an aralkyl group, etc.; $R^3$ is a hydrogen atom or an alkyl group, etc.; $R^4$ is a hydrogen atom or an alkyl group; $R^5$ is a hydrogen atom, an alkyl group or a halo lower alkyl group, etc.; $R^6$ is a hydrogen atom or an alkyl group; $R^7$ is a hydrogen atom, etc.; $R^8$ is a hydrogen atom, or an alkyl group, etc.; and E is $-NHCO-G-COR^{12}$, etc. (wherein G is a single bond or an alkylene group, and $R^{12}$ is a hydroxy group or an alkoxy group)].

EP 2 036 887 A1

**Description**

BACKGROUND OF THE INVENTION

Field of the invention

The present invention relates to a novel 6-5 system bicyclic heterocyclic derivative and its pharmaceutical utility. The compound has various pharmaceutical utilities as a medicament as a thyroid hormone receptor ligand.

Description of the Related Art

Thyroid hormones promote differentiation, growth, and energy metabolism etc in animal, and play an important role in the metabolic homeostasis including metabolic regulations such as lipids, carbohydrates, proteins, inorganic salts. Conditions reflecting abnormal thyroid hormone levels are classified as hypothyroidism or hyperthyroidism. Hypothyroidism causes, for example, increase in blood cholesterol, weight gain, lowering of body temperature, reduction in cardiac function, bradycardia, alopecia, and depression. On the other hand, hyperthyroidism causes, for example, reduction in blood cholesterol, weight loss, increase of body temperature, increase in cardiac output, tachycardia, arrhythmia, and promotion of bone absorption.

Thyroid hormones, 3,3',5,5'-tetraiodo-L-thyronine (T4) and 3,3',5-triiodo-L-thyronine (T3), are currently used in thyroid hormone replacement therapy which is a mainstay of treatment for patients with hypothyroidism, and also in thyroid-stimulating hormone (TSH) suppression therapy of patients with thyroid nodule or thyroid cancer. Further, thyroid hormones have been tried to be used for treating hyperlipidemia, obesity, goiter, thyroid cancer, depression, and skin disease etc. However, prior attempts to utilize thyroid hormones are clinically restricted by manifestations of thyrotoxicosis such as discomfort feelings, and in particular by cardiovascular toxicity such as arrhythmia, angina, and heart failure, which are frequently recognized at a dose for the replacement therapy or higher.

Many of actions exhibited by thyroid hormones are exerted by binding of T3, which is an active hormone, to thyroid hormone receptors (TRs) present mainly in a nucleus. That is, a complex of T3 and thyroid hormone receptor is bound to a site called a thyroid hormone responsive element (TRE) in a transcriptional regulatory domain of a target gene, and then expression of the target gene is activated or suppressed. The action mediated by thyroid hormone receptors is called a "genomic effect".

Thyroid hormone receptors consists of two subtypes of TRα and TRβ, and it is suggested that these have some different roles in vivo. For example, since it has been revealed that many or most cardiotoxicities by thyroid hormones are mediated through TRα isoform, a compound having a selective activity to TRβ is expected to be a medicine having a lesser effect of cardiotoxicity.

On the other hand, it is thought that a part of thyroid hormone actions is not mediated through thyroid hormone receptors, and this is called a "nongenomic effect". In addition to T4 and T3, it has been suggested that metabolites of thyroid hormone are also concerned with the effects, in fact it was revealed that these have a binding activity to some receptors and some enzymes.

Recently, it was shown that a phosphorylation of PI3K signaling cascade is activated by association of the thyroid hormone receptor present in a cytoplasm with a regulatory subunit of phosphatidylinositol-3-kinase (PI3K). It suggested that the part of the nongenomic effect is mediated through the thyroid hormone receptor. This PI3K phosphorylation signaling cascade plays an important role in a variety of cell functions such as cell proliferation, uptake of glucose and so on, and this action is thought to perform the functional regulation in cooperation with the genomic effect.

From the foregoing, a compound having or regulating all or a part of actions of thyroid hormones is expected to be useful as an agent for treating or preventing, for example, hyperlipemia, obesity, hypothyroidism, hyperthyroidism, goiter, thyroid cancer, cardiac arrhythmia, congestive heart failure, diabetes, depression, osteoporosis, skin disorder, glaucoma, or alopecia.

Compounds having the thyroid hormone action have previously been developed, but many of them have, as a basic structure, a thyronine structure similar to that of the thyroid hormones, or their analogues (see for example, Expert Opin. Ther. Patent, 14(8), 1169-1183(2004)). In recent years, as compounds having 6-5 system bicyclic heterocycles, indole derivatives (see WO 00/051971, WO 01/070687, WO 02/051805, EP 1297833 A, and WO 2004/018421), indazole derivatives (see WO 02/022586), and a benzofuran derivatives (see WO 02/079181 and WO 96/05190) have been reported. However, these compounds are different from the present compound in a substituted phenyl group having a crosslinked part and a binding position of a side chain on a carboxylic acid side, or a 6-5 system bicyclic heterocycle is a substitute for the substituted phenyl group. Those compounds are greatly different in a structure from the present compound in which a substituted phenyl group having a crosslinked part is bound at a 5-membered ring part of a 6-5 system bicyclic heterocycle, and a carboxylic acid side chain is bound at a 6-membered ring part.

Besides, as the compound having a 6-5 system bicyclic heterocycle, there are following reports, but there is

no description regarding the action on the thyroid hormone receptor. First, EP 780386 A describes indole derivatives having the blood sugar and blood lipid reducing action. These compounds are compounds having a thiazolidinedione skeleton which is said to act on a PPARγ receptor. In addition, WO 97/10219 describes benzimidazole derivatives. These compounds are a V-type $H^+$-ATPase inhibitor, which dose not have a carboxylic acid side chain on a benzene ring of benzimidazole. Substituents at positions corresponding to meta positions of a crosslinked part of a substituted phenyl group having a crosslinked part of the present compound are all a hydrogen atom. Similarly, WO 2004/108686 also describes benzimidazole derivatives. These compounds are described to act on a PPARγ receptor, and have the blood sugar and blood lipid reducing action, and substituents at positions corresponding to meta positions of a crosslinked part of a substituted phenyl group having a crosslinked part of the present compound are also all a hydrogen atom. Further, WO 01/066520, WO 03/022813 and WO 2004/078719 disclose indole derivatives having the prostaglandin D receptor antagonism. Also in these compound, substituents at positions corresponding to meta positions of a crosslinked part of a substituted phenyl group having a crosslinked part of the present compound are all a hydrogen atom.

Previously, many compounds having the thyroid hormone action have been reported, but all compounds cannot be said to be sufficient from a viewpoint of drug efficacy and safety, and are not satisfactory as medicaments. Therefore, creation of thyroid hormone receptor ligands which selectively exerts an objective action, and is sufficiently satisfactory as medicaments is strongly desired.

SUMMARY OF THE INVENTION

In view of the above points, the present inventors thought that, as a means for solving the aforementioned problem, a compound having a fundamental structure different from a thyronine structure of the thyroid hormone is effective, and intensively continued to study aiming at creation of a medicament as a novel thyroid hormone receptor ligand. As a result, it has been found out that a compound represented by the following general formula (I) characterized by a 6-5 system bicyclic heterocycle and a salt thereof exhibit affinity for the thyroid hormone receptor, resulting in completion of the present invention.

That is, according to the present invention, there is provided a compound represented by the following general formula (I):

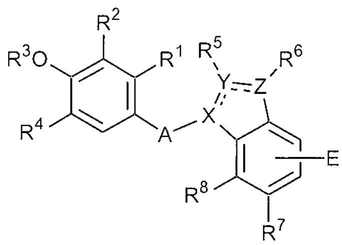

(I)

[wherein

means a single bond or a double bond;

A means $-CH_2-$ or -CO-;

X, Y, and Z each independently means a nitrogen atom or a carbon atom (provided that one or two of X, Y, and Z mean a nitrogen atom, and the rest means a carbon atom and, when Y and/or Z are a nitrogen atom, and form a double bond with one of the adjacent atoms, $R^5$ and/or $R^6$ are absent);

$R^1$ means a hydrogen atom or a C1-C6 alkyl group;

$R^2$ means a halogen atom, a C1-C6 alkyl group, a C2-C6 alkenyl group, a C3-C7 cycloalkyl group, a halo lower alkyl group, an alkanoyl group, an aryl group, a heteroaryl group, an aroyl group, an aralkyl group, a C1-C6 alkoxy group, an aryloxy group, an aralkyloxy group, $-(CH_2)_n-NR^9R^{10}$, $-CONR^9R^{10}$, $-NR^9COR^{11}$, $-S(O)_pR^{11}$, or $-SO_2NR^9R^{10}$, or means a 5- to 6-membered hydrocarbon ring which is formed by $R^1$ and $R^2$ together with the carbon atom to which they are bound (wherein n means an integer of 0 to 2, $R^9$ and $R^{10}$ each independently means a hydrogen atom, a C1-C6 alkyl group, a C2-C6 alkenyl group, a C3-C7 cycloalkyl group, an aryl group, a heteroaryl group, or an aralkyl group, or $R^9$ and $R^{10}$ mean a 5- to 6-membered heterocyclic ring which is formed by $R^9$ and $R^{10}$ together with the nitrogen to which they are bound, or alternatively, another nitrogen atom or oxygen atom, $R^{11}$ means a C1-C6 alkyl group, a C2-C6 alkenyl group, a C3-C7 cycloalkyl group, an aryl group, a heteroaryl group, or an aralkyl group, and p means an

integer of 0 to 2);
$R^3$ means a hydrogen atom, a C1-C6 alkyl group, or an acyl group;
$R^4$ means a hydrogen atom or a C1-C6 alkyl group;
$R^5$ means a hydrogen atom, a C1-C6 alkyl group, a halo lower alkyl group, or a cyano group;
$R^6$ means a hydrogen atom or a C1-C6 alkyl group;
$R^7$ means a hydrogen atom, a halogen atom, or a C1-C6 alkyl group;
$R^8$ means a hydrogen atom, a halogen atom, or a C1-C6 alkyl group; and
E means any group selected from the groups represented by the following formulae (a) to (f):

(a) $-NHCO-G-COR^{12}$,
(b) $-NH-G-COR^{12}$,
(c) $-O-G-COR^{12}$,
(d) $-CONH-G-COR^{12}$,
(e) -NHCO-G-tetrazolyl group, and
(f) $-G-COR^{12}$,

(wherein G means a single bond or a C1-C6 alkylene group, and $R^{12}$ means a hydroxy group or a C1-C6 alkoxy group], or a prodrug thereof, or a pharmacologically acceptable salt thereof, and these compounds are referred to as "present compound" hereinafter in the present specification. Various embodiments of the present compound are listed below.

A compound in which, in the general formula (I), $R^2$ is a halogen atom, a C1-C6 alkyl group, a C3-C7 cycloalkyl group, a halo lower alkyl group, an aryl group, a heteroaryl group, an aroyl group, an aralkyl group, or a C1-C6 alkoxy group, or a 5- to 6-membered hydrocarbon ring formed by $R^1$ and $R^2$ together with the carbon atom to which they are bound, $R^5$ is a hydrogen atom, a C1-C3 alkyl group, or a halo lower alkyl group, and $R^7$ is a hydrogen atom, or a halogen atom. A compound in which A in the general formula (I) is $-CH_2-$. A compound in which $R^1$, $R^3$, $R^4$, $R^6$, and $R^7$ in the general formula (I) are each a hydrogen atom. A compound in which $R^2$ in the general formula (I) is an i-propyl group, a s-butyl group, a 4-fluorobenzyl group, or a 4-(fluorophenyl)hydroxymethyl group. A compound in which $R^5$ in the general formula (I) is a methyl group or a trifluoromethyl group. A compound in which $R^8$ in the general formula (I) is a methyl group. A compound in which E in the general formula (I) is $-NHCO-G-COR^{12}$, wherein G is a single bond or $-CH_2-$, and $R^{12}$ is a hydroxy group or a C1-C3 alkoxy group. A compound in which one of X and Z in the general formula (I) is a nitrogen atom while the other is a carbon atom, and Y is a carbon atom. A compound described in the following general formula (II). A compound in which, in the following general formula (II), A is $-CH_2-$, $R^1$, $R^3$, $R^4$, $R^6$, and $R^7$ are each a hydrogen atom, $R^2$ is an i-propyl group, a s-butyl group, a 4-fluorobenzyl group, or a 4-(fluorophenyl)hydroxymethyl group, $R^5$ is a methyl group or a trifluoromethyl group, $R^8$ is a methyl group, and E is $-NHCO-G-OCR^{12}$ group, wherein G is a single group or $-CH_2-$, and
$R^{12}$ is a hydroxy group or a C1-C3 alkoxy group:

General formula (II)

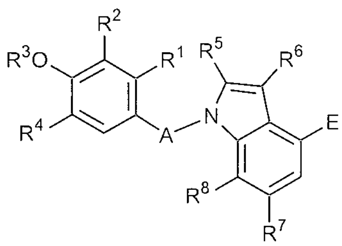

(I I)

[wherein all symbols are as defined in the general formula (I)].

The present invention further provides a pharmaceutical composition containing the present compound as an active ingredient. That is, the pharmaceutical composition of the present invention is used as an agent for treating or preventing a disease or a disorder, symptom of which is improved by cell functional regulation via a thyroid hormone receptor. Examples of the disease or the disorder, symptom of which is improved by cell functional regulation via a thyroid hormone receptor include hyperlipemia, obesity, hypothyroidism, hyperthyroidism, goiter, thyroid cancer, cardiac arrhythmia, congestive heart failure, diabetes, depression, osteoporosis, skin disorder, glaucoma, and alopecia.

When such the present invention is described by another expression, it results in use of the present compound for the manufacture of a medicament as a thyroid hormone receptor ligand. As used herein, the medicament as a thyroid

hormone receptor ligand is, for example, an agent for treating or preventing a disease or a disorder, symptom of which is improved by cell functional regulation via a thyroid hormone receptor. Such the disease or disorder is as described above.

Since the present compound has affinity for the thyroid hormone receptor, it can be used as a medicament as a thyroid hormone receptor ligand. Therefore, the present compound is useful as an agent for treating or preventing a disease or a disorder, symptom of which is improved by cell functional regulation via the thyroid hormone receptor, for example, hyperlipemia, obesity, hypothyroidism, hyperthyroidism, goiter, thyroid cancer, cardiac arrhythmia, congestive heart failure, diabetes, depression, osteoporosis, skin disorder, glaucoma, and alopecia. Since there are some compounds having high selectivity on and high affinity for TRβ of the thyroid hormone receptor among the present compounds, they are suitable for use as a medicament as a thyroid hormone receptor ligand having little side effect.

DETAILED DESCRIPTION OF THE INVENTION

The terms used in the present specification will be explained below.

The "thyroid hormone receptor ligand" means all compounds which bind to the thyroid hormone receptor, and the ligand may act as an agonist, an antagonist, a partial agonist or a partial antagonist, and is a so-called thyroid hormone receptor modulator. The thyroid hormone receptor is also called intranuclear T3 receptor.

The "C1-C6 alkyl group" means a straight or branched alkyl group consisting of 1 to 6 carbon atoms, and examples thereof include a methyl group, an ethyl group, a n-propyl group, an i-propyl group, a n-butyl group, an i-butyl group, a s-butyl group, a t-butyl group, a n-pentyl group, an i-pentyl group, a neo-pentyl group, a t-pentyl group, a n-hexyl group, an i-hexyl group, a 1-methylbutyl group, a 2-methylbutyl group, and a 1,2-dimethylpropyl group.

The "halogen atom" represents a fluorine atom, a chlorine atom, a bromine atom, or an iodine atom.

The "C2-C6 alkenyl group" means a non-cyclic straight or branched alkenyl group consisting of 2 to 6 carbon atoms, containing one or more double bonds, and examples thereof include a 2-propynyl group and a 3-butynyl group.

The "C3-C7 cycloalkyl group" means a cyclic hydrocarbon atom consisting of 3 to 7 carbon atoms, and examples thereof include a cyclopropyl group, a cyclobutyl group, a cyclopentyl group, and a cyclohexyl group.

The "halo lower alkyl group" means a C1-C6 alkyl group in which an arbitrary hydrogen atom of the C1-C6 alkyl group is substituted with 1 to 5 same or different kinds of halogen atoms, and examples thereof include a trifluoromethyl group and a 2,2,2-trifluoroethyl group.

The "alkanoyl group" means a group represented by (C1-C6 alkyl)-CO-, (C3-C7 cycloalkyl)-CO-, (halo lower alkyl)-CO-, or (aralkyl)-CO-, and examples thereof include an acetyl group, a propionyl group, an i-butyryl group, a pivaloyl group, a cyclopentylcarbonyl group, a cyclohexylcarbonyl group, a trifluoroacetyl group, and a phenylacetyl group.

The "aryl group" means a monocyclic or bicyclic hydrocarbon consisting of 6 to 10 carbon atoms, and an arbitrary hydrogen atom on a ring of the aryl group may be substituted with a halogen atom, a C1-C6 alkyl group, a halo lower alkyl group, a C2-C6 alkenyl group, a C2-C6 alkynyl group, a C3-C7 cycloalkyl group, a heterocycloalkyl group, a hydroxy group, a C1-C6 alkoxy group, an aryloxy group, an acyloxy group, an optionally substituted amino group, a mercapto group, a C1-C6 alkylthio group, an acyl group, a carboxylic acid group, a C1-C6 alkoxycarbonyl group, an optionally substituted aminocarbonyl group, a C1-C6 alkylsulfonyl group, an optionally substituted aminosulfonyl group, a nitro group, a cyano group, an aryl group, a heteroaryl group, an aralkyl group or the like. Examples include a phenyl group, a 4-fluorophenyl group, a 3,4-difluorophenyl group, a 2-chlorophenyl group, a 4-chlorophenyl group, a 4-trifluorophenyl group, a 4-hydroxyphenyl group, a 1-naphthyl group, and a 2-naphthyl group.

The "heteroaryl group" means a 5- or 6-membered monocyclic or bicyclic aromatic heterocycle consisting of 1 to 9 carbon atoms, and 1 to 4 hetero atoms such as nitrogen, oxygen and sulfur. Examples of the monocyclic aromatic heterocycle include a furyl group, a thienyl group, a pyrrolyl group, an imidazolyl group, a pyrazolyl group, a thiazolyl group, an isothiazolyl group, an oxazolyl group, a triazolyl group, a tetrazolyl group, a pyridyl group, a pyrimidinyl group, a pyridazinyl group, a pyrazinyl group, and an oxopyridazinyl group. Examples of the bicyclic aromatic heterocycle include benzofuranyl, benzothienyl, benzothiadiazolyl, benzothiazolyl, benzimidazolyl, indolyl, isoindolyl, indazolyl, quinolyl, isoquinolyl, cinnolinyl, quinazolinyl, quinoxalinyl, and benzodioxolyl. An arbitrary hydrogen atom on a ring of the heteroaryl group may be substituted with a halogen atom, a C1-C6 alkyl group, a halo lower alkyl group, a C2-C6 alkenyl group, a C2-C6 alkynyl group, a C3-C7 cycloalkyl group, a heterocycloalkyl group, a hydroxy group, a C1-C6 alkoxy group, an aryloxy group, an acyloxy group, an optionally substituted amino group, a mercapto group, a C1-C6 alkylthio group, an acyl group, a carboxylic acid group, a C1-C6 alkoxycarbonyl group, an optionally substituted aminocarbonyl group, a C1-C6 alkylsulfonyl group, an optionally substituted aminosulfonyl group, a nitro group, a cyano group, an aryl group, a heteroaryl group, an aralkyl group or the like.

The "aroyl group" means a group represented by (aryl)-CO-, or (heteroaryl)-CO-, and examples thereof include a benzoyl group, a 4-fluorobenzoyl group, a 1-naphthoyl group, a 2-naphthoyl group, and a pyridine-2-carbonyl group.

The "aralkyl group" means a C1-C6 alkyl group substituted with an aryl group or a heteroaryl group, and a hydrogen atom on a C1-C6 alkyl chain part may be substituted with a hydroxyl group if necessary. Examples of the

aralkyl group include a benzyl group, a 4-fluorobenzyl group, a 4-(fluorophenyl)hydroxymethyl group, a phenethyl group, and a (6-oxo-1,6-dihydropyridazin-3-yl)methyl group.

The "C1-C6 alkoxy group" means a group represented by (C1-C6 alkyl) -O-, and examples thereof include a methoxy group, an ethoxy group, a n-propoxy group, an i-propoxy group, a n-butoxy group, an i-butoxy group, a s-butoxy group, a t-butoxy group, a n-pentoxy group, an i-pentoxy group, a neo-pentoxy group, a t-pentoxy group, a 1-methylbutoxy group, a 2-methylbutoxy group, a 1,2-dimethylpropoxy group, and a n-hexyloxy group.

The "aryloxy group" means a group represented by (aryl)-O-, and examples thereof include a phenoxy group and a 4-fluorophenoxy group.

The "aralkyloxy group" means a group represented by (aralkyl)-O-, and examples thereof include a benzyloxy group and a phenethyloxy group.

The "acyl group" means a group represented by the alkanoyl group or the aroyl group.

The "C1-C6 alkylene group" means a straight or branched alkylene group consisting of 1 to 6 carbon atoms, and a hydrogen atom on the alkylene group is optionally substituted with a halogen atom, a C1-C6 alkyl group, or an aralkyl group. Examples thereof include a fluoromethylene group, a methylmethylene group, a benzylmethylene group, and a dimethylmethylene group.

The "C2-C6 alkynyl group" means a non-cyclic straight or branched alkynyl group consisting of 2 to 6 carbon atoms, and containing one or more triple bonds, and examples thereof include an ethynyl group, a 1-propynyl group, a 2-propynyl group, a 1-butynyl group, a 2-butynyl group, a 3-butynyl group, a 3-pentynyl group, a 2-hexynyl group, a 3-hexynyl group, and a 1-methyl-2-propynyl group.

The "acyloxy group" means a group represented by (alkanoyl)-O- or (aroyl)-O-, and examples thereof include an acetyloxy group and a benzoyloxy group.

The "heterocycloalkyl group" means a 3- to 6-membered saturated heterocycle containing at least one or more of a nitrogen atom, an oxygen atom or a sulfur atom in a ring, and examples thereof include a pyrrolidyl group, a piperidyl group, a morpholinyl group, a tetrahydrofuryl group, a tetrahydropyranyl group, and a tetrahydrothienyl group.

The "optionally substituted amino group" means an amino group, or an amino group in which 1 or 2 hydrogen atoms on the amino group are substituted with a C1-C6 alkyl group, a C3-C7 cycloalkyl group, an acyl group, or an aralkyl group, and examples thereof include an amino group, a methylamino group, a dimethylamino group, an ethylmethylamino group, an acetylamino group, a benzoylamino group, and a benzylamino group.

The "C1-C6 alkylthio group" means a group represented by (C1-C6 alkyl)-S-.

The "C1-C6 alkoxycarbonyl group" means a group represented by (C1-C6 alkoxy)-CO-.

The "optionally substituted aminocarbonyl group" means a group represented by (optionally substituted amino)-CO-.

The "C1-C6 alkylsulfonyl group" means a group represented by (C1-C6 alkyl)-$SO_2$-.

The "optionally substituted aminosulfonyl group" means a group represented by (optionally substituted amino)-$SO_2$-.

The "prodrug" means a compound which is converted into the general formula (I) by a reaction with an enzyme or gastric, etc. acid under the physiological condition in a living body. Such a prodrug is included in the scope of the present invention, and various prodrugs are known in the art. Examples of the prodrug when the compound represented by the general formula (I) has a carboxylic acid group include a compound in which the carboxylic acid group is esterified or amidated (for example, ethyl-esterified, carboxymethyl-esterified, pivaloyloxymethylated, or methyl-amidated). Examples of the prodrug when the compound represented by the general formula (I) has a hydroxy group include a compound in which the hydroxy group is alkylated, acylated, or phosphorylated (for example, methylated, acetylated, or succinylated). Examples of the prodrug when the compound represented by the general formula (I) has an amino group include a compound in which the amino group is acylated, alkylated, or phosphorylated (for example, eicosanoylated, alanylated, pentylaminocarbonylated, tetrahydrofuranylated, pyrrolidylmethylated, acetoxymethylated, or t-butylated).

The "pharmacologically acceptable salt" means a salt which retains the biological effectiveness and the property of the compound represented by the general formula (I), and is not disadvantageous in a biological or other viewpoints. Such the pharmacologically acceptable salt is included in the scope of the present invention. Examples of the pharmacologically acceptable salt include inorganic acid addition salts (salts with hydrochloric acid, hydrobromic acid, hydroiodic acid, sulfuric acid, phosphoric acid etc.), organic acid addition salts (for example, salts with methanesulfonic acid, benzenesulfonic acid, p-toluenesulfonic acid, formic acid, acetic acid, trifluoroacetic acid, oxalic acid, citric acid, malonic acid, fumaric acid, glutaric acid, adipic acid, maleic acid, tartaric acid, succinic acid, mandelic acid, malic acid, pantothenic acid, methylsulfuric acid or the like), salts with amino acids (for example, salts with lysine, arginine or the like), alkali metal addition salts (for example, salts with sodium, potassium, lithium or the like), alkaline earth metal addition salts (for example, salts with calcium, magnesium or the like), organic amine addition salts (for example, salts with ammonia, ethylamine, t-butylamine, diethylamine, diisopropylamine, triethylamine, tributylamine, dimethylpropylamine, morpholine, thiomorpholine, piperidine, pyrrolidine, monoethanolamine, diethanolamine or the like), and the like. A reaction of

forming these addition salts can be performed according to a conventional method.

The present invention will be described in detail below. The present compound is a compound represented by the following general formula (I), or a prodrug thereof, or a pharmacologically acceptable salt thereof. A particularly important place in order that the present compound has affinity for the thyroid hormone receptor is $R^2$, $OR^3$, and E, and the present compound can be discriminated from the prior art compounds by them, That is, $R^2$ is a substituent other than a hydrogen atom, and is preferably a substituent having a molecular size to some extent. When a substituent of $R^2$ is a hydrogen atom, affinity for the thyroid hormone receptor of the ligand is remarkably reduced. In addition, $R^3$ must be bound to a benzene ring via an oxygen atom. Further, E is required to be a carboxylic acid derivative or an equivalent thereof.

It is reported in "Progress of Medicinal Chemistry, 17, 151-183(1980) that a tetrazolyl group is equivalent to a carboxylic acid group. Enhancement of affinity, addition of selectivity, and improvement in pharmacokinetics due to conversion of a carboxylic acid group into a tetrazolyl group are reported, and it is expected also in the present compound that a compound having a tetrazolyl group has the similar effect.

When one or more asymmetric carbons are present in the present compound, the present invention includes both of isomers based on an asymmetric carbon, and a compound of an arbitrary combination of them. In addition, when a geometric isomer or a tautomer is present in the present compound, the present invention includes both of the geometric isomer and the tautomer. Further, the present compound includes a solvate with a pharmaceutically acceptable solvent such as water and ethanol.

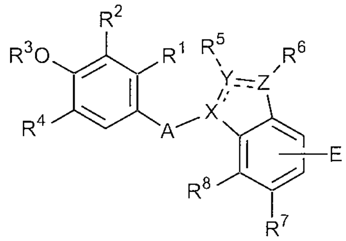

( I )

In the formula, 
means a single bond or a double bond.

A is $-CH_2-$ or -CO-, preferably $-CH_2-$.

X, Y, and Z are each independently a nitrogen atom or a carbon atom, one or two of X, Y, and Z are a nitrogen atom, and the rest is a carbon atom. When Y and/or Z are a nitrogen atom, and are taken together with one of the adjacent atoms to form a double bond, $R^5$ and/or $R^6$ are absent. Regarding X, Y, and Z, the case where one of X and Z is a nitrogen atom, and the other is a carbon atom, and Y is a carbon atom is preferable and, inter alia, the case where X is a nitrogen atom, and Y and Z are a carbon atom is optimal.

$R^1$ is a hydrogen atom or a C1-C6 alkyl group, preferably a hydrogen atom or a C1-C3 alkyl group and, inter alia, optimally a hydrogen atom.

$R^2$ is a halogen atom, a C1-C6 alkyl group, a C2-C6 alkenyl group, a C3-C7 cycloalkyl group, a halo lower alkyl group, an alkanoyl group, an aryl group, a heteroaryl group, an aroyl group, an aralkyl group, a C1-C6 alkoxy group, an aryloxy group, an aralkyloxy group, $-(CH_2)_n-NR^9R^{10}$, $-CONR^9R^{10}$, $-NR^9COR^{11}$, $-S(O)_pR^{11}$, or $-SO_2NR^9R^{10}$, or a 5- to 6-membered hydrocarbon ring which is formed by $R^1$ and $R^2$ together with the carbon atom to which they are bound. In this case, n is an integer of 0 to 2, and $R^9$ and $R^{10}$ are each independently a hydrogen atom, a C1-C6 alkyl group, a C2-C6 alkenyl group, a C3-C7 cycloalkyl group, an aryl group, a heteroaryl group, or an aralkyl group, or a 5- to 6-membered heterocycle which is formed by $R^9$ and $R^{10}$ together with the nitrogen atom to which they are bound or alternatively, together with another nitrogen atom or an oxygen atom. $R^{11}$ is a C1 - C6 alkyl group, a C2-C6 alkenyl group, a C3-C7 cycloalkyl group, an aryl group, a heteroaryl group, or an aralkyl group, and p is an integer of 0 to 2. Among them, $R^2$ is preferably a halogen atom; a C2-C5 alkyl group; a C3-C5 cycloalkyl group; a halo lower alkyl group; a phenyl group in which an arbitrary hydrogen atom of the phenyl group may be substituted with same or different one to two halogen atoms or a C1-C3 alkyl group; a pyridinyl group; an aralkyl group which is a C1-C3 alkyl group substituted with a phenyl group in which an arbitrary hydrogen atom of the phenyl group may be substituted with same or different one to two halogen atoms or C1-C3 alkyl group, and in which an arbitrary hydrogen atom of an alkyl chain part may be substituted with a hydroxy group; a C2-C5 alkoxy group; a phenoxy group in which an arbitrary hydrogen atom of a phenyl group may be substituted with same or different one to two halogen atoms or a C1-C3 alkyl group; a C3-C7 cycloalkylcarbamoyl group; a phenylcarbamonyl group; a piperidinecarbonyl group; a benzenesulfonyl group in which

an arbitrary hydrogen atom of a phenyl group may be substituted with same or different one to two halogen atoms or a C1-C3 alkyl group; or a C3-C7 cycloalkylsulfamoyl group and, inter alia, optimally an i-propyl group, a s-butyl group, a 4-fluorobenzyl group, or a 4-(fluorophenyl)hydroxymethyl group.

$R^3$ is a hydrogen atom, a C1-C6 alkyl group or an acyl group, among them, preferably a hydrogen atom or a C1-C3 alkyl group and, inter alia, optimally a hydrogen atom.

$R^4$ is a hydrogen atom or a C1-C6 alkyl group, among them, preferably a hydrogen atom or a C1-C3 alkyl group and, inter alia, optimally a hydrogen atom.

$R^5$ is a hydrogen atom, a C1-C6 alkyl group, a halo lower alkyl group or a cyano group, among them, preferably a hydrogen atom, a C1-C3 alkyl group, or a trifluoromethyl group, and, inter alia, optimally a methyl group or a trifluoromethyl group.

$R^6$ is a hydrogen atom or a C1-C6 alkyl group, among them, preferably a hydrogen atom or a C1-C3 alkyl group and, inter alia, optimally a hydrogen atom.

$R^7$ is a hydrogen atom, a halogen atom or a C1-C6 alkyl group, among them, preferably a hydrogen atom, a halogen atom or a C1-C3 alkyl group and, inter alia, optimally a hydrogen atom.

$R^8$ is a hydrogen atom, a halogen atom or a C1-C6 alkyl group, among them, preferably a hydrogen atom, a halogen atom or a C1-C3 alkyl group and, inter alia, optimally a methyl group.

E is -NHCO-G-COR$^{12}$, -NH-G-COR$^{12}$, -O-G-COR$^{12}$, -CONH-G-COR$^{12}$, a -NHCO-G-tetrazolyl group, or -G-COR$^{12}$. In this case, G is a single bond or a C1-C6 alkylene group, and $R^{12}$ is a hydroxy group or a C1-C6 alkoxy group. Among them, E is preferably -NHCO-G-COR$^{12}$ or a -NHCO-G-tetrazolyl group. G is preferably a single bond or a C1-C3 alkylene group and, among them, optimally a single bond or $-CH_2-$. $R^{12}$ is preferably a hydroxy group or a C1-C3 alkoxy group.

The present compound in which a substitution position of E, as well as X, Y, and Z are most preferable is described by the following general formula (II).

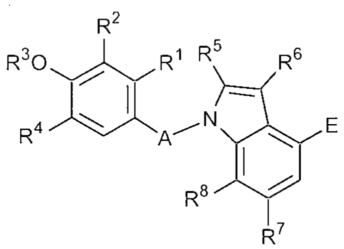

(I I)

A compound is optimal in which, in this general formula (II), A is $-CH_2-$, $R^1$, $R^3$, $R^4$, $R^6$, and $R^7$ are each a hydrogen atom, $R^2$ is an i-propyl group, a s-butyl group, a 4-fluorobenzyl group, or a 4-(fluorophenyl)hydroxymethyl group, $R^5$ is a methyl group or a trifluoromethyl group, $R^8$ is a methyl group, and E is- NHCO-G-COR$^{12}$ (wherein G is a single bond or a $-CH_2-$, and $R^{12}$ is a hydroxy group or a C1-C3 alkoxy group).

The compound represented by the general formula (I) which is the present compound can be produced by a method shown in the following reaction step formulae I to VII, a method described in Examples, or a combination of them and the known method.

[Reaction step formula I]

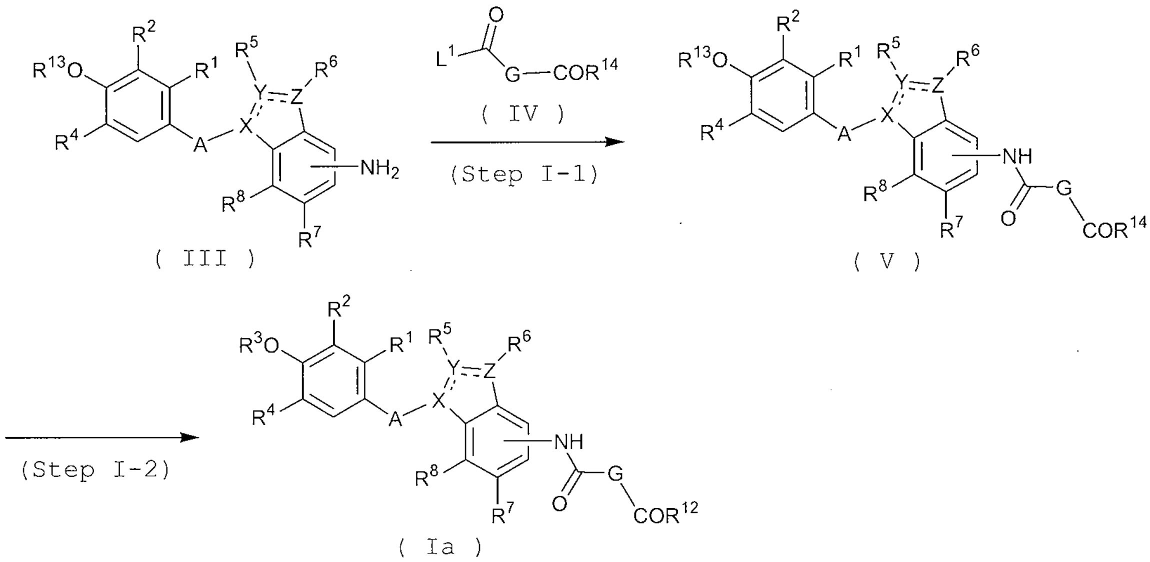


[wherein $R^{13}$ is a hydrogen atom, or a protecting group of a phenolic hydroxy group (for example, a C1-C6 alkyl group, an acyl group, a triisopropylsilyl group, a t-butyldimethylsilyl group, a benzyl group, a methoxymethyl group etc.), $L^1$ is a chlorine atom, a bromine atom, an iodine atom, a hydroxy group, or a C1-C6 alkoxy group, $R^{14}$ is a hydroxy group, or a protecting group of a carboxylic acid group (for example, a C1-C6 alkoxy group, a benzyloxy group etc.), and other symbols represent the same meanings as those of the aforementioned general formula.]

[Step I-1]

When $L^1$ of a compound represented by the general formula (IV) is a chlorine atom, a bromine atom or an iodine atom, a compound represented by the general formula (V) is obtained by reacting a compound represented by the general formula (III) and a compound represented by the general formula (IV) in a suitable solvent (for example, dichloromethane, N,N-dimethylformamide, tetrahydrofuran or the like) using a base (for example, triethylamine, pyridine or the like). A reaction temperature is -20°C to a boiling point of the solvent, and a reaction time is 30 minutes to 48 hours.

When $L^1$ of a compound represented by the general formula (IV) is a hydroxy group, a compound represented by the general formula (V) is obtained by reacting a compound represented by the general formula (III) and a compound represented by the general formula (IV) in a suitable solvent (for example, dichloromethane, N,N-dimethylformamide, tetrahydrofuran etc.) in the presence or the absence of an additive (for example, 4-dimethylaminopyridine, 1-hydroxybenzotriazole or the like) using a condensing agent (for example, 1-ethyl-3-(dimethylaminopropyl)carbodiimide, dicyclohexylcarbodiimide or the like). A reaction temperature is -20°C to a boiling point of the solvent, and a reaction time is 30 minutes to 48 hours.

When $L^1$ of a compound represented by the general formula (IV) is a C1-C6 alkoxy group, a compound represented by the general formula (V) is obtained by reacting a compound represented by the general formula (III) and a compound represented by the general formula (IV) without a solvent or in a suitable solvent (for example, toluene, xylene or the like). A reaction temperature is 80°C to a boiling point of the solvent, and a reaction time is 30 minutes to 48 hours.

Herein, the compound represented by the general formula (IV) is commercially available, or can be produced by a method described in Example or a known method.

[Step I-2]

When $R^{13}$ and/or $R^{14}$ of a compound represented by the general formula (V) are a protecting group, a compound represented by the general formula (Ia) in which $R^3$ is a hydrogen atom and/or $R^{12}$ is a hydroxy group is obtained by removing the protecting group according to the method described in "Protecting Groups in Organic Synthesis", 3rd Edition, Wiley (1999).

[Reaction step formula II]

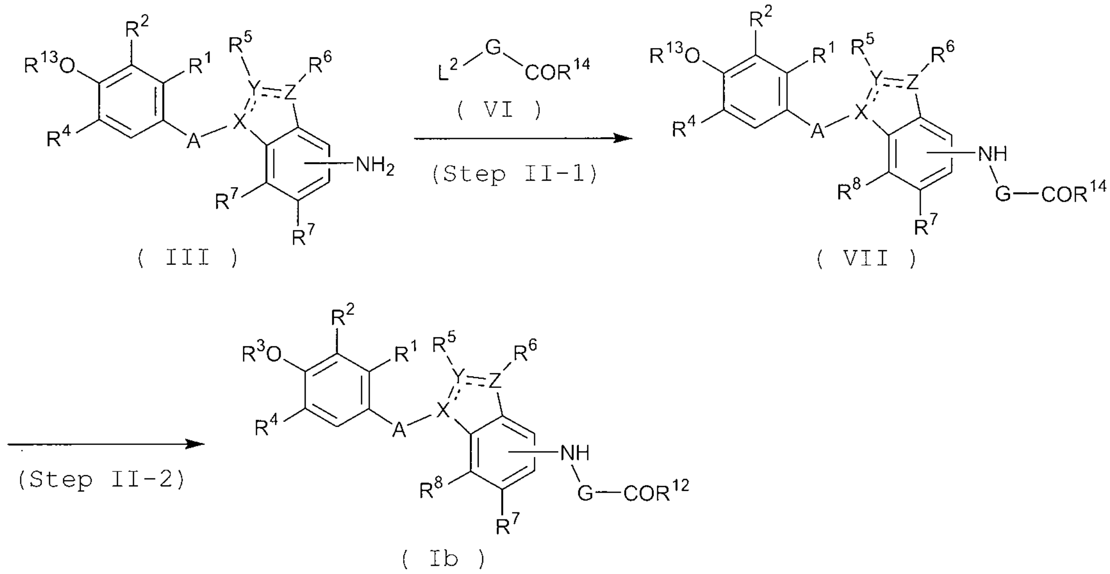


(wherein $L^2$ means a chlorine atom, a bromine atom or an iodine atom, and other symbols have the same meanings as those of the aforementioned general formula and the aforementioned reaction step formula.)

[Step II-1]

A compound represented by the general formula (VII) is obtained by reacting a compound represented by the general formula (III) and a compound represented by the general formula (VI) in a suitable solvent (for example, N,N-dimethylformamide, acetone, tetrahydrofuran, toluene, dichloromethane, acetonitrile, water or the like) using a base (for example, potassium carbonate, sodium hydride, sodium hydroxide, N,N-diisopropylethylamine or the like). A reaction temperature is -50°C to a boiling point of the solvent, and a reaction time is 30 minutes to 48 hours. The reaction may be performed using, as an additive, an iodide salt (for example, sodium iodide, potassium iodide or the like) and/or a phase transfer catalyst (for example, tetrabutylammonium bromide, tetrabutylammonium iodide or the like), if necessary. Herein, the compound represented by the general formula (VI) is commercially available, or can be produced using the known method.

[Step II-2]

A compound represented by the general formula (Ib) is obtained by reacting a compound represented by the general formula (VII) by the same method as that of the Step I-2.

[Reaction step formula III]

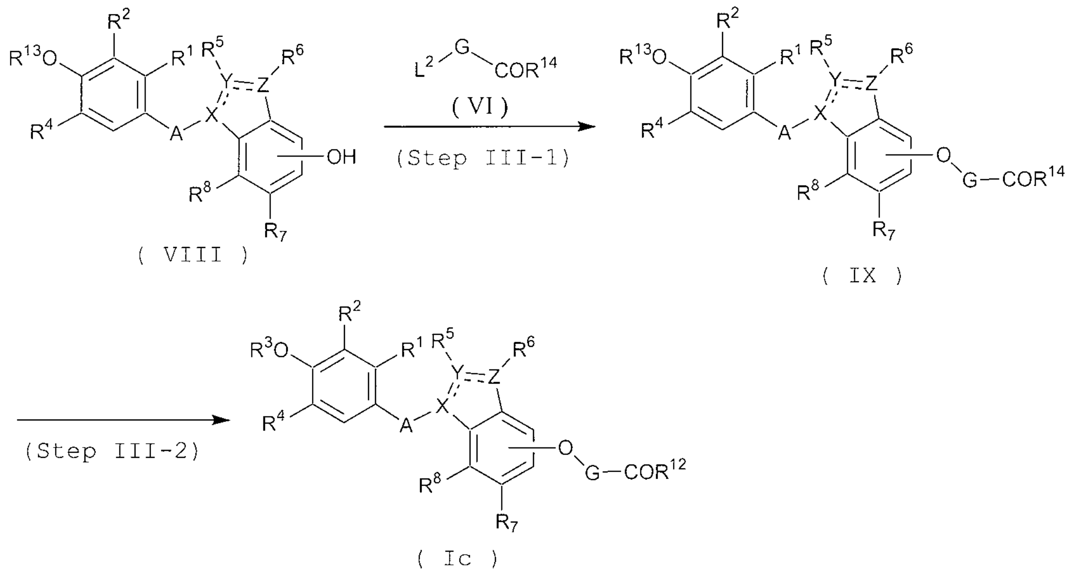


(wherein all symbols have the same meanings as those of the aforementioned general formula and the aforementioned reaction step formula.)

[Step III-1]

A compound represented by the general formula (IX) is obtained by reacting a compound represented by the general formula (VIII) and a compound represented by the general formula (VI) in a suitable solvent (for example, acetone, N,N-dimethylformamide, tetrahydrofuran, toluene, dichloromethane, acetonitrile or the like) using a base (for example, potassium carbonate, cesium carbonate, sodium bicarbonate, N,N-diisopropylethylamine or the like). A reaction temperature is room temperature to a boiling point of the solvent, and a reaction time is 30 minutes to 48 hours. The reaction may be performed using, as an additive, an iodide salt (for example, sodium iodide, potassium iodide or the like) and/or a phase transfer catalyst (for example, tetrabutylammonium bromide, tetrabutylammonium iodide or the like), if necessary.

[Step III-2]

A compound represented by the general formula (Ic) is obtained by reacting a compound represented by the general formula (IX) by the same method as that of the Step I-2.

[Reaction step formula IV]

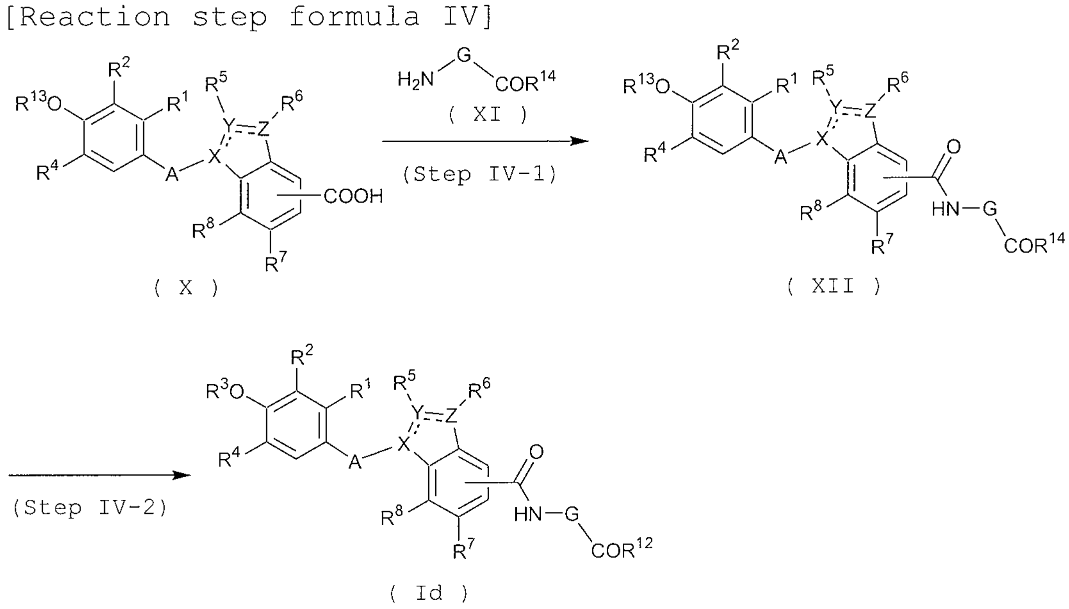


( X ) ( XII ) ( Id )

(wherein all symbols have the same meanings as those of the aforementioned general formula and the aforementioned reaction step formula.)

[Step IV-1]

A compound represented by the general formula (XII) is obtained by converting the general formula (X) into acid halide without a solvent or in a suitable solvent (for example, dichloromethane, toluene or the like) using a halogenating agent (for example, thionyl chloride, oxalyl chloride or the like), and reacting the acid halide with a compound represented by the general formula (XI) in a suitable solvent (for example, dichloromethane, N,N-dimethylformamide, tetrahydrofuran etc.) using a base (for example, triethylamine, pyridine or the like). A reaction temperature is -20°C to a boiling point of a solvent, and a reaction time is 30 minutes to 48 hours.

Alternatively, a compound represented by the general formula (XII) is obtained by reacting a compound represented by the general formula (X) and a compound represented by the general formula (XI) in a suitable solvent (for example, dichloromethane, N,N-dimethylformamide, tetrahydrofuran or the like) in the presence or the absence of an additive (for example, 4-dimethylaminopyridine, 1-hydroxybenzotriazole or the like) using a condensing agent (for example, 1-ethyl-3-(dimethylaminopropyl)carbodiimide, dicyclohexylcarbodiimide or the like). A reaction temperature is -20°C to a boiling point of the solvent, and a reaction time is 30 minutes to 48 hours.

Herein, the compound represented by the general formula (XI) is commercially available, or can be produced using the known method.

[Step IV-2]

A compound represented by the general formula (Id) is obtained by reacting a compound represented by the general formula (XII) by the same method as that of the Step I-2.

[Reaction step formula V]

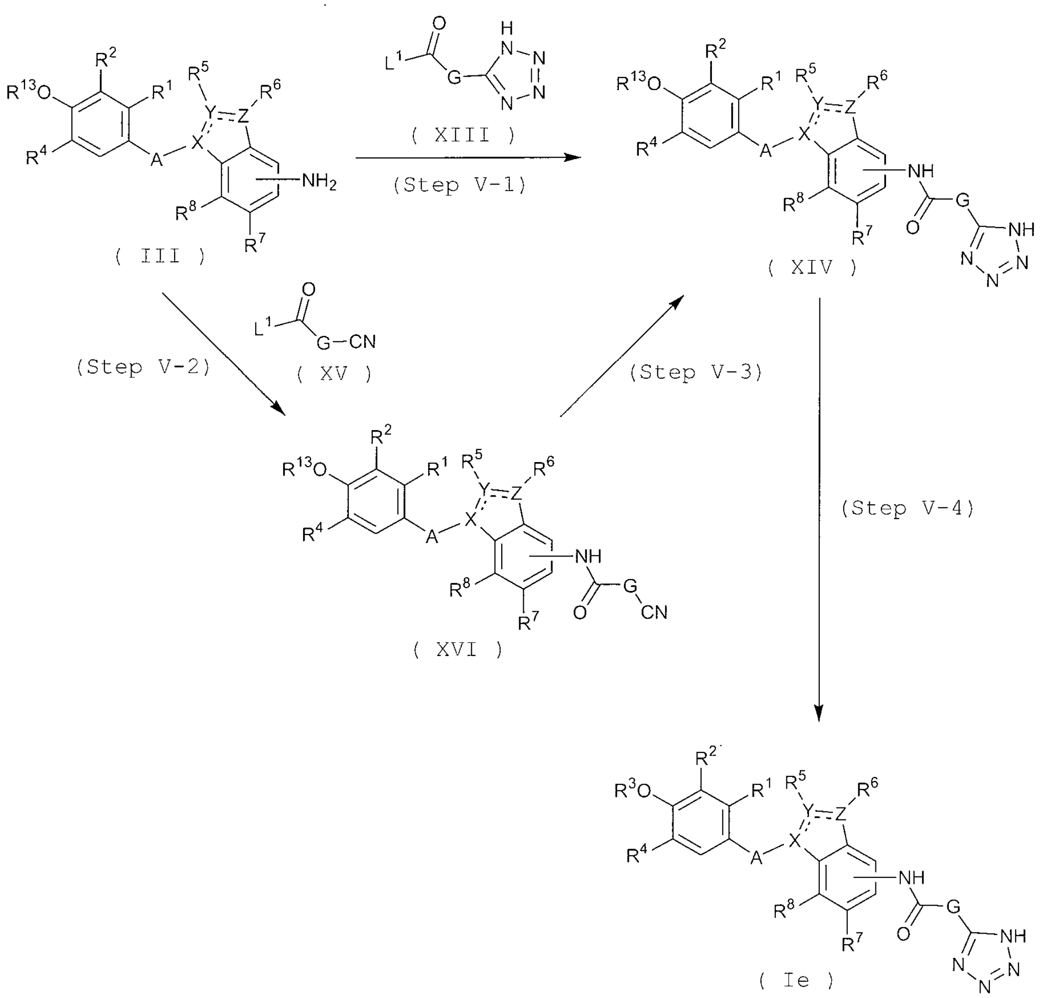


(wherein all symbols have the same meanings as those of the aforementioned general formula and the aforementioned reaction step formula.)

[Step V-1]

A compound represented by the general formula (XIV) is obtained by reacting a compound represented by the general formula (III) and a compound represented by the general formula (XIII) by the same method as that of the step I-1. Herein, the compound represented by the general formula (XIII) is commercially available, or can be produced using the known method.

[Step V-2]

A compound represented by the general formula (XIV) is obtained by reacting a compound represented by the general formula (III) and a compound represented by the general formula (XV) by the same method as that of the Step I-1. Herein, the compound represented by the general formula (XIV) is commercially available, or can be produced using the known method.

[Step V-3]

A compound represented by the general formula (XIV) is obtained by reacting a compound represented by the general formula (XIV) in a suitable solvent (for example, N,N-dimethylformamide, toluene, xylene etc.) using an azidating

agent (for example, sodium azide/ammonium chloride, tin azide compound (for example, tributyltin azide, trimethyltin azide or the like) or the like). A reaction temperature is 0°C to a boiling point of the solvent, and a reaction time is 30 minutes to 48 hours.

[Step V-4]

When $R^{13}$ of a compound represented by the general formula (XIV) is a protecting group, a compound represented by the general formula (Ie) in which $R^3$ is a hydrogen atom is obtained by removing the protecting group according to the method described in "Protecting Groups in Organic Synthesis", 3rd Edition, Wiley (1999).

[Reaction step formula VI]

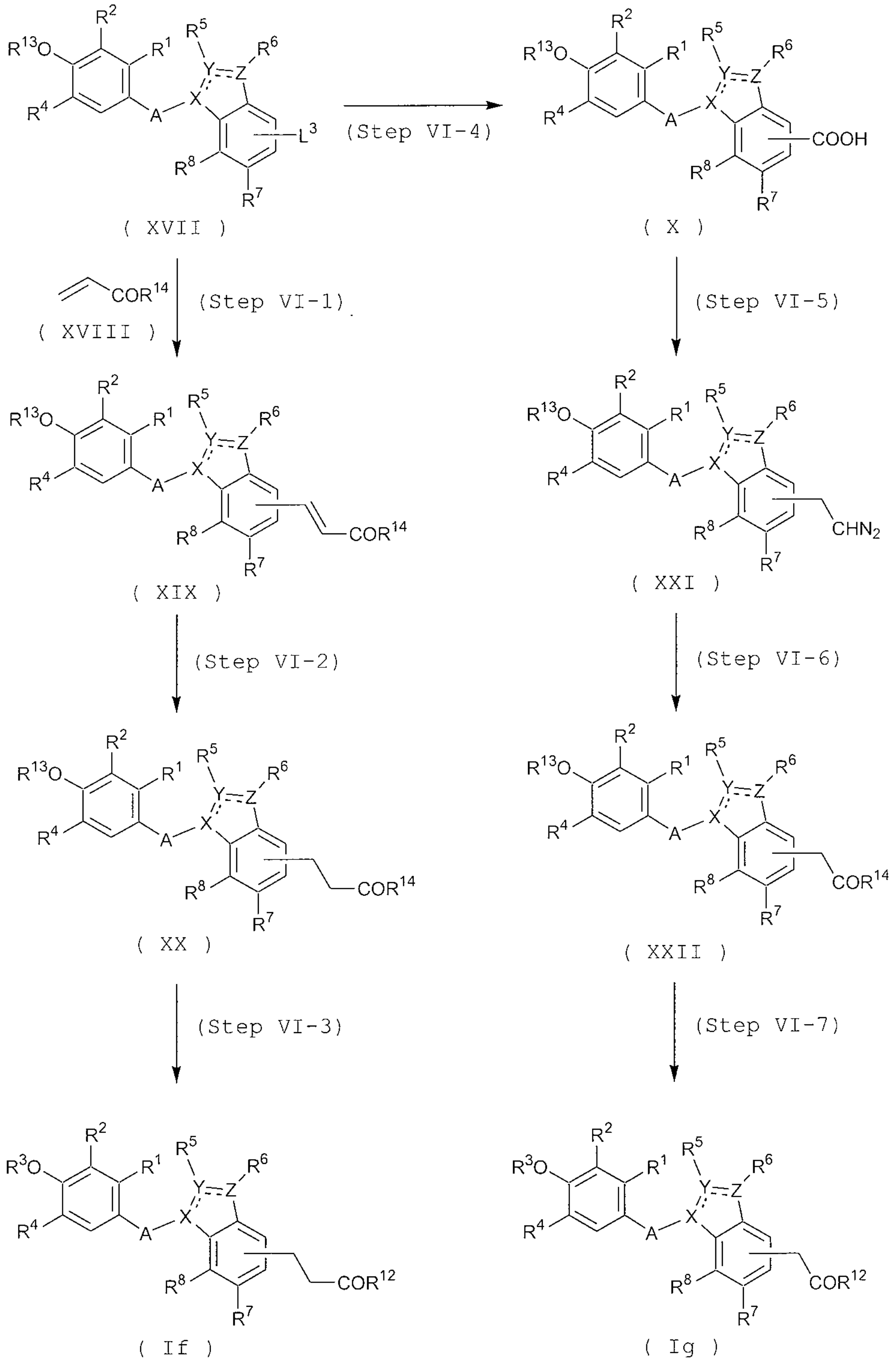


(wherein a $L^3$ group means a trifluoromethanesulfonyloxy group, a bromine atom, or an iodine atom, and other symbols have the same meanings as those of the aforementioned general formula and the aforementioned reaction step formula.)

[Step VI-1]

A compound represented by the general formula (XIX) is obtained by reacting a compound represented by the

general formula (XVII) and a compound represented by the general formula (XVIII) in a suitable solvent (for example, N,N-dimethylformamide, N,N-dimethylacetamide, acetonitrile or the like) using a palladium catalyst (for example, palladium acetate, tetrakis(triphenylphosphine)palladium (0) or the like), a ligand (for example, triphenylphosphine etc.), and a base (for example, triethylamine, potassium acetate or the like) according to the method described in "J. Med. Chem.", 46(9), 1580-1588(2003). A reaction temperature is 60°C to a boiling point of the solvent, and a reaction time is 30 minutes to 48 hours. Herein, the compound represented by the general formula (XVIII) is commercially available, or can be produced using the known method.

[Step VI-2]

A compound represented by the general formula (XX) is obtained by reacting a compound represented by the general formula (XIX) in a suitable solvent (for example, ethanol, methanol, tetrahydrofuran or the like) under the hydrogen atmosphere at 1 to 5 atm using a metal catalyst (for example, palladium/active carbon, platinum oxide or the like). A reaction temperature is 0°C to a boiling point of the solvent, and a reaction time is 30 minutes to 48 hours.

[Step VI-3]

A compound represented by the general formula (If) is obtained by reacting a compound represented by the general formula (XX) by the same method as that of the Step I-2.

[Step VI-4]

A compound represented by the general formula (X) is obtained by reacting a compound represented by the general formula (XVII) in a suitable solvent (for example, toluene, xylene, tetrahydrofuran, N,N-dimethylformamide, acetonitrile etc.) under the carbon monoxide atmosphere at 1 to 5 atm using a palladium catalyst (for example, bis (triphenylphosphine)dichloropalladium (II), bis(benzonitrile)palladium (II), palladium acetate or the like), a ligand (for example, triphenylphosphine etc.), and a base (for example, sodium hydroxide, triethylamine, sodium acetate etc.). A reaction temperature is 60°C to a boiling point of the solvent, and a reaction time is 30 minutes to 48 hours. The reaction may be performed using, as an additive, a phase transfer catalyst (for example, tetrabutylammonium iodide etc.), if necessary.

[Step VI-5]

A compound represented by the general formula (XXI) is obtained by converting a compound represented by the general formula (X) into acid halide without a solvent or in a suitable solvent (for example, dichloromethane, toluene etc.) using a halogenating agent (for example, thionyl chloride, oxalyl chloride etc.), and reacting the acid halide in a suitable solvent (for example, dichloromethane, diethyl ether, tetrahydrofuran etc.) using a diazotizing agent (for example, diazomethane, trimethylsilyldiazomethane etc.). A reaction temperature is 0°C to 50°C, and a reaction time is 30 minutes to 48 hours.

[Step VI-6]

A compound represented by the general formula (XXII) is obtained by reacting a compound represented by the general formula (XXI) without a solvent or in a suitable solvent (tetrahydrofuran, diethyl ether, dioxane etc.) using a silver salt (for example, silver nitrate, silver oxide etc.) and an alcohol (for example, ethanol, methanol, benzyl alcohol etc.). A reaction temperature is 0°C to a boiling point of the solvent, and a reaction time is 30 minutes to 48 hours.

[Step VI-7]

A compound represented by the general formula (Ig) by reacting a compound represented by the general formula (XXII) by the same method as that of the Step I-2.

[Reaction step formula VII]

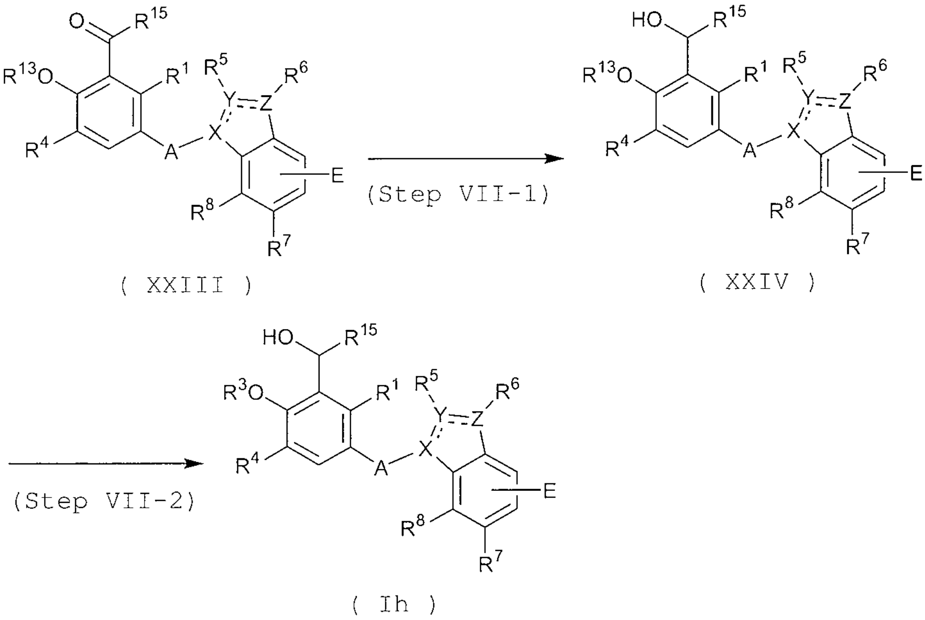


( XXIII ) ( XXIV ) ( Ih )

(wherein $R^{15}$ means a C1-C6 alkyl group, an aryl group, or a heteroaryl group, and other symbols have the same meanings as those of the aforementioned general formula and the aforementioned reaction step.)

[Step VII-1]

A compound represented by the general formula (XXIV) is obtained by reacting a compound represented by the general formula (XXIII) in a suitable solvent (for example, tetrahydrofuran, diethyl ether etc.) using acetic acid and sodium borohydride according to the method described in "Tetrahedron Lett.", 28(40), 4725-4728(1987). A reaction temperature is -50°C to a boiling point of the solvent, and a reaction time is 30 minutes to 48 hours.

[Step VII-2]

A compound represented by the general formula (Ih) is obtained by reacting a compound represented by the general formula (XXIV) by the same method as that of the Step I-2.

A compound represented by the general formula (III) used as a starting material in the Reaction step formulae I, II and V can be produced by a method shown in the following Reaction step formula VIII, a method described in Examples, or the known method.

[Reaction step formula VIII]

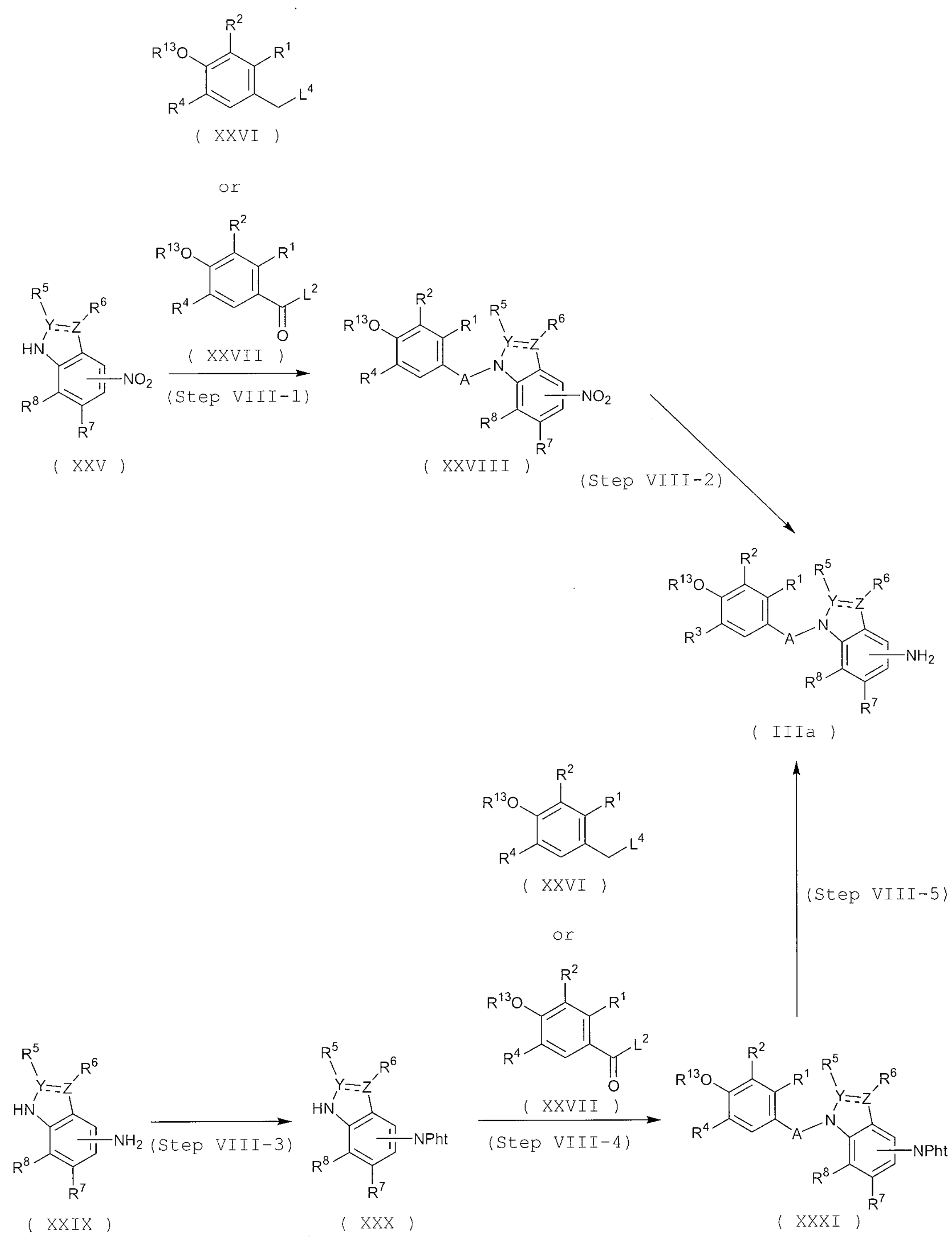


( XXVI )

or

( XXVII )

( XXV )

( XXVIII )

( IIIa )

( XXVI )

or

( XXVII )

( XXIX )

( XXX )

( XXXI )

(wherein $L^4$ means a chlorine atom, a bromine atom, an iodine atom, a methanesulfonyloxy group, or a p-toluenesulfonyloxy group, Pht means a phthaloyl group, and other symbols have the same meanings as those of the aforementioned general formula and the aforementioned reaction step formula.)

[Step VIII-1]

A compound represented by the general formula (XXVIII) is obtained by reacting a compound represented by the general formula (XXV), and a compound represented by the general formula (XXVI) or a compound represented by

the general formula (XXVII) in a suitable solvent (for example, acetone, N,N-dimethylformamide, tetrahydrofuran, toluene, dichloromethane, acetonitrile etc.) using a base (for example, sodium hydride, potassium carbonate, cesium carbonate, sodium bicarbonate, sodium hydroxide, N,N-diisopropylethylamine etc.). A reaction temperature is -78°C to a boiling point of the solvent, and a reaction time is 30 minutes to 48 hours. The reaction may be performed using, as an additive, an iodide salt (for example, sodium iodide, potassium iodide etc.) and/or a phase transfer catalyst (for example, tetrabutylammonium bromide, tetrabutylammonium iodide etc.), if necessary.

When the compound represented by the general formula (XXVIII) is an indoline compound, an indole compound is obtained by reacting the compound in a suitable solvent (for example, dioxane, toluene, chloroform etc.) using an oxidizing agent (for example, 2,3-dichloro-5,6-dicyano-1,4-benzoquinone, manganese dioxide, chloranil, sarcomine/oxygen etc.), if necessary. A reaction temperature is -20°C to a boiling point of the solvent, and a reaction time is 30 minutes to 48 hours.

[Step VIII-2]

A compound represented by the general formula (IIIa) is obtained by reacting a compound represented by the general formula (XXVIII) in a suitable solvent (for example, ethanol, tetrahydrofuran, ethyl acetate etc.) under the hydrogen atmosphere at 1 to 5 atm using a metal catalyst (for example, palladium/active carbon, platinum oxide, Raney nickel etc.). A reaction temperature is 0°C to a boiling point of the solvent, and a reaction time is 30 minutes to 48 hours. In addition, the compound represented by the general formula (IIIa) is also obtained by reacting the compound represented by the general formula (XXVIII) using the metal catalyst and a formate salt (for example, ammonium formate, potassium formate etc.) or a reducing metal salt or a metal (for example, tin dichloride, zinc, iron etc.).

[Step VIII-3]

A compound represented by the general formula (XXX) is obtained by introducing a phthaloyl group into an amino group of a compound represented by the general formula (XXIX) according to the method described in "Protecting Groups in Organic Synthesis", 3rd Edition, Wiley (1999).

[Step VIII-4]

A compound represented by the general formula (XXXI) is obtained by reacting a compound represented by the general formula (XXX), and a compound represented by the general formula (XXVI) or a compound represented by the general formula (XXVII) by the same reaction as that of the Step VIII-1.

[Step VIII-5]

A compound represented by the general formula (IIIa) is obtained by removing a phthaloyl group of the compound represented by the general formula (XXXI) according to the method described in "Protecting Groups in Organic Synthesis", 3rd Edition, Wiley (1999).

The compound represented by the general formula (VIII) used as a starting material in the Reaction step formula III can be produced by the method shown in the following Reaction step formula IX, a method described in Examples, or a combination with the known method.

[Reaction step formula IX]

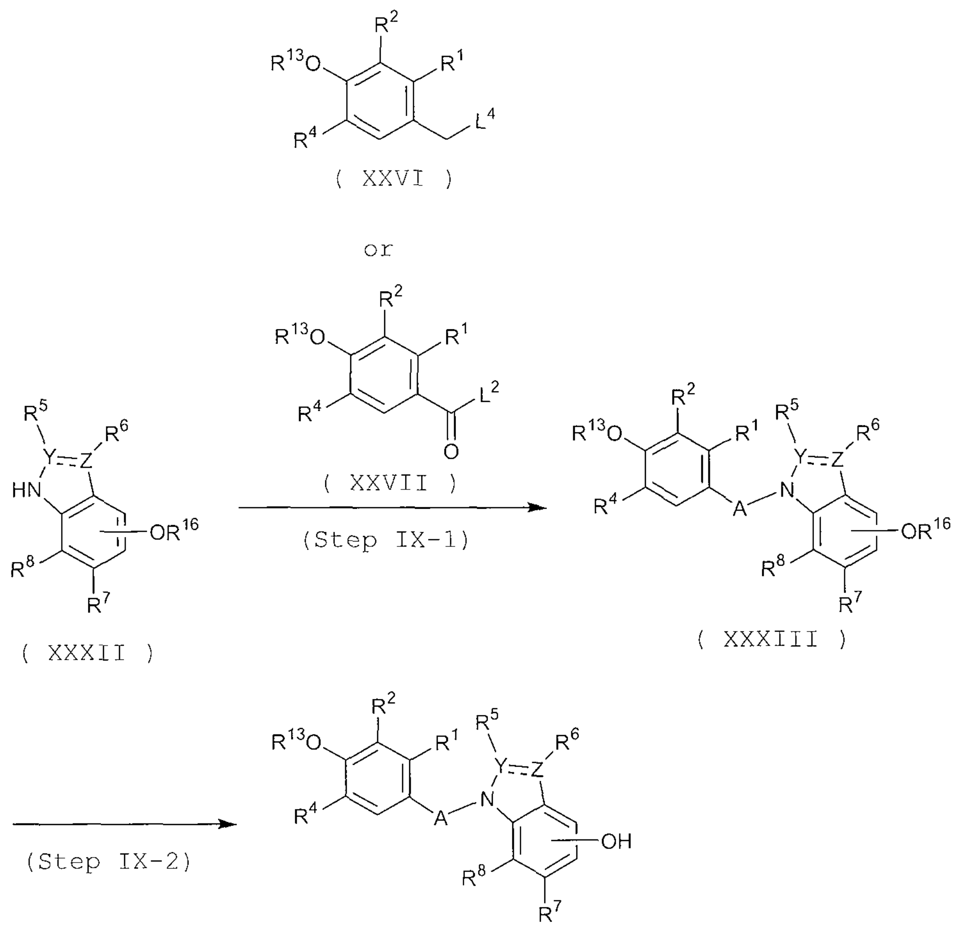


( XXVI ) or ( XXVII ) ( XXXII ) ( XXXIII ) ( VIIIa )

[wherein $R^{16}$ means a protecting group of a phenolic hydroxy group (for example, C1-C6 alkyl group, acyl group, triisopropylsilyl group, t-butyldimethylsilyl group, benzyl group, methoxymethyl group etc.), and other symbols have the same meanings as those of the aforementioned general formula and the aforementioned Reaction step formula.]

[Step IX-1]

A compound represented by the general formula (XXXIII) is obtained by reacting a compound represented by the general formula (XXXII), and a compound represented by the general formula (XXVI) or a compound represented by the general formula (XXVII) by the same method as that of the Step VIII-1.

[Step IX-2]

A compound represented by the general formula (VIIIa) is obtained by removing a protecting group $R^{16}$ of the compound represented by the general formula (XXXIII) according to the method described in "Protecting Groups in Organic Synthesis", 3rd Edition, Wiley (1999).

The compound represented by the general formula (X) used as a starting material in the Reaction step IV can be produced by the method shown in the following Reaction step formula X, a method described in Examples, or a combination with the known method.

[Reaction step formula X]

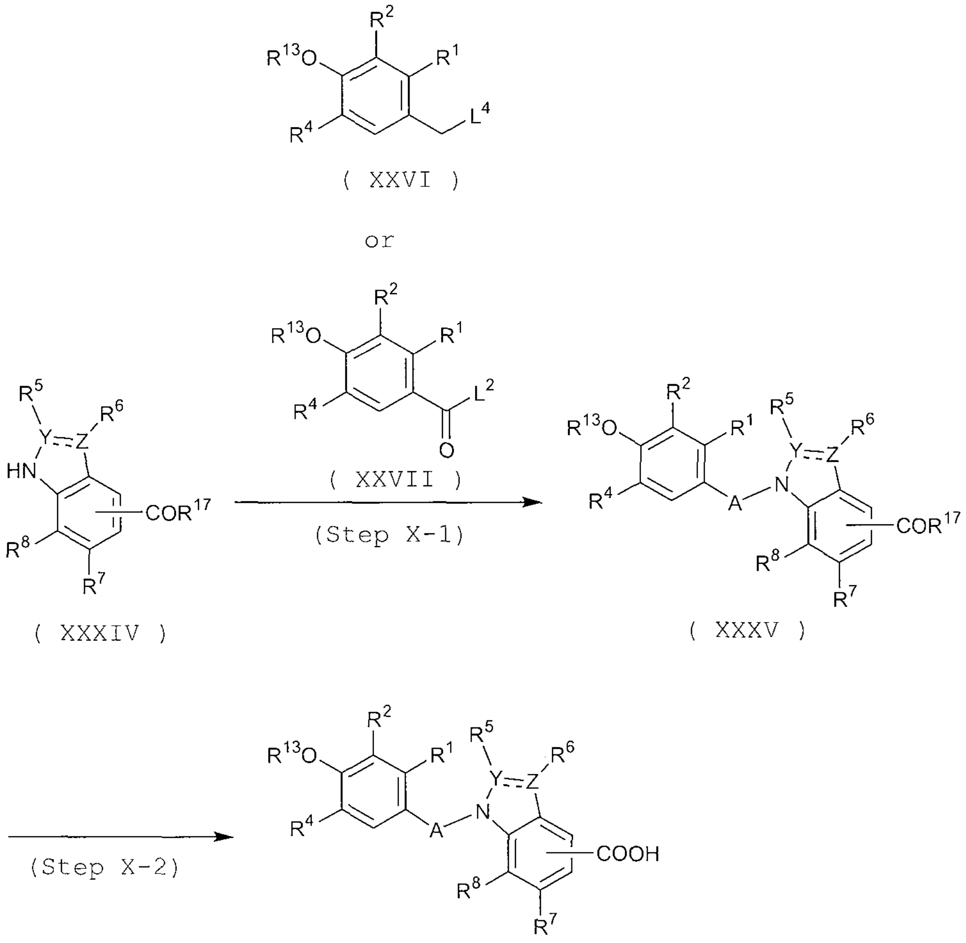

( XXVI )

or

( XXVII )

( XXXIV )

( XXXV )

( Xa )

[wherein $R^{17}$ means a protecting group of a carboxylic acid group (for example, C1-C6 alkoxy group, benzyloxy group etc.), and other symbols have the same meanings as those of the aforementioned general formula and the aforementioned Reaction step formula.]

[Step X-1]

A compound represented by the general formula (XXXV) is obtained by reacting a compound represented by the general formula (XXXIV), and a compound represented by the general formula (XXVI) or a compound represented by the general formula (XXVII) by the same reaction as that of the Step VIII-1.

[Step X-2]

A compound represented by the general formula (Xa) is obtained by removing a protecting group $R^{17}$ of the compound represented by the general formula (XXXV) according to the methods described in "Protecting Groups in Organic Synthesis", 3rd Edition, Wiley (1999).

The compound represented by the general formula (XVII) used as a starting material in the Reaction step formula VI can be produced by the method shown in the following Reaction step formula XI, a method described in Examples, or a combination with the known method.

[Reaction step formula XI]

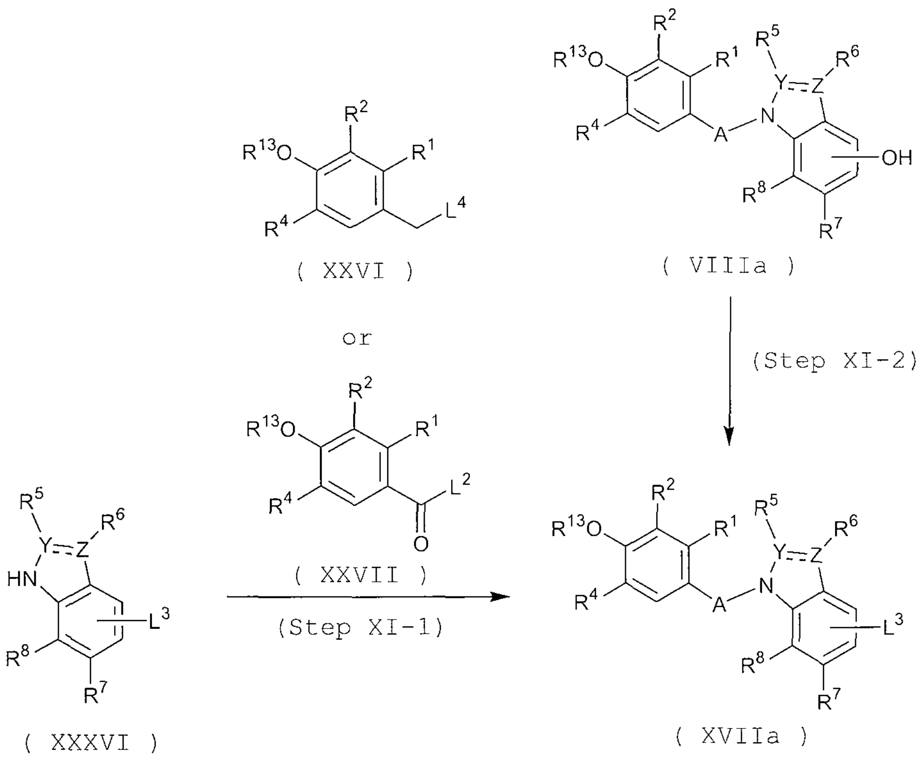


(XXVI) or (XXVII) (VIIIa) (XXXVI) (XVIIa)

(wherein all symbols have the same meanings as those of the aforementioned general formula and the aforementioned Reaction step formula.)

[Step XI-1]

A compound represented by the general formula (XVIIa) is obtained by reacting a compound represented by the general formula (XXXVI), and a compound represented by the general formula (XXVI) or a compound represented by the general formula (XXVII) by the same reaction as that of the Step VIII-1.

[Step XI-2]

A compound represented by the general formula (XVIIa) is obtained by reacting a compound represented by the general formula (VIIIa) in a suitable solvent (for example, dichloromethane, tetrahydrofuran, N,N-dimethylformamide etc.) using a base (for example, triethylamine, pyridine, 2,6-lutidine, dimethylaminopyridine etc.) and a trifluorosulfonating agent (for example, trifluoromethanesulfonic anhydride, trifluoromethanesulfonyl chloride etc.).

The compound represented by the general formula (III) and (VIII) used as a starting material in Reaction step formulae I to VI, which is represented by the general formula (XL), can be produced by the method shown in the following Reaction step formula (XII), a method described in Examples, or a combination with the known method.

[Reaction step formula XII]

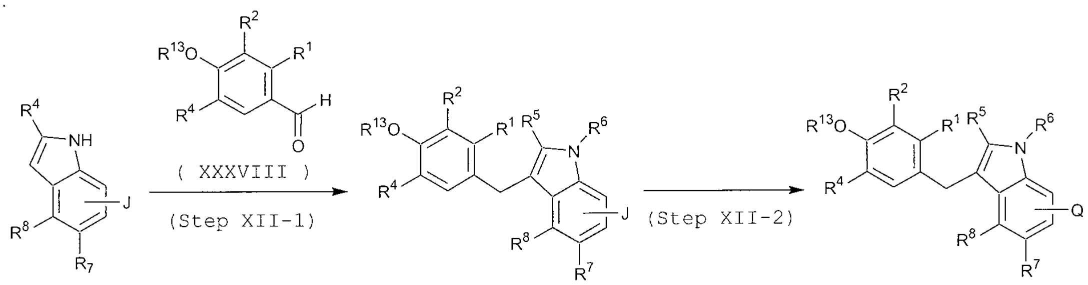


( XXXVII ) ( XXXIX ) ( XL )

(wherein J means a nitro group, -NPht, or $OR^{16}$, Q means an amino group or a hydroxy group, and other symbols have the same meaning as those of the aforementioned general formula and the aforementioned Reaction step formula.)

[Step XII-1]

A compound represented by the general formula (XXXIX) is obtained by reacting a compound represented by the general formula (XXXVII) and a compound represented by the general formula (XXXVIII) in dichloromethane using an acid (for example, trifluoroacetic acid, boron trifluoride diethyl ether complex etc.) and triethylsilane. A reaction temperature is -20°C to room temperature, and a reaction time is 10 minutes to 48 hours. A compound represented by the general formula (XXXIX) in which $R^5$ is a C1-C6 alkyl group is obtained by reacting the resulting compound in a suitable solvent (for example, N,N-dimethylformamide, tetrahydrofuran, toluene etc.) using a base (for example, sodium hydride, sodium hydroxide, potassium carbonate, cesium carbonate etc.) and C1-C6 alkyl halide (for example, methyl iodide, ethyl bromide etc.), if necessary. A reaction temperature is -20°C to a boiling point of a solvent, and a reaction time is 10 minutes to 48 hours.

[Step XII-2]

When J of the compound represented by the general formula (XXXIX) is a nitro group, a compound represented by the general formula (XL) in which Q is an amino group is obtained by reacting, using the same method as that of Step VIII-2.

When J of the compound represented by the general formula (XXXIX) is a NPht group, a compound represented by the general formula (XL) in which Q is an amino group is obtained by reacting, using the same method as that of the Step VIII-5.

When J represented by the general formula (XXXIX) is $-OR^{16}$, a compound represented by the general formula (XL) in which Q is a hydroxy group is obtained by reacting, using the same method as that of Step IX-2.

The compound represented by the general formula (III) and (VIII) used as a starting material in the Reaction step formulae I to VI, which is represented by the general formula (XLIII), can be produced by the method shown in the following Reaction step formula XIII, a method described in Examples or a combination with the known method.

[Reaction step formula XIII]

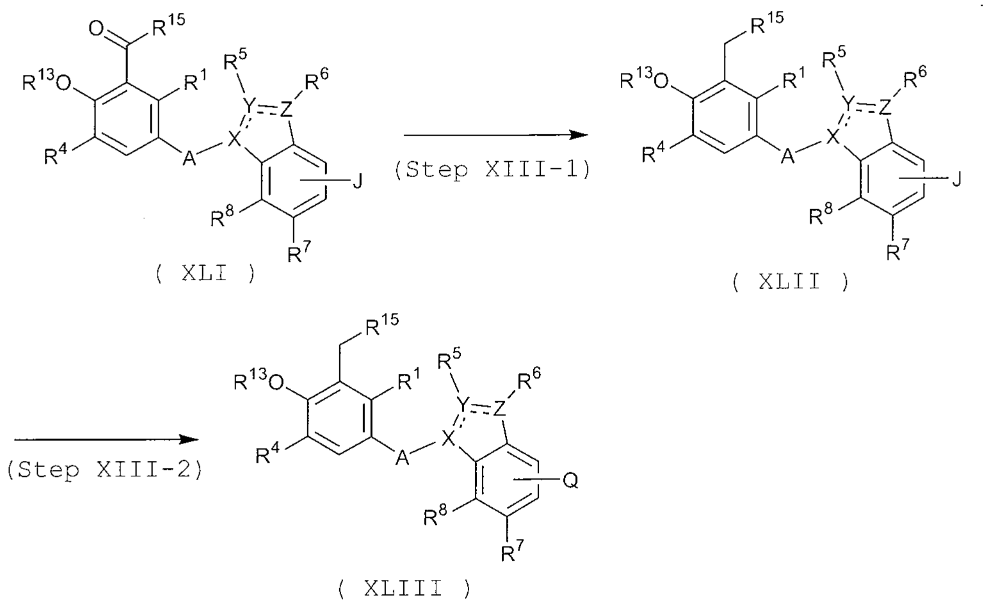


( XLI ) ( XLII ) ( XLIII )

(wherein all symbols have the same meanings as those of the aforementioned general formula and the aforementioned Reaction step formula.)

[Step XIII-1]

A compound represented by the general formula (XLII) is obtained by reacting a compound represented by the general formula (XLI) in trifluoroacetic acid using triethylsilane according to the method described in "J. Med. Chem.", 38(4), 695-707(1995). A reaction temperature is 0°C to a boiling point of the solvent, and a reaction time is 30 minutes to 48 hours.

[Step XIII-2]

A compound represented by the general formula (XLIII) is obtained by reacting a compound represented by the general formula (XLII) by the same method as that of the Step XII-2.

The compound represented by the general formula (III) and (VIII) used as a starting material in the Reaction step formulae I to VI, which is represented by the general formula (L), can be produced by the method shown in the following Reaction step formula XIV, a method described in Examples, or a combination with the known method.

[Reaction step formula XIV]

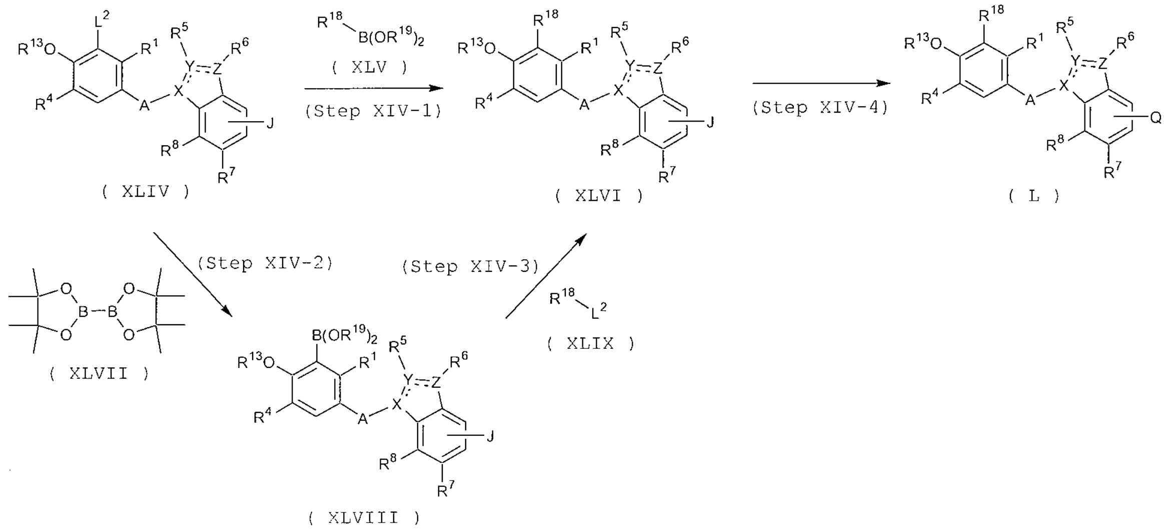


(wherein $R^{18}$ is a C1-C6 alkyl group, an aryl group, a heteroaryl group, an aralkyl group, or a styryl group, $R^{19}$ represents a hydrogen atom or a C1-C6 alkyl group, or two $R^{19}$s are taken together to form a ring represented by $-C(CH_3)_2C(CH_3)_2-$, and other symbols represent the same meanings as those of the aforementioned general formula and the aforementioned Reaction step formula.)

[Step XIV-1]

A compound represented by the general formula (XLVI) is obtained by reacting a compound represented by the general formula (XLIV) and a compound represented by the general formula (XLV) in a suitable solvent (for example, diethoxyethane, toluene, dimethyl sulfoxide etc.) using a palladium catalyst (for example, tetrakis(triphenylphosphine) palladium(0), [bis(diphenylphosphino)ferrocene]dichloropalladium (II) dichloromethane adduct etc.), and a base (for example, sodium carbonate, sodium phosphate, cesium carbonate, triethylamine etc.). A reaction temperature is 50°C to a boiling point of the solvent, and a reaction time is 30 minutes to 48 hours. Herein, the compound represented by the general formula (XLV) is commercially available, or can be produced using the known method.

[Step XIV-2]

A compound represented by the general formula (XLVIII) is obtained by reacting a compound represented by the general formula (XLIV) and bis(pinacolato)diborone (XLVII) in a suitable solvent (for example, dimethyl sulfoxide, toluene, diethoxyethane etc.) using a palladium catalyst (for example, [bis(diphenylphosphino)ferrocene]dichloropalladium (II) dichloromethane adduct, tetrakis(triphenylphosphine)palladium(0) etc.) and a base (for example, potassium acetate, cesium carbonate etc.). A reaction temperature is 50°C to a boiling point of the solvent, and a reaction time is 30 minutes to 48 hours.

[Step XIV-3]

A compound represented by the general formula (XLVI) is obtained by reacting a compound represented by the general formula (XLVIII) and a compound represented by the general formula (XLIX) by the same method as that of the Step XIV-1. Herein, the compound represented by the general formula (XLIX) is commercially available, or can be produced using the known method.

[Step XIV-4]

A compound represented by the general formula (L) is obtained by reacting a compound represented by the general formula (XLVI) by the same method as that of the Step XII-2.

The compounds represented by the general formulae (III) and (VIII) used as a starting material in the Reaction step formulae I to VI, which is represented by the general formula (LV), can be produced by the method shown in the

following Reaction step formula XV, a method described in Examples, or a combination with the known method.

[Reaction step formula XV]

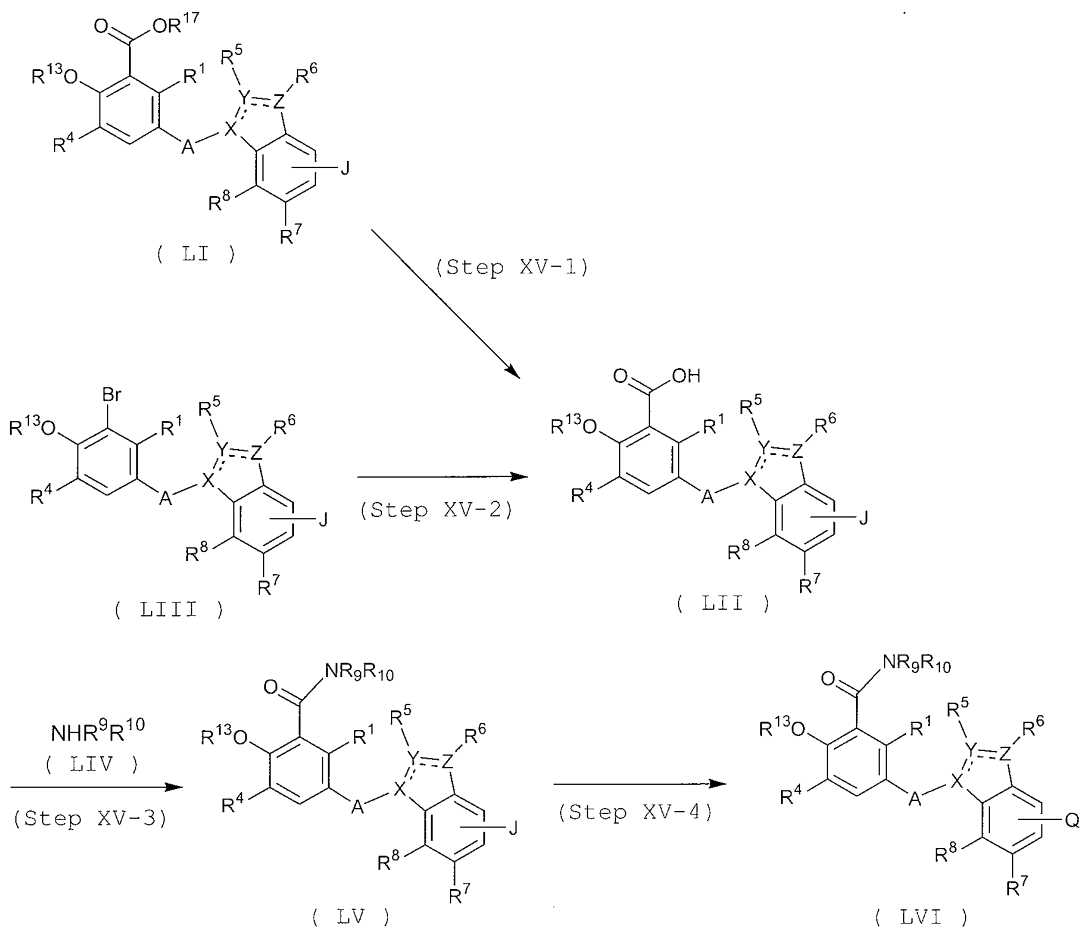


(wherein, all symbols have the same meanings as those of the aforementioned general formula and the aforementioned Reaction step formula.)

[Step XV-1]

A compound represented by the general formula (LII) is obtained by reacting a compound represented by the general formula (LI) in a suitable solvent (for example, ethanol, methanol, dioxane etc.) using a base (for example, sodium hydroxide, potassium hydroxide etc.). A reaction temperature is 0°C to a boiling point of the solvent, and a reaction time is 30 minutes to 48 hours.

[Step XV-2]

A compound represented by the general formula (LII) is obtained by reacting a compound represented by the general formula (LIII) by the same method as that of the Step VI-4.

[Step XV-3]

A compound represented by the general formula (LV) is obtained by reacting a compound represented by the general formula (LII) and a compound represented by the general formula (XLIV) by the same method as that of the

Step IV-1. Herein, the compound represented by the general formula (LIV) is commercially available, or can be produced using the known method.

[Step XV-4]

A compound represented by the general formula (LVI) is obtained by reacting a compound represented by the general formula (LV) by the same method as that of Step XII-2.

The compound represented by the general formula (XXV) used as a starting material in the Reaction step formula VIII can be produced by the method shown in the following Reaction step formulae XVI to XVIII, a method described in Examples, or a combination with the known method.

[Reaction step formula XVI]

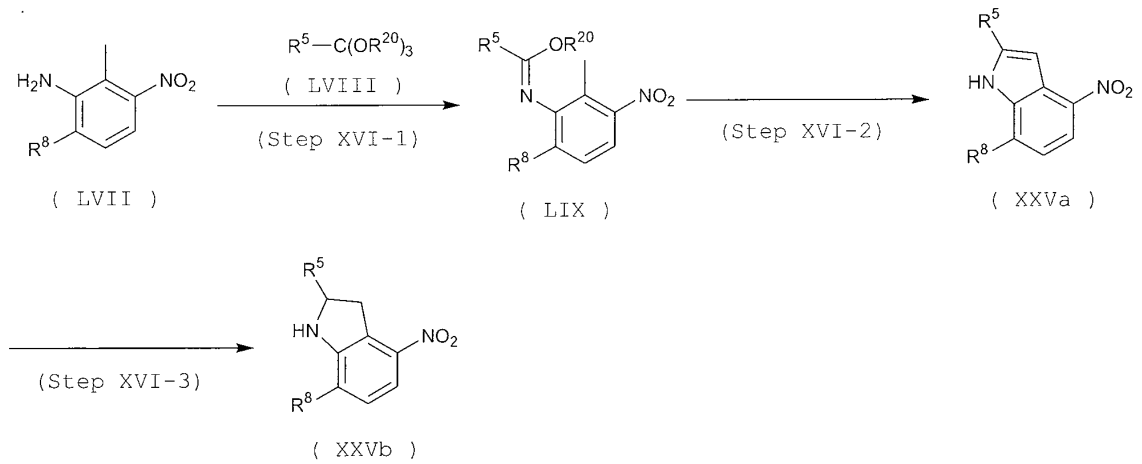


( LVII ) ( LIX ) ( XXVa ) ( XXVb )

(wherein $R^{20}$ means a C1-C6 alkyl group, and other symbols have the same meanings as those of the aforementioned general formula and the aforementioned Reaction step formula.)

[Step XVI-1]

A compound represented by the general formula (LIX) is obtained by reacting a compound represented by the general formula (LVII) and a compound represented by the general formula (LVIII) without a solvent using p-toluenesulfonic acid according to the method described in "Tetrahedron", 46(17), 6085-6112 (1990). A reaction temperature is 60°C to a boiling point of the solvent, and a reaction time is 30 minutes to 48 hours. Herein, the compounds represented by the general formulae (LVII) and (LVIII) are commercially available, or can be produced using the known method.

[Step XVI-2]

A compound represented by the general formula (XXVa) is obtained by reacting a compound represented by the general formula (LIX) in N,N-dimethylformamide using diethyl oxalate and potassium ethoxide according to the method described in "Tetrahedron", 46(17), 6085-6112 (1990). A reaction temperature is -50°C to 100°C, and a reaction time is 1 hour to 48 hours.

[Step XVI-3]

A compound represented by the general formula (XXVb) is obtained by reacting a compound represented by the general formula (XXVa) in trifluoroacetic acid using triethylsilane according to the method described in "Chem. Pharm. Bull. ", 49(1), 87-96(2001). A reaction temperature is room temperature to a boiling point of the solvent, and a reaction

time is 10 minutes to 48 hours.

[Reaction step formula XVII]

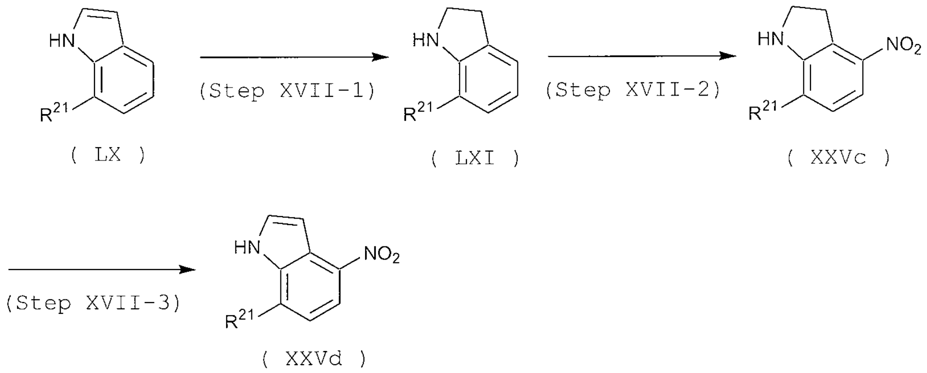


( LX ) ( LXI ) ( XXVc ) ( XXVd )

(wherein $R^{21}$ means a chlorine atom or a bromine atom.)

[Step XVII-1]

A compound represented by the general formula (LXI) is obtained by reacting a compound represented by the general formula (LX) by the same method as that of the Step XVI-3. Herein, a compound represented by the general formula (LX) is commercially available, or can be produced using the known method.

[Step XVII-2]

A compound represented by the general formula (XXVc) is obtained by reacting a compound represented by the general formula (LXI) in concentrated sulfuric acid using fuming nitric acid. A reaction temperature is -20°C to room temperature, and a reaction time is 30 minutes to 24 hours.

[Step XVII-3]

A compound represented by the general formula (XXVd) is obtained by reacting a compound represented by the general formula (XXVc) in a suitable solvent (for example, dioxane, ethyl acetate, chloroform etc.) using an oxidizing agent (for example, 2,3-dichloro-5,6-dicyano-1,4-benzoquinone, manganese dioxide, chloranil, sarcomine/oxygen etc.). A reaction temperature is -20°C to a boiling point of the solvent, and a reaction time is 30 minutes to 48 hours.

[Reaction step formula XVIII]

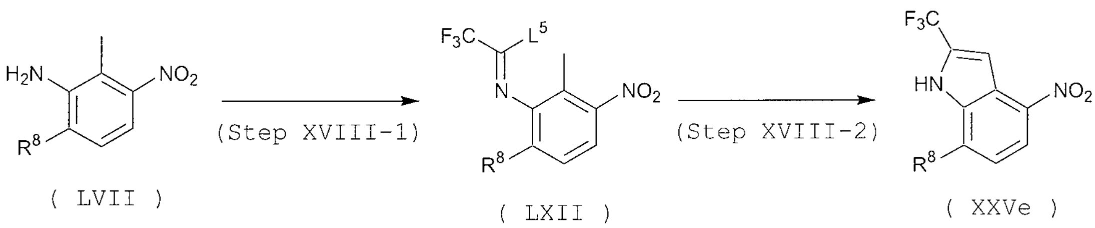


( LVII ) ( LXII ) ( XXVe )

(wherein $L^5$ means a chlorine atom or an iodine atom, and other symbols have the same meanings as those of the aforementioned general formula.)

[Step XVIII-1]

A compound represented by the general formula (LXII) in which $L^4$ is a chlorine atom is obtained by reacting a compound represented by the general formula (LVII) in carbon tetrachloride in the presence of triethylamine using triphenylphosphine and trifluoroacetic acid according to the method described in "Bull. Chem. Soc. Jpn.", 68(5), 1497-1507(1995). A reaction temperature is -50°C to a boiling point of the solvent, and a reaction time is 1 hour to 24 hours.

If necessary, following the aforementioned reaction, by reacting the compound in acetone using sodium iodide, a compound represented by the general formula (LXII) in which $L^4$ is an iodine atom is obtained. A reaction temperature is -50°C to a boiling point of a solvent, and a reaction time is 1 hour to 24 hours.

[Step XVIII-2]

A compound represented by the general formula (XXVe) is obtained by reacting a compound represented by the general formula (LXII) using the same method as that of the Step XVI-2.

Besides, compounds represented by the general formulae (XXV), (XXIX), (XXXII), (XXXIV), and (XXXVI) used as a starting material in the Reaction step formulae VIII to XI are commercially available, or can be produced according to the known method other than the aforementioned method (for example, "Tetrahedron", 60(2), 347-358(2004), "J. Chem. Soc. Perkin-I", (7), 1045-1075(2000), "Heterocycles", 38(11), 2415-2422 (1994), "Synthesis", (12), 1283-1286, (1992), "Comprehensive Heterocyclic Chemistry", Pergamon (1984) etc.).

The compound represented by the general formula (XXVI), (XXVII), or (XXXVIII) used as a starting material in the Reaction step formulae VIII to XII is commercially available, or can be produced by the method shown in the following Reaction step formula XIX, a method described in Examples, or a combination with the known method.

[Reaction step formula XIX]

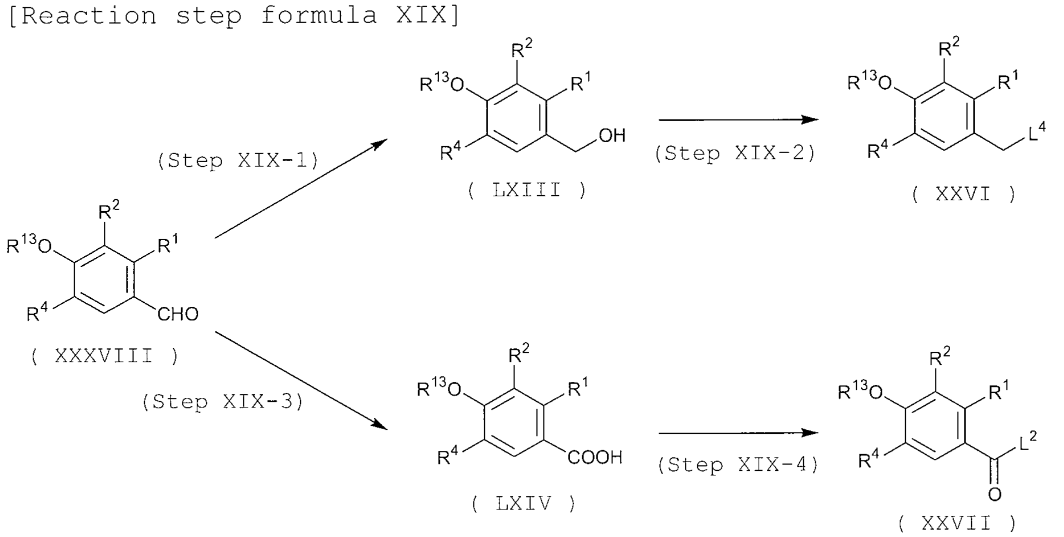


(XXXVIII) (LXIII) (XXVI) (LXIV) (XXVII)

(wherein all symbols have the same meanings as those of the aforementioned general formula and the aforementioned Reaction step formula.)

[Step XIX-1]

A compound represented by the general formula (LXIII) is obtained by reacting a compound represented by the general formula (XXXVIII) in a suitable solvent (for example, methanol, ethanol, tetrahydrofuran, diethyl ether etc.) using a reducing agent (for example, sodium borohydride etc.). A reaction temperature is -20°C to a boiling point of the solvent, and a reaction time is 10 minutes to 48 hours. When $R^{13}$ of the compound represented by the general formula (LXIII) is a hydrogen atom, a protecting group (for example, a triisopropylsilyl group, a t-butyldimethylsilyl group, a methoxymethyl group, a methyl group, a benzyl group etc.) is introduced according to the method described in "Protecting Groups in Organic Synthesis".

[Step XIX-2]

A compound represented by the general formula (XXVI) in which $L^4$ is a halogen atom is obtained by reacting a compound represented by the general formula (LXIII) in a suitable solvent (for example, dichloromethane, toluene, benzene etc.) using a halogenating agent (for example, thionyl chloride, phosphorus tribromide, triphenylphosphine/carbon tetrachlorideetc.). A reaction temperature is -20°C to a boiling point of the solvent, and a reaction time is 10 minutes to 48 hours.

A compound represented by the general formula (XXVI) in which $L^4$ is a methanesulfonyloxy group is obtained

by reacting a compound represented by the general formula (LXIII) in a suitable solvent (for example, dichloromethane, benzene etc.) in the presence of a base (for example, triethylamine, pyridine etc.) using methanesulfonyl chloride. A reaction temperature is -20°C to a boiling point of the solvent, and a reaction time is 10 minutes to 48 hours.

A compound represented by the general formula (XXVI) in which $L^4$ is a p-toluenesulfonyloxy group is obtained by reacting a compound represented by the general formula (LXIII) in a suitable solvent (for example, dichloromethane, diethyl ether, benzene etc.) in the presence of a base (for example, triethylamine, pyridine etc.) using p-toluenesulfonyl chloride. A reaction temperature is -20°C to a boiling point of the solvent, and a reaction time is 10 minutes to 48 hours.

[Step XIX-3]

A compound represented by the general formula (LXIV) is obtained by reacting a compound represented by the general formula (XXXVIII) in a suitable solvent (for example, acetone, water etc.) using potassium permanganate. A reaction temperature is 0°C to a boiling point of the solvent, and a reaction time is 10 minutes to 48 hours.

[Step XIX-4]

A compound represented by the general formula (XXVII) is obtained by reacting a compound represented by the general formula (LXIV) in a suitable solvent (for example, dichloromethane, benzene, toluene etc.) using a halogenating agent (for example, thionyl chloride, oxalyl chloride, thionyl bromide etc.). A reaction temperature is -20°C to a boiling point of the solvent, and a reaction time is 10 minutes to 48 hours.

The compounds represented by the general formulae (XXXVIII) and (LXIII) used as a starting material or a synthesis intermediate in the Reaction step formula XIX are commercially available, or can be produced by the method shown in the following Reaction step formula XX to XXI, a method described in Examples, or a combination with the known method.

[Reaction step formula XX]

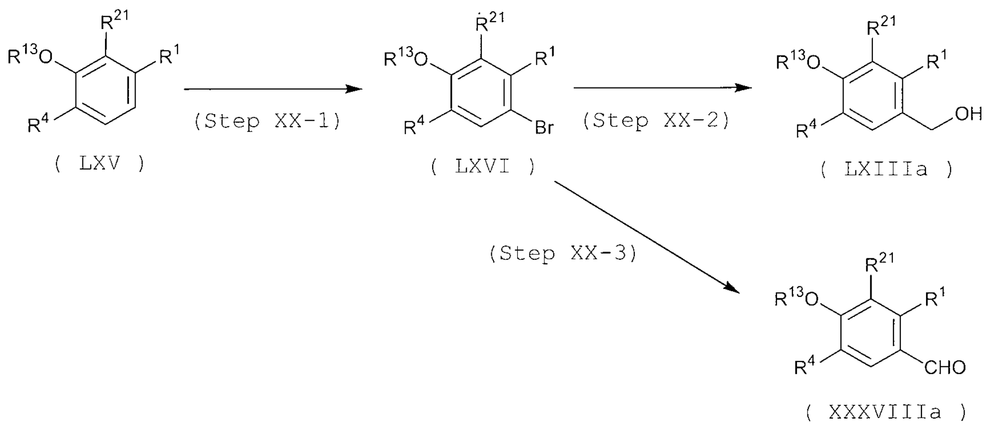


(wherein $R^{21}$ means a C1-C6 alkyl group, or a C3-C7 cycloalkyl group, and other symbols have the same meanings as those of the aforementioned general formula and the aforementioned Reaction step formula.)

[Step XX-1]

A compound represented by the general formula (LXVI) is obtained by reacting a compound represented by the general formula (LXV) in a suitable solvent (for example, acetonitrile, dichloromethane, carbon tetrachloride, diethyl ether, dimethyl sulfoxide etc.) using a brominating agent (for example, N-bromosuccinimide, bromine, bromine/dioxane complex, hydrobromic acid/acetic acid etc.). A reaction temperature is -50°C to a boiling point of the solvent, and a reaction time is 10 minutes to 48 hours. When $R^{13}$ of the compound represented by the general formula (LXVI) is a hydrogen atom, a protecting group (for example, triisopropylsilyl group, t-butyldimethylsilyl, methoxymethyl group, methyl group, benzyl group etc.) is introduced according to the method described in the above "Protecting Groups in Organic

Synthesis". Herein, the compound represented by the general formula (LIX) is commercially available, or can be produced using the known method.

[Step XX-2]

A compound represented by the general formula (LXIIIa) is obtained by lithiation-reacting a compound represented by the general formula (LXVI) in a suitable solvent (for example, tetrahydrofuran, diethyl ether etc.) using an organic lithium reagent (for example, t-butyllithium, n-butyllithium/TMEDA etc.), and reacting this using paraformaldehyde according to the method described in "Bioorg. Med. Chem. Lett.", 10(10), 2607-2611 (2000). A reaction temperature is -78°C to room temperature, and a reaction time is 10 minutes to 12 hours.
Alternatively, the compound represented by the general formula (LXIIIa) is obtained by Grignard-reacting a compound represented by the general formula (LXVI) in a suitable solvent (for example, tetrahydrofuran, diethyl ether etc.) using magnesium metal, and reacting this using paraformaldehyde. A reaction temperature is -78°C to room temperature, and a reaction time is 30 minutes to 12 hours.

[Step XX-3]

A compound represented by the general formula (XXXVIIIa) is obtained by lithiation-reacting a compound represented by the general formula (LXVI) in a suitable solvent (for example, tetrahydrofuran, diethyl ether etc.) using an organic lithium reagent (for example, t-butyllithium, n-butyllithium/TMEDA etc.) using N,N-dimethylformamide. A reaction temperature is 78°C to room temperature, and a reaction time is 10 minutes to 12 hours.
Alternatively, the compound represented by the general formula (XXXVIIIa) is obtained by Grignard-reacting a compound represented by the general formula (LXVI) in a suitable solvent (for example, tetrahydrofuran, diethyl ether etc.) using metal magnesium, and reacting this using N,N-dimethylformamide as in the Step XV-2. A reaction temperature is -78°C to room temperature, and a reaction time is 30 minutes to 12 hours.

[Reaction step formula XXI]

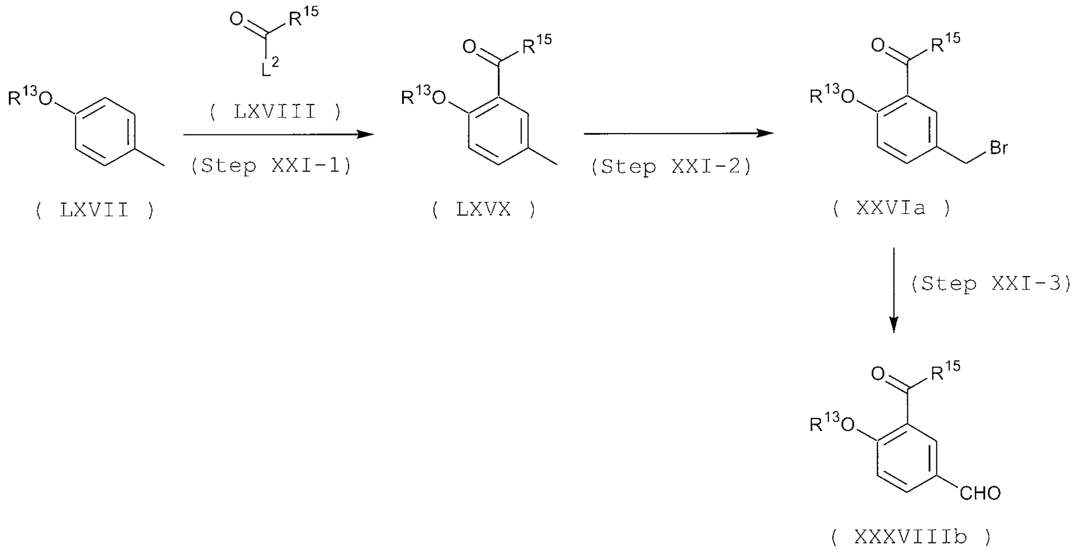

( LXVIII )
( LXVII )
( LXVX )
( XXVIa )
( XXXVIIIb )

(wherein all symbols have the same meanings as those of the reaction step formula.)

[Step XXI-1]

A compound represented by the general formula (LXV) is obtained by reacting a compound represented by the general formula (LXVII) and a compound represented by the general formula (LXVIII) in a suitable solvent (for example, dichloromethane, 1,2-dichloroethane, benzotrifluoride, nitrobenzene etc.) using a Lewis acid (for example, zinc chloride, aluminum chloride, titanium tetrachloride etc.). A reaction temperature is 0°C to a boiling point of the solvent, and a reaction time is 30 minutes to 7 days. Herein, compounds represented by the general formulae (LXVII) and (LXVIII) are commercially available, or can be produced using the known method.

[Step XXI-2]

A compound represented by the general formula (XXVIa) is obtained by reacting a compound represented by the general formula (LXIX) in a suitable solvent (for example, carbon tetrachloride, benzene, chlorobenzene etc.) in the presence of a radical initiation reagent (for example, α,α'-azobis(isobutyronitrile), dibenzoyl peroxide etc.) using a brominating reagent (for example, N-bromosuccinimide, bromine etc.). A reaction temperature is 0°C to a boiling point of the solvent, and a reaction time is 30 minutes to 24 hours.

[Step XXI-3]

A compound represented by the general formula (XXXVIIIb) is obtained by reacting a compound represented by the general formula (XXVIa) in dimethyl sulfoxide using a base (for example, sodium bicarbonate, triethylamine etc.). A reaction temperature is 0°C to a boiling point of the solvent, and a reaction time is 30 minutes to 24 hours.

In addition, a starting material, an intermediate, or a compound used as a reagent necessary for producing the present compound is commercially available, or can be produced according to the known method or the method described in, for example, "Comprehensive Organic Transformations: A Guide to Functional Group Preparation, 2nd Edition", John Wiley & Sons Inc (1999).

It is effective for producing a compound to introduce a suitable protecting group into a substituent contained in the present compound and a compound used for producing the compound (for example, a hydroxy group, an amino group, a carboxylic acid group etc.) at a stage of a raw material or an intermediate, and a protecting group described in "Protecting Groups in Organic Synthesis" may be appropriately selected for use, if necessary.

For isolation and purification of the present compound and a compound used for producing the compound from a reaction mixture, a normally used method can be used. For example, solvent extraction, ion exchange resin, column chromatography using silica gel, alumina or the like as a carrier, high performance liquid chromatography (HPLC) preparation, thin layer chromatography, scavenger resin, recrystallization and the like can be used, and these methods of isolation and purification can be performed alone, or in combination of them. Isolation and purification may be performed for every reaction or may be performed after completion of some reactions.

When a compound in the present specification has an asymmetric carbon, and there are optical isomers, these isomers can be resolved by a general method of optically resolving a racemic compound, for example, by a conventional method such as fractionation crystallization of recrystallization as a diastereomer salt with a general optically active compound, or chromatography. Alternatively, each optical isomer can be also isolated by high performance liquid chromatography (HPLC) preparation using a column for separating an optical active entity.

Since the present compound thus produced acts as the medicament as a thyroid hormone receptor ligand, it can be used as a pharmaceutical composition. The pharmaceutical composition is useful as an agent for preventing or treating a disease or a disorder, symptom of which is improved by cell functional regulation via the thyroid hormone receptor.

The disease or disorder, symptom of which is improved by cell functional regulation via the thyroid hormone receptor, means a disease or a disorder which can be effectively prevented or treated as recognized in the art by cell functional regulation via the thyroid hormone receptor, by the thyroid hormone and the medicament as a thyroid hormone receptor ligand. The cell functional regulation via the thyroid hormone receptor includes gene expression regulation via the thyroid hormone receptor or phosphorylation signaling cascade regulation. Such a disease or disorder specifically means a disease or a disorder due to a functional disorder of a metabolic pathway of a lipid, a sugar, a protein, in organic salts or the like, or metabolic organ thereof, or unbalance of the thyroid hormone, or thyroid function abnormality. That is, it is a disease or a disorder associated with cell functional regulation via the thyroid hormone receptor, and is not necessarily limited to a disease or a disorder developed due to the thyroid hormone. Examples of the disease or the disorder, symptom of which is improved by cell functional regulation via the thyroid hormone receptor include hyperlipemia, obesity, hypothyroidism, hyperthyroidism, goiter, thyroid cancer, cardiac arrhythmia, congestive heart failure, diabetes, depression, osteoporosis, skin disorder, glaucoma, and alopecia.

As an administration form when the present compound is used as a medicament, various administration forms described in "Japanese Pharmacopoeia", Preparations, the General Rule, can be selected depending on the purpose. For example, when molded into a form of a tablet, usually, orally ingestible components used in the art may be selected. Examples thereof include excipients such as lactose, crystalline cellulose, white sugar, and potassium phosphate. Further, if desired, various additives which are conventionally used in the pharmaceutical field such as binders, disintegrating agents, lubricants, and aggregation preventing agents may be incorporated.

An amount of the present compound contained in the present preparation as an active ingredient is not particularly limited, but is appropriately selected from a wide range. A dose of the active ingredient compound is appropriately determined depending on its usage, an age, a sex and other conditions of a patient, and severity of disease. In the case of oral administration, an amount of the present compound can be arbitrarily administered in a range of about 1 μg to

100 mg per 1 kg a day by dividing into 1 to 4 times a day. However, since a dose and number of doses are determined in view of relevant circumstances including severity of symptom to be treated, selection of a compound to be administered, and a selected administration route, the dose range and number of doses do not limit the scope of the present invention.

## EXAMPLES

The content of the present invention will be illustrated in more detail below by way of Examples, Reference Examples and Test Examples, but the technical scope of the present invention is not limited to the described content.

In a nuclear magnetic resonance ($^1$H NMR) spectrum in the following Examples and Reference Examples, a chemical shift value is described as a δ value (ppm) using tetramethylsilane as a standard substance. As a split pattern, singlet is indicated by "s", doublet is indicated by "d", triplet is indicated by "t", quartet is indicated by "q", multiplet is indicated by "m", and a broad ray is indicated by "br". Mass spectrometry is performed by an electrospray ionization method (ESI). In Tables, a methyl group is indicated by "Me", an ethyl group is indicated by "Et", a n-propyl group is indicated by "n-Pr", an isopropyl group is indicated by "i-Pr", a s-butyl group is indicated by "s-Bu", a cyclopentyl group is indicated by "c-Pen", a triisopropylsilyl group is indicated by "TIPS", a phenyl group is indicated by "Ph", a 3-pyridyl group is indicated by "3-Py", a benzyl group is indicated by "Bn", a phenethyl group is indicated by "PhEt", a styryl group is indicated by "Sty", a benzoyl group is indicated by "Bz", an ethoxy group is indicated by "OEt", and a phthaloyl group is indicated by "Pht". In addition, in the case of a substituted substituent, a substitution position is described before the substituent. For example, a 4-fluorobenzoyl group is described as "4-F-Bz".

### Reference Example 1

3-Bromo-4-triisopropylsilanyloxybenzaldehyde

3-Bromo-4-hydroxybenzaldehyde (3.0 g) was dissolved in N,N-dimethylformamide (50 ml), triisopropylsilyl chloride (4.8 mL) and imidazole (2.0 g) were added, and the mixture was stirred at room temperature for 24 hours. The reaction mixture was poured into ice water, followed by extraction with n-hexane. The organic layer was washed with water and an aqueous saturated sodium chloride solution, and dried with anhydrous sodium sulfate. A solvent was concentrated under reduced pressure to obtain the title compound (5.0 g).
$^1$H NMR ($CDCl_3$) δ (ppm): 1.01(18H,d), 1.29(3H,m), 7.11(1H,d), 7.74(1H,dd), 8.04(1H,d), 9.87(1H,s).

Compounds were synthesized according to the following reaction formula referring to the method of Reference Example 1. The synthesized compounds and data are shown in Table 1.

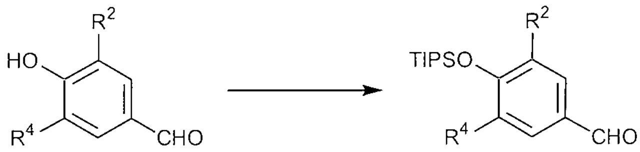

[Table 1]

| Reference Example | $R^2$ | $R^4$ | $^1$HNMR ($CDCl_3$) δ (ppm) |
|---|---|---|---|
| 2 | $CF_3$ | H | 1.01(18H,d),1.29(3H,m),7.00(1H,d),7.91(1H,dd), 8.07(1H,d),9.87(1H,s). |
| 3 | Me | Me | 1.10(18H,d),1.29(3H,m),2.35(6H,s),7.25(2H,s), 9.87(1H,s). |

### Reference Example 4

4-bromo-2-isopropylphenol

2-isopropylphenol (50.0 g) was dissolved in acetonitrile (500 mL), and N-bromosuccinimide (71.9 g) was added at 0°C, followed by stirring at room temperature for 4 hours. The reaction mixture was concentrated under reduced pressure, n-hexane was added, and the produced precipitate was filtered. The filtrate was washed with water, and an aqueous saturated sodium chloride solution, and dried with anhydrous sodium sulfate. The solvent was concentrated

under reduced pressure to obtain the title compound (84.0 g).
$^1H$ NMR ($CDCl_3$) δ (ppm): 1.14(6H,d), 3.17(1H,m), 6.73(1H,d), 7.13(1H,dd), 7.19(1H,d).

Compounds were synthesized according to the following reaction formula referring to the method of Reference Example 4. Synthesized compounds and data are shown in Table 2.

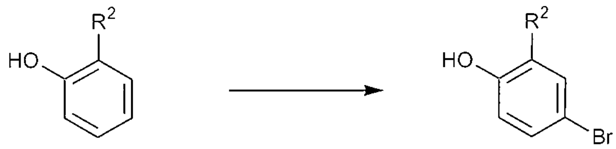

[Table 2]

| Reference Example | $R^2$ | $^1$HNMR ($CDCl_3$) δ (ppm) |
|---|---|---|
| 5 | *n*-Pr | 0.96(3H,t),1.62(2H,m),2.54(2H,t),4.82(1H,s),6.63(1H,d), 7.16(1H,dd),7.22(1H,d). |
| 6 | *s*-Bu | 0.86(3H,t),1.21(3H,d),1.52-1.70(2H,m),2.92(1H,m),4.68(1H,s), 6.63(1H,d),7.15(1H, dd),7.23(1H,d). |

Reference Example 7

4-Bromo-2-isopropyl-1-triisopropylsilanyloxybenzene

4-Bromo-2-isopropylphenol (84.8 g) was dissolved in N,N-dimethylformamide (848 mL), triisopropylsilyl chloride (101 mL) and imidazole (53.7 g) were added, and the mixture was stirred at room temperature for 24 hours. A reaction mixture was diluted with n-hexane, washed with water and an an aqueous saturated sodium chloride solution, and dried with anhydrous sodium sulfate. After the solvent was concentrated under reduced pressure, the resulting residue was crystallized with acetonitrile to obtain the title compound (98.0 g). $^1H$ NMR ($CDCl_3$) δ (ppm): 1.10(18H,d), 1.18(6H,d), 1.28(3H,m), 3.32(1H,m), 6.63(1H,d), 7.12(1H,dd), 7.25(1H,d).

Compounds were synthesized according to the following reaction formula referring to the method of Reference Example 7. Synthesized compounds and data are shown in Table 3.

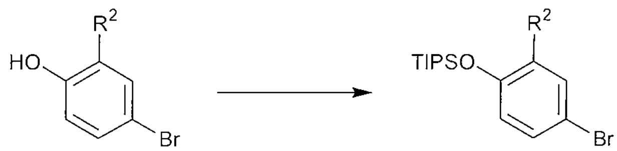

[Table 3]

| Reference Example | $R^2$ | $^1$HNMR ($CDCl_3$) δ (ppm) |
|---|---|---|
| 8 | *n*-Pr | 0.94(3H,t),1.10(18H,d),1.29(3H,m),1.58(2H,m),2.55(2H,t), 6.64(1H,d),7.12(1H,dd), 7.22(1H,d). |
| 9 | *s*-Bu | 0.85(3H,t),1.09-1.16(21H,m),1.29(3H,m),1.42-1.60(2H,m), 3.12(1H,m),6.64(1H,d), 7.11(1H,dd),7.22(1H,d). |
| 10 | c-Pen | 1.11(18H,d),1.30(3H,m),1.40-1.60(2H,m),1.60-1.72(2H,m), 1.72-1.84(2H,m), 1.94-2.06(2H,m),3.35(1H,m),6.64(1H,d), 7.10(1H,dd),7.28(1H,d). |

Reference Example 11

1-Benzyloxy-4-bromo-2-isopropylbenzene

4-Bromo-2-isopropylphenol (2.2 g) was dissolved in acetonitrile (10.0mL), followed by addition of benzyl chloride (1.7 g), sodium bicarbonate (1.0 g) and sodium iodide (0.2 g), and the mixture was stirred at 60°C for 18 hours. After a reaction mixture was returned to room temperature, and concentrated under reduced pressure, the resulting residue was suspended in water, and extracted with ethyl acetate. The organic layer was washed with a 3% aqueous potassium carbonate solution, a 5% aqueous citric acid solution, and an aqueous saturated sodium chloride solution, followed by being dried with anhydrous sodium sulfate, and the solvent was concentrated under reduced pressure. The resulting residue was purified by column chromatography (silica gel, developing solvent: n-hexane/ethyl acetate = 9/1) to obtain the title compound (2.7 g).
$^1$H NMR ($CDCl_3$) δ (ppm): 1.21(6H,d), 3.37(1H,m), 6.76(1H,d), 7.23(1H,dd), 7.31-7.43(6H,m).

Reference Example 12

4-bromo-2-isopropyl-1-methoxybenzene

4-bromo-2-isopropylphenol (8.0 g) was dissolved in N,N-dimethylformamide (30 mL), methyl iodide (10.6 g) and potassium carbonate (10.3 g) were added, and the mixture was stirred at 60°C for 3 hours. The reaction mixture was returned to room temperature, and diluted with n-hexane. The organic layer was washed with water and an aqueous saturated sodium chloride solution, followed by being dried with anhydrous sodium sulfate, and the solvent was concentrated under reduced pressure. The resulting residue was purified by column chromatography (silica gel, developing solvent: n-hexane) to obtain the title compound (6.9 g).
$^1$H NMR ($CDCl_3$) δ (ppm):1.22(6H,d), 3.26(1H,m), 3.82(3H,s), 6.70(1H,d), 7.24(1H,dd), 7.27(1H,d).

Reference Example 13

(3-isopropyl-4-triisopropylsilanyloxyphenyl)methanol

4-bromo-2-isopropyl-1-triisopropylsilanyloxybenzene (23.6 g) was dissolved in tetrahydrofuran (236 mL), and a 1.5 mol/L solution of t-butyllithium in n-pentane (100 mL) was added dropwise at -78°C over 1 hour. After stirred at -78°C for 1 hour, paraformaldehyde (3.9 g) was added at -78°C, and the mixture was stirred at -78°C for 1 hour, and subsequently, at room temperature for 3 hours. The reaction mixture was diluted with diethyl ether (300 mL), washed with 1 mol/L hydrochloric acid solution and an aqueous saturated sodium chloride solution, and dried with anhydrous sodium sulfate, and the solvent was concentrated under reduced pressure. The resulting residue was purified by flash chromatography (silica gel, developing solvent: n-hexane to n-hexane/ethyl acetate = 4/1) to obtain the title compound (9.4 g).
$^1$H NMR ($CDCl_3$) δ (ppm): 1.11(18H,d), 1.20(6H,d), 1.32(3H,m), 3.37(1H,m), 4.59(2H,s), 6.75(1H,d), 7.02(1H,dd), 7,19 (1H,d).

Compounds were synthesized according to the following reaction formula referring to the method of Reference Example 13. Synthesized compounds and data are shown in Table 4.

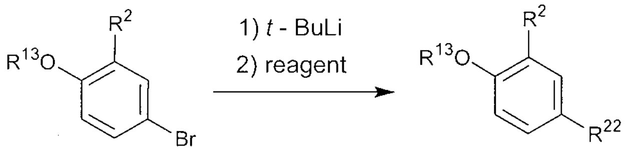


[Table 4]

| Reference Example | $R^2$ | $R^{13}$ | $R^{22}$ | reagent | $^1$HNNIR ($CDCl_3$) δ (ppm) |
|---|---|---|---|---|---|
| 14 | *n*-Pr | TIPS | $CH_2OH$ | paraforma ldehyde | 0.96(3H,t),1.11(18H,d),1.31(3H,m), 1.61(2H,m),2.58(2H,t),4.58(2H,d), 6.75(1H,d),7.03(1H,dd),7.12(1H,d). |

(continued)

| Reference Example | $R^2$ | $R^{13}$ | $R^{22}$ | reagent | $^1$HNNIR ($CDCl_3$) δ (ppm) |
|---|---|---|---|---|---|
| 15 | *i*-Pr | Me | $CH_2OH$ | paraforma ldehyde | 1.23(6H,d),3.36(1H,m),3.81(3H,s), 4.60(2H,s),6.80(1H,d),7.13(1H,dd), 7.19(1H,d),9.84(1H,s). |
| 16 | *i*-Pr | TIPS | CHO | N,N-dimet hylforma mide | 1.11(18H,d),1.23(6H,d),1.34(3H, m), 3.36(1H,m),6.85(1H,d),7.57(1H, dd), 7.75(1H,d),9.84(1H,s). |
| 17 | *s*-Bu | TIPS | $CH_2OH$ | paraforma ldehyde | 0.86(3H,t),1.11(18H,d),1.18(3H,d), 1.30(3H,m),1.48-1.68(2H,m),3.17 (1H,m),4.59(2H,d),6.76(1H,d),7.02 (1H,dd), 7.14(1H,d). |

Reference Example 18

4-benzyloxy-3-isopropylbenzaldehyde

1-benzyloxy-4-bromo-2-isopropylbenzene (160 mg) was dissolved in tetrahydrofuran (2.0 mL), tetramethylethylenediamine (150 μL) was added, and the mixture was cooled to -78°C. A 1.6 mol/L solution of n-butyl lithium in n-hexane (630 μL) was added dropwise over 1 hour, and the mixture was stirred at -78°C for 1 hour. Then, N,N-dimethylformamide (50 μL) was added at -78°C, and the mixture was stirred for 1 hour. To the reaction mixture was added an aqueous saturated ammonium chloride solution, followed by extraction with ethyl acetate. The organic layer was washed with an aqueous saturated sodium bicarbonate solution and an aqueous saturated sodium chloride solution, and dried with anhydrous sodium sulfate, and the solvent was concentrated under reduced pressure. The resulting residue was purified by flash chromatography (silica gel, developing solvent: n-hexane/ethyl acetate = 9/1) to obtain the title compound (110 mg).
$^1$H NMR ($CDCl_3$) δ (ppm): 1.26(6H,d), 3.41(1H,m), 5.17(2H,s), 7.00(1H,d), 7.34-7.44(5H,m), 7.68(1H,dd), 7.79(1H,s), 9.88(1H,s).

Reference Example 19

3-cyclopentyl-4-triisopropylsilanyloxybenzaldehyde

A reaction was performed using 4-bromo-2-cyclopentyl-1-triisopropylsilanyloxybenzene in place of 1-benzyloxy-4-bromo-2-isopropylbenzene by the same method as that of Reference Example 18, to obtain the title compound.
$^1$H NMR ($CDCl_3$) δ (ppm): 1.13(18H,d), 1.36(3H,m), 1.54-1.64(2H,m), 1.64-1.74(2H,m), 1.76-1.88(2H,m), 1.98-2.10(2H, m), 3.41(1H,m), 6.87(1H,d), 7.58(1H,dd), 7.77(1H,d), 9.85(1H,s).

Reference Example 20

(3-bromo-4-triisopropylsilanyloxyphenyl)methanol

3-bromo-4-triisopropylsilanyloxybenzaldehyde (3.0 g) was dissolved in ethanol (30 mL), sodium borohydride (159 mg) was added at 0°C, and the mixture was stirred at room temperature for 3 hours. To the reaction mixture were added acetic acid and water, and the mixture was concentrated under reduced pressure. The resulting residue was suspended in water, followed by extraction with ethyl acetate. The organic layer was washed with water and an aqueous saturated sodium chloride solution, and dried with anhydrous sodium sulfate, and the solvent was concentrated under reduced pressure. The resulting residue was purified by flash chromatography (silica gel, developing solvent: n-hexane to n-hexane/ethyl acetate = 4/1) to obtain the title compound (1.2 g).
$^1$H NMR ($CDCl_3$) δ (ppm): 1.14(18H,d), 1.30(3H,m), 4.58(2H,s), 6.86(1H,d), 7.13(1H,dd), 7.52(1H,d).
Compounds were synthesized according to the following reaction formula referring to the method of Reference Example 20. Synthesized compounds and data are shown in Table 5.

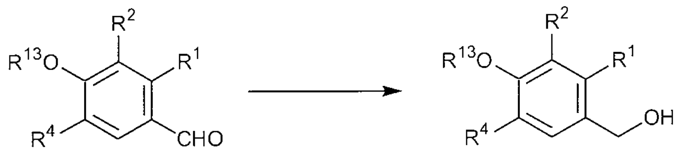

[Table 5]

| Reference Example | $R^1$ | $R^2$ | $R^4$ | $R^{13}$ | $^1$HNMR ($CDCl_3$) δ (ppm) |
|---|---|---|---|---|---|
| 21 | Me | Me | H | Me | 2.18(3H,s),2.31(3H,s),3.82(3H,s),4.66(2H,d), 6.70(1H,d), 7.13(1H,d). |
| 22 | H | *i*-Pr | H | Bn | 1.27(6H,d),3.44(1H,m),4.63(2H,s),5.10(2H,s), 6.90(1H,d), 7.15(1H,dd),7.25(1H,d),7.31-7.46 (6H,m). |
| 23 | H | c-Pen | H | TIPS | 1.11(18H,d),1.31(3H,m),1.42-1.60(2H,m),1.60-1.72(2H,m), 1.72-1.86(2H,m),1.96-2.08(2H,m), 3.40(1H,m),4.59(2H,d), 6.76(1H,d),7.02(1H, dd),7.21(1H,d). |
| 24 | H | $CF_3$ | H | TIPS | 1.09(18H,d),1.28(3H,m),4.62(2H,s),6.86(1H,d), 7.36(1H, dd),7.52(1H,d). |
| 25 | H | Me | Me | TIPS | 1.11(18H,d),1.28(3H,m),2.25(3H,s),4.55(2H,s), 6.96(2H,s). |

Reference Example 26

(3-ethoxy-4-triisopropylsilanyloxyphenyl)methanol

3-ethoxy-4-hydroxybenzaldehyde (500 mg) was dissolved in N,N-dimethylformamide (10 mL), triisopropylsilyl chloride (967 μL) and imidazole (410 mg) were added, and the mixture was stirred at room temperature for 24 hours. The reaction mixture was poured into ice water, followed by extraction with n-hexane. The organic layer was washed with water and an aqueous saturated sodium chloride solution, and dried with anhydrous sodium sulfate, and the solvent was concentrated under reduced pressure. Then, this was dissolved in ethanol (11 mL), sodium borohydride (57.1 mg) was added at 0°C, and the mixture was stirred at room temperature for 3 hours. To the reaction mixture were added acetic acid and water, and the mixture was concentrated under reduced pressure. The resulting residue was suspended in water, followed by extraction with ethyl acetate. The organic layer was washed with water and an aqueous saturated sodium chloride solution, and dried with anhydrous sodium sulfate, and the solvent was concentrated under reduced pressure. The resulting residue was purified by flash chromatography (silica gel, developing solvent: n-hexane to n-hexane/ethyl acetate = 4/1) to obtain the title compound (976 mg).
$^1$H NMR ($CDCl_3$) δ (ppm): 1.09(18H,d), 1.20-1.32(3H,m), 1.42(3H,t), 4.03(2H,q), 4.59(2H,d), 6.76(1H,dd), 6.84(1H,d), 6.87(1H,d).

Compounds were synthesized according to the following reaction formula referring to the method of Reference Example 26. Synthesized compounds and data are shown in Table 6.

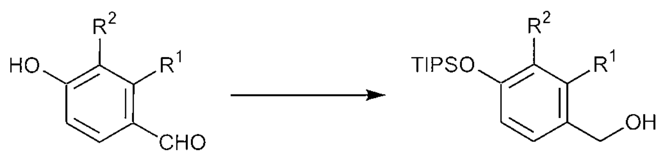

[Table 6]

| Reference Example | $R^1$ | $R^2$ | $^1$HNMR ($CDCl_3$) δ (ppm) |
|---|---|---|---|
| 27 | -CH=CH-CH=CH- | | 1.16(18H,d),1.42(3H,m),5.06(2H,d),6.82(1H,d),7.32 (1H,d),7.60-7.46 (2H,m),8.11(1H,d),8.33(1H,d). |
| 28 | H | Cl | 1.10(18H,d),1.24-1.33(3H,m),4.57(2H,d),6.86(1H,d), 7.07(1H,dd),7.34 (1H,d). |
| 29 | H | Me | 1.10(18H,d),1.29(3H,m),2.23(3H,s),4.55(2H,s),6.75 (1H,d),7.02(1H, dd),7.12(1H,d). |

Reference Example 30

4-benzyloxy-3-isopropylbenzoic acid

4-benzyloxy-3-isopropylbenzaldehyde (2.4 g) was suspended in water (20 mL), potassium permanganate (1.7 g) was added, and the mixture was stirred at room temperature for 2 hours. To the reaction mixture was added a 1 mol/L aqueous sodium hydroxide solution, and the mixture was filtered using Celite. The filtrate was neutralized with 1 mol/L hydrochloric acid, followed by extracted with ethyl acetate. The organic layer was washed with water and an aqueous saturated sodium chloride solution, and dried with anhydrous sodium sulfate, and the solvent was concentrated under reduced pressure. The resulting residue was purified by column chromatography (silica gel, developing solvent: n-hexane/ethyl acetate = 1/2) to obtain the title compound (1.7 g).
$^1$H NMR ($CDCl_3$) δ (ppm): 1.21(6H,d), 3.31(1H,m), 5.22(2H,s), 7.15(1H,d), 7.34-7.50(5H,m), 7.80(2H,m), 12.66(1H,brs).

Reference Example 31

(4-fluorophenyl)-(2-methoxy-5-methylphenyl)methanone

4-methylanisole (60.0 g) was dissolved in benzotrifluoride (300 mL), zinc chloride (1.3 g) and 4-fluorobenzoyl chloride (70.8 mL) were added, and the mixture was heated to reflux for 23 hours. The reaction mixture was concentrated under reduced pressure, and ethyl acetate and an aqueous saturated sodium bicarbonate solution were added to the resulting residue, followed by extraction with ethyl acetate. The organic layer was washed with water and an aqueous saturated sodium chloride solution, and dried with anhydrous sodium sulfate. The solvent was concentrated under reduced pressure, and the resulting residue was allowed to stand at room temperature to crystallize. The resulting crystal was washed with cooled n-hexane to obtain the title compound (82.4 g). $^1$H NMR ($CDCl_3$) δ (ppm): 2.32(3H,s), 3.69(3H, s), 6.88(1H,d), 7.08(1H,d), 7.10(1H,d), 7.16(1H,s), 7.26(1H,d), 7.83(1H,d), 7.84(1H,d).

Reference Example 32

(5-bromomethyl-2-methoxyphenyl)-(4-fluorophenyl)methanone

(4-fluorophenyl)-(2-methoxy-5-methylphenyl)methanone (30.0 g) was dissolved in carbon tetrachloride (300 mL), N-bromosuccinimide (24.1 g) and 2,2'-azobisisobutyronitrile (200 mg) were added, and the mixture was heated to reflux for 16 hours. The produced precipitate was filtered, and the filtrate was concentrated under reduced pressure, followed by crystallizing the resulting residue with diethyl ether/n-hexane to obtain the title compound (29.2 g).
$^1$H NMR ($CDCl_3$) δ (ppm): 3.74(3H,s), 4.50(2H,s), 6.96(1H,d), 7.11(2H,t), 7.39(1H,d), 7.51(1H,dd), 7.83(2H,m).

Reference Example 33

2,6-dimethyl-3-nitrophenylamine

2,6-xylidine (20.1 g) was dissolved in concentrated sulfuric acid (120 mL), fuming nitric acid (11.5 g) was added dropwise at 10 to 15°C, and the mixture was stirred at 15°C for 40 minutes. The reaction mixture was poured into ice water, and a 6 mol/L aqueous sodium hydroxide solution was added at 10°C to neutralize the solution. The produced precipitate was filtered, and sufficiently washed with water, and the resulting crystal was dried under reduced pressure to obtain the title compound (26.8 g).
$^1$H NMR ($CDCl_3$) δ (ppm): 2.23(3H,s), 2.29(3H,s), 3.86(1H,brs), 7.00(1H,d), 7.16(1H,d).

Reference Example 34

Ethyl N-(2-methyl-3-nitrophenyl)acetimidate

2-methyl-3-nitroaniline (5.1 g) was dissolved in triethyl orthoacetate (33.3 mL), p-toluenesulfonic acid monohydrate (316 mg) was added, and the mixture was stirred at 120°C for 1 hour. The reaction mixture was returned to room temperature, and concentrated under reduced pressure, and the resulting residue was then crystallized with cooled n-hexane. The resulting crystal was filtered, and dissolved again in n-hexane, and the solvent was then concentrated under reduced pressure to obtain the title compound (7.1 g).
$^1$H NMR ($CDCl_3$) δ (ppm): 1.37(3H,t), 1.78(3H,s), 2.26(3H,s), 4.28(2H,q), 6.88(1H,d), 7.22(1H,dd), 7.52(1H,d).
Compounds were synthesized according to the following reaction formula referring to the method of Reference Example 34. Synthesized compounds and data are shown in Table 7.

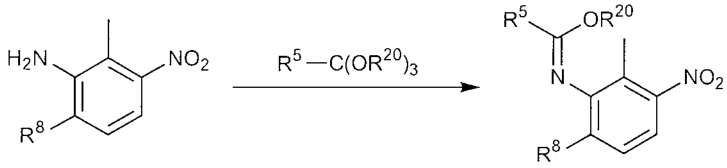


[Table 7]

| Reference Example | $R^5$ | $R^8$ | $R^{20}$ | $^1$HNMR ($CDCl_3$) δ (ppm) |
|---|---|---|---|---|
| 35 | Et | H | Et | 1.05(3H,t),1.36(3H,t),2.08(2H,q),2.24(3H,s),4.26(2H,q), 6.86(1H,d), 7.21(1H,t),7.50(1H,d). |
| 36 | H | Me | Me | 2.17(3H,s),2.28(3H,s),3.33(2H,d),3.97(3H,s),4.97(1H,s), 7.11(1H,d), 7.51(1H,d). |
| 37 | Me | Me | Et | 1.39(3H,t),1.67(3H,s),2.11(3H,s),2.23(3H,s),4.33(2H,q), 7.12(1H,d), 7.50(1H,d). |
| 38 | Et | Me | Et | 1.01(3H,t),1.37(3H,t),1.92(2H,q),2.08(3H,s),2.21(3H,s), 4.31(2H,q), 7.09(1H,d),7.46(1H,d). |

Reference Example 39

2-methyl-4-nitro-1H-indole

Diethyl oxalate (14 mL) was dissolved in N,N-dimethylformamide (70 mL), and potassium ethoxide (8.6 g) was added while cooling in an ice bath. After the reaction mixture was returned to room temperature, a solution of ethyl N-(2-methyl-3-nitrophenyl)acetimidate (15.4 g) in N,N-dimethylformamide (70 mL) was added dropwise, and the mixture was stirred at room temperature for 23 hours. The reaction mixture was poured into ice water, and the produced precipitate was filtered. The resulting precipitate was dissolved in chloroform, the organic layer was washed with water and dried with an anhydrous sodium sulfate, and the solvent was concentrated under reduced pressure. The resulting residue was purified by flash chromatography (silica gel, developing solvent: n-hexane to n-hexane/ethyl acetate = 7/3) to obtain the title compound (2.5 g).
$^1$H NMR ($CDCl_3$) δ (ppm): 2.55(3H,s), 7.02(1H,s), 7.18(1H,dd), 7.59(1H,d), 8.10(1H,d), 8.34(1H,brs).
Compounds were synthesized according to the following reaction formula referring to the method of Reference Example 39. Synthesized compounds and data are shown in Table 8.

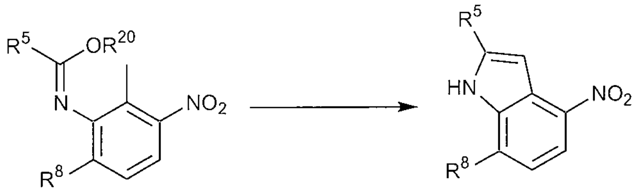

[Table 8]

| Reference Example | $R^5$ | $R^8$ | $R^{20}$ | $^1$HNMR δ (ppm) |
|---|---|---|---|---|
| 40 | Et | H | Et | (DNSO-$d_6$)1.33(3H,t),2.86(2H,q),6.83(1H,s),7.22(1H,t), 7.77(1H,d), 8.01(1H,d),11.87(1H,brs). |
| 41 | H | Me | Me | ($CDCl_3$)2.61(3H,s),7.08(1H,d),7.33(1H,m),7.48(1H,m), 8.09(1H,d),8.51 (1H,brs). |
| 42 | Me | Me | Et | (DMSO-$d_6$)2.51(3H,s),2.73(3H,s),6.81(1H,s),7.00(1H,d), 7.92(1H,d), 11.71(1H,brs). |
| 43 | Et | Me | Et | (DMSO-$d_6$)1.35(3H,t),2.60(3H,s),2.87(2H,q),6.85(1H,s), 7.03(1H,d), 7.94(1H,d),11.70(1H,brs). |

Reference Example 44

2,3-dimethyl-4-nitro-1H-indole

Dimethyl sulfoxide (25 mL) was added to potassium ethoxide (1.22 g), followed by addition of 2-butanone (1.3mL). 3-nitroaniline (1.00 g) was added, and the mixture was stirred at room temperature for 2 hours. The reaction mixture was poured into ice water, and the mixture was stirred at room temperature for 13 hours. The produced precipitate was filtered, and dissolved in ethyl acetate, the organic layer was washed with water and dried with anhydrous sodium sulfate, and the solvent was concentrated under reduced pressure. The resulting residue was purified by flash chromatography (silica gel, developing solvent: n-hexane to n-hexane/ethyl acetate = 7/3) to obtain the title compound (150 mg). $^1$H NMR ($CDCl_3$) δ (ppm): 2.28(3H,s), 2.43(3H,s), 7.11(1H,dd), 7.49(1H,dd), 7.73(1H,dd), 8.17(1H,s).

Reference Example 45

7-methyl-4-nitro-1H-indoline

7-methyl-4-nitro-1H-indole (500 mg) was dissolved in trifluoroacetic acid (10 mL), triethylsilane (907 μL) was added, and the mixture was heated to stir at 60°C for 2 hours. The reaction mixture was concentrated under reduced pressure, and an aqueous saturated sodium bicarbonate solution was then added to the resulting residue to be alkaline, followed by extraction with ethyl acetate. The organic layer was washed with water and dried with anhydrous sodium sulfate, and the solvent was concentrated under reduced pressure. The resulting residue was purified by flash chromatography (silica gel, developing solvent: n-hexane to n-hexane/ethyl acetate = 3/2) to obtain the title compound (461 mg). $^1$H NMR ($CDCl_3$) δ (ppm): 2.18(3H,s), 3.56(2H,dd), 3.69(2H,dd), 6.98(1H,d), 7.47(1H,d).

Compounds were synthesized according to the following reaction formula referring to the method of Reference Example 45. Synthesized compounds and data are shown in Table 9.

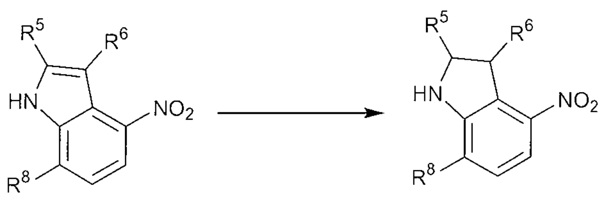

[Table 9]

| Reference Example | $R^5$ | $R^6$ | $R^8$ | $^1$HNMR ($CDCl_3$) δ (ppm) |
|---|---|---|---|---|
| 46 | Me | Me | H | 1.12(3H,d),1.33(3H,d),3.82(1H,m),4.03(1H,m),6.82(1H,d), 7.13(1H, dd),7.49(1H,dd). |
| 47 | Me | H | Me | 1.34(3H,d),2.17(3H,s),3.13(1H,dd),3.70(1H,dd),3.82 (1H,brs), 4.05-4.18(1H,m),6.96(1H,d),7.44(1H,d). |
| 48 | Et | H | Me | 1.00(3H,t),1.65(2H,m),2.16(3H,s),3.12-3.18(1H,m),3.64-3.71(1H,m), 3.86-3.93(2H,m),6.95(1H,dd),7.43(1H,d). |

Reference Example 49

7-bromo-5-nitro-1H-indoline

5-nitro-1H-indoline (2.0 g) was dissolved in acetic acid (15.0 mL), bromine (1.0 mL) was added, and the mixture was stirred at room temperature for 3 hours. The reaction mixture was poured into ice water, and this was neutralized with a 1 mol/L aqueous sodium hydroxide solution. The produced crystal was filtered and washed with water, and the resulting crystal was then dried under reduced pressure to obtain the title compound (3.3 g).
$^1$H NMR ($CDCl_3$) δ (ppm): 3.27(2H,t), 3.86(2H,t), 7.90(1H,s), 8.19(1H,s).

Reference Example 50

7-methyl-5-nitro-1H-indoline

7-bromo-5-nitro-1H-indoline (650 mg) was dissolved in N,N-dimethylformamide (10.0 mL), tetramethyltin (1.0 mL) and (triphenylphosphine)palladium (II) chloride (100 mg) were added, and the mixture was stirred at 140°C for 12 hours. The reaction mixture was concentrated under reduced pressure, and the resulting residue was then purified by flash chromatography (silica gel, developing solvent: n-hexane to n-hexane/ethyl acetate = 4/1) to obtain the title compound (250 mg).
$^1$H NMR ($CDCl_3$) δ (ppm): 2.15(3H,s), 3.14(2H,t), 3.78(2H,t), 7.86(1H,s), 7.87(1H,s).

Reference Example 51

7-methyl-5-nitro-1H-indole

7-methyl-5-nitro-1H-indoline (188 mg) was dissolved in dioxane (5.0 mL), 2,3-dichloro-5,6-dicyano-1,4-benzoquinone (263 mg) was added, and the mixture was stirred at room temperature for 3 hours. The reaction mixture was diluted with dichloromethane, the organic layer was washed with an aqueous saturated sodium bicarbonate solution, and an aqueous saturated sodium chloride solution, followed by dried with anhydrous sodium sulfate, and the solvent was concentrated under reduced pressure. The resulting residue was purified by flash chromatography (silica gel, developing solvent: n-hexane to n-hexane/ethyl acetate = 2/1) to obtain the title compound (167 mg).
$^1$H NMR ($CDCl_3$) δ (ppm): 2.56(3H,s), 6.73(1H,dd), 7.35(1H,t), 7.93(1H,d), 8.45(1H,d).

Reference Example 52

7-chloro-1H-indoline

7-chloro-1H-indole (1.3 g) was dissolved in trifluoroacetic acid (20 mL), triethylsilane (2. 7 mL) was added, and the mixture was stirred at 60°C for 2 hours. The reaction mixture was returned to room temperature, and concentrated under reduced pressure. To the resulting residue were added water and 1 mol/L hydrochloric acid to be acidic, followed by extraction with ethyl acetate. The organic layer was washed with an aqueous saturated sodium chloride solution, and dried with anhydrous sodium sulfate. The solvent was concentrated under reduced pressure to obtain the title compound (980 mg). $^1$H NMR ($CDCl_3$) δ (ppm): 3.11(2H,t), 3.62(2H,t), 3.98(1H,brs), 6.62(1H,t), 6.99(2H,t).

Reference Example 53

7-chloro-4-nitro-1H-indoline

After fuming nitric acid (920 μL) was added to concentrated sulfuric acid (23.1 g), 7-chloroindoline (1.9 g) was added at 0°C, and the mixture was stirred at room temperature for 5 hours. The reaction mixture was cooled to 0°C, and a 5 mol/L aqueous sodium hydroxide solution (96 mL) was added to neutralize the solution. The produced precipitate was filtered and washed with toluene, and the filtrate was then extracted with toluene. The organic layer was dried with anhydrous sodium sulfate, and the solvent was concentrated under reduced pressure. The resulting residue was purified by column chromatography (silica gel, developing solvent: n-hexane to n-hexane/ethyl acetate = 9/1) to obtain the title compound (700 mg).
$^1$H NMR ($CDCl_3$) δ (ppm): 3.20(2H,t), 3.75(2H,t), 4.31(1H,brs), 7.03(1H,d), 7.26(1H,d).

Reference Example 54

7-chloro-4-nitro-1H-indole

7-chloro-4-nitro-1H-indoline (1.7 g) was dissolved in methanol (20 mL), sarcomine (630 mg) was added, and the mixture was stirred at 30°C for 18 hours while the air was blown therein. After the reaction mixture was concentrated under reduced pressure, the resulting residue was purified by column chromatography (silica gel, developing solvent: n-hexane to n-hexane/ethyl acetate =1/1) to obtain the title compound (540 mg).
$^1$H NMR ($CDCl_3$) δ (ppm): 7.29(1H,d), 7.36(1H,t), 7.53(1H,t), 8.12(1H,d), 8.77(1H,brs).

Reference Example 55

2,2,2-trifluoro-N-(2-methyl-3-nitrophenyl)acetimidoyl chloride

Triphenylphosphine (12.9 g) and triethylamine (2.7 mL) were dissolved in carbon tetrachloride (16.0 mL), trifluoroacetic acid (1.5 mL) was added dropwise at 0°C, and the mixture was stirred for 10 minutes. Further, 2-methyl-3-nitroaniline (2.5 g) was added at room temperature, and the mixture was stirred at 70°C for 5 hours. After the reaction mixture was returned to room temperature, n-hexane was added, and the produced precipitate was filtered. After the filtrate was concentrated under reduced pressure, n-hexane was added to the resulting residue again, and the produced precipitate was filtered. The filtrate was concentrated under reduced pressure to obtain the title compound (3.1 g).
$^1$H NMR ($CDCl_3$) δ (ppm): 2.32(3H,s), 7.13(1H,d), 7.42(1H,t), 7.80(1H,d).

Reference Example 56

N-(2,6-dimethyl-3-nitrophenyl)-2,2,2-trifluoroacetimidoyl chloride

According to the same manner as that of Reference Example 55 using 2,6-dimethyl-3-nitrophenylamine in place of 2-methyl-3-nitroaniline, a reaction was performed to obtain the title compound.
$^1$H NMR ($CDCl_3$) δ (ppm): 2.12(3H,s), 2.21(3H,s), 7.23(1H,d), 7.71(1H,d).

Reference Example 57

4-nitro-2-trifluoromethyl-1H-indole

Potassium ethoxide (1.4 g) was dissolved in N,N-dimethylformamide (5.0 mL), and diethyl oxalate (1.6 mL) were added. Then, a solution of 2,2,2-trifluoro-N-(2-methyl-3-nitrophenyl)acetimidoyl chloride (2.1 g) in N,N-dimethylformamide (5.0 mL) was added dropwise at 0°C, and the mixture was stirred at 40°C for 5 hours. The reaction mixture was poured into ice water, followed by extraction with ethyl acetate. The organic layer was washed with water and an aqueous saturated sodium chloride solution, and dried with anhydrous sodium sulfate, and the solvent was concentrated under reduced pressure. The resulting residue was purified by flash chromatography (silica gel, developing solvent: n-hexane/ethyl acetate = 9/1 to 4/1) to obtain the title compound (1.1 g).
$^1$H NMR ($CDCl_3$) δ (ppm): 7.47(1H,t), 7.71(1H,d), 7.80(1H,d), 8.23(1H,d), 8.87(1H,brs).

Reference Example 58

7-methyl-4-nitro-2-trifluoromethyl-1H-indole

According to the same manner as that of Reference Example 57 using N-(2,6-dimethyl-3-nitrophenyl)-2,2,2-trifluoroacetimidoyl chloride in place of 2,2,2-trifluoro-N-(2-methyl-3-nitrophenyl)acetimidoyl chloride, a reaction was performed to obtain the title compound.
$^1H$ NMR ($CDCl_3$) δ (ppm): 2.66(3H,s), 7.24(1H,d), 7.72(1H,s), 8.16(1H,d), 8.73(1H,s).

Reference Example 59

6-bromo-4-phthalimido-1H-indole

4-amino-6-bromo-1H-indole (300 mg) was dissolved in acetic acid (10.0 mL), phthalic anhydride (316 mg) was added, and the mixture was stirred at 90°C for 4 hours. The reaction mixture was returned to room temperature, and then neutralized with an aqueous saturated sodium bicarbonate solution, followed by extraction with ethyl acetate. The organic layer was washed with water and an aqueous saturated sodium chloride solution, and dried with anhydrous sodium sulfate. The solvent was concentrated under reduced pressure to obtain the title compound (399 mg).
$^1H$ NMR (DMSO-$d_6$) δ (ppm): 6.35(1H,s), 7.29(1H,d), 7.44(1H,t), 7.73(1H,s), 7.94-7.96(2H,m), 8.00-8.03(2H,m), 11.51 (1H,brs).

Reference Example 60

1-(3-isopropyl-4-triisopropylsilanyloxybenzyl)-4-nitro-1H-i ndole

(3-isopropyl-4-triisopropylsilanyloxyphenyl)methanol (478 mg) was dissolved in dichloromethane (5.0 mL), thionyl chloride (162 μL) was added dropwise, and the mixture was stirred at room temperature for 2 hours. A reaction mixture was concentrated under reduced pressure to obtain (4-chloromethyl-2-isopropylphenoxy)triisopropylsilane.
60% sodium hydride (59.2 mg) was suspended in N,N-dimethylformamide (1.0 mL), and a solution of 4-nitro-1H-indole (200 mg) in N,N-dimethylformamide (1.0 mL) was added dropwise at 5°C. After stirred at 5°C for 1 hour, a solution of the previously obtained (4-chloromethyl-2-isopropylphenoxy)triisopropylsilane in N,N-dimethylformamide (2.0 mL) was added dropwise at 5°C, and the mixture was stirred for 3 hours. The reaction mixture was poured into ice water, followed by extraction with ethyl acetate. The organic layer was washed with water and an aqueous saturated sodium chloride solution, and dried with anhydrous sodium sulfate, and the solvent was concentrated under reduced pressure. The resulting residue was purified by flash chromatography (silica gel, developing solvent: n-hexane/ethyl acetate = 9/1) to obtain the title compound (481 mg).
$^1H$ NMR ($CDCl_3$) δ (ppm): 1.08(18H,d), 1.09(6H,d), 1.21-1.34(3H,m), 3.36(1H,m), 5.33(2H,s), 6.70(1H,s), 7.06(1H,d), 7.26(3H,m), 7.38(1H,d), 7.66(1H,d), 8.15(1H,d).
Compounds were synthesized according to the following reaction formula referring to the method of Reference Example 60. Synthesized compounds are shown in Table 10, and data are shown in Table 11.

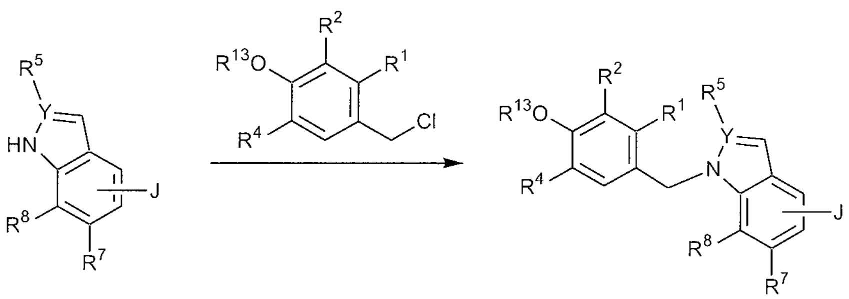

[Table 10]

| Reference Example | $R^1$ | $R^2$ | $R^4$ | $R^5$ | $R^7$ | $R^8$ | $R^{13}$ | Y | J |
|---|---|---|---|---|---|---|---|---|---|
| 61 | H | *i*-Pr | H | H | H | H | Bn | C | 4-COOMe |
| 62 | H | *i*-Pr | H | Me | H | H | TIPS | C | 4-$NO_2$ |
| 63 | H | *i*-pr | H | Et | H | H | TIPS | C | 4-$NO_2$ |
| 64 | H | *i*-Pr | H | $CF_3$ | H | H | TIPS | C | 4-$NO_2$ |
| 65 | H | *i*-Pr | H | H | Br | H | TIPS | C | 4-NPht |
| 66 | H | *i*-Pr | H | H | H | Cl | TIPS | C | 4-$NO_2$ |
| 67 | H | *i*-Pr | H | H | H | Me | TIPS | C | 4-$NO_2$ |
| 68 | H | *i*-Pr | H | $CF_3$ | H | Me | TIPS | C | 4-$NO_2$ |
| 69 | H | c-Pen | H | H | H | Me | TIPS | C | 4-$NO_2$ |
| 70 | H | Me | Me | H | H | Me | TIPS | C | 4-$NO_2$ |
| 71 | H | *i*-Pr | H | H | H | H | TIPS | C | 5-$NO_2$ |
| 72 | H | *i*-Pr | H | Me | H | H | TIPS | C | 5-$NO_2$ |
| 73 | H | *i*-Pr | H | H | H | Me | TIPS | C | 5-$NO_2$ |
| 74 | H | *i*-Pr | H |  | H | H | TIPS | N | 4-$NO_2$ |

[Table 11]

| Reference Example | $^1$HNMR ($CDCl_3$) δ (ppm) |
|---|---|
| 61 | 1.19(6H,d),3.38(1H,m),3.99(3H,s),5.04(2H,s),5.30(2H,s),6.80(2H,s), 7.08(1H,s),7.16(1H,d), 7.20-7.26(2H,m),7.30-7.42(5H,m),7.52(1H,d), 7.91(1H,d). |
| 62 | 1.07(18H,d),1.14(6H,d),1.26(3H,m),2.48(3H,s),3.32(1H,m),5.29(2H,s), 6.42(1H,dd),6.62(1H, d),6.94(1H,d),7.10(1H,s),7.15(1H,dd),7.54(1H,d), 8.10(1H,d). |
| 63 | 1.09(18H,d),1.13(6H,d),1.25(3H,m),1.26(3H,t),1.38(3H,t),2.79(2H,q), 3.32(1H,m),5.30(2H, s),6.35(1H,s),6.42(1H,dd),6.62(1H,d),6.91(1H,d), 7.12-7.17(2H,m),7.53(1H,d),8.10(1H,d). |
| 64 | 1.07(18H,d),1.13(6H,d),1.28(3H,m),3.34(1H,m),5.46(2H.s),6.58(1H,dd), 6.65(1H,d),6.98 (1H,d),7.24(1H,d),7.36(1H,t),7.61(1H,d),7.75(1H,s), 8.18(1H,d). |
| 65 | 1.09(18H,d),1.18(6H,d),1.25-1.30(3H,m),3.35(1H,m),5.19(2H,s),6.26 (1H,d),6.70(1H,s),6.71 (1H,d),7.07-7.09(2H,m),7.23(1H,d),7.56(1H,s), 7.79-7.82(2H,m),7.97-8.00(2H,m). |
| 66 | 1.08(18H,d),1.15(6H,d),1.28(3H,m),3.34(1H,m),5.72(2H,s),6.63-6.70 (2H,m), 6.99(1H,d), 7.20(1H,d),7.33(2H,s),8.05(1H,d). |
| 67 | 1.08(18H,d),1.13(6H,d),1.26(3H,m),2.63(3H,s),3.33(1H,m),5.55(2H,s), 6.42(1H,dd),6.65(1H, d),6.86(1H,d),6.94(1H,d), 7.32(2H,dd),8.04(1H,d). |
| 68 | 1.07(18H,d),1.13(6H,d),1.28(3H,m),2.58(3H,s),3.34(1H,m),5.65(2H.s), 6.24(1H,dd),6.60(1H, d),6.98(1H,d),7.09(1H,d),7.15(1H,d),7.83(1H,s), 8.08(1H,d). |
| 69 | 1.10(18H,d),1.28(3H,m),1.36-2.14(8H,m),2.62(3H,s),3.36(1H,m),5.53 (2H,s),6.44(1H,dd), 6.66(1H,d),6.82(1H,d),6.93(1H,d),7.30(1H,d),7.33 (1H,d),8.04(1H,d). |
| 70 | 1.08(18H,d),1.25(3H,m),2.15(6H,s),2.60(3H,s),5.47(2H,s),6.49(2H,s), 6.91(1H,d),7.26(1H, d),7.31(1H,d),8.01(1H,d). |
| 71 | 1.09(18H,d),1.15(6H,m),1.29(3H,m),3.34(1H,m),5.65(2H,s),6.70(3H,m), 7.02(1H,s), 7.33-7.36(2H,m),8.09(1H,dd),8.61(1H,d). |
| 72 | 1.06(18H,d),1.14(6H,m),1.26(3H,m),2.41(3H,s),3.32(1H,m),5.26(2H,s), 6.45(1H,dd),6.48 (1H,s),6.63(1H,d),6.93(1H,d),7.25(1H,d),8.02(1H,dd), 8.50(1H,d). |

(continued)

| Reference Example | $^1$HNMR ($CDCl_3$) δ (ppm) |
|---|---|
| 73 | 1.09(18H,d),1.12(6H,m),1.28(3H,m),2.61(3H,s),3.33(1H,m),5.53(2H,s), 6.45(1H,dd),6.66 (1H,d),6.71(1H,d),7.19(1H,d),7.81(1H,s),8.44(1H,s). |
| 74 | 1.07(18H,d),1.11(6H,d),1.32(3H,m),3.34(1H,m),5.60(2H,s),6.68(1H,d), 6.82(1H,dd),1.11 (1H,d),7.43(1H,t),7.69(1H,d),8.13(1H,m),8.66(1H,s). |

Reference Example 75

1-(3-bromo-4-triisopropylsilanyloxybenzyl)-2,7-dimethyl-4-n itro-1H-indoline

(3-bromo-4-triisopropylsilanyloxyphenyl)methanol (497 mg) was dissolved in dichloromethane (2.0 mL), thionyl chloride (151 μL) was added dropwise, and the mixture was stirred at room temperature for 2 hours. The reaction mixture was concentrated under reduced pressure to obtain (2-bromo-4-chloromethylphenoxy)triisopropylsilane.

2,7-dimethyl-4-nitro-1H-indoline (221 mg) was dissolved in acetone (10 mL), and potassium carbonate (239 mg) and sodium iodide (10 mg) were added. Then, a solution of the previously obtained (2-bromo-4-chloromethylphenoxy) triisopropylsilane (522 mg) in acetone (5.0 mL) was added dropwise, and the mixture was heated to reflux for 14 hours. The reaction mixture was filtered, and the filtrate was concentrated under reduced pressure. To the resulting residue was added water, followed by extraction with ethyl acetate. The organic layer was washed with water and an aqueous saturated sodium chloride solution, and dried with anhydrous sodium sulfate, and the solvent was concentrated under reduced pressure. The resulting residue was purified by flash chromatography (silica gel, developing solvent: n-hexane/ ethyl acetate = 4/1) to obtain the title compound (312 mg).
$^1$H NMR ($CDCl_3$) δ (ppm): 1.11(18H,d), 1.20(3H,d), 1.27(3H,m), 2.32(3H,s), 2.98(1H,m), 3.66-3.79(2H,m), 4.35(1H,d), 4.45(1H,d), 6.82(1H,d), 6.96(1H,d), 7.04(1H,dd), 7.45-7.49(2H,m).

Compounds were synthesized according to the following reaction formula referring to the method of Reference Example 75. Synthesized compounds are shown in Table 12, and data are shown in Table 13.

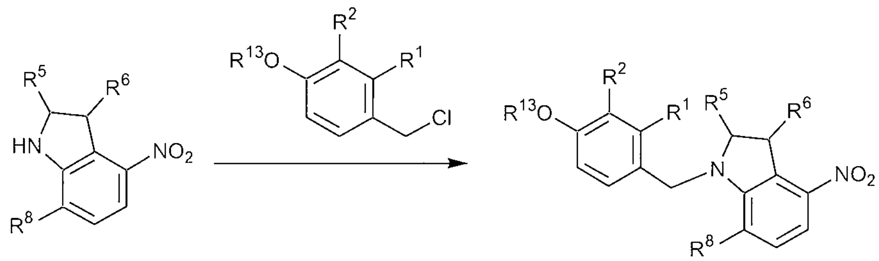

[Table 12]

| Reference Example | $R^1$ | $R^2$ | $R^5$ | $R^6$ | $R^8$ | $R^{13}$ |
|---|---|---|---|---|---|---|
| 76 | Me | Me | Me | H | Me | Me |
| 77 | -CH=CH-CH=CH- | | Me | H | Me | TIPS |
| 78 | H | Cl | Me | H | Me | TIPS |
| 79 | H | Me | Me | H | Me | TIPS |
| 80 | H | *n*-Pr | Me | H | Me | TIPS |
| 81 | H | *i*-Pr | Me | Me | H | TIPS |
| 82 | H | *i*-Pr | Me | H | Me | Me |
| 83 | H | *i*-Pr | Me | H | Me | TIPS |
| 84 | H | *i*-Pr | Et | H | Me | TIPS |
| 85 | H | *s*-Bu | Me | H | Me | TIPS |

(continued)

| Reference Example | $R^1$ | $R^2$ | $R^5$ | $R^6$ | $R^8$ | $R^{13}$ |
|---|---|---|---|---|---|---|
| 86 | H | $CF_3$ | Me | H | Me | TIPS |
| 87 | H | OEt | Me | H | Me | TIPS |

[Table 13]

| Reference Example | $^1$HNMR ($CDCl_3$) δ (ppm) |
|---|---|
| 76 | 1.23(3H,d),2.19(3H,s),2.20(3H,s),2.23(3H,s),3.04(1H,m),3.76-3.86(5H,m). 4.36(1H,d),4.57 (1H,d),6.68(1H,d),6.91(1H,d),7.18(1H,d),7.41(1H,d). |
| 77 | 1.15(18H,d),1.25(3H,d),1.39(3H,m),2.22(3H,s),3.07(1H,m),3.78-3.92(2H,m), 4.81(1H,d), 5.04(1H,d),6.82(1H,d),6.91(1H,d),7.38(1H,d),7.43(1H,d),7.48-7.58 (2H,m),7.90-7.96(1H,m), 8.32-8.38(1H,m). |
| 78 | 1.11(18H,d),1.20(3H,d),1.27(3H,m),2.31(3H,s),2.96(1H,m),3.64-3.78 (2H,m),4.34(1H,d), 4.44(1H,d),6.80(1H,d),6.87(1H,d),7.05(1H,dd),7.35 (1H,d),7.49(1H,s). |
| 79 | 1.09(18H,d),1.19(3H,d),1.23-1.34(3H,m),2.21(3H,s),2.35(3H,s),6.70(1H,d), 6.89(1H,dd), 6.95(1H,d),7.00(1H,d),7.44(1H,d). |
| 80 | 0.89(3H,t),1.10(18H,d),1.19(3H,d),1.22-1.34(3H,m),1.48-1.65(2H,m),2.35 (3H,s),2.54(2H,t), 2.96(1H,m),3.64-3.78(2H,m),4.41(2H,s),6.69(1H,d),6.89 (1H,brd),6.95(1H,d),6.98(1H,brs), 7.45(1H,d). |
| 81 | 1.05-1.36(33H,m),3.34(1H,m),3.73(1H,m),3.85(1H,m),4.04(1H,d),4.43(1H,d), 6.61(1H,d), 6.69(1H,d),6.91(1H,dd);7.09-7.13(2H,m),7.42(1H,d). |
| 82 | 1.14(6H,d),1.20(3H,d),1.30(3H,m),2.36(3H,s),2.96(1H,m),3.33(1H,m),3.64-3.73 (2H,m),3.81 (3H,s),4.42(2H,m),6.69(1H,d),6.88(1H,dd),6.96(1H,d),7.03(1H,d); 7.46(1H,d). |
| 83 | 1.10(18H,d),1.14(6H,d),1.24(3H,d),1.30(3H,m),2.36(3H,s),2.96(1H,m),3.33 (1H,m), 3.64-3.73(2H,m),4.42(2H,m),6.69(1H,d),6.88(1H,dd),6.96(1H,d), 7.03(1H,d), 7.46(1H,d). |
| 84 | 0.85(3H,t),1.07-1.12(24H,m),1.22-1.32(3H,m),1.50-1.57(2H,m),2.36(3H,s), 3.00(1H,m),3.31 (1H,m),3.51-3.61(2H,m),4.37(2H,q),6.67(1H,d),6.84(1H,dd), 6.94-6.97(2H,m),7.46(1H,d). |
| 85 | 0.14-0.85(3H,m),1.05-1.15(21H,m),1.19(3H,d),1.24-1.32(3H,m),1.40-1.70 (2H,m),2.32-2.38 (3H,m),2.90-3.02(1H,m),3.06-3.20(1H,m),3.62-3.78(2H,m), 4.34-4.48(2H,m),6.79(1H,brd), 6.86(1H,dd),6.92-7.00(2H,m),7.46(1H,brd). |
| 86 | 1.09(18H,d),1.20(3H,d),1.30(3H,m),2.31(3H,s),2.98(1H,dd),3.65-3.78(2H,m), 4.36(1H,d), 4.48(1H,d),6.83(1H,d),6.96(1H,d),7.26(1H,dd),7.46-7.48(2H,m). |
| 87 | 1.08(18H,d),1.19(3H,d),1.18-1.30(3H,m),1.37(3H,t),2.34(3H,s),2.96(1H,m), 3.64-3.80(2H, m),3.91(2H,q),4.37(1H,d),4.44(1H,d),6.66(1H,dd),6.73(1H,d), 6.79(1H,d),6.95(1H,d),7.45 (1H,d), |

Reference Example 88

<u>(4-fluorophenyl)-[2-methoxy-5-(7-methyl-4-nitro-1H-indolin-1-ylmethyl)phenyl]methanone</u>

7-methyl-4-nitro-1H-indoline (300 mg) was dissolved in toluene (5.0 mL), followed by addition of potassium carbonate (349 mg), sodium iodide (20 mg), and (5-bromomethyl-2-methoxyphenyl)-(4-fluorophenyl)methanone (653 mg), and the mixture was heated to reflux for 6 hours. After the reaction mixture was returned to room temperature and filtered, the filtrate was concentrated under reduced pressure. To the resulting residue were added ethyl acetate and water, followed by extraction with ethyl acetate. The organic layer was washed with water and an aqueous saturated sodium chloride solution, and dried with anhydrous sodium sulfate, and the solvent was concentrated under reduced pressure. The resulting residue was purified by column chromatography (silica gel, developing solvent: n-hexane to n-hexane/ethyl acetate = 10/1) to obtain the title compound (352 mg).
$^1$H NMR ($CDCl_3$) δ (ppm) : 2.37(3H,s), 3.40-3.50(4H,m), 3.71(3H,s), 4.45(2H,s), 6.95(2H,d), 7.09(2H,t), 7.29(1H,d), 7.41(1H,dd), 7.46(1H,d), 7.80(2H,dd).

Reference Example 89

(4-fluorophenyl)-[2-methoxy-5-(2,7-dimethyl-4-nitro-1H-indo lin-1-ylmethyl)phenyl]methanone

2,7-dimethyl-4-nitro-1H-indoline (10.0 g) was dissolved in acetonitrile (200 mL), followed by addition of cecium carbonate (20.4 g), sodium iodide (779 mg), and (5-bromomethyl-2-methoxyphenyl)-(4-fluorophenyl)methanone (20.2 g), and the mixture was heated to reflux for 6 hours. After the reaction mixture was returned to room temperature and filtered, the filtrate was concentrated under reduced pressure. To the resulting residue were added ethyl acetate and water, followed by extraction with ethyl acetate. The organic layer was washed with water and an aqueous saturated sodium chloride solution, and dried with anhydrous sodium sulfate, and the solvent was concentrated under reduced pressure. The resulting residue was purified by column chromatography (silica gel, developing solvent: n-hexane to n-hexane/ethyl acetate = 10/1) to obtain the title compound (21.5 g).
$^1$H NMR ($CDCl_3$) δ (ppm): 1.21(3H,d), 2.32(3H,s), 2.97(1H,m), 3.70-3.76(5H,m), 4.41(1H,d), 4.51(1H,d), 6.93(2H,m), 7.07(2H,t), 7.25(2H,t), 7.38(1H,d), 7.45(1H,d), 7.78(2H,dd).

Reference Example 90

1-[3-(4-fluorobenzyl)-4-methoxybenzyl]-2,7-dimethyl-4-nitro -1H-indoline

(4-fluorophenyl)-[2-methoxy-5-(2,7-dimethyl-4-nitro-1 H-indoline-1-ylmethyl)phenyl]methanone (262 mg) was dissolved in dichloromethane (1.6 mL), trifluoroacetic acid (0.8 mL) and triethylsilane (337 μL) were added, and the mixture was stirred at room temperature for 15 hours. The reaction mixture was concentrated under reduced pressure, and the resulting residue was dissolved in ethyl acetate. The organic layer was washed with water and an aqueous saturated sodium chloride solution, and dried with anhydrous sodium sulfate. After the solvent was concentrated under reduced pressure, the resulting residue was purified by flash chromatography (silica gel, developing solvent: n-hexane to n-hexane/ethyl acetate = 5/1) to obtain the title compound (187 mg).
$^1$H NMR ($CDCl_3$) δ (ppm): 1.18(3H,d), 2.29(3H,s), 2.95(1H,dd), 3.62-3.68(2H,m), 3.80(3H,s), 3.90(2H,s), 4.36(1H,d), 4.46(1H,d), 6.79(1H,d), 6.84(1H,d), 6.88-6.92(3H,m), 7.03-7.07(3H,m), 7.44(1H,d).

Reference Example 91

[5-(7-methyl-4-nitro-1H-indolin-1-ylmethyl)-2-hydroxyphenyl ]-(4-fluorophenyl)methanone

(4-fluorophenyl)-[2-methoxy-5-(7-methyl-4-nitro-1H-in dolin-1-ylmethyl)phenyl]methanone (498 mg) was dissolved in dichloromethane (10.0 mL), a 1 mol/L solution of boron tribromide in dichloromethane (4.7 mL) was added dropwise at 0°C, and the mixture was stirred at room temperature for 3 hours. To the reaction mixture was added 1 mol/L hydrochloric acid, and this was stirred at room temperature for 16 hours, followed by extraction with dichloromethane. The organic layer was washed with water and an aqueous saturated sodium chloride solution, and dried with anhydrous sodium sulfate, and the solvent was concentrated under reduced pressure. The resulting residue was purified by column chromatography (silica gel, developing solvent: n-hexane to n-hexane/ethyl acetate = 4/1) to obtain the title compound (441 mg).
$^1$H NMR ($CDCl_3$) δ (ppm) : 2.35(3H,s), 3.39-3.49(4H,m), 4.41(2H,s), 6.95(1H,d), 7.08(1H,d), 7.14(2H,t), 7,43-7.50(3H, m), 7.63(2H,dd), 11,82(1H,s). Compounds were synthesized according to the following reaction formula referring to the method of Reference Example 91. Synthesized compounds and data are shown in Table 14.

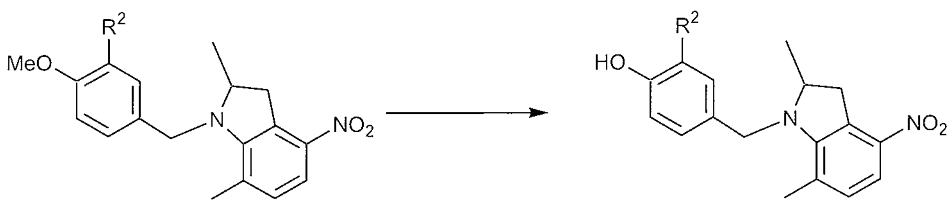

[Table 14]

| Reference Example | $R^2$ | $^1$HNMR ($CDCl_3$) δ (ppm) |
|---|---|---|
| 92 | 4-F-Bz | 1.21(3H,d),2.28(3H,s),2.96(1H,m),3.64-3.72(2H,m),4.44(2H,dd), 6.92(1H,d), 7.05-7.13(3H,m),7.41-7.47(3H,m),7.57(2H,m), 11.80(1H,s). |
| 93 | 4-F-Bn | 1.18(3H,d),2.31(3H,s),2.95(1H,dd),3.64-3.72(2H,m),3.90(2H,s), 4.40(2H,dd),4.48 (1H,s),6.72(1H,d),6.90-6.96(4H,m),7.00(1H,dd), 7.07-7.11(2H,m),7.45(1H,d). |

Reference Example 94

1-[3-(4-fluorobenzyl)-4-triisopropylsilanyloxybenzyl]-2,7-d imethyl-4-nitro-1H-indoline

1-[3-(4-fluorobenzyl)-4-hydroxybenzyl]-2,7-dimethyl-4 -nitro-1H-indoline (117 mg) was dissolved in N,N-dimethylformamide (2.0 mL), triisopropylsilyl chloride (73.9 μL) and imidazole (39.2 mg) were added, and the mixture was stirred at room temperature for 48 hours. The reaction mixture was poured into ice water, followed by extraction with n-hexane. The organic layer was washed with water and an aqueous saturated sodium chloride solution, and dried with anhydrous sodium sulfate. The solvent was concentrated under reduced pressure, and the resulting residue was purified by flash chromatography (silica gel, developing solvent: n-hexane to n-hexane/ethyl acetate = 4/1) to obtain the title compound (126 mg).
$^1$H NMR ($CDCl_3$) δ (ppm): 1.06(18H,d), 1.16(3H,d), 1.23-1.31(3H,m), 2.28(3H,s), 2.90-2.95(1H,m), 3.59-3.70(2H,m), 3.90(2H,s), 4.31(1H,d), 4.43(1H,d), 6.73-6.78(2H,m), 6.88-6.95(4H,m), 7.00-7.04(2H,m), 7.43(1H,d).

Reference Example 95

1-(3-bromo-4-triisopropylsilanyloxybenzyl)-2,7-dimethyl-4-n itro-1H-indole

1-(3-bromo-4-triisopropylsilanyloxybenzyl)-2,7-dimeth yl-4-nitro-1H-indoline (300 mg) was dissolved in 1,4-dioxane (10 mL), 2,3-dichloro-5,6-dicyano-1,4-benzoquinone (140 mg) was added, and the mixture was stirred at 60°C for 1 hour. After the reaction mixture was concentrated under reduced pressure, the resulting residue was dissolved in ethyl acetate. The organic layer was then washed with an aqueous saturated sodium bicarbonate solution, water and an aqueous saturated sodium chloride solution, and dried with anhydrous sodium sulfate, and the solvent was concentrated under reduced pressure. The resulting residue was purified by flash chromatography (silica gel, developing solvent: n-hexane/ethyl acetate = 3/1) to obtain the title compound (277 mg).
$^1$H NMR ($CDCl_3$) δ (ppm): 1.08(18H,d), 1.25(3H,m), 2.39(3H,s), 2.57(3H,s), 5.47(2H,s), 6.41(1H,dd), 6.75(1H,d), 6.85 (1H,d), 7.10(1H,d), 7.16(1H,d), 7.98(1H,d).

Compounds were synthesized according to the following reaction formula referring to the method of Reference Example 95. Synthesized compounds are shown in Table 15, and data are shown in Table 16.

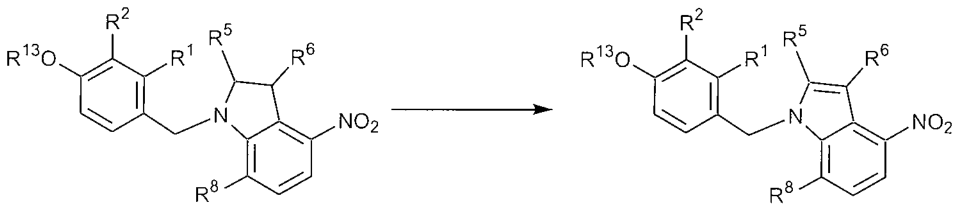

[Table 15]

| Reference Example | $R^1$ | $R^2$ | $R^5$ | $R^6$ | $R^8$ | $R^{13}$ |
|---|---|---|---|---|---|---|
| 96 | Me | Me | Me | H | Me | Me |
| 97 | -CH=CH-CH=CH- | | Me | H | Me | TIPS |
| 98 | H | Cl | Me | H | Me | TIPS |

(continued)

| Reference Example | $R^1$ | $R^2$ | $R^5$ | $R^6$ | $R^8$ | $R^{13}$ |
|---|---|---|---|---|---|---|
| 99 | H | Me | Me | H | Me | TIPS |
| 100 | H | *n*-Pr | Me | H | Me | TIPS |
| 101 | H | *i*-Pr | Me | Me | H | TIPS |
| 102 | H | *i*-Pr | Me | H | Me | Me |
| 103 | H | *i*-Pr | Me | H | Me | TIPS |
| 104 | H | *i*-Pr | Et | H | Me | TIPS |
| 105 | H | *s*-Bu | Me | H | Me | TIPS |
| 106 | H | $CF_3$ | Me | H | Me | TIPS |
| 107 | H | 4-F-Bz | H | H | Me | H |
| 108 | H | 4-F-Bz | Me | H | Me | H |
| 109 | H | 4-F-Bn | Me | H | Me | TIPS |
| 110 | H | OEt | Me | H | Me | TIPS |

[Table 16]

| Reference Example | $^1$HNMR ($CDCl_3$) δ (ppm) |
|---|---|
| 96 | 2.22(3H,s),2.32(3H,s),2.40(3H,s),2.49(3H,s),3.73(3H,s),5.44(2H,s),5.80 (1H,d),6.48(1H,d), 6.84(1H,d),7.21(1H,s),8.00(1H,d). |
| 97 | 1.10(18H,d),1.35(3H,m),2.42(3H,s),2.45(3H,s),5.92(2H,brs),6.03(1H,d), 6.63(1H,d),6.85(1H, d),7.20-7.30(1H,m),7.59(1H,m),7.65(1H,m),7.97(1H,d), 8.02(1H,d),8.39(1H,brd). |
| 98 | 1.09(18H,d),1.27(3H,m),2.41(3H,d),2.57(3H,s),5.49(2H,s),6.41(1H,dd), 6.70(1H,d),6.87(1H, d),7.05(1H,d),7.18(1H,d),8.00(1H,d). |
| 99 | 1.07(18H,d),1.20-1.32(3H,m),2.16(3H,s),2.42(3H,s),2.60(3H,s),5.48(2H,s), 6.38(1H,dd), 6.65-6.67(2H,m),6.86(1H,d),7.17(1H,s),8.00(1H,d). |
| 100 | 0.87(3H,t),1.07(18H,d),1.24(3H,m),1.48-1.60(2H,m),2.43(3H,s),2.50(2H,t), 2.59(3H,s),5.48 (2H,s),6.35(1H,dd),6.64(1H,d),6.66(1H,d),6.85(1H,d), 7.17(1H,brs),8.00(1H,d). |
| 101 | 1.05(18H,d),1.13(6H,d),1.18-1.26(3H,m),2.29(3H,s),2.34(3H,s),3.31(1H,m), 5.25(2H,s),6.36 (1H,dd),6.59(1H,d),6.94(1H,d),1.07(1H,dd),7.45(1H,dd), 7.68(1H,dd). |
| 102 | 1.12(6H,d),2.43(3H,s),2.59(3H,s),3.32(1H,m),3.77(3H,s),5.51(2H,s),6.35 (1H,d),6.68(1H,d), 6.81-6.86(2H,m),7.19(1H,s),7.99(1H,d). |
| 103 | 1.07(18H,d),1.12(6H,d),1.25(3H,m),2.43(3H,s),2.59(3H,s),3.32(1H,m), 5.49(2H,s),6.26(1H, d),6.62(1H,d),6.81-6.86(2H,m),7.17(1H,s),7.98(1H,d). |
| 104 | 1.07(18H,d),1.11(6H,d),1.26(3H,m),1.38(3H,t),2.59(3H,s),2.73(2H,q), 3.32(1H,m),5.50(2H, s),6.23(1H,dd),6.61(1H,d),6.19(1H,d),6.86(1H,d), 7.20(1H,s),8.00(1H,d). |
| 105 | 0.77(3H,t),1.04-1.16(21H,m),1.26(3H,m),1.34-1.70(2H,m),2.43(3H,s), 2.59(3H,s),3.14-3.20 (1H,m),5.49(2H,s),6.28(1H,dd),6.63(1H,d),6.71(1H,d), 6.85(1H,d),7.17(1H,brs),7.99(1H,d). |
| 106 | 1.12(18H,d),1.29(3H,m),2.43(3H,s),2.61(3H,s),5.55(2H,s),6.56(1H,dd), 6.78(1H,d),6.90(1H, d),7.21(1H,s),7.24(1H,d)8.02(1H,d). |
| 107 | 2.61(3H,s),5.54(2H,s),6.77(1H,d),6.95(3H,t),7.05(1H,d),7.17(1H,dd), 7.23-7.36(4H,m),8.05 (1H,d),11.81(1H,s). |
| 108 | 2.37(3H,s),2.58(3H,s),5.49(2H,s),6.62(1H,brs),6.86-6.94(3H,m),7.03-7.32(3H,m),8.00(1H, d),11.78(1H,s). |

(continued)

| Reference Example | $^1$HNMR ($CDCl_3$) δ (ppm) |
|---|---|
| 109 | 1.03(18H,d),1.20-1.27(3H,m),2.38(3H,s),2.54(3H,s),3.87(2H,s),5.42(2H,s), 6.40-6.42(2H, m),6.69(1H,d),6.82-6.91(3H,m),6.96-7.00(2H,m),7.13(1H,s), 7.98(1H,s). |
| 110 | 1.06(18H,d),1.22(3H,m),1.33(3H,t),2.42(3H,s),2.57(3H,s),3.82(2H,q), 5.49(2H,s),6.15(1H, dd),6.30(1H,d),6.75(1H,d),6.85(1H,d),7.17(1H,s), 7.99(1H,d). |

Reference Example 111

2,7-dimethyl-4-nitro-1-[3-phenyl-4-triisopropylsilanyloxybe nzyl]-1H-indole

1-[3-bromo-4-triisopropylsilanyloxybenzyl]-2,7-dimeth yl-4-nitro-1H-indole (200 mg), tetrakis(triphenylphosphine)palladium (0)(21.4 mg), phenylboronic acid (55.1 mg), and potassium phosphate (239 mg) were dissolved in a mixed solution of diethoxyethane (3.0 mL) and ethanol (1.0 mL), and the mixture was stirred at 80°C for 24 hours. The reaction mixture was returned to room temperature, and filtered with a Celite pad, and the filtrate was concentrated under reduced pressure. The resulting residue was dissolved in ethyl acetate, and washed with water and an aqueous saturated sodium chloride solution, followed by dried with anhydrous sodium sulfate. The solvent was concentrated under reduced pressure, and the resulting residue was purified by flash chromatography (silica gel, developing solvent: n-hexane to n-hexane/ethyl acetate = 5/1) to obtain the title compound (95.4 mg).
$^1$H NMR ($CDCl_3$) δ (ppm): 0.91(18H,d), 1.03-1.11(3H,m), 2.45(3H,s), 2.63(3H,s), 5.55(2H,s), 6.46(1H,dd), 6.79(1H,d), 6.87(1H,d), 6.91(1H,d), 7.18-7.45(6H,m), 7.99(1H,d).
Compounds were synthesized according to the following reaction formula referring to the method of Reference Example 111. Synthesized compounds and data are shown in Table 17.

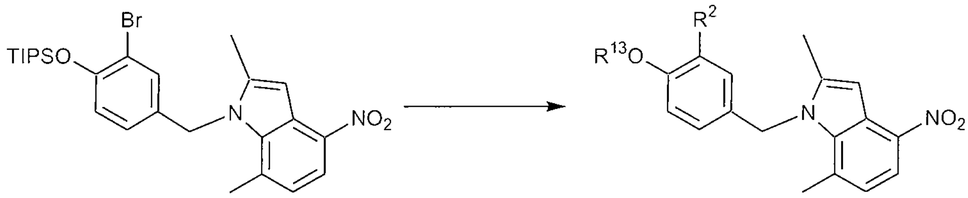

[Table 17]

| Reference Example | $R^2$ | $R^{13}$ | $^1$HNMR ($CDCl_3$) δ (ppm) |
|---|---|---|---|
| 112 | 3-Py | H | 2.46(3H,s),2.65(3H,s),5.31(1H,s),5.58(2H,s),6.62(1H,dd), 6.84-6.90(3H,m), 7.20(1H,d),7.37(1H,dd),7.77(1H,dt),8.01 (1H,d),8.60(1H,dd),8.70(1H,d). |
| 113 | Sty | H | 2.46(3H,s),2.63(3H,s),5.55(2H,s),6.42(1H,d),6.72(1H,d), 6.88(1H,d),7.02 (1H,d),7.09(1H,s),7.21-7.37(5H,m),7.50(2H,d), 8.02(1H,d). |

Reference Example 114

1-(3-isopropyl-4-benzyloxybenzoyl)-7-methyl-4-nitro-1H-indo le

4-benzyloxy-3-isopropylbenzoic acid (552 mg) was dissolved in dichloromethane (2.0 mL), oxalyl dichloride (151 μL) was added dropwise, and the mixture was stirred at room temperature for 2 hours. The reaction mixture was concentrated under reduced pressure to obtain 4-benzyloxy-3-isopropylbenzoyl chloride.
60% sodium hydride (81.7 mg) was suspended in N,N-dimethylformamide (1.0 mL), a solution of 7-methyl-4-nitro-1H-indole (300 mg) in N,N-dimethylformamide (1.0 mL) was added dropwise at 5 °C, and the mixture was stirred for 1 hour. A solution of 4-benzyloxy-3-isopropylbenzoyl chloride (590 mg) in N,N-dimethylformamide (1.0 mL) was added dropwise at 5°C, and the mixture was stirred for 3 hours. The reaction mixture was poured into ice water, followed by extraction with ethyl acetate. The organic layer was washed with water and an aqueous saturated sodium chloride solution, and dried with anhydrous sodium sulfate, and the solvent was concentrated under reduced pressure. The

resulting residue was purified by flash chromatography (silica gel, developing solvent: n-hexane to n-hexane/ethyl acetate = 19/1) to obtain the title compound (646 mg).
$^1$H NMR ($CDCl_3$) δ (ppm): 1.27(6H,d), 2.54(3H,s), 3.45(1H,m), 5.22(2H,s), 7.03(1H,s), 7.25(1H,d), 7.35-7.50(7H,m), 7.77(1H,dd), 7.88(1H,d), 8.20(1H,d).

Reference Example 115

3-(3-isopropyl-4-triisopropylsilanyloxybenzyl)-7-nitro-1H-i ndole

7-nitro-1H-indole (300 mg) was dissolved in dichloromethane (5.0 mL), and a solution of 3-isopropyl-4-triisopropylsilanyloxybenzaldehyde (652 mg) in dichloromethane (5.0 mL) was added at room temperature. Further, a solution of trifluoroacetic acid (206 μL) and triethylsilane (887 μL) in dichloromethane (1.0 mL) was added dropwise at 0°C, and the mixture was stirred for 2 hours. The reaction mixture was neutralized with a 1 mol/L aqueous sodium hydroxide solution, and extracted with dichloromethane. The organic layer was washed with water and an aqueous saturated sodium chloride solution, and dried with anhydrous sodium sulfate, and the solvent was concentrated under reduced pressure. The resulting residue was purified by flash chromatography (silica gel, developing solvent: n-hexane to n-hexane/ethyl acetate = 19/1) to obtain the title compound (175 mg).
$^1$H NMR ($CDCl_3$) δ (ppm): 1.14(18H,d), 1.18(6H,d), 1.31(3H,m), 3.35(1H,m), 4.07(2H,s), 6.69(1H,d), 6.87(1H,dd), 7.09-7.17(2H,m), 7.84(1H,d), 8.15(1H,d), 9.73(1H,brs).

Reference Example 116

1-(3-bromo-4-triisopropylsilanyloxybenzyl)-2,7-dimethyl-1H-indole-4-ylamine

1-(3-bromo-4-triisopropylsilanyloxybenzyl)-2,7-dimeth yl-4-nitro-1H-indole (273 mg) was dissolved in acetic acid (15 mL), distilled water (93 μL), and iron (287 mg) were added, and the mixture was heated to stir at 60°C for 6 hours. The reaction mixture was returned to room temperature, and filtered using Celite. To the filtrate was added ethyl acetate, the organic layer was washed with water and an aqueous saturated sodium chloride solution, followed by dried with anhydrous sodium sulfate, and the solvent was concentrated under reduced pressure. The resulting residue was purified by flash chromatography (silica gel, developing solvent: n-hexane/ethyl acetate = 3/1) to obtain the title compound (165 mg).
$^1$H NMR ($CDCl_3$) δ (ppm): 1.09(18H,d), 1.27(3H,m), 2.30(3H,d), 2.42(3H,s), 5.30(brs), 5.42(2H,s), 6.24(1H,d), 6.31(1H, d), 6.48(1H,dd), 6.64(1H,d), 6.73(1H,d), 7.16(1H,d).

Compounds were synthesized according to the following reaction formula referring to the method of Reference Example 116. Synthesized compounds and data are shown in Table 18.

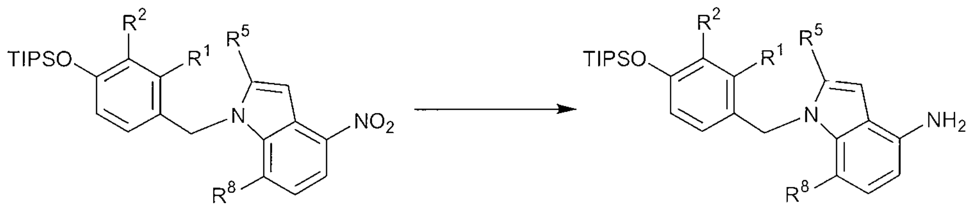

[Table 18]

| Reference Example | $R^1$ | $R^2$ | $R^5$ | $R^8$ | $^1$HNMR ($CDCl_3$) δ (ppm) |
|---|---|---|---|---|---|
| 117 | -CH=CH-CH=CH- | | Me | Me | 1.10(18H,d),1.34(3H,m),2.29(3H,s),2.32 (3H,s),5.85(2H, brs),6.15(1H,d),6.32(1H,s), 6.36(1H,d),6.63(2H,d),7.55 (1H,m),7.62 (1H,m),7.98(1H,d),8.36(1H,d). |
| 118 | H | Cl | Me | Me | 1.08(18H,d),1.27(3H,m),2.31(3H,d),2.44 (3H,s),5.35 (brs),5.41(2H,s),6.22(1H,d),6.30 (1H,d),6.45(1H,dd), 6.64(1H,d),6.73(1H,d), 7.13(1H,d). |

(continued)

| Reference Example | $R^1$ | $R^2$ | $R^5$ | $R^8$ | $^1$HNMR ($CDCl_3$) δ (ppm) |
|---|---|---|---|---|---|
| 119 | H | *i*-Pr | H | Cl | 1.08(18H,d),1.15(6H,d),1.24-1.30(3H,m), 3.33(1H,m), 3.93(2H,brs),5.64(2H,s),6.29 (1H,d),6.34(1H,d),6.40(1H, d),6.64-6.71 (2H,m),6.96(1H,d),7.04(1H,d). |

Reference Example 120

6-bromo-1-(3-isopropyl-4-triisopropylsilanyloxybenzyl)-1H-i ndol-4-ylamine

6-bromo-1-(3-isopropyl-4-triisopropylsilanyloxybenzyl )-4-nitro-1H-indole (144 mg) was dissolved in ethanol (2.0 mL), hydrazine monohydrate (22 μL) was added, and the mixture was stirred at 70°C for 1 hour. After the reaction mixture was returned to room temperature, ethyl acetate and water were added, followed by extraction with ethyl acetate. The organic layer was washed with water and an aqueous saturated sodium chloride solution, and dried with anhydrous sodium sulfate. The solvent was concentrated under reduced pressure to obtain the title compound (153 mg).
$^1$H NMR ($CDCl_3$) δ (ppm): 1.08(18H,d), 1.16(6H,d), 1.20-1.31(3H,m), 3.33(1H,m), 3.96(2H,brs), 5.12(2H,s), 6.37(1H, dd), 6.51(1H,s), 6.65-6.68(2H,m), 6.93-6.95(2H,m), 7.03(1H,d), 7.56(1H,s), 7.79-7.82(2H,m), 7.97-8.00(2H,m).

Reference Example 121

2,7-dimethyl-1-(3-trifluoromethyl-4-triisopropylsilanyloxyb enzyl)-1H-indol-4-ylamine

2,7-dimethyl-4-nitro-1-(3-trifluoromethyl-4-triisopro pylsilanyloxybenzyl)-1H-indole (311mg) was dissolved in a mixed solution of tetrahydrofuran (5.0 mL) and ethanol (5.0 mL), 10% palladium carbon (93.3 mg) was added, and the mixture was stirred in the hydrogen atmosphere at room temperature for 3 hours. The catalyst was filtered, and the filtrate was concentrated under reduced pressure. The resulting residue was purified by flash chromatography (silica gel, developing solvent: n-hexane to n-hexane/ethyl acetate = 4/1) to obtain the title compound (257 mg).
$^1$H NMR ($CDCl_3$) δ (ppm): 1.11(18H,d), 1.29(3H,m), 2.33(3H,s), 2.44(3H,s), 3.78(2H,brs), 5.48(2H,s), 6.27(1H,d), 6.33 (1H,d), 6.62(1H,d), 6.66(1H,d), 6.75(1H,d).

Compounds were synthesized according to the following reaction formula referring to the method of Reference Example 121. Synthesized compounds are shown in Table 19, and data are shown in Table 20 and Table 21.

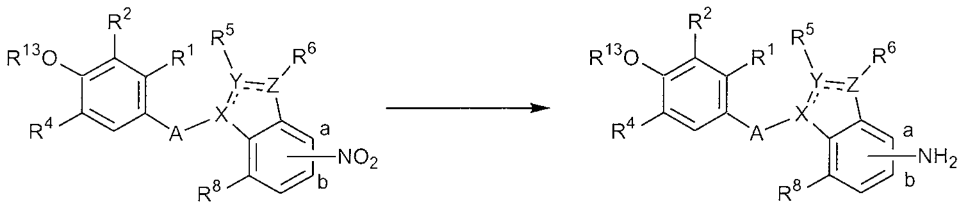

[Table 19]

| Reference Example | $R^1$ | $R^2$ | $R^4$ | $R^5$ | $R^6$ | $R^8$ | $R^{13}$ | A | -X-Y-Z- | $NH_2$ |
|---|---|---|---|---|---|---|---|---|---|---|
| 122 | Me | Me | H | Me | H | Me | Me | $CH_2$ | -N-C=C- | a |
| 123 | H | Me | H | Me | H | Me | TIPS | $CH_2$ | -N-C=C- | a |
| 124 | H | *n*-Pr | H | Me | H | Me | TIPS | $CH_2$ | -N-C=C- | a |
| 125 | H | *i*-Pr | H | H | H | H | TIPS | $CH_2$ | -N-C=C- | a |
| 126 | H | *i*-Pr | H | Me | H | H | TIPS | $CH_2$ | -N-C=C- | a |
| 127 | H | *i*-Pr | H | Et | H | H | TIPS | $CH_2$ | -N-C=C- | a |
| 128 | H | *i*-Pr | H | $CF_3$ | H | H | TIPS | $CH_2$ | -N-C=C- | a |
| 129 | H | *i*-Pr | H | H | H | Me | TIPS | $CH_2$ | -N-C=C- | a |

(continued)

| Reference Example | $R^1$ | $R^2$ | $R^4$ | $R^5$ | $R^6$ | $R^8$ | $R^{13}$ | A | -X-Y-Z- | $NH_2$ |
|---|---|---|---|---|---|---|---|---|---|---|
| 130 | H | *i*-Pr | H | Me | Me | H | TIPS | $CH_2$ | -N-C=C- | a |
| 131 | H | *i*-Pr | H | Me | H | Me | TIPS | $CH_2$ | -N-C=C- | a |
| 132 | H | *i*-Pr | H | Me | H | Me | Me | $CH_2$ | -N-C=C- | a |
| 133 | H | *i*-Pr | H | Et | H | Me | TIPS | $CH_2$ | -N-C=C- | a |
| 134 | H | *i*-Pr | H | $CF_3$ | H | Me | TIPS | $CH_2$ | -N-C=C- | a |
| 135 | H | *s*-Bu | H | Me | H | Me | TIPS | $CH_2$ | -N-C=C- | a |
| 136 | H | Ph | H | Me | H | Me | TIPS | $CH_2$ | -N-C=C- | a |
| 137 | H | 3-Py | H | Me | H | Me | H | $CH_2$ | -N-C=C- | a |
| 138 | H | 4-F-Bz | H | H | H | Me | H | $CH_2$ | -N-C=C- | a |
| 139 | H | 4-F-Bz | H | Me | H | Me | H | $CH_2$ | -N-C=C- | a |
| 140 | H | 4-F-Bn | H | Me | H | Me | TIPS | $CH_2$ | -N-C=C- | a |
| 141 | H | OEt | H | Me | H | Me | TIPS | $CH_2$ | -N-C=C- | a |
| 142 | H | Me | Me | H | H | Me | TIPS | $CH_2$ | -N-C=C- | a |
| 143 | H | *i*-Pr | H | H | H | H | TIPS | $CH_2$ | -N-C=C- | b |
| 144 | H | *i*-Pr | H | Me | H | H | TIPS | $CH_2$ | -N-C=C- | b |
| 145 | H | *i*-Pr | H | H | H | Me | TIPS | $CH_2$ | -N-C=C- | b |
| 146 | H | *i*-Pr | H | Me | H | Me | TIPS | $CH_2$ | -N-CH-CH- | a |
| 147 | H | *i*-Pr | H |  | H | H | TIPS | $CH_2$ | -N-N=C- | a |
| 148 | H | *i*-Pr | H | H | H | H | TIPS | $CH_2$ | -C=C-N- | a |
| 149 | H | *i*-Pr | H | H | H | Me | H | CO | -N-C=C- | a |

[Table 20]

| Reference Example | $^1$HNMR ($CDCl_3$) δ (ppm) |
|---|---|
| 122 | 2.21(3H,s),2.30(6H,s),2.33(3H,s),3.72(3H,s),5.38(2H,s),5.93(1H,d), 6.26(1H,s),6.30(1H,d), 6.47(1H,d),6.61(1H,d). |
| 123 | 1.07(18H,d),1.19-1.28(3H,m),2.16(3H,s),2.31(3H,s),2.44(3H,s),3.75(1H,s), 5.41(2H,s),6.23 (1H,d),6.30(1H,d),6.44(1H,dd),6.62-6.65(2H,m),6.71(1H,d). |
| 124 | 0.89(3H,t),1.07(18H,d),1.24(3H,m),1.48-1.60(2H,m),2.31(3H,s),2.44(3H,s), 2.51(2H,t),5.41 (2H,s),6.22(1H,brs),6.29(1H,d),6.40(1H,dd),6.63(1H,d), 6.64(1H,d),6.73(1H,d). |
| 125 | 1.07(18H,d),1.15(6H,d),1.24(3H,m),3.33(1H,m),5.19(2H,s),6.39(1H,d), 6.41(1H,d),6.64(1H, d),6.70(1H,dd),6.81(1H,d),6.98-7.02(2H,m), 7.05(1H,d). |
| 126 | 1.09(18H,d),1.16(6H,d),1.27(3H,m),3.33(1H,m),3.49(1H,brs),5.16(2H,s), 6.33(1H,d), 6.63-6.68(3H,m),6.94(1H,d),7.01(1H,d),7.04(1H,s),7.12(1H,d). |
| 127 | 1.07(18H,d),1.17(6H,d),1.20-1.31(9H,m),2.68(2H,q),3.33(1H,m),5.19(2H,s), 6.23(1H,s),6.38 (1H,d),6.46(1H,dd),6.59(1H,d),6.74(2H,d),6.93(1H,t), 6.98(1H,d). |
| 128 | 1.06(18H,d),1.12(6H,d),3.30(3H,m),3.32(1H,m),3.99(2H,brs),5.31(2H,s), 6.40(1H,d),6.60 (1H,s),6.70(1H,d),6.91(1H,s),6.99(1H,s),7.06(1H,t), 7.23(1H,d). |
| 129 | 1.08(18H,d),1.15(6H,d),1.26(3H,m),2.48(3H,s),3.33(1H,m),3.81(2H,brs), 5.47(2H,s),6.43 (1H,d),6.47(1H,dd),6.62(1H,d),6.69(1H,d),6.94(1H,d), 6.96(1H,d). |

(continued)

| Reference Example | $^1$HNMR ($CDCl_3$) δ (ppm) |
|---|---|
| 130 | 1.07(18H,d),1.17(6H,d),1.20-1.32(3H,m),2.23(3H,s),2.51(3H,s),3.33(1H,m), 4.05(2H,s),5.13 (2H,s),6.29(1H,dd),6.46(1H,dd),6.59(1H,d),6.70(1H,dd), 6.87(1H,dd),7.01(1H,d). |
| 131 | 1.07(18H,d),1.13(6H,d),1.25(3H,m),2.32(3H,s),2.44(3H,s),3.32(1H,m), 5.42(2H,s),6.22(1H, s),6.29(1H,d),6.59(1H,d),6.62(1H,d),6.89(1H,d). |
| 132 | 1.07(18H,d),1.13(6H,d),1.25(3H,m),2.32(3H,s),2.44(3H,s),3.32(1H,m), 5.42(2H,s),6.22(1H, s),6.29(1H,d),6.59(1H,d),6.62(1H,d),6.89(1H,d). |
| 133 | 1.06(18H,d),1.14(6H,d),1.21-1.32(6H,m),2.44(3H,s),2.64(2H,q),3.32(1H,m), 3.76(2H,s),5.43 (2H,s),6.24-6.31(3H,m),6.58(1H,d),6.63(1H,d),6.87(1H,d). |
| 134 | 1.07(18H,d),1.11(6H,d),3.30(3H,m),2.40(3H,s),3.32(1H,m),3.89(2H,brs), 5.57(2H,s),6.30 (1H,dd),6.35(1H,d),6.58(1H,d),6.57-6.59(2H,m),6.95(1H,s). |
| 135 | 0.80(3H,t),1.12-1.14(21H,m),1.24(3H,m),1.36-1.70(2H,m),2.32(3H,s), 2.44(3H,s),3.04-3.18 (1H,m),3.74(2H,brs),5.42(2H,s),6.22(1H,brs), 6.26-6.34(2H,m),6.59(1H,d),6.63(1H,d),6.82 (1H,d). |
| 136 | 1.05(18H,d),1.18-1.28(3H,m),2.28(3H,s),2.40(3H,s),3.87(2H,s),5.36(2H,s), 6.19(1H,d),6.29 (1H,d),6.45(1H,dd),6.56(1H,d),6.61(1H,d),6.66(1H,d), 6.88-6.93(2H,m),1.00-1.03(2H,m). |
| 137 | 2.34(3H,s),2.47(3H,s),5.49(2H,s),6.24(1H,s),6.30(1H,d),6.62-6.65(2H,m), 6.78(1H,d),6.89 (1H,d),7.35(1H,dd),7.80(1H,dd),8.55(1H,dd),8.71(1H,d). |

[Table 21]

| Reference Example | $^1$HNMR ($CDCl_3$) δ (ppm) |
|---|---|
| 138 | 2.39(3H,s),3.82(2H,brs),5.44(2H,s),6.35(1H,s),6.35(1H,d),6.71-6.85(2H,m), 6.89(1H,d), 6.96-7.04(3H,m),7.24-7.30(4H,m). |
| 139 | 2.27(3H,s),2.41(3H,s),3.86(2H,brs),5.42(2H,s),6.14(1H,s),6.34(1H,d), 6.60-6.65(3H,m), 6.97-7.04(3H,m),7.03-7.21(3H,m),11.86(1H,s) |
| 140 | 1.05(18H,d),1.18-1.28(3H,m),2.28(3H,s),2.40(3H,s),3.87(2H,s),5.36(2H,s), 6.19(1H,d),6.29 (1H,d),6.45(1H,dd),6.56(1H,d),6.61(1H,d),6.66(1H,d), 6.88-6.93(2H,m),7.00-7.03(2H,m). |
| 141 | 1.05(18H,d),1.22(3H,m),1.33(3H,t),2.31(3H,s),2.41(3H,s),3.84(2H,q), 5.41(2H,s),6.18-6.24 (2H,m),6.29(1H,d),6.39(1H,d),6.63(1H,d),6.72(1H,d). |
| 142 | 1.07(18H,d),1.25(3H,m),2.15(6H,s),2.44(3H,s),5.40(2H,s),6.30(1H,d), 6.41(1H,d),6.56(2H, s),6.67(1H,d),6.94(1H,d). |
| 143 | 1.09(18H,d),1.16(6H,d),1.27(3H,m),3.33(1H,m),3.49(1H,brs),5.16(2H,s), 6.33(1H,d), 6.63-6.68(3H,m),6.94(1H,d),7.01(1H,d),7.04(1H,s),7.12(1H,d). |
| 144 | 1.07(18H,d),1.15(6H,d),1.25(3H,m),2.33(3H,s),3.32(1H,m),3.46(1H,brs), 5.15(2H,s),6.12 (1H,s),6.44(1H,dd),6.55-6.60(2H,m),6.87(1H,d),6.98(1H,d), 7.03(1H,d). |
| 145 | 1.07(18H,d),1.14(6H,d),1.25(3H,m),2.46(3H,s),3.32(1H,m),5.41(2H,s), 6.32(1H,d),6.36(1H, s),6.43(1H,dd),6.62(1H,d),6.78(1H,d),6.90(1H,d), 6.95(1H,m). |
| 146 | 1.06(18H,d),1.14-1,17(9H,m),1.28(3H,m),2.22(3H,s),2.28(1H,dd),3.04 (1H,dd),3.30-3.73 (3H,m),3.58-3.64(1H,m),4.28(1H,d),4.42(1H,d),6.11 (1H,d),6.69(1H,t),6.95(1H,dd),7.11(1H, d). |
| 147 | 1.08(18H,d),1.15(6H,d),3.32(3H,m),4.12(2H,brs),5.45(2H,s),6.32(1H,d), 6.65(1H,d), 6.77-6.82(2H,m),7.11-7.15(2H,m),7.96(1H,s). |
| 148 | 1.10(18H,d),1.18(6H,d),1.30(3H,m),3.34(1H,m),4.00(2H,s),6.57(1H,d), 6.66(1H,d),6.79(1H, d),6.88(1H,dd),6.93(1H,t),7.09(1H,d),7.14(1H,d), 7.80(1H,brs). |

(continued)

| Reference Example | $^1$HNMR ($CDCl_3$) δ (ppm) |
|---|---|
| 149 | 1.28(6H,d),2.34(3H,s),3.26(1H,m),6.54(1H,d),6.58(1H,d),6.82(1H,d), 6.97(1H,d),7.18(1H,d), 7.64(1H,dd),7.84(1H,d). |

Reference Example 150

1-(4-hydroxy-3-phenetylbenzyl)-2,7-dimethyl-1H-indol-4-ylam ine

According to the same manner as that of Reference Example 121 using 1-(4-hydroxy-3-styrylbenzyl)-2,7-dimethyl-4-nitro-1H-indole in place of 2,7-dimethyl-4-nitro-1-(3-trifluoromethyl-4-triisopropylsil anyloxybenzyl)-1H-indole, a reaction was performed to obtain the title compound.
$^1$H NMR ($CDCl_3$) δ (ppm): 2.26(3H,s), 2.41(3H,s), 2.85(4H,s), 5.37(2H,s), 6.22(1H,s), 6.31(1H,d), 6.37(1H,dd), 6.52(1H, d), 6.63(1H,m), 6.67(1H,d), 7.12-7.14(2H,m), 7.18(1H,m), 7.23-7.27(2H,m).

Reference Example 151

1-(4-benzyloxy-3-isopropylbenzyl)-1H-indole-4-carboxylic acid

Methyl 1-(4-benzyloxy-3-isopropylbenzyl)-1H-indole-4-carboxylate (300 mg) was dissolved in a mixed solution of 1,4-dioxane (1.0 mL) and methanol(1.0 mL), a 5 mol/l aqueous sodium hydroxide solution (218 μL) was added, and the mixture was stirred at 80°C for 5 hours. The reaction mixture was returned to room temperature, and concentrated under reduced pressure, and the resulting residue was then dissolved in water. The solution was acidified with 1 mol/L hydrochloric acid, followed by extraction with ethyl acetate. The organic layer was washed with water and an aqueous saturated sodium chloride solution, and dried with anhydrous sodium sulfate. After concentrated under reduced pressure, the resulting residue was crystallized with diethyl ether/hexane to obtain the title compound (256 mg). $^1$H NMR (DMSO-$d_6$) δ (ppm): 1.13(6H,d), 3.26(1H,m), 5.07(2H,s), 5.40(2H,s), 6.96(2H,s), 6.98(1H,d), 7.18-7.22(2H,m), 7.31-7.44(5H, m), 7.65(1H,d), 7.72(1H,d), 7.79(1H,d), 12.64(1H,brs).

Reference Example 152

Ethyl N-[2,7-dimethyl-1-(3-trifluoromethyl-4-triisopropylsilanylo xybenzyl)-1H-indol-4-yl]oxamate

2,7-dimethyl-1-(3-trifluoromethyl-4-triisopropylsilan yloxybenzyl)-1H-indol-4-ylamine (304 mg) was dissolved in diethyl oxalate (3.0 mL), and the mixture was stirred at 100°C for 3 hours. The reaction mixture was returned to room temperature, and concentrated under reduced pressure. The resulting residue was purified by flash chromatography (silica gel, developing solvent: n-hexane to n-hexane/ethyl acetate = 4/1) to obtain the title compound (276 mg).
$^1$H NMR ($CDCl_3$) δ (ppm): 1.06(18H,d), 1.28(3H,m), 1.45(3H,s), 2.33(3H,s), 2.49(3H,s), 4.44(2H,q), 5.48(2H,s), 6.35 (1H,s), 6.56(1H,dd), 6.73(1H,d), 6.84(1H,d), 7.22(1H,d), 7.75(1H,d), 9.07(1H,s).

Compounds were synthesized according to the following reaction formula referring to the method of Reference Example 152. Synthesized compounds are shown in Table 22, and data are shown in Table 23 and Table 24.

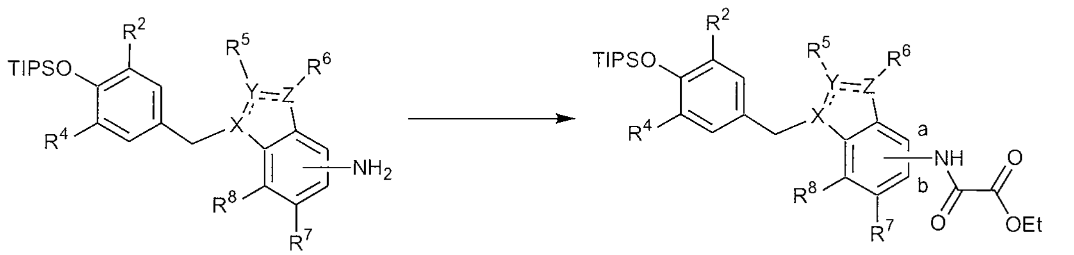

[Table 22]

| Reference Example | $R^2$ | $R^4$ | $R^5$ | $R^6$ | $R^7$ | $R^8$ | -X-Y-Z- | NHCOCOOEt |
|---|---|---|---|---|---|---|---|---|
| 153 | Me | H | Me | H | H | Me | -N-C=C- | a |
| 154 | *n*-Pr | H | Me | H | H | Me | -N-C=C- | a |
| 155 | *i*-Pi | H | H | H | H | H | -N-C=C- | a |
| 156 | *i*-Pr | H | Me | H | H | H | -N-C=C- | a |
| 157 | *i*-Pr | H | Et | H | H | H | -N-C=C- | a |
| 158 | *i*-Pr | H | $CF_3$ | H | H | H | -N-C=C- | a |
| 159 | *i*-pr | H | H | H | Br | H | -N-C=C- | a |
| 160 | *i*-Pr | H | H | H | H | Cl | -N-C=C- | a |
| 161 | *i*-Pr | H | H | H | H | Me | -N-C=C- | a |
| 162 | *i*-Pr | H | Me | Me | H | H | -N-C=C- | a |
| 163 | *i*-Pr | H | Me | H | H | Me | -N-C=C- | a |
| 164 | *s*-Bu | H | Me | H | H | Me | -N-C=C- | a |
| 165 | 4-F-Bn | H | Me | H | H | Me | -N-C=C- | a |
| 166 | OEt | H | Me | H | H | Me | -N-C=C- | a |
| 167 | Me | Me | H | H | H | Me | -N-C=C- | a |
| 168 | *i*-Pr | H | H | H | H | H | -N-C=C- | b |
| 169 | *i*-Pr | H | Me | H | H | H | -N-C=C- | b |
| 170 | *i*-Pr | H | H | H | H | Me | -N-C=C- | b |
| 171 | *i*-Pr | H | Me | H | H | Me | -N-CH-CH- | a |
| 172 | *i*-Pr | H | - | H | H | H | -N-N=C- | a |

[Table 23]

| Reference Example | $^1$HNMR ($CDCl_3$) δ (ppm) |
|---|---|
| 153 | 1.07(18H,d),1.19-1.29(3H,m),1.46(3H,t),2.16(3H,s),2.35(3H,s),2.51(3H,s), 4.45(2H,q),5.44 (2H,s),6.33(1H,s),6.40(1H,dd),6.64-6.68(2H,m),6.83(1H,d), 7.76(1H,d),9.09(1H,s). |
| 154 | 0.88(3H,t),1.06(18H,d),1.25(3H,m),1.45(3H,t),1.49-1.60(2H,m),2.35(3H,s), 2.46-2.54(5H,m), 4.45(2H,q),5.44(2H,s),6.33(1H,brs),6.37(1H,dd),6.63 (1H,d),6.69(1H,d),6.83(1H,d),7.75(1H, d),9.09(1H,brs). |
| 155 | 1.06(18H,d),1.13(6H,d),1.26(3H,m),1.43(3H,t),3.31(1H,m),4.42(2H,q),5.21 (2H,s),6.51(1H, d),6.64(1H,d),6.68(1H,dd),7.01(1H,d),7.11(1H,d),7.17 (1H,d),7.90(1H,m),9.16(1H,brs). |
| 156 | 1.07(18H,d),1.15(6H,d),1.26(3H,m),1.46(3H,t),2.40(3H,s),3.32(1H,m),4.45 (2H,q),5.23(2H, s),6.33(1H,s),6.45(1H,dd),6.61(1H,d),6.96(1H,d),7.13 (2H,m),7.86-7.90(1H,m),9.13(1H,s). |
| 157 | 1.06(18H,d),1.14(6H,d),1.24-1.37(9H,m),2.72(2H,q),3.31(1H,m),4.43 (2H,q),5.24(2H,s),6.37 (1H,s),6.44(1H,dd),6.60(1H,d),6.96(1H,d),7.04-7.12 (2H,m),7.89(1H,d),9.17(1H,s). |
| 158 | 1.07(18H,d),1.12(6H,d),1.25(3H,m),1.46(3H,t),3.31(1H,m),4.46(2H,q),5.38 (2H,s),6.58(1H, dd),6.62(1H,d),6.97(1H,d),7.04(1H,d),7.13(1H,d),7.28 (1H,t),7.91(1H,d),9.17(1H,s). |
| 159 | 1.07(18H,d),1.15(6H,d),1.23-1.31(3H,m),1.44(3H,t),3.33(1H,m),4.43(2H,q), 5.16(2H,s),6.48 (1H,d),6.67(1H,s),7.01(1H,s),7.07(1H,d),7.34(1H,s),8.09 (1H,s),9.11(1H,s). |
| 160 | 1.07(18H,d),1.13(6H,d),1.26(3H,m),1.44(3H,t),3.31(1H,m),4.44(2H,q),5.68 (2H,s),6.52(1H, d),6.66(1H,s),7.00(1H,d),7.09(1H,d),7.14(1H,s),7.85(1H,d), 9.11(1H,brs). |

(continued)

| Reference Example | $^1$HNMR ($CDCl_3$) δ (ppm) |
|---|---|
| 161 | 1.07(18H,d),1.13(6H,d),1.25(3H,m),1.46(3H,t),2.55(3H,s),3.32(1H,m),4.45 (2H,q),5.50(2H, s),6.44(1H,dd),6.53(1H,d),6.64(1H,d),6.89-6.91(2H,m),7.08 (1H,d),7.81(2H,d),9.14(1H,s). |
| 162 | 1.05(18H,d),1.14(6H,d),1.19-1.26(3H,m),1.44(3H,t),2.27(3H,s),2.56(3H,s), 3.30(1H,m),4.41 (2H,q),5.29(2H,s),6.40(1H,dd),6.58(1H,d),6.95(1H,d), 7.07-7.10(2H,m),7.89-7.93(1H,m), 9.76(1H,s). |
| 163 | 1.06(18H,d),1.13(6H,d),1.25(3H,m),1.46(3H,t),2.36(3H,s),2.51(3H,s),3.32 (1H,m),4.45(2H, q),5.45(2H,s),6.25(1H,dd),6.33(1H,s),6.60(1H,d),6.82-6.85 (2H,m),7.76(1H,d),9.10(1H,s). |
| 164 | 0.79(3H,t),1.00-1.15(21H,m),1.25(3H,m),1.34-1.60(5H,m),2.36(3H,s), 2.50(3H,s),3.05-3.20 (1H,m),4.45(2H,q),5.44(2H,s),6.25-6.35(2H,m), 6.61(1H,d),6.76(1H,d),6.82(1H,d),7.76(1H, d),9.09(1H,brs). |
| 165 | 1.05(18H,d),1.19-1.30(3H,m),1.46(3H,t),2.30(3H,s),2.46(3H,s),3.85(2H,s), 4.45(2H,q),5.39 (2H,s),6.28(1H,s),6.41-6.44(2H,m),6.68(1H,d),6.81(1H,d), 6.87-6.91(2H,m),6.96-7.00(2H, m),7.75(1H,d),9.07(1H,s). |
| 166 | 1.05(18H,d),1.22(3H,m),1.33(3H,t),1.46(3H,t),2.35(3H,s),2.49(3H,s), 3.82(2H,q),4.45(2H,q), 5.45(2H,s),6.16(1H,dd),6.33(2H,brs),6.74(1H,d), 6.82(1H,d),7.75(1H,d),9.08(1H,brs). |
| 167 | 1.08(18H,d),1.24(3H,m),1.44(3H,t),2.15(6H,s),2.52(3H,s),4.42(2H,q),5.43(2 H,s),6.52(1H,s), 6.89(1H,d),7.07(1H,d),7.79(1H,d),9.13(1H, brs). |

[Table 24]

| Reference Example | $^1$HNMR ($CDCl_3$) δ (ppm) |
|---|---|
| 168 | 1.08(18H,d),1.15(6H,d),1.27(3H,m),1.43(3H,t),3.32(1H,m),4.41(2H,q), 5.21(2H,s),6.51(1H, d),6.64-6.67(2H,m),7.02(1H,s),7.11(1H,d),7.26-7.33 (3H,m),8.01(1H,d),8.90(1H,brs). |
| 169 | 1.07(18H,d),1.15(6H,d),1.26(3H,m),1.44(3H,t),2.37(3H,s),3.32(1H,m), 4.42(2H,q),5.23(2H, s),6.33(1H,s),6.45(1H,dd),6.61(1H,d),6.96(1H,d), 7.13(2H,m),7.86-7.90(1H,m),9.13(1H,s). |
| 170 | 1.09(18H,d),1.15(6H,d),1.26(3H,m),1.42(3H,t),2.52(3H,s),3.31(1H,m), 4.41(2H,q),5.45(2H, s),6.41(1H,dd),6.51(1H,d),6.61(1H,d),6.86(1H,d), 6.99(1H,s),7.05(1H,d),7.88(1H,s),8.81(1H, brs). |
| 171 | 1.12-1.19(24H,m),1.31(3H,m),1.44(3H,t),2.31(3H,s),2.44(1H,dd),3.18 (1H,dd),3.34(1H,m), 3.61-3.72(1H,m),4.39-4.49(4H,m),6.69(1H,d),6.91-6.93 (2H,m),7.08(1H,d),7.35(1H,d),8.56 (1H,brs). |
| 172 | 1,05(18H,d),1.12(6H,d),1.23(3H,m),1.44(3H,t),3.28(3H,m),4.44(2H,q), 5.49(2H,s),6.63(1H, d),6.77(1H,dd),7.08(1H,d),7.18(1H,d),7.31(1H,t), 7.79(1H,d),8.09(1H,d),9.18(1H,brs). |

Reference Example 173

Ethyl N-[1-(3-bromo-4-triisopropylsilanyloxybenzyl)-2,7-dimethyl-1H-indol-4-yl]malonamate

1-(3-bromo-4-triisopropylsilanyloxybenzyl)-2,7-dimeth yl-1H-indol-4-ylamine (160 mg) was dissolved in dichloromethane (10 mL), triethylamine (111 μL) was added, followed by addition of ethylmalonyl chloride (56 μL) at 0°C, and the mixture was stirred at room temperature for 30 minutes. The resulting mixture was diluted with ethyl acetate, washed with water and an aqueous saturated sodium chloride solution, and dried with anhydrous sodium sulfate, and the solvent was concentrated under reduced pressure. The resulting residue was purified by flash chromatography (silica gel, developing solvent: n-hexane/ethyl acetate = 4/1) to obtain the title compound (108 mg).
$^1$H NMR ($CDCl_3$) δ (ppm): 1.08(18H,d), 1.25(3H,m), 1.36(3H,t), 2.33(3H,s), 2.48(3H,s), 3.55(2H,s), 4.30(2H,q), 5.44(2H, s), 6.39(1H,s), 6.44(1H,dd), 6.73(1H,d), 6.80(1H,d), 7.14(1H,d), 7.69(1H,d), 9.60(1H,s).

Compounds were synthesized according to the following reaction formula referring to the method of Reference Example 173. Synthesized compounds are shown in Table 25, and data are shown in Table 26 and Table 27.

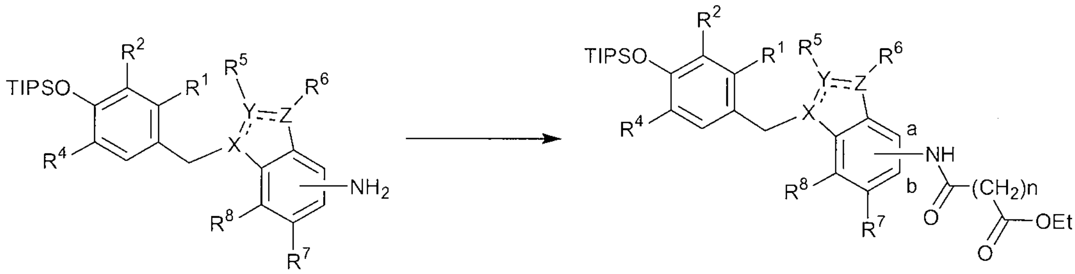

[Table 25]

| Reference Example | $R^1$ | $R^2$ | $R^4$ | $R^5$ | $R^6$ | $R^7$ | $R^8$ | -X-Y-Z- | NHCO | n |
|---|---|---|---|---|---|---|---|---|---|---|
| 174 | -CH=CH-CH=CH- | | H | Me | H | H | Me | -N-C=C- | a | 1 |
| 175 | H | Cl | H | Me | H | H | Me | -N-C=C- | a | 1 |
| 176 | H | Me | H | Me | H | H | Me | -N-C=C- | a | 1 |
| 177 | H | *n*-Pr | H | Me | H | H | Me | -N-C=C- | a | 1 |
| 178 | H | *i*-Pr | H | H | H | H | H | -N-C=C- | a | 1 |
| 179 | H | *i*-Pr | H | Me | H | H | H | -N-C=C- | a | 1 |
| 180 | H | *i*-Pr | H | Et | H | H | H | -N-C=C- | a | 1 |
| 181 | H | *i*-Pr | H | $CF_3$ | H | H | H | -N-C=C- | a | 1 |
| 182 | H | *i*-Pr | H | H | H | Br | H | -N-C=C- | a | 1 |
| 183 | H | *i*-Pr | H | H | H | H | Cl | -N-C=C- | a | 1 |
| 184 | H | *i*-Pr | H | H | H | H | Me | -N-C=C- | a | 1 |
| 185 | H | *i*-Pr | H | Me | Me | H | H | -N-C=C- | a | 1 |
| 186 | H | *i*-Pr | H | Me | H | H | Me | -N-C=C- | a | 1 |
| 187 | H | *i*-Pr | H | Me | H | H | Me | -N-C=C- | a | 2 |
| 188 | H | *i*-Pr | H | Et | H | H | Me | -N-C=C- | a | 1 |
| 189 | H | *i*-Pr | H | $CF_3$ | H | H | Me | -N-C=C- | a | 1 |
| 190 | H | $CF_3$ | H | Me | H | H | Me | -N-C=C- | a | 1 |
| 191 | H | *s*-Bu | H | Me | H | H | Me | -N-C=C- | a | 1 |
| 192 | H | *c*-Pen | H | H | H | H | Me | -N-C=C- | a | 1 |
| 193 | H | *4*-F-Bn | H | Me | H | H | Me | -N-C=C- | a | 1 |
| 194 | H | EtO | H | Me | H | H | Me | -N-C=C- | a | 1 |
| 195 | H | Me | Me | H | H | H | Me | -N-C=C- | a | 1 |
| 196 | H | *i*-Pr | H | H | H | H | H | -N-C=C- | b | 1 |
| 197 | H | *i*-Pr | H | H | H | H | Me | -N-C=C- | b | 1 |
| 198 | H | *i*-Pr | H | Me | H | H | Me | -N-CH-CH- | a | 1 |
| 199 | H | *i*-Pr | H | | H | H | H | -N-N=C- | a | 1 |

[Table 26]

| Reference Example | $^{1}$HNMR ($CDCl_3$) δ (ppm) |
|---|---|
| 174 | 1.10(18H,d),1.34(3H,m),1.36(3H,t),2.34(3H,s),2.35(3H,s),3.57(2H,s), 4.32(2H,q),5.87(2H, brs),6.06(1H,d),6.46(1H,s),6.61(1H,d),6.78(1H,d), 7.56(1H,m),7.63(1H,m),7.69(1H,d),7.98 (1H,d),8.37(1H,m),9.62(1H,brs). |
| 175 | 1.07(18H,d),1.24(3H,m),1.36(3H,t),2.33(3H,s),2.47(3H,s),3.53(2H,s), 4.29(2H,q),5.43(2H,s), 6.39(1H,s),6.44(1H,dd),6.70(1H,d),6.80(1H,d), 7.12(1H,d),7.60(1H,d),9.60(1H,s). |
| 176 | 1.06(18H,d),1.20-1.28(3H,m),1.35(3H,t),2.15(3H,s),2.34(3H,s),2.49(3H,s), 3.54(2H,s),4.30 (2H,q),5.42(2H,s),6.37-6.41(2H,m),6.63(1H,d),6.67(1H,s), 6.79(1H,d),7.67(1H,d),9.57(1H, s). |
| 177 | 0.88(3H,t),1.07(18H,d),1.25(3H,m),1.35(3H,t),1.48-1.60(2H,m),2.35(3H,s), 2.46-2.54(5H,m), 3.55(2H,s),4.31(2H,q),5.43(2H,s),6.34-6.40(2H,m), 6.62(1H,d),6.70(1H,d),6.79(1H,d),7.68 (1H,d),9.57(1H,brs). |
| 178 | 1.09(18H,d),1.16(6H,d),1.26(3H,m),1.35(3H,t),3.33(1H,m),3.55(2H,s), 4.30(2H,q),5.23(2H, s),6.59(1H,d),6.65-6.70(2H,m),7.05(1H,s),7.13-7.19 (2H,m),7.87(1H,d),9.74(1H,s). |
| 179 | 1.07(18H,d),1.15(6H,d),1.25(3H,m),1.35(3H,t),2.39(3H,s),3.31(1H,m), 3.55(2H,s),4.31(2H, q),5.21(2H,s),6.37(1H,s),6.45(1H,d),6.59(1H,d), 6.97(1H,s),7.05-7.11(2H,m),7.82(1H,d), 9.66(1H,s). |
| 180 | 1.08(18H,d),1.14(6H,d),1.24-1.37(9H,m),2.72(2H,q),3.30(1H,m),3.59(2H,s), 4.31(2H,q),5.24 (2H,s),6.37(1H,s),6.44(1H,dd),6.59(1H,d),6.93(1H,d), 7.04-7.12(2H,m),7.81(1H,d),9.61(1H, s). |
| 181 | 1.07(18H,d),1.13(6H,d),1.28(3H,m),1.36(3H,t),3.31(1H,m),3.55(2H,s), 4.31(2H,q),5.37(2H, s),6.58-6.63(2H,m),6.98(1H,s),7.05-7.08(2H,m), 7.21-7.27(1H,m),7.89(1H,d),9.89(1H,brs). |
| 182 | 1.08(18H,d),1.15(6H,d),1.21-1.35(6H,m),3.34(1H,m),3.53(2H,s),4.29(2H,q), 5.16(2H,s),6.55 (1H,d),6.67(1H,s),7.01(1H,s),7.04(1H,d),7.28(1H,s), 8.08(1H,d),9.85(1H,s). |
| 183 | 1.08(18H,d),1.15(6H,d),1.30(3H,m),1.33(3H,t),3.33(1H,m),3.53(2H,s), 4.28(2H,q),5.67(2H, s),6.61(1H,d),6.66(1H,d),7.02(H,s),7.06(1H,d), 7.10(1H,d),7.80(1H,d),9.81(1H,s) |
| 184 | 1.08(18H,d),1.14(6H,d),1.26(3H,m),1.35(3H,t),2.53(3H,s),3.33(1H,m), 3.55(2H,s),4.30(2H, q),5.50(2H,s),6.42(1H,dd),6.59(1H,d),6.62(1H,d), 6.86(1H,d),6.91(1H,d),7.05(1H,d),7.73 (1H,d),9.69(1H,s). |
| 185 | 1.06-1.36(30H,m),2.27(3H,s),2.48(3H,s),3.32(1H,m),3.56(2H,s),4.28(2H,q), 5.19(2H,s),6.41 (1H,dd),6.59(1H,d),6.99-7.10(3H,m),7.52-7.55(1H,m), 9.34(1H,s). |
| 186 | 1.07(18H,d),1.12(6H,d),1.25(3H,m),1.35(3H,t),2.35(3H,s),2.49(3H,s), 3.32(1H,m),3.54(2H, s),4.30(2H,q),5.44(2H,s),6.25(1H,d),6.38(1H,s), 6.59(1H,d),6.79(1H,d),6.86(1H,s),7.68(1H, d),9.57(1H,brs). |
| 187 | 1.07(18H,d),1.14(6H,d),1.22-1.30(6H,m),2.34(3H,s),2.48(3H,s),2.75-2.82 (4H,m),3.32(1H, m),4.20(2H,q),5.43(2H,s),6.24-6.33(2H,m),6.59(1H,d), 6.78(1H,d),6.86(1H,s),7.58(1H,d), 7.75(1H,s). |
| 188 | 1.06(18H,d),1.12(6H,d),1.22-1.37(9H,m),2.48(3H,s),2.66(2H,q),3.31(1H,m), 3.54(2H,s),4.30 (2H,q),5.45(2H,s),6.23(1H,dd),6.37(1H,s),6.57(1H,d), 6.79(1H,d),6.83(1H,d),7.67(1H,d), 9.52(1H,s). |

[Table 27]

| Reference Example | $^{1}$HNMR ($CDCl_3$) δ (ppm) |
|---|---|
| 189 | 1.05(18H,d),1.09(6H,d),1.24(3H,m),1.35(3H,t),2.45(3H,s),3.29(1H,m), 3.55(2H,s),4.31(2H, q),5.59(2H,s),6.24(1H,dd),6.57(1H,d),6.76(1H,d), 6.96(1H,d),7.07(1H,s),7.76(1H,d),9.82 (1H,s). |

(continued)

| Reference Example | $^1$HNMR ($CDCl_3$) δ (ppm) |
|---|---|
| 190 | 1.07(18H,d),1.26(3H,m),1.36(3H,t),2.34(3H,s),2.48(3H,s),4.31(2H,q), 5.49(2H,s),6.41(1H,s), 6.56(1H,dd),6.73(1H,d),6.82(1H,d),7.24(1H,d), 7.70(1H,d),9.61(1H,brs). |
| 191 | 0.79(3H,t),1.04-1.12(21H,m),1.25(3H,m),1.35(3H,t),1.38-1.70(2H,m), 2.35(3H,s),2.49(3H,s), 3.06-3.16(1H,m),3.55(2H,s),4.31(2H,q),5.44(2H,s), 6.28(1H,dd),6.38(1H,s),6.60(1H,d), 6.76-6.82(2H,m),7.68(1H,d), 9.57(1H,brs). |
| 192 | 1.07(18H,d),1.20-1.31(3H,m),1.35(3H,t),1.39-2.04(8H,m),2.52(3H,s), 3.36(1H,m),3.61(2H, s),4.30(2H,q),5.48(2H,s),6.44(1H,dd),6.59(1H,d), 6.63(1H,d),6.84(1H,d),6.88(1H,d),7.04 (1H,d),7.72(1H,d),9.66(1H,brs). |
| 193 | 1.01(18H,d),1.19-1.30(3H,m),1.36(3H,t),2.30(3H,s),2.44(3H,s),3.55(2H,s), 3.85(2H,s),4.31 (2H,q),5.38(2H,s),6.33(1H,s),6.44(2H,d),6.67(1H,d), 6.77(1H,d),6.87-6.91(2H,m),6.96-7.00 (2H,m),7.68(1H,d),9.57(1H,s). |
| 194 | 1.05(18H,d),1.22(3H,m),1.33(3H,t),1.35(3H,t),2.34(3H,s),2.47(3H,s), 3.55(2H,s),3.82(2H,q), 4.31(2H,q),5.44(2H,s),6.18(1H,dd),6.35(1H,d), 6.37(1H,s),6.73(1H,d),6.79(1H,d),7.67(1H, d),9.58(1H,brs). |
| 195 | 1.07(18H,d),1.24(3H,m),2.14(6H,s),2.49(3H,s),3.53(2H,s),4.27(2H,q), 5.42(2H,s),6.52(2H, s),6.57(1H,d),6.83(1H,d),7.02(1H,d),7.69(1H,d), 9.66(1H,brs). |
| 196 | 1.12(18H,d),1.19(6H,d),1.28-1.39(3H,m),1.30(3H,t),3.36(1H,m),3.53(2H,s), 4.30(2H,q),5.25 (2H,s),6.52(1H,d),6.68-6.73(2H,m),7.08(1H,s),7.14(1H,d), 7.26-7.31(2H,m),1.93(1H,s),9.15 (1H,brs). |
| 197 | 1.09(18H,d),1.15(6H,d),1.30(3H,m),1.35(3H,t),2.54(3H,s),3.34(1H,m), 3.49(2H,s),4.28(2H, q),5.48(2H,s),6.42(1H,dd),6.50(1H,d),6.64(1H,d), 6.90(1H,d),6.95(1H,s),7.06(1H,d),7.78 (1H,d),9.05(1H,brs). |
| 198 | 1.07-1.19(27H,m),1.26-1.35(6H,m),2.29(3H,s),2.44(1H,dd),3.21(1H,dd), 3.35(1H,m),3.47 (2H,s),3.60-3.69(1H,m),4.26(2H,q),4.38(2H,dd),6.69(1H,d), 6.87(1H,d),6.93(1H,dd),7.10 (1H,d),7.35(1H,d),9.05(1H,brs). |
| 199 | 1.05(18H,d),1.12(6H,d),1.25(3H,m),1.33(3H,t),3.29(1H,m),3.54(2H,s), 4.29(2H,q),5.49(2H, s),6.62(1H,d),6.77(1H,dd),7.08(1H,d),7.11(1H,d), 7.28(1H,t),7.79(1H,d),8.09(1H,s),9.94(1H, brs). |

Reference Example 200

Monoethyl 2,2-dimethylmalonate

Diethyl 2, 2-dimethylmalonate (1.01 mL) was dissolved in ethanol (4.0 mL), and a solution of potassium hydroxide (318 mg) in ethanol (3.18 mL) was added. The mixture was stirred at room temperature for 3 hours and, further stirred at 5°C for 18 hours. The reaction mixture was concentrated under reduced pressure, dissolved in water, and washed with diethyl ether. To the aqueous layer was added concentrated hydrochloric acid (0.6 mL), and the mixture was stirred at room temperature for 10 minutes. This was extracted with diethyl ether, and dried with anhydrous sodium sulfate. The solvent was concentrated under reduced pressure to obtain the title compound (610 mg). $^1$H NMR (DMSO-$d_6$) δ (ppm): 1.17(3H,t), 1.31(6H,s), 4.12(2H,q). Reference Example 201

Ethyl N-[1-(3-isopropyl-4-triisopropylsilanyloxybenzyl)-7-methyl-1H-indol-4-yl]-2,2-dimethylmalonamate

Monoethyl 2, 2-dimethylmalonate (170 mg) was dissolved in N,N-dimethylformamide (3.0 mL), and 1-ethyl-3-(dimethylaminopropyl)carbodiimide (203 mg) was added. A solution of 1-(3-isopropyl-4-triisopropylsilanyloxybenzyl)-7-methyl-1H-indol-4-ylamine (436 mg) in N,N-dimethylformamide (2.5 mL) was added at 0°C, and the mixture was stirred at room temperature for 3 hours. The reaction mixture was diluted with ethyl acetate, washed with water and an aqueous saturated sodium chloride solution, and dried with anhydrous sodium sulfate, and the solvent was concentrated under reduced pressure. The resulting residue was purified by preparative thin layer chromatography (silica gel, developing solvent: n-hexane/ethyl acetate = 3/1) to obtain the title compound

(105 mg).
$^1$H NMR ($CDCl_3$) δ (ppm): 1.08(18H,d), 1.14(6H,d), 1.26(3H,m), 1.33(3H,t), 1.61(6H,s), 2.52(3H,s), 3.33(1H,m), 4.28 (2H,q), 5.49(2H,s), 6.41(1H,dd), 6.49(1H,d), 6.62(1H,d), 6.86(1H,d), 6.92(1H,d), 7.04(1H,d), 7.69(1H,d), 9.01(1H,s).

Reference Example 202

Ethyl N-[1-(3-isopropyl-4-triisopropylsilanyloxybenzyl)-2,7-dimet hyl-1H-indol-4-yl]-2,2-dimethylmalonamate

According to the same manner as that of Reference Example 201 using 1-(3-isopropyl-4-triisopropylsilanyloxybenzyl)-2,7-dimethyl -1H-indol-4-ylamine in place of 1-(3-isopropyl-4-triisopropylsilanyloxybenzyl)-7-methyl-1H-indol-4-ylamineme, a reaction was performed to obtain the title compound.
$^1$H NMR ($CDCl_3$) δ (ppm): 1.07(18H,d), 1.14(6H,d), 1.24(3H,m), 1.33(3H,t), 1.61(6H,s), 2.35(3H,s), 2.49(3H,s), 3.32(1H, m), 4.28(2H,q), 5.43(2H,s), 6.24(1H,dd), 6.28(1H,s), 6.59(1H,d), 6.79(1H,d), 6.88(1H,d), 7.65(1H,d), 8.91(1H,s).

Reference Example 203

Ethyl [1-(3-isopropyl-4-triisopropylsilanyloxybenzyl)-2-methyl-1H -indol-4-ylamino]acetate

1-(3-isopropyl-4-triisopropylsilanyloxybenzyl)-2-meth yl-1H-indol-4-ylamine (183 mg) was dissolved in N, N-dimethylformamide (2.0 mL), potassium carbonate (84 mg) and ethyl bromoacetate (84 μL) were added, and the mixture was stirred at 60°C for 1 hour. The reaction mixture was filtered, and the filtrate was concentrated under reduced pressure. The resulting residue was purified by flash chromatography (silica gel, developing solvent: n-hexane to n-hexane/ethyl acetate = 4/1) to obtain the title compound (190 mg).
$^1$H NMR ($CDCl_3$) δ (ppm): 1.07(18H,d), 1.16(6H,d), 1.26(3H,m), 1.31(3H,t), 2.35(3H,s), 3.32(1H,m), 4.06(2H,s), 4.27 (2H,q), 5.18(2H,s), 6.18(1H,d), 6.29(1H,s), 6.47(1H,dd), 6.59(1H,d), 6.76(1H,d), 6.97-7.00(2H,m).

Reference Example 204

Ethyl 3-[1-(3-isopropyl-4-triisopropylsilanyloxybenzyl)-7-methyl-1H-indol-4-ylamino]propionate

According to the same manner as that of Reference Example 203 using 1-(3-isopropyl-4-triisopropylsilanyloxybenzyl)-7-methyl-1H-indol-4-ylamine in place of 1-(3-isopropyl-4-triisopropylsilanyloxybenzyl)-2-methyl-1H-indol-4-ylamine, and using ethyl bromopropionate in place of ethyl bromoacetate, a reaction was performed to obtain the title compound.
$^1$H NMR ($CDCl_3$) δ (ppm): 1.08(18H,d), 1.15(6H,d), 1.24-1.28(6H,m), 2.48(3H,s), 2.70(2H,t), 3.33(1H,m), 3.59(2H,t), 4.16(2H,q), 5.46(2H,s), 6.21(1H,d), 6.40(1H,d), 6.47(1H,dd), 6.62(1H,d), 6.75(1H,d), 6.93-6.95(2H,m).

Reference Example 205

Ethyl N-[3-(3-isopropyl-4-triisopropylsilanyloxybenzyl)-1H-indol-7-yl]malonamate

3-(3-isopropyl-4-triisopropylsilanyloxybenzyl)-1H-ind ol-7-ylamine (55 mg) was dissolved in diethyl malonate (0.5 mL), and the mixture was stirred at 140°C for 5 hours. The reaction mixture was concentrated under reduced pressure, and the resulting residue was purified by flash chromatography (silica gel, developing solvent: n-hexane to n-hexane/ethyl acetate = 4/1) to obtain the title compound (56 mg).
$^1$H NMR ($CDCl_3$) δ (ppm): 1.11(18H,d), 1.18(6H,d), 1.29(3H,m), 1.35(3H,t), 3.35(1H,m), 3.54(2H,s), 4.03(2H,s), 4.29 (2H,q), 6.67(1H,d), 6.85-6.89(3H,m), 7.00(1H,t), 7.15(1H,d) 7.40(1H,d), 9.51(1H,brs).

Reference Example 206

Ethyl (2-methyl-1H-indol-4-yloxy)acetate

4-hydroxy-2-methyl-1H-indole (1.0 g) was dissolved in acetone (50.0 mL), potassium carbonate (1.4 g), sodium iodide (101 mg), and ethyl bromoacetate (829 μL) were added, and the mixture was stirred at room temperature for 16 hours. The reaction mixture was filtered, and the filtrate was concentrated under reduced pressure. To the resulting residue was added water, followed by extraction with ethyl acetate. The organic layer was washed with water and an aqueous saturated sodium chloride solution, and then dried with anhydrous sodium sulfate, and the solvent was concentrated under reduced pressure. The resulting residue was crystallized with n-hexane/diethyl ether to obtain the title compound (1.6 g). $^1$H NMR ($CDCl_3$) δ (ppm): 1.28(3H,t), 2.41(3H,s), 4.26(2H,q), 4.74(2H,s), 6.37-6.40(2H,m), 6.93-7.00 (2H,m).

Reference Example 207

Ethyl [1-(4-benzyloxy-3-isopropylbenzyl)-2-methyl-1H-indol-4-ylox y]acetate

(4-benzyloxy-3-isopropylphenyl)methanol (1000 mg) was dissolved in dichloromethane (10.0 mL), thionyl chloride (427 μL) was added dropwise, and the mixture was stirred at room temperature for 2 hours. The reaction mixture was concentrated under reduced pressure to obtain 1-benzyloxy-4-chloromethyl-2-isopropylbenzene.

60% sodium hydride (156 mg) was suspended in N,N-dimethylformamide (5.0 mL), and a solution of ethyl (2-methyl-1H-indol-4-yloxy)acetate (758 mg) in N,N-dimethylformamide (1.0 mL) was added dropwise at 5°C. After stirred at 5°C for 1 hour, a solution of the previously obtained 1-benzyloxy-4-chloromethyl-2-isopropylbenzene in N,N-dimethylformamide (5.0 mL) was then added dropwise at 5°C, and the mixture was stirred for 3 hours. The reaction mixture was poured into ice water, followed by extraction with ethyl acetate. The organic layer was washed with water and an aqueous saturated sodium chloride solution, and then dried with anhydrous sodium sulfate, and the solvent was concentrated under reduced pressure. The resulting residue was purified by flash chromatography (silica gel, developing solvent: n-hexane to n-hexane/ethyl acetate = 4/1) to obtain the title compound (482 mg).
$^1$H NMR ($CDCl_3$) δ (ppm): 1.17(6H,d), 1.30(3H,t), 2.36(3H,s), 3.33(1H,m), 4.27(2H,q), 4.76(2H,s), 5.00(2H,s), 5.21(2H, s), 6.40(1H,d), 6.47(1H,s), 6.56(1H,dd), 6.72(1H,d), 6.89(1H,d), 6.98(1H,d), 7.00(1H,d), 7.27-7.41(6H,m).

Reference Example 208

Ethyl {[1-(3-isopropyl-4-benzyloxybenzyl)-1H-indole-4-carbonyl]am inoacetate

1-(3-isopropyl-4-benzyloxybenzyl)-1H-indole-4-carboxy lic acid (244 mg) was dissolved in N,N-dimethylformamide (1.0 mL), followed by addition of 1-hydroxybenzotriazole (122 mg), glycineethyl hydrochloride (111 mg), triethylamine (128 μL), and N-(3-dimethylaminopropyl)-N'-ethylcarbodiimide hydrochloride (152 mg), and the mixture was stirred at room temperature for 5 hours. The reaction mixture was poured into ice water, followed by extraction with ethyl acetate. The organic layer was washed with an aqueous saturated sodium bicarbonate solution, 1 mol/L hydrochloric acid, water, and an aqueous saturated sodium chloride solution, and dried with anhydrous sodium sulfate. After concentrated under reduced pressure, the resulting residue was crystallized with n-hexane/diethyl ether to obtain the title compound (277 mg). $^1$H NMR ($CDCl_3$) δ (ppm): 1.19(6H,d), 1.33(3H,t), 3.37(1H,m), 4.29(2H,q), 4.34(2H,d), 5.04(2H,s), 5.30(2H,s), 6.80-6.83(3H,m), 7.01(1H,d), 7.09(1H,s), 7.20-7.26(2H,m), 7.30-7.42(5H,m), 7.47(1H,d), 7.58(1H,d).

Reference Example 209

N-[1-(3-isopropyl-4-triisopropylsilanyloxybenzyl)-7-methyl-1H-indol-4-yl]-2-(1H-tetrazol-5-yl)acetamide

1-(3-isopropyl-4-triisopropylsilanyloxybenzyl)-7-meth yl-1H-indol-4-ylamine (150 mg) was dissolved in tetrahydrofuran (2.0 mL), 1H-tetrazole-5-acetic acid (51.2 mg) and 1-ethyl-3-(dimethylaminopropyl)carbodiimide (95.7 mg) were added, and the mixture was stirred at room temperature for 16 hours. The reaction mixture was diluted with ethyl acetate, washed with an aqueous saturated sodium bicarbonate solution, water and an aqueous saturated sodium chloride solution, and dried with anhydrous sodium sulfate, and the solvent was concentrated under reduced pressure. The resulting residue was purified by preparative thin layer chromatography (silica gel, developing solvent: ethyl acetate) to obtain the title compound (47.3 mg).
$^1$H NMR (DMSO-$d_6$) δ (ppm): 1.05(18H,t), 1.08(6H,d), 1.24-1.32(6H,m), 2.44(3H,s), 3.16(1H,m), 4.27(2H,s), 5.55(2H, s), 6.48(1H,dd), 6.68(1H,d), 6.66(1H,s), 6.74(1H,d), 6.79(1H,d), 6.92(1H,d), 7.37(1H,d), 7.45(1H,d), 10.02(1H,s).

Compounds were synthesized according to the following reaction formula referring to the method of Reference Example 209. Synthesized compounds and data are shown in Table 28.

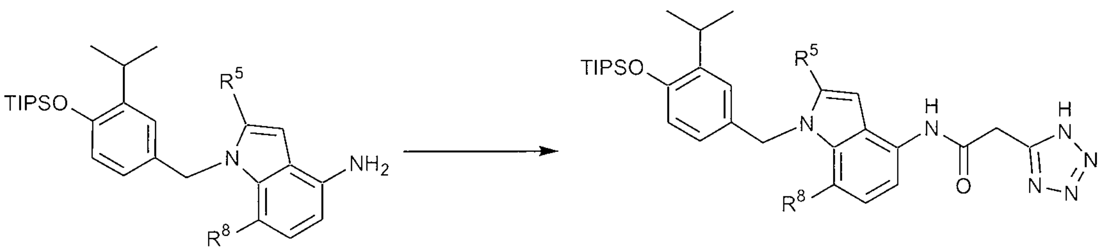

[Table 28]

| Reference Example | $R^5$ | $R^8$ | $^1$HNMR (DMSO-$d_6$) δ (ppm) |
|---|---|---|---|
| 210 | Et | H | 1.04(18H,t),1.09(6H,d)1.24-1.32(6H,m),2.73(2H,q),3.16(1H,m), 4.27(2H,s), 5.31(2H,s),6.57(1H,dd),6.61(1H,s),6.66(1H,d), 6.97-7.01(2H,m),7.18(1H,d), 7.58(1H,d),10.04(1H,s). |
| 211 | Me | Me | 1.04(18H,t),1.08(6H,d)1.24-1.32(6H,m),2.34(3H,s),2.43(3H,s), 3.16(1H,m), 4.22(2H,s),5.48(2H,s),6.34(1H,dd),6.58(1H,s), 6.66(1H,s),6.68(1H,s)6.89(1H, s),7.41(1H,d),10.02(1H,s). |

Reference Example 212

Ethyl 1-[1-(4-benzyloxy-3-isoporpylbenzyl-1H-indol-4-yl)acetate

1-(4-benzyloxy-3-isopropylbenzyl)-1H-indole-4-caboxyl ic acid (244 mg) was dissolved in dichloromethane (1.0 mL), oxalyl dichloride (79.4 μL) was added, and the mixture was stirred at room temperature for 2 hours. The reaction mixture was concentrated under reduced pressure to obtain 1-(4-benzyloxy-3-isopropylbenzyl)-1H-indole-4-carboxylic acid chloride.

A 0.6 mol/L solution of trimethylsilyldiazomethane in hexane (1.4 mL), and triethylamine (113 μL) were dissolved in a mixed solution of tetrahydrofuran (1.0 mL) and acetonitrile (1.0 mL). 1-(4-benzyloxy-3-isopropylbenzyl)-1H-indole-4-carboxylic acid chloride was dissolved in a mixed solution of tetrahydrofuran (1.0 mL) and acetonitrile (1.0 mL), which was added thereto, and the mixture was stirred at 0°C for 16 hours. The reaction mixture was concentrated under reduced pressure, the resulting residue was dissolved in ethanol (2.0 mL), 2,4,6-trimethylpyridine (1.0 mL) was added, and this was refluxed for 5 hours. The reaction mixture was returned to room temperature, and concentrated under reduced pressure. The resulting residue was dissolved in ethyl acetate, washed with water and an aqueous saturated sodium chloride solution, and then dried with anhydrous sodium sulfate. After concentrated under reduced pressure, the resulting residue was purified by flash chromatography (silica gel, developing solvent: n-hexane to n-hexane/ethyl acetate = 4/1) to obtain the title compound (76.3 mg).
$^1$H NMR ($CDCl_3$) δ (ppm): 1.19(6H,d), 1.33(3H,t), 3.37(1H,m), 3.51(2H,s), 4.29(2H,q), 5.04(2H,s), 5.30(2H,s), 6.40-6.43 (2H,m), 6.56(1H,dd), 6.76(1H,d), 6.85(1H,d), 6.96(1H,d), 7.00(1H,d), 7.28-7.45(6H,m).

Example 1

Ethyl N-[1-(4-hydroxy-3-isopropylbenzyl)-1H-indol-4-yl]oxamate Ethyl

N-[1-(3-isopropyl-4-triisopropylsilanyloxybenzyl)-1H-indol-4-yl] oxamate (112 mg) was dissolved in tetrahydrofuran (1.0mL), a 1 mol/L tetrabutylammonium fluoride in tetrahydrofuran (230μ L) was added, and the mixture was stirred at room temperature for 30 minutes. The reaction mixture was diluted with ethyl acetate, washed with water and an aqueous saturated sodium chloride solution, and dried with anhydrous sodium sulfate, and the solvent was concentrated under reduced pressure. The resulting residue was purified by flash chromatography (silica gel, developing solvent: n-hexane to n-hexane/ethyl acetate = 4/1) to obtain the title compound (33.1 mg).
ESI/MS (m/z) :381 $(M+H)^+$, 379$(M-H)^-$.
$^1$H NMR ($CDCl_3$) δ (ppm): 1.20(6H,d), 1.45(3H,t), 3.17(1H,m), 4.45(2H,q), 5.24(2H,s), 6.54(1H,d), 6.65(1H,d), 6.74(1H, dd), 7.04(1H,d), 7.13(1H,d), 7.19-7.20(2H,m), 7.92(1H,m), 9.18(1H,brs).

Compounds were synthesized according to the following reaction formula referring to the method of Example 1. Synthesized compounds are shown in Table 29, and data are shown in Table 30 to Table 33.

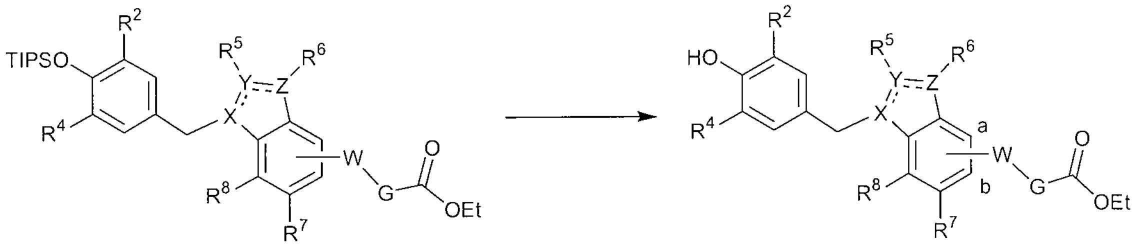

[Table 29]

| Example | $R^2$ | R4 | R5 | $R^6$ | $R^7$ | $R^8$ | -X-Y-Z- | W | | G |
|---|---|---|---|---|---|---|---|---|---|---|
| 2 | Br | H | Me | H | H | Me | -N-C=C- | a | -NHCO- | $CH_2$ |
| 3 | Cl | H | Me | H | H | Me | -N-C=C- | a | -NHCO- | $CH_2$ |
| 4 | Me | H | Me | H | H | Me | -N-C=C- | a | -NHCO- | bond |
| 5 | Me | H | Me | H | H | Me | -N-C=C- | a | -NHCO- | $CH_2$ |
| 6 | *n*-Pr | H | Me | H | H | Me | -N-C=C- | a | -NHCO- | bond |
| 7 | *i*-Pr | H | H | H | H | H | -N-C=C- | a | -NHCO- | $CH_2$ |
| 8 | *i*-Pr | H | Me | H | H | H | -N-C=C- | a | -NHCO- | bond |
| 9 | *i*-Pr | H | Me | H | H | H | -N-C=C- | a | -NHCO- | $CH_2$ |
| 10 | *i*-Pr | H | Me | H | H | H | -N-C=C- | a | -NH- | $CH_2$ |
| 11 | *i*-Pr | H | Et | H | H | H | -N-C=C- | a | -NHCO- | $CH_2$ |
| 12 | *i*-Pr | H | $CF_3$ | H | H | H | -N-C=C- | a | -NHCO- | $CH_2$ |
| 13 | *i*-Pr | H | H | H | Br | H | -N-C=C- | a | -NHCO- | $CH_2$ |
| 14 | *i*-Pr | H | H | H | H | Cl | -N-C=C- | a | -NHCO- | $CH_2$ |
| 15 | *i*-Pr | H | H | H | H | Me | -N-C=C- | a | -NHCO- | bond |
| 16 | *i*-Pr | H | H | H | H | Me | -N-C=C- | a | -NHCO- | $CH_2$ |
| 17 | *i*-Pr | H | H | H | H | Me | -N-C=C- | a | -NHCO- | $C(Me)_2$ |
| 18 | *i*-Pr | H | H | H | H | Me | -N-C=C- | a | -NH- | $CH_2CH_2$ |
| 19 | *i*-Pr | H | Me | Me | H | H | -N-C=C- | a | -NHCO- | bond |
| 20 | *i*-Pr | H | Me | Me | H | H | -N-C=C- | a | -NHCO- | $CH_2$ |
| 21 | *i*-Pr | H | Me | H | H | Me | -N-C=C- | a | -NHCO- | bond |
| 22 | *i*-Pr | H | Me | H | H | Me | -N-C=C- | a | -NHCO- | $CH_2$ |
| 23 | *i*-Pr | H | Me | H | H | Me | -N-C=C- | a | -NHCO- | $C(Me)_2$ |
| 24 | *i*-Pr | H | Me | H | H | Me | -N-C=C- | a | -NHCO- | $CH_2CH_2$ |
| 25 | *i*-Pr | H | Et | H | H | Me | -N-C=C- | a | -NHCO- | $CH_2$ |
| 26 | *i*-Pr | H | $CF_3$ | H | H | Me | -N-C=C- | a | -NHCO- | $CH_2$ |
| 27 | $CF_3$ | H | Me | H | H | Me | -N-C=C- | a | -NHCO- | $CH_2$ |
| 28 | Ph | H | Me | H | H | Me | -N-C=C- | a | -NHCO- | $CH_2$ |
| 29 | 4-F-Bn | H | Me | H | H | Me | -N-C=C- | a | -NHCO- | $CH_2$ |
| 30 | Me | Me | H | H | H | Me | -N-C=C- | a | -NHCO- | bond |
| 31 | Me | Me | H | H | H | Me | -N-C=C- | a | -NHCO- | $CH_2$ |

(continued)

| Example | $R^2$ | R4 | R5 | $R^6$ | $R^7$ | $R^8$ | -X-Y-Z- | W | | G |
|---|---|---|---|---|---|---|---|---|---|---|
| 31 | *i*-Pr | H | H | H | H | H | -N-C=C- | b | -NHCO- | bond |
| 33 | *i*-Pr | H | H | H | H | H | -N-C=C- | b | -NHCO- | $CH_2$ |
| 34 | *i*-Pr | H | Me | H | H | H | -N-C=C- | b | -NHCO- | bond |
| 35 | *i*-Pr | H | H | H | H | Me | -N-C=C- | b | -NHCO- | bond |
| 36 | *i*-Pr | H | H | H | H | Me | -N-C=C- | b | -NHCO- | $CH_2$ |
| 37 | *i*-Pr | H | Me | H | H | Me | -N-CH-CH- | a | -NHCO- | $CH_2$ |
| 38 | *i*-Pr | H | - | H | H | H | -N-N=C- | a | -NHCO- | bond |
| 39 | *i*-Pr | H | - | H | H | H | -N-N=C- | a | -NHCO- | $CH_2$ |
| 40 | *i*-Pr | H | H | H | H | H | -C=C-N- | a | -NHCO- | $CH_2$ |

[Table 30]

| Example | Data |
|---|---|
| 2 | ESI/MS(m/z):459(M+H)+,457(M-H)-. $^1$HNMR($CDCl_3$)δ(ppm):1.36(3H,t),2.34(3H,s),2.49(3H,s),3.55(2H, s),4.31 (2H,q),5.45(2H,s),5.51(1H,s),6.41(1H,s),6.64(1H,dd),6.81(1H,d),6.90(1H,d), 7.69(1H,d),9.62 (1H,s). |
| 3 | ESI/MS(m/z):415(M+H)+,413(M-H)-. $^1$HNMR($CDCl_3$)δ(ppm):1.36(3H,t),2.33(3H,s),2.49(3H,s),3.54(2H, s),4.30 (2H,q),5.45(2H,s),5.51(1H,s),6.41(1H,s),6.60(1H,dd),6.80(1H,d),6.85(1H,d), 7.63(1H,d),9.61 (1H,s). |
| 4 | ESI/MS(m/z):459(M+H)$^+$,457(M-H)$^-$. $^1$HNMR($CDCl_3$) δ(ppm):1.36(3H,t),2.34(3H,s),2.49(3H,s),3.55(2H, s),4.31 (2H,q),5.45(2H,s),5.51(1H,s),6.41(1H,s),6.64(1H,dd),6.81(1H,d),6.90(1H,d), 7.69(1H,d),9.62 (1H,s). |
| 5 | ESI/MS(m/z):459(M+H)$^+$,457(M-H)$^-$. $^1$HNMR($CDCl_3$) δ(ppm):1.36(3H,t),2.34(3H,s),2.49(3H,s),3.55(2H, s),4.31 (2H,q),5.45(2H,s),5.51(1H,s),6.41(1H,s),6.64(1H,dd),6.81(1H,d);6.90(1H,d), 7.69(1H,d),9.62 (1H,s). |
| 6 | ESI/MS(m/z):409(M+H)+,407(M-H)-. $^1$HNMR($CDCl_3$) δ(ppm):0.90(3H,t),1.45(3H,t),1.56(2H,m),2.34 (3H,s), 2.46-2.56(5H,m),4.44(2H,q),4.81(1H,brs),5.45(2H,s),6.34(1H,brs),6.42 (1H,dd),6.63(1H,d),6.66 (1H,d),6.83(1H,d),7.75(1H,d),9.09(1H,brs). |
| 7 | ESI/MS(m/z):395(M+H)+,393(M-H)-. $^1$HNMR($CDCl_3$) δ(ppm):1.19(6H,d),1.46(3H,t),2.55(3H,s),3.17 (1H,m), 3.55(2H,s),4.30(2H,q),4.92(1H,s),5.23(2H,s),6.60(1H,d),6.65(1H,d),6.74 (1H,dd),7.04(1H,d), 7.09(1H,d),7.11-7.19(2H,m),7.86(1H,d),9.75(1H,s). |
| 8 | ESI/MS(m/z):395(M+H)+,393(M-H)-. $^1$HNMR($CDCl_3$) δ(ppm):1.19(6H,d),1.46(3H,t),2.40(3H,s),3.15 (1H,m), 4.45(2H,q),4.66(1H,s),5.24(2H,s),6.34(1H,s),6.50(1H,dd),6.59(1H,d),6.95 (1H,d),7.09-7.15(2H, m),7.87(1H,dd),9.12(1H,s). |
| 9 | ESI/MS(m/z):409(M+H)+,407(M-H)-. $^1$HNMR($CDCl_3$) δ(ppm):1.20(6H,d),1.35(3H,t),2.39(3H,s),3.16 (1H,m), 3.55(2H,s),4.30(2H,q),4.83(1H,s),5.22(2H,s),6.37(1H,s),6.49(1H,dd),6.58 (1H,d),6.96(1H,d), 7.02-7.11(2H,m),7.81(1H,d),9.68(1H,s). |
| 10 | ESI/MS(m/z):381(M+H)+,379(M-H)-. $^1$HNMR($CDCl_3$) δ(ppm):1.19(6H,d),1.30(3H,t),2.34(3H,s),3.15 (1H,m), 4.05(2H,s),4.26(2H,q),4.51(brs),4.89(brs),5.17(2H,s),6.17(1H,d),6.28(1H,s), 6.49(1H,dd),6.53 (1H,d),6.72(1H,d),6.95-6.99(2H,m). |
| 11 | ESI/MS(m/z):423(M+H)+,421(M-H)-. $^1$HNMR(DMSO-$d_6$) δ (ppm):1.11(6H,t),1.23-1.30(6H,m),2.74(2H, q), 3.16(1H,m),3.60(2H,s),4.17(2H,q),5.27(2H,s),6.52(1H,dd),6.56(1H,s), 6.65(1H,d),6.92-7.01(2H,m), 7.16(1H,d),7.59(1H,d),9.13(1H,s),9.74(1H,s). |

(continued)

| Example | Data |
|---|---|
| 12 | ESI/MS(m/z):463(M+H)+,461(M-H)-. $^1$HNMR($CDCl_3$) δ (ppm):1.06(6H,d),1.21(3H,t),3.11(1H,m),3.60 (2H,s), 4.14(2H,q),5.41(2H,s),6.52(1H,dd),6.63(1H,d),6.93(1H,s),7.22-7.27(2H,m), 7.46(1H,s),7.75(1H, dd),9.26(1H,s),10.04(1H,brs). |

[Table 31]

| Example | Data |
|---|---|
| 13 | ESI/MS(m/z):474(M+H)$^+$,472(M-H)$^-$. $^1$HNMR(DMSO-$d_6$) δ(ppm):1.12(6H,d),1.22(3H,t),3.15(1H,m),3.62 (2H,s), 4.14(2H,q),5.26(2H,s),6.69(1H,d),6.77-6.80(2H,m),7.12(1H,s),7.45(1H,d), 7.52(1H,s),7.90(1H, d),9.29(1H,s),9.98(1H,s). |
| 14 | ESI/MS(m/z):429(M+H)$^+$,427(M-H)$^-$. $^1$HNMR($CDCl_3$) δ(ppm):1.08(18H,d),1.15(6H,d),1.24-1.32(3H,m), 1.33(3H,t), 3.33(1H,m),3.53(2H,s),4.28(2H,q),5.67(2H,s),6.61(1H,d),6.66(1H,d),7.02 (1H,s),7.06(1H,d), 7.10(1H,d),7.80(1H,d),9.81(1H,s). |
| 15 | ESI/MS(m/z):395(M+H)$^+$,393(M-H)$^-$. $^1$HNMR($CDCl_3$) δ(ppm):1.19(6H,d),1.46(3H,t),2.55(3H,s),3.16(1H, m),4.45 (2H,q),4.69(1H,s),5.51(2H,s),6.48(1H,dd),6.54(1H,d),6.62(1H,d),6.88-6.91 (2H,m),7.08(1H,d), 7.16(1H,d),7.81(1H,d),9.14(1H,s). |
| 16 | ESI/MS(m/z):409(M+H)$^+$,407(M-H)$^-$. $^1$HNMR($CDCl_3$) δ(ppm):1.19(6H,d),1.34(3H,t),2.52(3H,s),3.16(1H, m),3.55 (2H,s),4.30(2H,q),4.93(1H,s),5.49(2H,s),6.46(1H,d),6.59-6.61(2H,m),6.85 (1H,d),6.90(1H,s), 7.04(1H,d),7.72(1H,d),9.70(1H,s). |
| 17 | ESI/MS(m/z):437(M+H)+,435(M-H)- . $^1$HNMR($CDCl_3$) δ(ppm):1.17(6H,d),1.30(3H,t),1.60(6H,s),2.50 (3H,s),3.19 (1H,m),4.26(2H,q),5.44(2H,s),5.98(1H,s),6.38(1H,dd),6.4(1H,d),6.57(1H,d), 6.81(1H,d), 6.89(1H,d),7.00(1H,d),7.63(1H,d),9.05(1H,s). |
| 18 | EST/MS(m/z):395(M+H)+,393(M-H)-. $^1$HNMR($CDCl_3$) δ(ppm):1.09(6H,d),1.26(3H,d),2.47(3H,s),2.70 (2H,t),3.16 (1H,m),3.59(2H,t),4.16(2H,q),4.86(1H,brs),5.46(2H,s),6.22(1H,d), 6.41(1H,d),6.49(1H,dd), 6.57(1H,d),6.57(1H,d),6.91-6.94(1H,m). |
| 19 | ESI/MS(m/z):409(M+H)+,407(M-H)-. $^1$HNMR($CDCl_3$) δ(ppm):1.19(6H,d),1.45(3H,t),2.27(3H,s),2.56 (3H,s),3.14 (1H,m),4.44(2H,q),4.63(1H,s),5.19(2H,s),6.45(1H,dd),6.57(1H,d),6.94(1H,d) 7.05-7.10(2H, m),7.92(1H,dd),9.76(1H,s). |
| 20 | ESI/MS(m/z):423(M+H)+,421(M-H)- . $^1$HNMR($CDCl_3$) δ(ppm):1.21(6H,d),1.34(3H,t),2.27(3H,s),2.48 (3H,s),3.16 (1H,m),3.56(2H,s),4.28(2H,q),5.20(2H,s),6.46(1H,dd),6.57(1H,d),6.97(1H,d), 7.05-7.06(2H, m),7.53(1H,dd),9.35(1H,s). |
| 21 | ESI/MS(m/z):409(M+H)+,407(M-H)- . $^1$HNMR($CDCl_3$) δ(ppm):1.18(6H,d),1.46(3H,t),2.35(3H,s),2.51 (3H,s),3.15 (1H,m),4.45(2H,q),4.68(1H,s),5.46(2H,s),6.30(1H,dd),6.34(1H,s),6.58(1H,d), 6.82-6.84(2H, m),7.75(1H,d),9.10(1H,s). |
| 22 | ESI/MS(m/z):423(M+H)+,421(M-H)- . $^1$HNMR($CDCl_3$) δ(ppm):1.18(6H,d),1.34(3H,t),2.33(3H,s),2.49 (3H,s),3.16 (1H,m),4.30(2H,q),5.14(1H,brs),5.44(2H,s),6.28(1H,dd),6.37(1H,d), 6.56(1H,d),6.78(1H,d), 6.84(1H,d),7.67(1H,d),9.60(1H,brs). |
| 23 | ESI/MS(m/z):451(M+H)+,449(M-H)- . $^1$H NMR(DMSO-$d_6$) δ(ppm):1.09(6H,t),1.24(3H,t),1.51(6H,s),2.32 (3H,s), 2.47(3H,s),3.16(1H,m),4.18(2H,q),5.43(2H,s),6.23(1H,dd),6.28(1H,d),6.63 (1H,d),6.69(1H,d), 6.85(1H,d),6.98(1H,d),9.12(1H,s),9.17(1H,s). |

[Table 32]

| Example | Data |
|---|---|
| 24 | ESI/MS(m/z):437(M+H)$^+$,435(M-H)$^-$. $^1$HNMR($CDCl_3$) δ(ppm):1.18(6H,d),1.27(3H,t),2.33(3H,s),2.48(3H, s),2.73-2.81(4H,m),3.16(1H,m),4.19(2H,q),5.44(2H,s),6.27-6.29(2H,m),6.56(1H,d), 6.77(1H,d),6.85 (1H,s),7.57(1H,d),7.77(1H,s). |

(continued)

| Example | Data |
|---|---|
| 25 | ESI/MS(m/z):437(M+H)$^+$,435(M-H)$^-$. 1.16(6H,d),1.30-1.36(6H,m),2.47(3H,s),2.64(2H,q),3.13(1H,m),3.54(2H,s), 4.30(2H,q),5.45(2H,s),6.27(1H,dd),6.36(1H,s),6.54(1H,d),6.78(1H,d),6.81 (1H,d),7.66(1H,d),9.53(1H,s). |
| 26 | ESI/MS(m/z):477(M+H)$^+$,475(M-H). $^1$HNMR($CDCl_3$) δ(ppm):1.15(6H,d),1.35(3H,t),2.46(3H,s),3.13(1H,m),3.55 (2H,s),4.31(2H,q),4.82(1H,brs),5.60(2H,s),6.27(1H,dd),6.55(1H,d),6.76 (1H,d),6.96(1H,d),7.08 (1H,s),7.75(1H,d),9.85(1H,s). |
| 27 | ESI/MS(m/z):425(M+H)$^+$,423(M-H)$^-$. $^1$HNMR(DMSO-$d_6$) δ(ppm):1.22(3H,t),2.32(3H,s),2.41(3H,s),3.57 (2H,s), 4.13(2H,q),5.51(2H,s),6.57(1H,s),6.68(1H,d),6.75(1H,d),6.93(1H,d), 7.04(1H,d),7.42(1H,d),9.68 (1H,s),10.50(1H,s). |
| 28 | ESI/MS(m/z):457(M+H)+,455(M-H)- . $^1$HNMR(DMSO-$d_6$) δ(ppm):1.24(3H,t)2.37(3H,s),2.49(3H,s),3.57 (2H,s), 4.16(2H,q),5.51(2H,s),6.52-6.55(2H,m),6.69(1H,d),6.81(1H,d),6.86(1H,d), 7.24-7.28(1H,m), 7.34-7.44(5H,m),9.43(1H,s),9.62(1H,s). |
| 29 | ESI/MS(m/z):489(M+H)+,487(M-H)-. $^1$HNMR($CDCl_3$) δ (ppm):1.35(3H,t)2.31(3H,s),2.46(3H,s),3.55(2H,s),3.84 (2H,s),4.31(2H,q),5.41(2H,s),6.36(1H,s),6.48-6.53(2H,s),6.64(1H,d),6.7 (1H,d),6.92(2H,t), 7.03-7.07(2H,m),7.67(1H,d),9.60(1H,s). |
| 30 | ESI/MS(m/z):381(M+H)+,379(M-H)-. $^1$HNMR($CDCl_3$) δ(ppm):1.48(3H,t),2.17(6H,s),2.57(3H,s),4.45 (2H,q),4.60 (1H,s),5.46(2H,s),6.54(1H,d),6.55(1H,d).6.90(1H,d),7.08(1H,d),7.80(1H,d), 9.15(1H,brs). |
| 31 | ESI/MS(m/z):395(M+H)+,393(M-H)- . $^1$HNMR($CDCl_3$) δ(ppm):1.33(3H,t),2.14(6H,s),2.52(3H,s),3.53 (2H,s),4.27 (2H,q),4.53(1H,s),5.43(2H,s),6.54(2H,s),6.58(1H,d),6.84(1H,d),7.02(1H,d), 7.69(1H,d),9.66 (1H,brs). |
| 32 | ESI/MS(m/z):381(M+H)+,3 79(M-H)- . $^1$HNMR($CDCl_3$) δ(ppm):1.19(6H,d),1.42(3H,t),3.15(1H,m),4.41 (2H,q),4.90 (1H,s),5.21(2H,s),6.51(1H,d),6.64(1H,d),6.72(1H,dd),7.02(1H,d),7.11(1H,d), 7.27(1H,s), 7.32(1H,dd),8.00(1H,d),8.90(1H,brs). |
| 33 | ESI/MS(m/z):395(M+H)+,393(M-H)- . $^1$HNMR($CDCl_3$) δ(ppm):1.17(6H,d),1.31(3H,t),3.14(1H,m),3.46 (2H,s),4.25 (2H,q),5.13(1H,s),5.18(2H,s),6.46(1H,d),6.61(1H,d),6.68(1H,dd),7.01(1H,s), 7.06(1H,d), 7.21(2H,m),7.86(1H,s),9.12(1H,brs). |
| 34 | ESI/MS(m/z):395(M+H)+,393(M-H)- . $^1$HNMR($CDCl_3$) δ(ppm):1.19(6H,d),1.39(3H,t),2.36(3H,s),3.18 (1H,m),4.39 (2H,q),5.18(2H,s),5.58(1H,s),6.29(1H,s),6.45(1H,dd),6.61(1H,d),6.94(1H,d), 7.16(1H,d), 7.24(1H,m),7.89(1H,d),8.93(1H.s). |

[Table 33]

| Example | Data |
|---|---|
| 35 | ESI/MS(m/z):395(M+H)+,393(M-H). $^1$HNMR($CDCl_3$) δ(ppm):1.16(6H,d),1.41(3H,t),2.52(3H,s),3.16(1H,m),4.40 (2H,q),4.76(1H,s),5.45(2H,s),6.45(1H,dd),6.51(1H,d),6.60(1H,d),6.85(1H,d), 6.98(1H,s),7.04 (1H,d),7.89(1H,s),8.82(1H,brs). |
| 36 | ESI/MS(m/z):409(M+H)$^+$,407(M-H). $^1$HNMR($CDCl_3$) δ(ppm):1.16(6H,d),1.31(3H,t),2.49(3H,s),3.15(1H,m),3.46 (2H,s),4.24(2H,q),5.00(1H,brs),5.44(2H,s),6.42-6.46(2H,m),6.59(1H,d),6.86 (1H,d),6.91(1H,s), 7.01(1H,d),7.75(1H,d),9.04(1H,brs). |
| 37 | ESI/MS(m/z):425(M+H)$^+$,423(M-H)- . $^1$HNMR($CDCl_3$) δ(ppm):1.18-1.22(6H,m),1.33(3H,t),2.27(3H,s), 2.44(1H,dd), 3.15-3.24(2H,m),3.47(2H,s),3.60-3.67(1H,m),4.26(2H,q),4.38(2H,dd), 6.66(1H,d),6.87 (1H,d),6.96(1H,dd),7.10(1H,d),7.33(1H,d),9.08(1H,brs). |
| 38 | ESI/MS(m/z):382(M+H)+,380(M-H)-. $^1$HNNIR($CDCl_3$) δ(ppm):1.14(6H,d),1.43(3H,t),3.21(3H,m),4.44 (2H,q), 5.47(2H,s),6.68(1H,d),6.81(1H,dd),7.0 7(1H,d),7.19(1H,dd),7.30(1H,td), 7.76(1H,t),8.09(1H,s), 9.38(1H,brs). |

(continued)

| Example | Data |
|---|---|
| 39 | ESI/MS(m/z):396(M+H)+,394(M-H)- . $^1$HNMR($CDCl_3$) δ(ppm):1.21(6H,d),1.35(3H,t),3.17(1H,m),3.56 (2H,s), 4.31(2H,q),5.19(1H,brs),5.50(2H,s),6.61(1H,d),6.82(1H,dd),7.10(1H,d), 7.14(1H,d),7.31(1H,t), 7.80(1H,d),8.12(1H,s),9.99(1H,brs). |
| 40 | ESI/MS(m/z):395(M+H)+,393(M-H)- . $^1$HNMR($CDCl_3$) δ(ppm):1.24(6H,d),1.35(3H,t),3.18(1H,m),3.56 (2H,s), 4.04(2H,s),4.30(2H,q),4.68(1H,brs),6.64(1H,d),6.86(1H,d),6.91-6.95(3H,m), 7.01(1H,t),7.15(1H, d),7.39(1H,d),9.54(1H,brs). |

Compounds were synthesized according to the following reaction formula referring to the method of Example 1. Synthesized compounds and data are shown in Table 34.

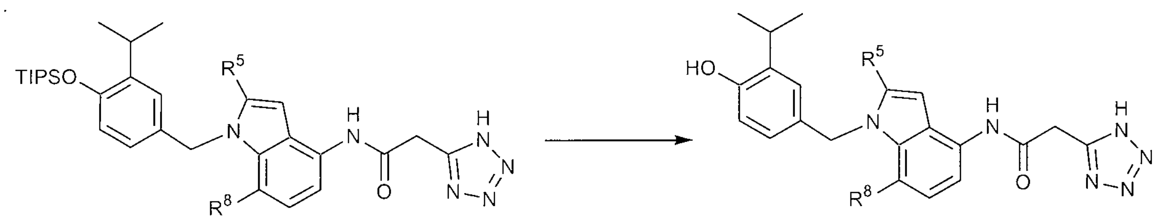

[Table 34]

| Example | $R^5$ | $R^8$ | Data |
|---|---|---|---|
| 41 | Et | H | ESI/MS(m/z):419(M+H)$^+$,417(M-H)$^-$. $^1$HNMR(DMSO-$d_6$) δ(ppm):1.09(6H,t),1.29(3H,t), 2.74(2H,q),3.14 (1H,m),4.30(2H,s),5.27(2H,s),6.51(1H,dd),6.62-6.65(2H,m),6.93 (1H,d), 6.98(1H,t),7.18(1H,d),7.60(1H,d),9.20(1H,s),10.05(1H,s). |
| 42 | H | Me | ESI/MS(m/z):405(M+H)+,403(M-H)- . $^1$HNMR(DMSO-$d_6$) δ(ppm):1.07(6H,d),2.48(3H,s), 3.15(1H,m), 4.28(2H,s),5.50(2H,s),6.43(1H,dd),6.66(1H,d),6.74(1H,d),6.79 (1H,d),6.85 (1H,d),7.34(1H,d),7.45(1H,d),9.14(1H,s),9.96(1H,s). |
| 43 | Me | Me | ESI/MS(m/z):419(M+H)$^+$,417(NI-H)$^-$. $^1$HNMR(DMSO-$d_6$) δ(ppm):1.06(6H,d),2.34(3H,s), 2.45(3H,s),3.13 (1H,m),4.26(2H,s),5.44(2H,s),6.26(1H,dd),6.58(1H,s),6.64(1H,d), 6.68 (1H,d),6.79(1H,s),7.38(1H,d),9.15(1H,s),9.89(1H,s). |

Reference Example 213

1-(4-hydroxy-3-isopropylbenzyl)-2,7-dimethyl-1H-indol-4-yla mine

According to the same manner as that of Example 1 using 1-(3-isopropyl-4-triisopropylsilanyloxybenzyl)-2,7-dimethyl -1H-indol-4-ylamine in place of ethyl N-[1-(3-isopropyl-4-triisopropylsilanyloxybenzyl)-1H-indol-4-yl]oxamidate, a reaction was performed to obtain the title compound.
$^1$H NMR ($CDCl_3$) δ (ppm): 1.19(6H,d), 2.29(3H,s), 2.42(3H,s), 3.17(1H,m), 5.41(2H,s), 6.20-6.21(2H,m), 6.30(1H,d), 6.40(1H,d), 6.62(1H,d), 6.88(1H,d).

Example 44

Benzyl N-[1-(4-hydroxy-3-isopropylbenzoyl)-7-methyl-1H-indol-4-yl] malonamate

1-(4-hydroxy-3-isopropylbenzoyl)-7-methyl-1H-indol-4-ylamine (100 mg) was dissolved in tetrahydrofuran (5.0 mL), malonic acid monobenzyl ester (58.6 mg), and N-(3-dimethylaminopropyl)-N'-ethylcarbodiimide hydrochloride (75. 6 mg) were added, and the mixture was stirred at room temperature for 2 hours. The reaction mixture was diluted with ethyl acetate, washed with an aqueous saturated sodium bicarbonate solution and an aqueous saturated sodium chloride

solution, and dried with anhydrous sodium sulfate, and the solvent was concentrated under reduced pressure. The resulting residue was purified by flash chromatography (silica gel, developing solvent: n-hexane to n-hexane/ethyl acetate = 4/1) to obtain the title compound (130 mg).
ESI/MS(m/z): 485(M+H)+, 483(M-H)-.
$^1$H NMR ($CDCl_3$) δ (ppm): 1.23(6H,d), 2.34(3H,s), 3.25(1H,m), 3.62(2H,s), 5.26(2H,s), 6.00(1H,m), 6.60(1H,d), 6.67(1H, d), 7.09(1H,d), 7.39(5H,m), 7.53(1H,d), 7.73(1H,d), 7.82(1H,s), 9.57(1H,s).

Example 45

Ethyl N-[1-(3-isopropyl-4-methoxybenzyl)-2,7-dimethyl-1H-indol-4-yl]malonamate

1-(3-isopropyl-4-methoxybenzyl)-2,7-dimethyl-1H-indol -4-ylamine (93 mg) was dissolved in diethyl malonate (930 mg), and the solution was stirred at 140°C for 2 hours. The reaction mixture was returned to room temperature, and concentrated under reduced pressure. The resulting residue was purified by flash chromatography (silica gel, developing solvent: n-hexane to n-hexane/ethyl acetate = 4/1) to obtain the title compound (79 mg).
ESI/MS (m/z): 437(M+H)+, 435(M-H)-.
$^1$H NMR ($CDCl_3$) δ (ppm): 1.13(6H,d), 1.35(3H,t), 2.35(3H,s), 2.49(3H,s), 3.32(1H,m), 3.54(2H,s), 3.76(3H,s), 4.30(2H, q), 5.45(2H,s), 6.38(1H,s), 6.64(1H,d), 6.78(1H,d), 6.86(1H,s), 7.67(1H,d), 9.57(1H,brs).

Example 46

Ethyl N-{1-[3-(4-fluorobenzoyl)-4-hydroxybenzyl]-7-methyl-1H-indo 1-4-yl}oxamate

According to the same manner as that of Example 45 using [5-(4-amino-7-methyl-1H-indol-1-ylmethyl)-2-hydroxyphenyl]-(4-fluorophenyl)methanone in place of 1-(3-isopropyl-4-methoxybenzyl)-2,7-dimethyl-1H-indol-4-yla mine, and using diethyl oxalate in place of diethyl malonate, a reaction was performed to obtain the title compound.
ESI/MS(m/z): 475(M+H)+, 473(M-H)-.
$^1$H NMR ($CDCl_3$) δ (ppm): 1.48(3H,t), 2.49(3H,s), 4.48(2H,q), 5.50(2H,s), 6.46(1H,d), 6.58(1H,d), 6.90-6.94(3H,m), 7.01-7.06(2H,m), 7.22-7.27(3H,m), 7.90(1H,d), 9.10(1H,s), 11.80(1H,s).

Example 47

Ethyl

2-fluoro-N-[1-(4-hydroxy-3-isopropylbenzyl)-2,7-dimethyl-1H -indol-4-yl]malonamate

According to the same manner as that of Example 45 using 1-(4-hydroxy-3-isopropylbenzyl)-2,7-dimethyl-1H-indol-4-yla mine in place of
1-(3-isopropyl-4-methoxybenzyl)-2,7-dimethyl-1H-indol-4-yla mine, and using diethyl fluoromalonate in place of diethyl malonate, a reaction was performed to obtain the title compound.
ESI/MS(m/z): 441(M+H)+, 439(M-H)-.
$^1$H NMR ($CDCl_3$) δ (ppm): 1.16(6H,d), 1.35(3H,t), 2.32(3H,s), 2.48(3H,s), 3.13(1H,m), 4.36(2H,m), 4.81(1H,s), 5.43(2H, s), 5.43(1H,d), 6.25-6.27(2H,m), 6.55(1H,d), 6.78(1H,d), 6.81(1H,d), 7.56(1H,d), 8.18(1H,s).

Example 48

Ethyl N-[1-(4-hydroxy-3-isopropylbenzyl)-2,7-dimethyl-1H-indol-4-yl]-2-methyl-malonamate

According to the same manner as that of Example 45 using 1-(4-hydroxy-3-isopropylbenzyl)-2,7-dimethyl-1H-indol-4-yla mine in place of
1-(3-isopropyl-4-methoxybenzyl)-2,7-dimethyl-1H-indol-4-yla mine, and using diethyl methylmalonate in place of diethyl malonate, a reaction was performed to obtain the title compound. ESI/MS(m/z): 437(M+H)+, 435(M-H)-.
$^1$H NMR ($CDCl_3$) δ (ppm): 1.18(6H,d), 1.34(3H,t), 1.61(3H,d), 2.34(3H,s), 2.49(3H,s), 3.17(1H,m), 3.52(1H,q), 4.29(2H, q), 5.44(2H,s), 6.27(1H,d), 6.32(1H,s), 6.56(1H,d), 6.78(1H,d), 6.86(1H,d), 7.64(1H,d), 9.00(1H,s).

Example 49

Ethyl 2-[1-(4-hydroxy-3-isopropylbenzyl)-2,7-dimethyl-1H-indol-4-ylcarbamoyl]-3-methyl-butylate

According to the same manner as that of Example 45 using 1-(4-hydroxy-3-isopropylbenzyl)-2,7-dimethyl-1H-indol-4-yla mine in place of
1-(3-isopropyl-4-methoxybenzyl)-2,7-dimethyl-1H-indol-4-yla mine, and using diethyl isopropylmalonate in place of diethyl malonate, a reaction was performed to obtain the title compound.
ESI/MS (m/z) : 465$(M+H)^+$, 463$(M-H)^-$.
$^1H$ NMR ($CDCl_3$) δ (ppm): 1.08(3H,d), 1.13(3H,d), 1.19(6H,d), 1.34(3H,t), 2.34(3H,s), 2.41-2.45(1H,m), 2.49(3H,s), 3.13-3.20(2H,m), 4.23-4.34(2H,m), 4.93(1H,s), 5.44(2H,s), 6.28(1H,dd), 6.33(1H,s), 6.56(1H,d), 6.78(1H,d), 6.87 (1H, d), 7.66(1H,d), 9.09(1H,s).

Example 50

Ethyl 2-benzyl-N-[1-(4-hydroxy-3-isopropylbenzyl)-2,7-dimethyl-1H -indol-4-yl]malonamate

According to the same manner as that of Example 45 using 1-(4-hydroxy-3-isopropylbenzyl)-2,7-dimethyl-1H-indol-4-yla mine in place of
1-(3-isopropyl-4-methoxybenzyl)-2,7-dimethyl-1H-indol-4-yla mine, and using diethyl benzylmalonate in place of diethyl malonate, a reaction was performed to obtain the title compound. ESI/MS(m/z): 513$(M+H)^+$, 511$(M-H)^-$.
$^1H$ NMR ($CDCl_3$) δ (ppm): 1.15(3H,t), 1.19(6H,d), 2.32(3H,s), 2.49(3H,s), 3.16(1H,m), 3.32(1H,dd), 3.44(1H,dd), 3.71 (1H,dd), 4.14(2H,q), 4.80(1H,brs), 5.44(2H,s), 6.22(1H,s), 6.57(1H,d), 6.78(1H,d), 6.86(1H,d), 7.21-7.31(8H,m), 7.62 (1H,d), 8.89(1H,s).

Example 51

Ethyl N-[1-(4-hydroxy-3-pyridin-3-ylbenzyl)-2,7-dimethyl-1H-indol -4-yl]oxamate

According to the same manner as that of Example 45 using 1-(4-hydroxy-3-pyridin-3-ylbenzyl)-2,7-dimethyl-1H-indol-4-ylamine in place of
1-(3-isopropyl-4-methoxybenzyl)-2,7-dimethyl-1H-indol-4-yla mine, and using diethyl oxalate in place of diethyl malonate, a reaction was performed to obtain the title compound. ESI/MS(m/z): 444$(M+H)^+$, 442$(M-H)^-$.
$^1H$ NMR (DMSO-$d_6$) δ (ppm): 1.13(3H,t), 2.35(3H,s), 2.50(3H,s), 4.29(2H,q), 5.52(2H,s), 6.38(1H,s), 6.56(1H,dd), 6.74 (1H,d), 6.86-6.89(2H,m), 7.19(1H,d), 7.39(1H,dd), 7.83(1H,dd), 8.45(1H,dd), 8.59(1H,d), 9.77(1H,s), 10.29(1H,s).

Example 52

Ethyl N-[1-(4-hydroxy-3-pyridin-3-ylbenzyl)-2,7-dimethyl-1H-indol -4-yl]malonamate

According to the same manner as that of Example 45 using 1-(4-hydroxy-3-pyridin-3-ylbenzyl)-2,7-dimethyl-1H-indol-4-ylamine in place of
1-(3-isopropyl-4-methoxybenzyl)-2,7-dimethyl-1H-indol-4-yla mine, a reaction was performed to obtain the title compound.
ESI/MS(m/z): 458$(M+H)^+$, 456$(M-H)^-$.
$^1H$ NMR (DMSO-$d_6$) δ (ppm): 1.23(3H,t), 2.37(3H,s), 2.49(3H,s), 3.58(2H,s), 4.15(2H,q), 5.52(2H,s), 6.54-6.58(2H,m), 6.70(1H,d), 6.87-6.90(2H,m), 7.39-7.44(2H,m), 7.85(1H,dd), 8.47(1H,dd), 8.61(1H,d), 9.68(1H,s), 9.76(1H,s).

Example 53

Ethyl N-[1-(4-hydroxy-3-phenethylbenzyl)-2,7-dimethyl-1H-indol-4-yl]malonamate

According to the same manner as that of Example 45 using 1-(4-hydroxy-3-phenethylbenzyl)-2,7-dimethyl-1H-indol-4-yla mine in place of
1-(3-isopropyl-4-methoxybenzyl)-2,7-dimethyl-1H-indol-4-yla mine, a reaction was performed to obtain the title compound.
ESI/MS(m/z): 485$(M+H)^+$, 483$(M-H)^-$.
$^1H$ NMR ($CDCl_3$) δ (ppm): 1.32(3H,t), 2.26(3H,s), 2.43(3H,s), 2.84(4H,s), 3.53(2H,s), 4.27(2H,q), 5.34(2H,s), 6.30-6.33(1H,m), 6.36(1H,s), 6.58-6.61(2H,m), 6.76(1H,d), 7.09-7.23(5H,m), 7.65(1H,d), 9.63(1H,s).

Example 54

Ethyl N-{1-[3-(4-fluorobenzoyl)-4-hydroxybenzyl]-2,7-dimethyl-1H-indol-4-yl}malonamate

[5-(4-amino-2,7-dimethyl-1H-indol-1-ylmethyl)-2-hydro xyphenyl]-(4-fluorophenyl)methanone (18.4 g) was dissolved in dichloromethane (200 mL), pyridine (4.6 mL), and a solution of ethylmalonyl chloride (10.3 g) in dichloromethane (70 mL) were added at 0°C, and the mixture was stirred at room temperature for 2 hours. The resulting mixture was diluted with dichloromethane, washed with 1 mol/L hydrochloric acid, an aqueous saturated sodium bicarbonate solution, and an aqueous saturated sodium chloride solution, and dried with anhydrous sodium sulfate, and the solvent was concentrated under reduced pressure. The resulting residue was purified by flash chromatography (silica gel, developing solvent: n-hexane to n-hexane/ethyl acetate = 3/1) to obtain the title compound (19.5 g).
ESI/MS(m/z): 503$(M+H)^+$, 501$(M-H)^-$.
$^1$H NMR ($CDCl_3$) δ (ppm): 1.36(3H,t), 2.28(3H,s), 2.44(3H,s), 3.57(2H,s), 4.31(2H,q), 5.42(2H,s), 6.27(1H,d), 6.34(1H, d), 6.79(1H,d), 6.88(2H,t), 7.03(1H,d), 7.16(2H,d), 7.22(1H,dd), 7.79(1H,d), 9.66(1H,s), 11.81(1H,s).

Example 55

Ethyl N-[1-(4-methoxy-2,3-dimethylbenzyl)-2,7-dimethyl-1H-indol-4 -yl]malonamate

According to the same manner as that of Example 54 using 1-(4-methoxy-2,3-dimethylbenzyl)-2,7-dimethyl-1H-indol-4-yl amine in place of [5-(4-amino-2,7-dimethyl-1H-indol-1-ylmethyl)-2-hydroxyphen yl]-(4-fluorophenyl)methanone, a reaction was performed to obtain the title compound.
ESI/MS (m/z) : 423$(M+H)^+$, 421$(M-H)^-$.
$^1$H NMR ($CDCl_3$) δ (ppm): 1.36(3H,t), 2.21(3H,s), 2.31(3H,s), 2.33(3H,s), 2.39(3H,s), 3.55(2H,s), 3.72(3H,s), 4.31(2H, q), 5.40(2H,s), 5.85(1H,d), 6.41(1H,s), 6.46(1H,d), 6.77(1H,d), 7.67(1H,d), 9.59(1H,brs).

Example 56

Ethyl N-(1-{3-[(4-fluorophenyl)hydroxymethyl]-4-hydroxybenzyl}-7-methyl-1H-indol-4-yl)oxamate

Sodium borohydride (4.8 mg) was suspended in tetrahydrofuran (1.0 mL), and acetic acid (14.4 μL) was added at 0°C. After stirred at room temperature for 1 hour, a solution of ethyl N-{1-[3-(4-fluorobenzoyl)-4-hydroxybenzyl]-7-methyl-1H-indo 1-4-yl}oxamate (54.2mg) in tetrahydrofuran (0.5mL) was added, and the mixture was stirred at room temperature for 2 hours. The reaction mixture was neutralized with an aqueous saturated sodium bicarbonate solution, followed by extraction with ethyl acetate. The organic layer was washed with water, and an aqueous saturated sodium chloride solution, and dried with anhydrous sodium sulfate, and the solvent was concentrated under reduced pressure. The resulting residue was purified by flash chromatography (silica gel, developing solvent: n-hexane to n-hexane/ethyl acetate = 3/1), and crystallized with ethyl acetate/n-hexane to obtain the title compound (24.4 mg). ESI/MS(m/z): 477 $(M+H)^+$, 475$(M-H)^-$.
$^1$H NMR (DMSO-$d_6$) δ (ppm): 1.33(3H,t), 2.45(3H,s), 4.32(2H,q), 5.51(2H,s), 5.68(1H,d), 5.88(1H,d), 6.48(1H,d), 6.56 (1H,d), 6.64(1H,d), 6.78(1H,d), 7.04(2H,t), 7.16(1H,s) 7.24-7.28(3H,m), 7.35(1H,d), 9.40(1H,s), 10.33(1H,s).

Example 57

Ethyl N-(1-{3-[(4-fluorophenyl)hydroxymethyl]-4-hydroxybenzyl}-2, 7-dimethyl-1H-indol-4-yl)malonamate

According to the same manner as that of Example 56 using ethyl N-{1-[3-(4-fluorobenzoyl)-4-hydroxybenzyl]-2,7-dimethyl-1H-indol-4-yl}malonamate in place of ethyl N-{1-[3-(4-fluorobenzoyl)-4-hydroxybenzyl]-7-methyl-1H-indo 1-4-yl}oxamate, a reaction was performed to obtain the title compound.
ESI/MS(m/z): 505$(M+H)^+$, 503$(M-H)^-$.
$^1$H NMR (DMSO-$d_6$) δ (ppm): 1.22(3H,t), 2.31(3H,s), 2.41(3H,s), 3.57(2H,s), 4.14(2H,q), 5.43(2H,s), 5.67(1H,brs), 5.88 (1H,s), 6.34(1H,dd), 6.54(1H,s), 6.64(1H,d), 6.67(1H,d), 7.05(1H,t), 7.10(1H,d), 7.26(2H,m), 7.41(1H,d), 9.38(1H,brs), 9.66(1H,s).

Example 58

Ethyl (+)-N-(1-{3-[(4-fluorophenyl)hydroxymethyl]-4-hydroxybenzyl }-2,7-dimethyl-1H-indol-4-yl)malonamate

Ethyl N-(1-{3-[(4-fluorophenyl)hydroxymethyl]-4-hydroxybenzyl}-2, 7-dimethyl-1H-indol-4-yl)malonamate was resolved by high performance liquid chromatography (developing solvent; n-hexane/ethanol = 3/2) with a chiral column (Daicel, CHIRALCEL OJ-H) to obtain the title compound having a retention time of 5.2 minutes and $[\alpha]^{24}_D$ = +17.3° (c = 1.0, methanol). ESI/MS(m/z): 505$(M+H)^+$, 503$(M-H)^-$.
$^1$H NMR(DMSO-$d_6$) δ (ppm): 1.22(3H,t), 2.31(3H,s), 2.41(3H,s), 3.57(2H,s), 4.14(2H,q), 5.43(2H,s), 5.67(1H,brs), 5.88 (1H,s), 6.34(1H,dd), 6.54(1H,s), 6.64(1H,d), 6.67(1H,d), 7.05(1H,t), 7.10(1H,d) 7.26(2H,m), 7.41(1H,d), 9.38(1H,brs), 9.66(1H,s).

Example 59

Ethyl (-)-N-(1-{3-[(4-fluorophenyl)hydroxymethyl]-4-hydroxybenzyl}-2,7-dimethyl-1H-indol-4-yl)malonamate

Ethyl N-(1-{3-[(4-fluorophenyl)hydroxymethyl]-4-hydroxybenzyl}-2, 7-dimethyl-1H-indol-4-yl)malonamate was resolved by high performance liquid chromatography (developing solvent; n-hexane/ethanol = 3/2) with a chiral column (Daicel, CHIRALCEL OJ-H) to obtain the title compound having a retention time of 6.9 minutes and $[\alpha]^{24}_D$ = -15.7° (c = 1.0, methanol). ESI/MS(m/z): 505$(M+H)^+$, 503$(M-H)^-$.
$^1$H NMR(DMSO-$d_6$) δ (ppm): 1.22(3H,t), 2.31(3H,s), 2.41(3H,s), 3.57(2H,s), 4.14(2H,q), 5.43(2H,s), 5.67(1H,brs), 5.88 (1H,s), 6.34(1H,dd), 6.54(1H,s), 6.64(1H,d), 6.67(1H,d), 7.05(1H,t), 7.10(1H,d) 7.26(2H,m), 7.41(1H,d), 9.38(1H,brs), 9.66(1H,s).

Example 60

Ethyl [1-(4-hydroxy-3-isopropylbenzyl)-2-methyl-1H-indol-4-yloxy] acetate

Ethyl [1-(4-benzyloxy-3-isopropylbenzyl)-2-methyl-1H-indol-4-ylox y]acetate (470 mg) was dissolved in ethanol (5.0 mL), 10% palladium carbon (94 mg) was added, and the mixture was stirred at room temperature for 3 hours in the hydrogen atmosphere. The catalyst was filtered, and the filtrate was concentrated under reduced pressure. The resulting residue was purified by flash chromatography (silica gel, developing solvent; n-hexane to n-hexane/ethyl acetate = 4/1) to obtain the title compound (288 mg).
ESI/MS(m/z): 382$(M+H)^+$, 380$(M-H)^-$.
$^1$H NMR($CDCl_3$) δ (ppm): 1.19(6H,d), 1.29(3H,t), 2.34(3H,s), 3.15(1H,m), 4.27(2H,q), 4.75(2H,s), 5.18(2H,s), 6.39(1H, d), 6.46-6.50(2H,m), 6.55(1H,dd), 6.88(1H,d), 6.94-6.98(2H,m).

Example 61

Ethyl {[1-(4-hydroxy-3-isopropylbenzyl)-1H-indol-4-carbonyl]amino }acetate

According to the same manner as that of Example 60 using ethyl {[1-(4-benzyloxy-3-isopropylbenzyl)-1H-indol-4-carbonyl]ami no}acetate in place of ethyl [1-(4-benzyloxy-3-isopropylbenzyl)-2-methyl-1H-indol-4-ylox y]acetate, a reaction was performed to obtain the title compound.
ESI/MS(m/z): 395$(M+H)^+$, 393$(M-H)^-$.
$^1$H NMR(DMSO-$d_6$) δ (ppm): 1.13(6H,d), 1.23(3H,t), 3.14(1H,m) 4.03(2H,d), 4.15(2H,q), 5.32(2H,s), 6.68(1H,d) 6.81 (1H,dd), 6.92(1H,d), 7.14(1H,d) 7.19(1H,t), 7.47(1H,d), 7.57(1H,s), 7.69(1H,d), 8.62(1H,t), 9.28(1H,brs).

Example 62

Ethyl [1-(4-hydroxy-3-isopropylbenzyl)-1H-indol-4-yl]acetate

According to the same manner as that of Example 60 using ethyl [1-(4-benzyloxy-3-isopropylbenzyl)-1H-indol-4-yl]acetate in place of ethyl [1-(4-benzyloxy-3-isopropylbenzyl)-2-methyl-1H-indol-4-ylox y]acetate, a reaction was performed to obtain the title compound.
ESI/MS(m/z): 352$(M+H)^+$, 350$(M-H)^-$.

$^1H$ NMR(DMSO-$d_6$) δ (ppm): 1.14(6H,d), 1.22(3H,t), 3.14(1H,m), 3.50(1H,s), 4.14(2H,q), 5.31(2H,s), 6.65(1H,d), 6.80 (1H,dd), 6.90-7.35(1H,m).

Example 63

N-[1-(4-hydroxy-3-isopropylbenzyl)-1H-indol-4-yl]oxamic acid

Ethyl N-[1-(4-hydroxy-3-isopropylbenzyl)-1H-indol-4-yl]oxamate (33 mg) was dissolved in ethanol (0.5 mL), a 1 mol/L aqueous sodium hydroxide solution (173 μL) was added, and the mixture was stirred at room temperature for 1 hour. The reaction mixture was concentrated under reduced pressure, and the resulting residue was dissolved in water. 1 mol/L hydrochloric acid was added to be acidic, followed by extraction with ethyl acetate. The organic layer was washed with water, and an aqueous saturated sodium chloride solution, and dried with anhydrous sodium sulfate, and the solvent was concentrated under reduced pressure. The resulting residue was crystallized with diethyl ether/n-hexane to obtain the title compound (22 mg).
ESI/MS(m/z): 353$(M+H)^+$, 351$(M-H)^-$.
$^1H$ NMR (DMSO-$d_6$) δ (ppm): 1.11(6H,d), 3.12(1H,m), 5.27(2H,s), 6.56(1H,d), 6.67(1H,d), 6.81(1H,dd), 7.09(1H,t), 7.13 (1H,d), 7.36(1H,d), 7.42(1H,d), 7.44(1H,d), 9.27(1H,s), 10.35(1H,s).
Compounds were synthesized according to the following reaction formula referring to the method of Example 63. Synthesized compounds are shown in Table 35 and Table 36, and data are shown in Table 37 to Table 41.

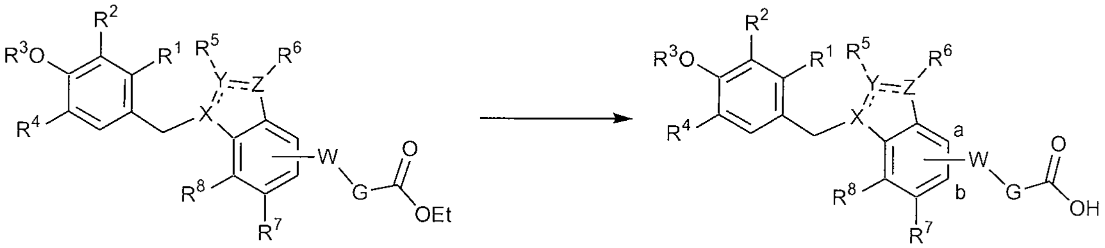

[Table 35]

| Example | $R^1$ | $R^2$ | $R^3$ | $R^4$ | $R^5$ | $R^6$ | $R^7$ | $R^8$ | -X-Y-Z- | | W | G |
|---|---|---|---|---|---|---|---|---|---|---|---|---|
| 64 | Me | Me | Me | H | Me | H | H | Me | -N-C=C- | a | -NHCO- | $CH_2$ |
| 65 | H | Br | H | H | Me | H | H | Me | -N-C=C- | a | -NHCO- | $CH_2$ |
| 66 | H | Cl | H | H | Me | H | H | Me | -N-C=C- | a | -NHCO- | $CH_2$ |
| 67 | H | Me | H | H | Me | H | H | Me | -N-C=C- | a | -NHCO- | bond |
| 68 | H | Me | H | H | Me | H | H | Me | -N-C=C- | a | -NHCO- | $CH_2$ |
| 69 | H | *i*-Pr | H | H | H | H | H | H | -N-C=C- | a | -NHCO- | $CH_2$ |
| 70 | H | *i*-Pr | H | H | H | H | H | H | -N-C=C- | a | -CONH- | $CH_2$ |
| 71 | H | *i*-Pr | H | H | H | H | H | H | -N-C=C- | a | bond | $CH_2$ |
| 72 | H | *i*-Pr | H | H | Me | H | H | H | -N-C=C- | a | -NHCO- | bond |
| 73 | H | *i*-Pr | H | H | Me | H | H | H | -N-C=C- | a | -NHCO- | $CH_2$ |
| 74 | H | *i*-Pr | H | H | Me | H | H | H | -N-C=C- | a | -0- | $CH_2$ |
| 75 | H | *i*-Pr | H | H | Et | H | H | H | -N-C=C- | a | -NHCO- | $CH_2$ |
| 76 | H | *i*-Pr | H | H | $CF_3$ | H | H | H | -N-C=C- | a | -NHCO- | $CH_2$ |
| 77 | H | *i*-Pr | H | H | H | H | Br | H | -N-C=C- | a | -NHCO- | $CH_2$ |
| 78 | H | *i*-Pr | H | H | H | H | H | Cl | -N-C=C- | a | -NHCO- | $CH_2$ |
| 79 | H | *i*-Pr | H | H | H | H | H | Me | -N-C=C- | a | -NHCO- | bond |

(continued)

| Example | $R^1$ | $R^2$ | $R^3$ | $R^4$ | $R^5$ | $R^6$ | $R^7$ | $R^8$ | -X-Y-Z- | | W | G |
|---|---|---|---|---|---|---|---|---|---|---|---|---|
| 80 | H | *i*-Pr | H | H | H | H | H | Me | -N-C=C- | a | -NHCO- | $CH_2$ |
| 81 | H | *i*-Pr | H | H | H | H | H | Me | -N-C=C- | a | -NHCO- | $C(Me)_2$ |
| 82 | H | *i*-Pr | H | H | H | H | H | Me | -N-C=C- | a | -NH- | $CH_2CH_2$ |
| 83 | H | *i*-Pr | H | H | Me | Me | H | H | -N-C=C- | a | -NHCO- | bond |
| 84 | H | *i*-Pr | H | H | Me | Me | H | H | -N-C=C- | a | -NHCO- | $CH_2$ |
| 85 | H | *i*-Pr | H | H | Me | H | H | Me | -N-C=C- | a | -NHCO- | bond |
| 86 | H | *i*-Pr | H | H | Me | H | H | Me | -N-C=C- | a | -NHCO- | $CH_2$ |
| 87 | H | *i*-Pr | Me | H | Me | H | H | Me | -N-C=C- | a | -NHCO- | $CH_2$ |
| 88 | H | *i*-Pr | H | H | Me | H | H | Me | -N-C=C- | a | -NHCO- | CHF |
| 89 | H | *i*-Pr | H | H | Me | H | H | Me | -N-C=C- | a | -NHCO- | CH(Me) |
| 90 | H | *i*-Pr | H | H | Me | H | H | Me | -N-C=C- | a | -NHCO- | CH(*i*-Pr) |
| 91 | H | *i*-Pr | H | H | Me | H | H | Me | -N-C=C- | a | -NHCO- | CH(Bn) |
| 92 | H | *i*-Pr | H | H | Me | H | H | Me | -N-C=C- | a | -NHCO- | $C(Me)_2$ |
| 93 | H | *i*-Pr | H | H | Me | H | H | Me | -N-C=C- | a | -NHCO- | $CH_2CH_2$ |
| 94 | H | *i*-Pr | H | H | Et | H | H | Me | -N-C=C- | a | -NHCO- | $CH_2$ |
| 95 | H | *i*-Pr | H | H | $CF_3$ | H | H | Me | -N-C=C- | a | -NHCO- | $CH_2$ |
| 96 | H | $CF_3$ | H | H | Me | H | H | Me | -N-C=C- | a | -NHCO- | $CH_2$ |
| 97 | H | Ph | H | H | Me | H | H | Me | -N-C=C- | a | -NHCO- | $CH_2$ |
| 98 | H | 3-Py | H | H | Me | H | H | Me | -N-C=C- | a | -NHCO- | bond |
| 99 | H | 3-Py | H | H | Me | H | H | Me | -N-C=C- | a | -NHCO- | $CH_2$ |
| 100 | H | 4-F-Bn | H | H | Me | H | H | Me | -N-C=C- | a | -NHCO- | $CH_2$ |
| 101 | H | PhEt | H | H | Me | H | H | Me | -N-C=C- | a | -NHCO- | bond |
| 102 | H | PhEt | H | H | Me | H | H | Me | -N-C=C- | a | -NHCO- | $CH_2$ |
| 103 | H | Me | H | Me | H | H | H | Me | -N-C=C- | a | -NHCO- | bond |
| 104 | H | Me | H | Me | H | H | H | Me | -N-C=C- | a | -NHCO- | $CH_2$ |

[Table 36]

| Example | $R^1$ | $R^2$ | $R^3$ | $R^4$ | $R^5$ | $R^6$ | $R^7$ | $R^8$ | -X-Y-Z- | | W | G |
|---|---|---|---|---|---|---|---|---|---|---|---|---|
| 80 | H | *i*-Pr | H | H | H | H | H | Me | -N-C=C- | a | -NHCO- | $CH_2$ |
| 81 | H | *i*-Pr | H | H | H | H | H | Me | -N-C-C- | a | -NHCO- | $C(Me)_2$ |
| 82 | H | *i*-Pr | H | H | H | H | H | Me | -N-C=C- | a | -NH- | $CH_2CH_2$ |
| 83 | H | *i*-Pr | H | H | Me | Me | H | H | -N-C=C- | a | -NHCO- | bond |
| 84 | H | *i*-Pr | H | H | Me | Me | H | H | -N-C=C- | a | -NHCO- | $CH_2$ |
| 85 | H | *i*-Pr | H | H | Me | H | H | Me | -N-C=C- | a | -NHCO- | bond |
| 86 | H | *i*-Pr | H | H | Me | H | H | Me | -N-C=C- | a | -NHCO- | $CH_2$ |
| 87 | H | *i*-Pr | Me | H | Me | H | H | Me | -N-C=C- | a | -NHCO- | $CH_2$ |
| 88 | H | *i*-Pr | H | H | Me | H | H | Me | -N-C=C- | a | -NHCO- | CHF |
| 89 | H | *i*-Pr | H | H | Me | H | H | Me | -N-C=C- | a | -NHCO- | CH(Me) |

(continued)

| Example | $R^1$ | $R^2$ | $R^3$ | $R^4$ | $R^5$ | $R^6$ | $R^7$ | $R^8$ | -X-Y-Z- | | W | G |
|---|---|---|---|---|---|---|---|---|---|---|---|---|
| 90 | H | *i*-Pr | H | H | Me | H | H | Me | -N-C=C- | a | -NHCO- | CH(*i*-Pr) |
| 91 | H | *i*-Pr | H | H | Me | H | H | Me | -N-C=C- | a | -NHCO- | CH(Bn) |
| 92 | H | *i*-Pr | H | H | Me | H | H | Me | -N-C=C- | a | -NHCO- | $C(Me)_2$ |
| 93 | H | *i*-Pr | H | H | Me | H | H | Me | -N-C=C- | a | -NHCO- | $CH_2CH_2$ |
| 94 | H | *i*-Pr | H | H | Et | H | H | Me | -N-C=C- | a | -NHCO- | $CH_2$ |
| 95 | H | *i*-Pr | H | H | $CF_3$ | H | H | Me | -N-C=C- | a | -NHCO- | $CH_2$ |
| 96 | H | $CF_3$ | H | H | Me | H | H | Me | -N-C=C- | a | -NHCO- | $CH_2$ |
| 97 | H | Ph | H | H | Me | H | H | Me | -N-C=C- | a | -NHCO- | $CH_2$ |
| 98 | H | 3-Py | H | H | Me | H | H | Me | -N-C=C- | a | -NHCO- | bond |
| 99 | H | 3-Py | H | H | Me | H | H | Me | -N-C=C- | a | -NHCO- | $CH_2$ |
| 100 | H | 4-F-Bn | H | H | Me | H | H | Me | -N-C=C- | a | -NHCO- | $CH_2$ |
| 101 | H | PhEt | H | H | Me | H | H | Me | -N-C=C- | a | -NHCO- | bond |
| 102 | H | PhEt | H | H | Me | H | H | Me | -N-C=C- | a | -NHCO- | $CH_2$ |
| 103 | H | Me | H | Me | H | H | H | Me | -N-C=C- | a | -NHCO- | bond |
| 104 | H | Me | H | Me | H | H | H | Me | -N-C=C- | a | -NHCO- | $CH_2$ |
| 105 | H | *i*-Pr | H | H | H | H | H | H | -N-C=C- | b | -NHCO- | bond |
| 106 | H | *i*-Pr | H | H | H | H | H | H | -N-C=C- | b | -NHCO- | $CH_2$ |
| 107 | H | *i*-Pr | H | H | Me | H | H | H | -N-C=C- | b | -NHCO- | bond |
| 108 | H | *i*-Pr | H | H | H | H | H | Me | -N-C=C- | b | -NHCO- | bond |
| 109 | H | *i*-Pr | H | H | H | H | H | Me | -N-C=C- | b | -NHCO- | $CH_2$ |
| 110 | H | *i*-Pr | H | H | Me | H | H | Me | -N-CH-CH- | a | -NHCO- | $CH_2$ |
| 111 | H | *i*-Pr | H | H | - | H | H | H | -N-N=C- | a | -NHCO- | bond |
| 112 | H | *i*-Pr | H | H | - | H | H | H | -N-N=C- | a | -NHCO- | $CH_2$ |
| 113 | H | *i*-Pr | H | H | H | H | H | H | -C=C-N- | a | -NHCO- | $CH_2$ |

[Table 37]

| Example | Data |
|---|---|
| 64 | ESI/MS(m/z):395$(M+H)^+$,393(M-H)-. $^1$HNMR(DMSO-$d_6$) δ(ppm):2.14(3H,s),2.28(3H,s),2.29(3H,s),2.30 (3H,s), 3.48(2H,s),3.65(3H,s),5.42(2H,s),5.59(1H,d),6.54-6.60(2H,m),6.64(1H,d), 7.45(1H,d),9.69(1H, brs). |
| 65 | ESI/MS(m/z):431$(M+H)^+$,429$(M-H)^-$. $^1$HNMR(DMSO-$d_6$) δ(ppm):2.31(3H,s),2.42(3H,s),3.46(2H,s),5.45 (2H,s), 6.56-6.57(2H,m),6.69(1H,d),6.86(1H,d),6.92(1H,s),7.45(1H,d),9.71(1H,s), 10.12(1H,s),12.53 (1H,brs). |
| 66 | ESI/MS(m/z):387$(M+H)^+$,385$(M-H)^-$. $^1$HNMR(DMSO-$d_6$) δ(ppm):2.31(3H,s),2.42(3H,s),3.46(2H,s),5.45 (2H,s), 6.54-6.65(3H,m),6.86-6.95(2H,m),7.43(1H,d),9.70(1H,s),10.11(1H,s), 12.51(1H,brs). |
| 67 | ESI/MS(m/z):353$(M+H)^+$,351(M-H)- . $^1$HNMR(DMSO-$d_6$) δ(ppm):2.03(3H,s),2.32(3H,s),2.47(3H,s), 5.43(2H,s), 6.35(1H,dd),6.38(1H,d),6.63(1H,d),6.67(1H,d),6.73(1H,d),7.27(1H,d), 9.22(1H,s),10.15(1H, s). |

(continued)

| Example | Data |
|---|---|
| 68 | ESI/MS(m/z):367(M+H)$^+$,365(M-H)- . $^1$HNMR(DMSO-$d_6$) δ(ppm):2.01(3H,s),2.30(3H,s),2.42(3H,s), 3.43(2H,s), 5.40(2H,s),6.32(1H,dd),6.53(1H,s),6.60(1H,d),6.64-6.67(2H,m),7.45(1H,d), 9.19(1H,s). |
| 69 | ESI/MS(m/z):367(M+H)$^+$,365(M-H)$^-$. $^1$HNMR(DMSO-$d_6$) δ(ppm):1.10(6H,d),3.13(1H,m),3.48(2H,s), 5.24(2H,s), 6.66(1H,d),6.70(1H,d),6.79(1H,dd),7.03(1H,dd),7.09(1H,d),7.23(1H,d), 7.38(1H,d),7.62(1H, d),9.18(1H,s),9.80(1H,s). |
| 70 | ESI/MS(m/z):367(M+H)$^+$,365(M-H)$^-$. $^1$HNMR(DMSO-$d_6$) δ(ppm):1.10(6H,d),3.13(1H,m),3.87(2H,d), 5.30(2H,s), 6.66(1H,d),6.79(1H,dd),6.92(1H,d),7.12(1H,d),7.16(1H,dd),7.45(1H,d), 7.56(1H,d),7.66(1H, d),8.32(1H,s),9.27(1H,s). |
| 71 | ESI/MS(m/z):352(M+H)$^+$,350(M-H)$^-$. $^1$HNMR(DMSO-$d_6$)δ(ppm):1.14(6H,d),3.13(1H,m),3.51(1H,s),5.31 (2H,s), 6.64(1H,d),6.81(1H,dd),6.90-7.35(1H,m). |
| 72 | ESI/MS(m/z):367(M+H)$^+$,365(M-H)$^-$. $^1$HNMR(DMSO-$d_6$) δ(ppm):1.09(6H,d),2.37(3H,s),3.13(1H,m), 5.25(2H,s), 6.34(1H,s),6.53(1H,dd),6.64(1H,d),6.98-7.03(2H,m),1.26(1H,d),7.36(1H,d), 9.15(1H,s), 10.19(1H,s). |
| 73 | ESI/MS(m/z):381(M+H)$^+$,379(M-H)$^-$. $^1$HNMR(DMSO-$d_6$) δ(ppm):1.09(6H,d),2.37(3H,s),3.12(1H,m), 3.27(2H,s), 5.23(2H,s),6.52-6.50(2H,m),6.63(1H,d),6.94-6.97(2H,m), 7.15(1H,d), 7.57(1H,d),9.14(1H, s),9.70(1H,s) |
| 74 | ESI/MS(m/z):354(M+H)$^+$,352(M-H)$^-$. $^1$HNMR(DMSO-$d_6$) δ(ppm):1.09(6H,d),2.37(3H,s),3.14(1H,m), 4.75(2H,s), 5.23(2H,s),6.30(1H,s),6.37(1H,d),6.50(2H,dd),6.65(1H,d),6.93(1H,t), 7.00(1H,d),7.03(1H, d). |

[Table 38]

| Example | Data |
|---|---|
| 75 | ESI/MS(m/z):395(M+H)$^+$,393(M-H)$^-$. $^1$HNMR(DMSO-$d_6$) δ(ppm):1.09(6H,t),1.28(3H,t),2.73(2H,q),3.13 (1H,m), 3.51(2H,s),5.26(2H,s),6.51(1H,dd),6.57(1H,s),6.64(1H,d),6.93(1H,d),6.98 (1H,t),7.14(1H,d), 7.62(1H,d),9.12(1H,s),9. 72(1H,s),12.50(1H,brs). |
| 76 | ESI/MS(m/z):435(M+H)$^+$,433(M-H)$^-$. $^1$HNMR(DMSO-$d_6$) δ(ppm):1.07(6H,d),3.12(1H,m),3.51(2H,s), 5.42(2H,s), 6.52(1H,dd),6.63(1H,d),6.95(1H,d),7.23-7.27(2H,m),7.49(1H,s),7.79(1H,m) 9.27(1H,s), 10.02(1H,brs). |
| 77 | ESI/MS(m/z):446(M+H)$^+$,444(M-H)$^-$. $^1$HNMR(DMSO-$d_6$) δ(ppm):1.10(6H,d),3.13(1H,m),3.50(2H,s), 5.24(2H,s), 6.66(1H,d),6.75-6.80(2H,m),7.11(1H,s),7.43(1H,d),7.49(1H,s),7.91(1H,s), 9.26(1H,s),9.93 (1H,s),12.67(1H,brs). |
| 78 | ESI/MS(m/z):401(M+H)$^+$,399(M-H)$^-$. $^1$HNMR(DMSO-$d_6$) δ(ppm):1.07(6H,d),3.12(1H,m),3.49(2H,s), 5.61(2H,s), 6.60-6.66(2H,m),6.83(1H,d),6.96(1H,s),7.06(1H,d),7.44(1H,d),7.63(1H,d), 9.13(1H,s),9.82 (1H,s). |
| 79 | ESI/MS(m/z):367(M+H)$^+$,365(M-H)$^-$. $^1$HNMR(DMSO-$d_6$) δ(ppm):1.07(6H,d),2.49(3H,s),3.13(1H,m), 5.49(2H,s), 6.40(1H,dd),6.57(1H,d),6.65(1H,d),6.78(1H,d),6.87(1H,d),7.30(1H,d),7.33 (1H,d),9.15(1H, s),10.16(1H,s). |
| 80 | ESI/MS(m/z):381(M+H)$^+$,379(M-H)$^-$. $^1$HNMR(DMSO-$d_6$) δ(ppm):1.07(6H,d),2.46(3H,s),3.13(1H,m), 3.48(2H,s), 5.48(2H,s),6.39(1H,dd),6.64(1H,d),6.72-6.74(2H,m),6.85(1H,d),7.30(1H,d), 7.48(1H,d), 9.14(1H,s),9.68(1H,s). |
| 81 | ESI/MS(m/z):409(M+H)$^+$,407(M-H)$^-$. $^1$HNMR(DMSO-$d_6$) δ(ppm):1.08(6H,d),1.46(6H,s),2.46(3H,s),3.13 (1H,m), 5.48(1H,s),6.36(1H,dd),6.46(1H,d),6.63(1H,d),6.73(1H,d),6.88(1H,d),7.14 (1H,d),7.30(1H,d), 9.14(1H,s),9.66(1H,brs). |
| 82 | ESI/MS(m/z):367(M+H)$^+$,365(M-H)$^-$. $^1$HNMR(DMSO-$d_6$) δ(ppm):1.08(6H,d),2.36(3H,s),2.57(2H,t),3.13 (1H,m), 3.35(2H,t),5.41(2H,s),5.98(1H,d),6.37(1H,dd),6.55-6.58(2H,m),6.62(1H,d), 6.87(1H,d),7.09 (1H,d),9.10(1H,s). |

(continued)

| Example | Data |
|---|---|
| 83 | ESI/MS(m/z):381(M+H)+,379(M-H)-. 1HNMR(DMSO-$d_6$) δ(ppm):1.10(6H,d),2.26(3H,s),2.41(3H,s),3.12(1H,m), 5.21(2H,s),6.44(1H,dd),6.61(1H,d),6.93-7.01(2H,m),7.17(1H,d),7.50(1H,m), 9.17(1H,s),10.51(1H,s). |
| 84 | ESI/MS(m/z):395(M+H)+,393(M-H)-. 1HNMR(DMSO-$d_6$) δ(ppm):1.10(6H,d),2.26(3H,s),2.31(3H,s),3.12(1H,m), 3.37(1H,s),5.22(2H,s),6.45(1H,d),6.62(1H,d),6.91-7.00(3H,m),7.23(1H,d), 9.18(1H,s),9.83(1H,s). |
| 85 | ESI/MS(m/z):381(M+H)+,379(M-H)-. 1HNMR(DMSO-$d_6$) δ(ppm):1.08(6H,d),2.32(3H,s),2.46(3H,s),3.13(1H,m), 5.43(2H,s),6.21(1H,d),6.39(1H,s),6.64(1H,d),6.73(1H,d),6.84(1H,s), 7.26(1H,d),9.22(1H,s), 10.15(1H,s). |

[Table 391

| Example | Data |
|---|---|
| 86 | ESI/MS(m/z):395(M+H)+,393(M-H)-. 1HNMR(DMSO-$d_6$) δ(ppm):1.07(6H,d),2.32(3H,s),2.44(3H,s),3.13(1H,m), 3.48(2H,s),5.42(2H,s),6.21(1H,d),6.55(1H,s),6.63(1H,d),6.67(1H,d), 6.81(1H,s),7.45(1H,d), 9.18(1H,s),9.63(1H,s),12.59(1H,brs). |
| 87 | ESI/MS(m/z):409(M+H)+,407(M-H)-. 1HNMR(DMSO-$d_6$) δ(ppm):1.08(6H,d),2.33(3H,s),2.43(3H,s),3.18(1H,m), 3.49(2H,s),3.72(3H,s),5.49(2H,s),6.33(1H,dd),6.58(1H,s),6.67(1H,d), 6.81(1H,d),7.46(1H,d), 9.66(1H,s). |
| 88 | ESI/MS(m/z):413(M+H)+,411(M-H)-. 1HNMR(DMSO-$d_6$) δ(ppm):1.08(6H,d),2.32(3H,s),2.43(3H,s),3.13(1H,m), 5.23(1H,d),5.43(2H,s),6.18-6.22(1H,m),6.37(1H,s),6.62-6.68(2H,m), 6.82(1H,d),7.53(1H,d), 9.20(1H,s),11.34(1H,s). |
| 89 | ESI/MS(m/z):409(M+H)+,407(M-H)-. 1HNMR(DMSO-$d_6$) δ(ppm):1.07(6H,d),1.30(3H,d),2.31(3H,s),2.44(3H,s), 3.13(1H,m),3.73(1H,q),5.42(2H,s),6.21(1H,dd),6.53(1H,s),6.62(1H,d), 6.66(1H,d),6.81(1H,d), 7.38(1H,d),9.12(1H,s),9.60(1H,s),12.48(1H,brs). |
| 90 | ESI/MS(m/z):437(M+H)+,435(M-H)-. 1HNMR(DMSO-$d_6$) δ(ppm):0.97(3H,d),1.01(3H,d),1.07(6H,d),2.31(3H,s), 2.44(3H,s),3.13(1H,m),3.37(1H,d),5.42(2H,s),6.21(1H,dd),6.50(1H,s), 6.62(1H,d),6.66(1H,d), 6.82(1H,d),7.33(1H,d),9.13(1H,s),9.58(1H,s). |
| 91 | ESI/MS(m/z):485(M+H)+,483(M-H)-. 1HNMR(DMSO-$d_6$) δ(ppm):1.07(6H,d),2.29(3H,s),2.42(3H,s), 3.09-3.15 (3H,m),5.40(2H,s),6.19(1H,dd),6.34(1H,s),6.61-6.65(2H,m),6.80(1H,d), 7.18(1H,m), 7.25-7.28(5H,m),9.12(1H,s),9.55(1H,s). |
| 92 | ESI/MS(m/z):423(M+H)+,421(M-H)-. 1HNMR(DMSO-$d_6$) δ(ppm):1.10(6H,t),1.50(6Hs),2.32(3H,s),2.47(3H,s), 3.15(1H,m),5.44(2H,s),6.23(1H,dd),6.30(1H,s),6.63(1H,d),6.69(1H,d), 6.85(1H,d),7.09(1H,d), 9.11(1H,s),9.20(1H,s),12.71(1H,brs). |
| 93 | ESI/MS(m/z):409(M+H)+,407(M-H)-. 1HNMR(DMSO-$d_6$) δ(ppm):1.06(6H,d),2.30(3H,s),2.42(3H,s),2.53(2H,t), 2.66(2H,t),3.12(1H,m),5.40(2H,s),6.21(1H,d),6.56(1H,s),6.60-6.64(2H,m), 6.79(1H,s),7.38(1H,d),9.11(1H,s),9.42(1H,s). |
| 94 | ESI/MS(m/z):449(M+H)+,447(M-H)-. 1HNMR(DMSO-$d_6$) δ(ppm):1.07(6H,d),1.26(3H,t),2.44(3H,s),2.65(2H,q), 3.13(1H,m),3.30(2H,s),5.44(2H,s),6.19(1H,dd),6.57(1H,s),6.62(1H,d), 6.67(1H,d),6.80(1H,d), 7.56(1H,d),9.20(1H,s). |
| 95 | ESI/MS(m/z):449(M+H)+,447(M-H)-. 1HNMR(DMSO-$d_6$) δ(ppm):1.03(6H,d),2.42(3H,s),3.11(1H,m), 3.50(2H,s), 5.57(2H,s),6.17(1H,dd),6.61(1H,dd),6.62(1H,d),6.13(1H,d),6.96(1H,d), 7.53(1H,s),7,66(1H,d),9.25(1H,s),9.94(1H,s). |
| 96 | ESI/MS(m/z):422(M+H)+,420(M-H)-. 1HNMR(DMSO-$d_6$) δ(ppm):2.06(6H,s),3.50(2H,s),5.45(2H,s),6.50(2H,s), 6.82(2H,d),7.33(1H,d),7.50(1H,d),8.18(1H,s),9.73(1H,s),12.64(1H,brs). |

[Table 40]

| Example | Data |
|---|---|
| 97 | ESI/MS(m/z):429$(M+H)^+$,427$(M-H)^-$. $^1$HNMR(DMSO-$d_6$) δ(ppm):2.36(3H,s),2.49(3H,s),3.48(2H,s),5.51 (2H,s), 6.53-6.55(2H,m),6.68(1H,d),6.82-6.86(2H,m),7.24-7.28(1H,m),7.34-7.44 (5H,m),9.44(1H,s), 9.61(1H,s). |
| 98 | ESI/MS(m/z):416$(M+H)^+$,414$(M-H)^-$. $^1$HNMR(DMSO-$d_6$) δ(ppm):2.37(3H,s),2.49(3H,s),5.53(2H,s),6.36 (1H,s), 6.52-6.56(1H,m),6.72(1H,d),6.88(1H,dd),6.93(1H,d),7.39-7.42(1H,m), 7.49-7.51(1H,m), 7.84-7.87(1H,m),8.47(1H,dd),8.63(1H,d),9.76(1H,s), 10.11(1H,s). |
| 99 | ESI/MS(m/z):430$(M+H)^+$. $^1$HNMR(DMSO-$d_6$) δ(ppm):2.35(3H,s),2.47(3H,s),3.42(2H,s),5.51(2H,s), 6.55(2H,m),6.68(1H,d),6.81(2H,m),7.38(1H,m),1.45(1H,d),7.83(1H,m), 8.46(1H,m),8.60(1H,m),9.69 (1H,s),9.86(1H,s). |
| 100 | ESI/MS(m/z):461$(M+H)^+$,459$(M-H)^-$. $^1$HNMR(DMSO-$d_6$) δ(ppm):2.27(3H,s),2.39(3H,s),3.46(2H,s),3.75 (2H,s), 5.37(2H,s),6.31(1H,s),6.51(1H,s),6.63-6.68(3H,m),6.69(2H,t),7.02(2H,t), 7.09-7.13(2H,m),7.43 (1H,d),9.23(1H,s),9.60(1H,s),12.56(1H,s). |
| 101 | ESI/MS(m/z):43$(M+H)^+$,441$(M-H)^-$. $^1$HNMR(DMSO-$d_6$) δ(ppm):2.27(3H,s),2.41(3H,s),2.74(4H,m),5.3 (2H,s), 6.29-6.32(2H,m),6.62(1H,d),6.67-6.10(2H,m),7.09-1.15(3H,m),7.20-7.23 (2H,m),7.50(1H,d), 9.24(1H,s),10.04(1H,s). |
| 102 | ESI/MS(m/z):457$(M+H)^+$,455$(M-H)^-$. $^1$HNMR(DMSO-$d_6$) δ(ppm):2.26(3H,s),2.40(3H,s),2.74(4H,m), 3.44(2H,s), 5.37(2H,s),6.30(1H,m),6.52(1H,s),6.61(1H,s),6.66-6.68(2H,m),7.09-7.16 (3H,m),7.20-7.24 (2H,m),7.44(1H,d),9.22(1H,s),9.80(1H,s). |
| 103 | ESI/MS(m/z):353$(M+H)^+$,351$(M-H)^-$. $^1$HNMR(DMSO-$d_6$) δ(ppm):2.07(6H,s),2.51(3H,s),5.47(2H,s),6.51 (2H,s), 6.58(1H,d),6.79(1H,d),7.29(1H,d),7.36(1H,d),8.18(1H,s),10.27(1H,bis). |
| 104 | ESI/MS(m/z):367$(M+H)^+$,365$(M-H)^-$. $^1$HNMR(DMSO-$d_6$) δ(ppm):2.06(6H,s),2.51(3H,s),3.50(2H,s),5.45 (2H,s), 6.50(2H,s),6.74(1H,d),6.75(1H,d),7.34(1H,d),7.50(1H,d),8.18(1H,s), 9.73(1H,s),12.64(1H,brs). |
| 105 | ESI/MS(m/z):353$(M+H)^+$,351$(M-H)^-$. $^1$HNMR(DMSO-$d_6$) δ(ppm):1.10(6H,d),3.12(1H,m),5.24(2H,s), 6.43(1H,d), 6.66(1H,d),6.80(1H,dd),7.11(1H,d),7.39-7.46(3H,m),8.00(1H,d),9.26(1H,s), 10.53(1H,s). |
| 106 | ESI/MS(m/z):367$(M+H)^+$,365$(M-H)^-$. $^1$HNMR(DMSO-$d_6$) δ(ppm):1.11(6H,d),3.14(1H,m),3.34(2H,s), 5.23(2H,s), 6.40(1H,d),6.67(1H,d),6.79(1H,dd),7.16-7.28(3H,m),7.40-7.43(2H,m), 7.88(1H,d),9.25(1H, s),9.96(1H,s). |
| 107 | ESI/MS(m/z):367$(M+H)^+$,365$(M-H)^-$. $^1$HNMR(DMSO-$d_6$) δ(ppm):1.09(6H,d),2.35(3H,s),3.12(1H,m), 5.22(2H,s), 6.23(1H,s),6.52(1H,d),6.64(1H,d),6.95(1H,d),7.33(2H,s),7.88(1H,s), 9.14(1H,s),10.39(1H, s). |

[Table 41]

| Example | Data |
|---|---|
| 108 | ESI/MS(m/z):367$(M+H)^+$,365$(M-H)^-$. $^1$HNMR(DMSO-$d_6$) δ(ppm):1.06(6H,d),2.45(3H,s),3.12(1H,m), 5.46(2H,s), 6.36(1H,dd),6.45(1H,d),6.63(1H,d),6.82(1H,d),7.11(1H,s),7.35(1H,d),7.84 (1H,d),9.20(1H, s),10.42(1H,s). |
| 109 | ESI/MS(m/z):381$(M+H)^+$,379$(M-H)^-$. $^1$HNMR(DMSO-$d_6$) δ(ppm):1.05(6H,d),2.44(3H,s),3.12(1H,m), 3.31(2H,s), 5.45(2H,s),6.36-6.41(2H,m),6.63(1H,d),6.83(1H,d),7.31(1H,s),7.74(1H,d), 9.20(1H,s),9.86 (1H,s). |
| 110 | ESI/MS(m/z):395$(M+H)^+$,393$(M-H)^-$. $^1$HNMR(DMSO-$d_6$) δ(ppm):1.09-1.12(9H,m),2.22(3H,s),2.44(1H, dd),3.21 (1H,dd),3.35(1H,m),3.41(2H,s),3.60-3.69(1H,m),4.33(2H,dd),6.69(1H,d), 6.76-6.82(2H,m), 6.90(1H,dd),7.05(1H,d),9.09(1H,s),9.59(1H,s),12.51(1H,s). |
| 111 | ESI/MS(m/z):354$(M+H)^+$,352 $(M-H)^-$ $^1$HNMR(DMSO-$d_6$) δ(ppm):1.10(6H,d),3.13(1H,m),5.49(2H,s),6.66 (1H,d), 6.84(1H,dd), 7.14(1H,d),7.35(1H,t),7.46(1H,d),7.53(1H,d),8.18(1H,s),9.28 (1H,s),10.82(1H,s). |

(continued)

| Example | Data |
|---|---|
| 112 | ESI/MS(m/z):368(M+H)+,366(M-H)-. 1HNMR(DMSO-$d_6$) δ(ppm):1.10(6H,d),3.12(1H,m),3.50(2H,s), 5.48(2H,s), 6.66(1H,d),6.82(1H,dd),7.11(1H,d),7.30(1H,t),7.41(1H,d),7.68(1H,d), 8.26(1H,s),9.27(1H, brs),10.19(1H,s). |
| 113 | ESI/MS(m/z):367(M+H)+,365(M-H)-. 1HNMR(DMSO-$d_6$) δ(ppm):1.14(6H,d),3.15(1H,m),3.44(2H,s), 3.90(2H,s), 6.64(1H,d),6.84(1H,dd),6.90(1H,t),7.06(1H,d),7.11(1H,d),7.24(1H,d), 7.36(1H,d),8.98(1H, s),9.89(1H,s),10.33(1H,s). |

Example 114

N-[1-(4-hydroxy-3-trifluoromethylbenzyl)-2,7-dimethyl-1H-in dol-4-yl]oxamic acid

Ethyl N-[2,7-dimethyl-1-(3-trifluoromethyl-4-triisopropylsilanylo xybenzyl)-1H-indol-4-yl]oxamate (270 mg) was dissolved in tetrahydrofuran (3.0 mL), a 1 mol/L solution of tetrabutylammonium fluoride in tetrahydrofuran (503 μL) was added, and the mixture was stirred at room temperature for 30 minutes. The reaction mixture was diluted with ethyl acetate, washed with water, and an aqueous saturated sodium chloride solution, and dried with anhydrous sodium sulfate, and the solvent was concentrated under reduced pressure. The resulting residue was dissolved in ethanol (3.0 mL), an aqueous sodium hydroxide solution (893 μL) was added, and the mixture was stirred at room temperature for 1 hour. The reaction mixture was concentrated under reduced pressure, and the resulting residue was dissolved in water. This was neutralized with 1 mol/L hydrochloric acid, followed by extraction with ethyl acetate. The organic layer was washed with an aqueous saturated sodium bicarbonate solution, and an aqueous saturated sodium chloride solution, and dried with anhydrous sodium sulfate, and the solvent was concentrated under reduced pressure. The resulting residue was crystallized with diethyl ether/n-hexane to obtain the title compound (153 mg). ESI/MS(m/z): 407(M+H)+, 405(M-H)-.

$^1$H NMR(DMSO-$d_6$) δ (ppm): 2.33(3H,s), 2.45(3H,s), 5.54(2H,s), 6.43(1H,s), 6.75(1H,d), 6.77(1H,d), 6.96(1H,s), 7.09 (1H,d), 7.26(1H,d), 10.22(1H,s), 10.56(1H,s), 14.12(1H,brs).

Compounds were synthesized according to the following reaction formula referring to the method of Example 114. Synthesized compounds are shown in Table 42, and data are shown in Table 43 and Table 44.

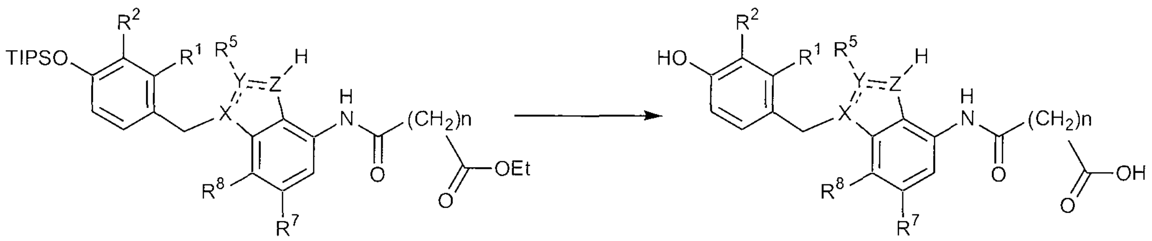

[Table 42]

| Example | $R^1$ | $R^2$ | $R^5$ | $R^7$ | $R^8$ | -X-Y-Z- | n |
|---|---|---|---|---|---|---|---|
| 115 | -CH=CH-CH=C H- | | Me | H | Me | -N-C=C- | 1 |
| 116 | H | *n*-Pr | Me | H | Me | -N-C=C- | 0 |
| 117 | H | *n*-Pr | Me | H | Me | -N-C=C- | 1 |
| 118 | H | *i*-Pr | Et | H | H | -N-C=C- | 0 |
| 119 | H | *i*-Pr | $CF_3$ | H | H | -N-C=C- | 0 |
| 120 | H | *i*-Pr | H | Br | H | -N-C=C- | 0 |
| 121 | H | *i*-Pr | H | H | Cl | -N-C=C- | 0 |
| 122 | H | *s*-Bu | Me | H | Me | -N-C=C- | 0 |

(continued)

| Example | $R^1$ | $R^2$ | $R^5$ | $R^7$ | $R^8$ | -X-Y-Z- | n |
|---|---|---|---|---|---|---|---|
| 123 | H | *s*-Bu | Me | H | Me | -N-C=C- | 1 |
| 124 | H | c-Pen | H | H | Me | -N-C=C- | 1 |
| 125 | H | 4-F-Bn | Me | H | Me | -N-C=C- | 0 |
| 126 | H | OEt | Me | H | Me | -N-C=C- | 0 |
| 127 | H | OEt | Me | H | Me | -N-C=C- | 1 |
| 128 | H | *i*-Pr | Me | H | Me | -N-CH-CH- | 0 |

[Table 43]

| Example | Data |
|---|---|
| 115 | ESI/MS(m/z):403$(M+H)^+$,401$(M-H)^-$. $^1$HNMR(DMSO-$d_6$) δ(ppm):2.26(3H,s),2.31(3H,s),3.49(2H,s), 5.82-5.92 (3H,m),6.60-6.68(3H,m),7.47(1H,d),7.55(1H,m),1.64(1H,m),8.15(1H,d), 8.21(1H,d),9.74(1H, brs),10.08(1H,s). |
| 116 | ESI/MS(m/z):381$(M+H)^+$,379$(M-H)^-$. $^1$HNMR(DMSO-$d_6$) δ(ppm):0.82(3H,t),1.45(2H,m),2.31(3H,s),2.39 (2H,t),2.45 (3H,s),5.42(2H,s),5.75(1H,s),6.32(1H,dd),6.37(1H,s),6.62-6.68(2H,m),6.71 (1H,d),7.29(1H, d),9.16(1H,s),10.13(1H,s). |
| 117 | ESI/MS(m/z):395$(M+H)^+$,393$(M-H)^-$. $^1$HNMR(DMSO-$d_6$) δ(ppm):0.81(3H,t),1.44(2H,m),2.31(3H,s),2.38 (2H,t),2.43 (3H,s),3.46(2H,s),5.41(2H,s),6.31(1H,brd),6.54(1H,s),6.61(1H,brs),6.65 (1H,d),6.66(1H,d), 7.45(1H,d),9.15(1H,s),9.73(1H,brs). |
| 118 | ESI/MS(m/z):381(M+H)+,379$(M-H)^-$. 1HNMR(DMSO-d6) ■ ■ (ppm):1.10(6H,t),1.27(3H,t),2.74(2H,q), 3.15(1H,m), 5.28(2H,s),6.42(1H,s),6.51(1H,dd),6.64(1H,d),6.95(1H,d),7.03(1H,t),7.25 (1H,d),7.41(1H, d),9.13(1H,s),10.21(1H,s). |
| 119 | ESI/MS(m/z):421(M+H)+,419$(M-H)^-$. 1HNMR(DMSO-d6) ■ ■ (ppm):1.07(6H,d),3.12(1H,m),5.43(2H,s), 6.54(1H,d), 6.64(1H,d),6.97(1H,d),7.30(1H,t),7.33(1H,s),7.37(1H,d),7.53(1H,d),9.28 (1H,s),10.70(1H, s). |
| 120 | ESI/MS(m/z):432$(M+H)^+$,430$(M-H)^-$. 1HNMR(DMSO-d6) ■ ■ (ppm):1.09(6H,d),3.14(1H,m),5.25(2H,s), 6.61(1H,d), 6.67(1H,d),6.79(1H,dd),7.14(1H,s),7.45(1H,d),7.62(2H,brs),9.27(1H,s),10.48 (1H,s). |
| 121 | ESI/MS(m/z):383$(M+H)^+$,381$(M-H)^-$. 1HNMR(DMSO-d6) ■ ■ (ppm):1.09-1.12(9H,m),2.22(3H,s),2.44 (1H,dd), 3.21(1H,dd),3.35(1H,m),3.60-3.69(1H,m),4.33(2H,dd),6.69(1H,d),6.76-6.82 (2H,m),6.90(1II, dd),7.05(1H,d),9.09(1H,s),9.16(1H,s),10.00(1H,s). |
| 122 | ESI/MS(m/z):395(M+H)+,393$(M-H)^-$. 1HNMR(DMSO-d6) ■ ■ (ppm):0.71(3H,t),1.04(3H,d),1.44(2H,m), 2.32(3H,s), 2.45(3H,s),2.91(1H,m),5.43(2H,s),6.23(1H,dd),6.38(1H,s),6.64(1H,d), 6.68-6.76(2H,m), 7.25(1H,d),9.13(1H,s),10.13(1H,s). |
| 123 | ESI/MS(m/z):409$(M+H)^+$,407$(M-H)^-$. 1HNMR(DMSO-d6) ■ ■ (ppm):0.71(3H,t),1.03(3H,d),1.44-1.54 (2H,m),2.32 (3H,s),2.43(3H,s),2.91(1H,m),3.47(2H,s),5.42(2H,s),6.23(1H,dd),6.55(1H,s), 6.63(1H,d), 6.66(1H,d),6.72(1H,d),7.44(1H,d),9.13(1H,s),9.67(1H,brs). |
| 124 | ESI/MS(m/z):407$(M+H)^+$,405$(M-H)^-$. 1HNMR(DMSO-d6) ■ ■ (ppm):1.32-1.45(2H,m),1.45-1.70(4H,m), 1.75-1.90 (2H,m),2.45(3H,s),3.10-3.46(1H,m),3.49(2H,s),5.47(2H,s),6.40(1H,brd),6.63 (1H,d), 6.68-6.75(2H,m),6.81(1H,brs),7.32(1H,d),7.50(1H,d),9.19(1H,s),9.71 (1H,brs). |
| 125 | ESI/MS(m/z):447$(M+H)^+$,445$(M-H)^-$. 1HNMR(DMSO-d6) ■ ■ (ppm):2.27(3H,s),2.41(3H,s),3.75(2H,s), 5.39(2H,s), 6.34-6.36(2H,m),6.62(1H,d),6.69(2H,t),7.01(2H,t),7.08-7.12(2H,m),7.25 (1H,d),9.28(1H,s), 10.02(1H,s). |

[Table 44]

| Example | Data |
|---|---|
| 126 | ESI/MS(m/z):383(M+H)$^+$,381(M-H)$^-$. $^1$HNMR(DMSO-$d_6$) δ(ppm):1.25(3H,t),2.32(3H,s),2.46(3H,s),3.88 (2H,q),5.43 (2H,s),5.75(1H,s),6.01(1H,dd),6.38(1H,s),6.54(1H,d),6.65(1H,d),6.72(1H,d), 7.24(1H,d), 8.81(1H,s),10.15(1H,s). |
| 127 | ESI/MS(m/z):397(M+H)$^+$,395(M-H)$^-$. $^1$HNMR(DMSO-$d_6$) δ(ppm):1.25(3H,t),2.31(3H,s),2.43(3H,s),3.46 (2H,s),3.87 (2H,q),5.42(2H,s),6.00(1H,brd),6.50-6.58(2H,m),6.60-6.10(2H,m),7.45(1H,d), 8.80(1H,s), 9.72(1H,brs). |
| 128 | ESI/MS(m/z):387(M+H)$^+$,385(M-H)$^-$. $^1$HNMR(DMSO-$d_6$) δ(ppm):1.11(6H,d),3.14(1H,m),5.64(2H,s), 6.64-6.69 (3H,m),7.01(1H,s),7.13(1H,d),7.44(1H,d),7.51(1H,d),10.34(1H,s),10.43 (1H,s). |

Example 129

N-(1-{3-[(4-fluorophenyl)hydroxymethyl]-4-hydroxybenzyl}-2, 7-dimethyl-1H-indol-4-yl)malonamic acid

Ethyl N-(1-{3-[(4-fluorophenyl)hydroxymethyl]-4-hydroxybenzyl}-2, 7-dimethyl-1H-indol-4-yl)malonamate (2.7 g) was dissolved in tetrahydrofuran (5.0 mL), 0.1 mol/L aqueous sodium hydroxide solution (134.3 mL) was added, and the mixture was stirred at room temperature for 5 hours. 1 mol/L hydrochloric acid was added to be acidic at 0 °C, followed by extraction with ethyl acetate. The organic layer was washed with water and an aqueous saturated sodium chloride solution, and dried with anhydrous sodium sulfate, and the solvent was concentrated under reduced pressure. The resulting residue was crystallized with n-hexane/diethyl ether to obtain the title compound (2.0 g).
ESI/MS(m/z): 477(M+H)$^+$, 475(M-H)$^-$.
$^1$H NMR(DMSO-$d_6$) δ (ppm): 2.32(3H,s), 2.43(3H,s), 3.49(2H,s), 5.44(2H,s), 5.68(1H,d), 5.89(1H,d), 6.33(1H,dd), 6.56 (1H,s), 6.63(1H,d), 6.67(1H,d), 7.06(1H,t), 7.12(1H,d), 7.26(2H,dd), 7.46(1H,d), 9.39(1H,brs), 9.66(1H,s).

Example 130

(+)-N-(1-{3-[(4-fluorophenyl)hydroxymethyl]-4-hydroxybenzyl }-2,7-dimethyl-1H-indol-4-yl)malonamic acid

According to the same manner as that of Example 129 using ethyl (+)-N-(1-{3-[(4-fluorophenyl)hydroxymethyl]-4-hydroxybenzyl }-2,7-dimethyl-1H-indol-4-yl)malonamate in place of ethyl N-(1-{3-[(4-fluorophenyl)hydroxymethyl]-4-hydroxybenzyl}-2, 7-dimethyl-1H-indol-4-yl)malonamate, a reaction was performed to obtain the title compound.
[α]$2^3{}_D$: +9.5° (c = 1.0, methanol)
ESI/MS(m/z): 477(M+H)$^+$, 475(M-H)$^-$.
$^1$H NMR(DMSO-$d_6$) δ (ppm): 2.32(3H,s), 2.43(3H,s), 3.49(2H,s), 5.44(2H,s), 5.68(1H,d), 5.89(1H,d), 6.33(1H,dd), 6.56 (1H,s), 6.63(1H,d), 6.67(1H,d), 7.06(1H,t), 7.12(1H,d), 7.26(2H,dd), 7.46(1H,d), 9.39(1H,brs), 9.66(1H,s).

Example 131

(-)-N-(1-{3-[(4-fluorophenyl)hydroxymethyl]-4-hydroxybenzyl }-2,7-dimethyl-1H-indol-4-yl)malonamic acid

According to the same manner as that of Example 129 using ethyl (-)-N-(1-{3-[(4-fluorophenyl)hydroxymethyl]-4-hydroxybenzyl }-2,7-dimethyl-1H-indol-4-yl)malonamate in place of ethyl N-(1-{3-[(4-fluorophenyl)hydroxymethyl]-4-hydroxybenzyl}-2, 7-dimethyl-1H-indol-4-yl)malonamate, a reaction was performed to obtain the title compound.
[α]$^{23}{}_D$: -7.9° (c = 1.0, methanol)
ESI/MS(m/z): 477(M+H)$^+$, 475(M-H)$^-$.
$^1$H NMR(DMSO-$d_6$) δ (ppm): 2.32(3H,s), 2.43(3H,s), 3.49(2H,s), 5.44(2H,s), 5.68(1H,d), 5.89(1H,d), 6.33(1H,dd), 6.56 (1H,s), 6.63(1H,d), 6.67(1H,d), 7.06(1H,t), 7.12(1H,d), 7.26(2H,dd), 7.46(1H,d), 9.39(1H,brs), 9.66(1H,s).

Example 132

Ethyl N-{1-[3-(4-fluorobenzoyl)-4-hydroxybenzyl]-2,7-dimethyl-1H-indol-4-yl}oxamate

[5-(4-Amino-2,7-dimethyl-1H-indol-1-ylmethyl)-2-hydro xyphenyl]-(4-fluorophenyl)methanone (135 mg) was dissolved in diethyl oxalate (1.4 g), and the mixture was stirred at 100°C for 3 hours. The reaction mixture was returned to room temperature, and concentrated under reduced pressure. The resulting residue was purified by flash chromatography (silica gel, developing solvent: n-hexane to n-hexane/ethyl acetate = 2/1) to obtain the title compound (95.4 mg). ESI/MS(m/z): 489$(M+H)^+$, 487$(M-H)^-$.
$^1$H NMR($CDCl_3$) δ (ppm): 1.47(3H,t), 2.29(3H,s), 2.47(3H,s), 4.46(2H,q), 5.44(2H,s), 6.22(1H,d), 6.44(1H,d), 6.84-6.91 (3H,m), 7.03(1H,d), 7.18-7.24(4H,m), 7.84(1H,d), 9.02(1H, brs), 11.79(1H,s)

Example 133

N-[1-(4-hydroxy-3-isopropylbenzoyl)-7-methyl-1H-indol-4-yl] malonamic acid

Benzyl N-[1-(4-hydroxy-3-isopropylbenzoyl)-7-methyl-1H-indol-4-yl] malonamate (100 mg) was dissolved in tetrahydrofuran (mL), 10% palladium carbon (20 mg) was added, and the mixture was stirred at room temperature for 2 hours in the hydrogen atmosphere. The catalyst was filtered, and the filtrate was concentrated under reduced pressure. The resulting residue was crystallized with n-hexane/ethyl acetate to obtain the title compound (87 mg). ESI/MS (m/z) : 395$(M+H)^+$, 393$(M-H)^-$.
$^1$H NMR(DMSO-$d_6$) δ (ppm): 1.20(6H,s), 2.29(3H,s), 3.36(1H,m), 3.49(2H,s), 6.89-6.91(1H,m), 7.00(1H,d), 7.07(1H,d), 7.41(1H,d), 7.62-7.72(3H,m), 9.95(1H,s), 10.51(1H,s).

Example 134

[1-(4-Hydroxy-3-isopropylbenzyl)-1H-indol-4-yl]carboxylic acid

According to the same manner as that of Example 133 using [1-(4-benzyloxy-3-isopropylbenzyl)-1H-indol-4-yl]carboxylic acid in place of benzyl
N-[1-(4-hydroxy-3-isopropylbenzoyl)-7-methyl-1H-indol-4-yl] malonamate, a reaction was performed to obtain the title compound.
ESI/MS(m/z): 310$(M+H)^+$.
$^1$H NMR(DMSO-$d_6$) δ (ppm): 1.10(6H,d), 3.13(1H,m), 5.31(2H,s), 6.67(1H,d), 6.80(1H,dd), 6.97(1H,d), 7.11(1H,d), 7.18 (1H,dd), 7.57(1H,d), 7.70(1H,d), 7.75(1H,d), 9.21(1H,brs).

Example 135

N-[1-(4-acetoxy-3-isopropylbenzyl)-2,7-dimethyl-1H-indol-4-yl]malonamic acid

N-[1-(4-hydroxy-3-isopropylbenzyl)-2,7-dimethyl-1H-in dol-4-yl]malonamic acid (100 mg) was suspended in acetic anhydride (2.0 mL), pyridine (0.2 mL) was added, and the mixture was stirred at room temperature for 30 minutes. The reaction mixture was concentrated under reduced pressure, and the resulting residue was purified by preparative thin layer chromatography (silica gel, developing solvent: chloroform/methanol/acetic acid = 80/20/1) to obtain the title compound (15 mg).
ESI/MS(m/z): 437($[M+H]^+$), 435($[M-H]^-$).
$^1$H NMR(DMSO-$d_6$) δ (ppm): 1.05(6H,d), 2.26(3H,s), 2.33(3H,s), 2.42(3H,s), 2.92(1H,m), 3.46(2H,s), 5.55(2H,s), 6.43 (1H,dd), 6.58(1H,s), 6.68(1H,d), 6.90(1H,d), 7.00(1H,d), 7.45(1H,d), 9.71(1H,s), 12.5(1H,brs).

Example 136

Sodium N-[1-(4-hydroxy-3-isopropylbenzyl)-2-methyl-1H-indol-4-yl]o xamate

N-[1-(4-hydroxy-3-isopropylbenzyl)-2-methyl-1H-indol-4-yl]oxamic acid (103 mg) was suspended in water (12.0 mL)/ethanol (8.0 mL), a 1 mol/L aqueous sodium hydroxide solution (281 μL) was added, and the mixture was stirred at room temperature for 1 hour. The reaction mixture was lyophilized to obtain the title compound (106 mg). $^1$H NMR (DMSO-$d_6$) δ (ppm): 1.09(6H,d), 2.38(3H,s), 3.13(1H,m), 5.23(2H,s), 6.26(1H,s), 6.51(1H,d), 6.64(1H,d), 6.97-6.98(2H, m), 7.11(1H,d), 7.76(1H,d), 9.13(1H,brs), 10.09(1H,s).

Compounds were synthesized according to the following reaction formula referring to the method of Example 136.

Synthesized compounds and data are shown in Table 45.

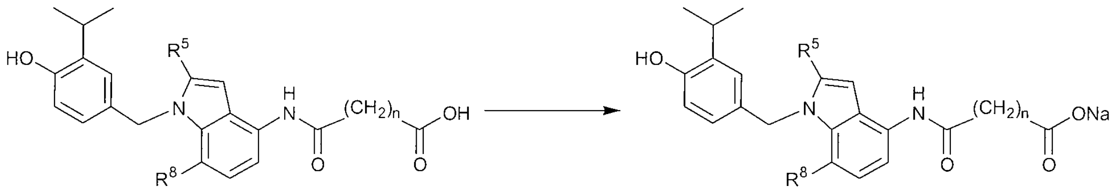

[Table 45]

| Example | $R^5$ | $R^8$ | n | $^1$HNMR (DMSO-$d_6$) δ(ppm) |
|---|---|---|---|---|
| 137 | Me | H | 1 | 1.09(6H,d),2.36(3H,s),2.88(2H,s),3.13(1H,m),5.22(2H,s), 6.39(1H,s),6.50(1H, dd),6.64(1H,d),6.91(1H,dd),6.95(1H,d), 7.03(1H,d),7.78(1H,d),9.16(1H,brs). |
| 138 | $CF_3$ | H | 0 | 1.07(6H,d),3.12(1H,m),5.41(2H,s),6.51(1H,d),6.64(1H,dd), 6.97(1H,s),7.23-7.26 (3H,m),7.77(1H,brs),9.31(1H,brs), 10.30(1H,brs). |
| 139 | H | Me | 0 | 1.07(6H,d),2.46(3H,s),3.13(1H,m),5.49(2H,s),6.40(1H,dd), 6.46(1H,d),6.65(1H, d),6.74(1H,d),6.86(1H,d),7.33(1H,d), 7.66(1H,d),10.08(1H,s). |
| 140 | H | Me | 1 | 1.07(6H,d),2.44(3H,s),2.98(2H,s),3.13(1H,m),5.47(2H,s), 6.38(1H,dd),6.64-6.69 (3H,m),6.85(1H,d),7.29(1H,d), 7.65(1H,d),9.20(1H,s). |
| 141 | Me | Me | 0 | 1.07(6H,d),2.33(3H,s),2.43(3H,s),3.13(1H,m),5.42(2H,s), 6.22(1H,dd),6.30(1H, s),6.63(1H,d),6.68(1H,d),6.81(1H,d), 7.63(1H,d),9.15(1H,s),10.05(1H,s). |
| 142 | Me | Me | 1 | 1.07(6H,d),2.30(3H,s),2.41(3H,s),2.90(2H,s),3.12(1H,m), 5.41(2H,s),6.21(1H, dd),6.45(1H,s),6.61-6.64(2H,m), 6.80(1H,s),7.64(1H,d),9.19(1H,s). |
| 143 | $CF_3$ | Me | 1 | 1.04(6H,d),2.41(3H,s),2.97(3H,s),2.97(2H,s),5.58(2H,s), 6.19(1H,dd),6.63(1H,d), 6.74(1H,d),6.93(1H,d),7.27(1H,s), 7.80(1H,d),9.32(1H,s). |

Example 144

Calcium N-[1-(4-hydroxy-3-isopropylbenzyl)-7-methyl-1H-indol-4-yl]m alonamate

N-[1-(4-hydroxy-3-isopropylbenzyl)-7-methyl-1H-indol-4-yl]malonamic acid (1140 mg) was suspended in ethanol (5.0 mL), a 1 mol/L aqueous sodium hydroxide solution (3.0 mL) was added, and the mixture was stirred at room temperature for 10 minutes. Then, a solution of calcium chloride (1332 mg) in water (10.0 mL) and water (15.0 mL) were added, and the mixture was stirred at room temperature for 1 hour. This was extracted with ethyl acetate, and the organic layer was dried with anhydrous sodium sulfate. The solvent was concentrated under reduced pressure to obtain the title compound (1120 mg).
$^1$H NMR(DMSO-$d_6$) δ (ppm): 1.05(6H,d), 2.43(3H,s), 3.03(2H,s), 3.11(1H,m), 5.46(2H,s), 6.38(1H,d), 6.63(1H,d), 6.64 (1H,s), 6.68(1H,d), 6.83(1H,s), 7.29(1H,d), 7.64(1H,d), 9.17(1H,s), 12.28(1H,s).

Example 145

N-[1-(4-hydroxy-3-isopropylbenzyl)-2,7-dimethyl-1H-indol-4-yl]malonamic acid L-lysine salt

N-[1-(4-hydroxy-3-isopropylbenzyl)-2,7-dimethyl-1H-in dol-4-yl]malonamic acid (1.02 g) and L-lysine (378 mg) were dissolved in ethanol (5.0mL) and water (25 mL). Lyophilization afforded the title compound (1.40 g).
$^1$H NMR(DMSO-$d_6$) δ (ppm): 1.07(6H,d), 1.37-1.73(6H,m), 2.31(3H,s), 2.42(3H,s), 2.75(2H,t), 2.98(2H,s), 3.12(1H,m),

3.20(1H,t), 5.41(2H,s), 6.20(1H,dd), 6.45(1H,s), 6.61-6.65(2H,m), 6.80(1H,s), 7.62(1H,d), 12.44(1H,s).

Test Example 1

Binding affinity of the synthesized present compound and the thyroid hormone receptor was obtained by using a protein having a ligand binding domain of human TRα or human TRβ expressed using Escherichia coli, and $^{125}$I-T3, and measuring an amount of a complex with $^{125}$I-T3 obtained by substituting $^{125}$I-T3 with the thyroid hormone receptor ligand from the formed complex.

Thyroid hormone receptor binding test

(1) Preparation of fused protein having ligand binding region of thyroid hormone receptor

Human TRα and human TRβ ligand binding domains were expressed in Escherichia coli as a fused protein (recombinant human TRα or recombinant human TRβ) having TRα and TRβ ligand binding domains at a C-terminus of His-Patch Thioredoxin using the His-Patch Thioredoxin fusing protein expressing system

(Invitorogen).

A plasmid expressing the fused protein was made by inserting, into a multiple cloning site of pThioHis, a cDNA encoding an amino acid sequence corresponding to 190$^{th}$ to 410$^{th}$ from a N-terminus of Accession No. CAA38749, and 244$^{th}$ to 461$^{st}$ from a N-terminal of Accession No. P10828 reported in GenBank as human TRα and human TRβ ligand binding domains using a procedure of gene recombination. A nucleotide sequence of a region corresponding to the fused protein was confirmed by DNA sequence analysis.

The fused protein was produced by culturing Escherichia coli (JM 109) transformed with the prepared plasmid, and adding IPTG during proliferation to induce expression. Escherichia coli by which the fused protein had been subjected to expression inducement were collected by a centrifugation method, and ground using ultrasound to prepare a ground cell liquid containing the fused protein. The ground cell liquid was centrifuged at 15000 rpm for about 30 minutes, and a solution containing the fused protein from the supernatant using a nickel chelating column was obtained. An NAP-10 column was replaced with a binding solution (20 mM, Tris-HCl, 0.15 M NaCl, 8% Glycerol, 0.1% BSA, 1 mM EDTA, 10 mM 2-melcaptethanol). The resulting binding solution containing the fused protein was frozen and stored at -70°C or lower.

(2) Binding affinity for thyroid hormone receptor

$^{125}$I-T3 (Perkin Elmer, Cat. No. NEX110X, AS62450) was added to the binding solution containing recombinant human TRα or recombinant human TRβ to 0.16 nM, and this was allowed to stand at room temperature for 1 to 3 hours to form a complex of recombinant human TRβ or recombinant human TRα and $^{125}$I-T3. This binding solution (60 μL) was added to all wells of a 96-well plate (MILLIPORE MultiScreen-HV, Cat. No. MANVN4550, Lot. No. F4SN86613). 90 μL of a dilution series solution made so that a test substance or non-labeled T3 became 1.67-fold a final concentration in the binding solution was added to the binding solution in which the above complex had been formed, to a total solution volume of each well of 150 μL. This mixed solution was incubated at 25°C for 2 to 3 hours, whereby formation of a complex with $^{125}$I-T3 was sufficiently inhibited by a test substance or non-labeled T3. 80 μL of SephadexG25 was added to each well of MultiScreen-HV (MILLIPORE, Cat. No. MANVN4550, Lot. No. F4SN86613), the binding solution was added to sufficiently swell, and this was centrifuged at 600xg for 1 minute to prepare a column of SephadexG25. 25 μL of the mixed solution (150 μL) with the test substance or non-labeled T3 added was taken, overlaid on the SephadexG25 column, and this was immediately centrifuged at 600xg for 1 minute. Again, 25 μL untreated binding solution was overlaid on the SephadexG25 column, and this was immediately centrifuged at 600xg for 1 minute. A separated solution containing a complex with $^{125}$I-T3 which had passed through the column by two times centrifugation was recovered into in Isoplate (PS) (Perkin Elmer, Cat. No. 1450-514). 200 μL of Optiphase Super Mix (Perkin Elmer, Cat. No. 1200-439) was added to each well containing the separated solution, and radioactivity was measured with 1450 MICROBETA TRILUX (Perkin Elmer). An amount of $^{125}$I-T3 converted from the radioactivity was adopted as an amount of a complex of recombinant human TRα or recombinant human TRβ and $^{125}$I-T3. As an amount of recombinant human TRα or recombinant human TRβ used in the present test, an amount by which proper radioactivity is obtained in a range where an addition amount of the receptor and an amount of the complex in the above test system, was used.

From a value obtained by subtracting remaining radioactivity when an excessive amount of T3 (0.256 μM) was added (rate of formation of complex with $^{125}$I-T3, 0%) as non-specific binding from radioactivity when a test substance or T3 was not added (rate of formation of complex with $^{125}$I-T3, 100%), a true total amount of a complex of recombinant human TRα or recombinant human TRβ and $^{125}$I-T3 was obtained by conversion as an approximate value. An amount

of a complex of recombinant human TRα or recombinant human TRβ and $^{125}$I-T3 remaining at each concentration of a test substance or T3 (remaining complex amount) was obtained by conversion from a value obtained by subtracting remaining radioactivity from the measured radioactivity. This was divided by the total complex amount to calculate a binding rate as the following equation:

$$\text{Binding rate} = \text{remaining complex amount}/\text{total complex amount}$$

A drug concentration ($IC_{50}$) showing a 50% inhibition rate was obtained by performing linear approximation using the binding rate, and a logarithmic value of each concentration of the test substance or T3, and making a concentration at a binding rate=0.5. For linear approximation, only each concentration of the test substance or T3 in a range of a binding rate of 0.1 to 0.9 was used. $IC_{50}$ of Example compounds is shown in Table 46.

[Table 46]

| Compound (Example) | TRα ($IC_{50}$,nM) | TRβ ($IC_{50}$,nM) | Compound (Example) | TRα ($IC_{50}$,nM) | TRβ ($IC_{50}$,nM) |
|---|---|---|---|---|---|
| 1 | 1500 | 220 | 93 | 650 | 260 |
| 8 | 370 | 72 | 95 | 98 | 5.0 |
| 15 | 490 | 60 | 96 | 5800 | 210 |
| 19 | 280 | 56 | 100 | 600 | 28 |
| 21 | 37 | 6.3 | 101 | 2200 | 390 |
| 26 | 3400 | 310 | 103 | 3200 | 860 |
| 32 | 980 | 350 | 105 | 970 | 260 |
| 41 | >10000 | 650 | 108 | 190 | 54 |
| 42 | 8200 | 150 | 109 | >10000 | 600 |
| 43 | 450 | 8.5 | 110 | >10000 | 610 |
| 56 | 480 | 84 | 111 | >10000 | 1400 |
| 57 | 3800 | 24 | 113 | >10000 | 320 |
| 63 | 1200 | 170 | 114 | 180 | 49 |
| 65 | >10000 | 470 | 115 | 3600 | 130 |
| 69 | >10000 | 430 | 116 | 52 | 9.2 |
| 72 | 230 | 40 | 117 | 640 | 36 |
| 73 | 5100 | 210 | 118 | 280 | 91 |
| 74 | 2700 | 200 | 119 | 57 | 15 |
| 75 | >10000 | 550 | 120 | 380 | 63 |
| 76 | 610 | 67 | 122 | 24 | 2.6 |
| 77 | >10000 | 570 | 123 | 170 | 9.4 |
| 78 | 230 | 36 | 125 | 66 | 8.7 |
| 79 | 5200 | 130 | 126 | 1900 | 320 |
| 82 | 1600 | 1100 | 128 | 320 | 47 |
| 83 | 85 | 27 | 129 | 260 | 7.5 |
| 85 | 24 | 3.1 | 130 | 5200 | 150 |
| 86 | 670 | 16 | 131 | 99 | 7.1 |
| 88 | 120 | 13 | | | |
| 89 | 870 | 120 | T3 | 3.2 | 2.9 |

The $IC_{50}$ value of T3 according to the present method was 3.2 nM and 2.9 nM, respectively, for recombinant human TRα and recombinant human TRβ. On the other hand, the present compound which was tested this time exhibited an $IC_{50}$ value of 20 nM to >10000 nM for human TRα, and an $IC_{50}$ value of 2 nM to 2000 nM for human TRβ, and it was found that the compound acts as a thyroid hormone receptor ligand. The present compounds which were tested this time all have higher affinity for TRβ than for TRα, and are expected to be advantageous from a viewpoint of side effect.

[Industrial Applicability]

Since the present compound has affinity for the thyroid hormone receptor, it can be used as the medicament as a thyroid hormone receptor ligand. Therefore, it is useful as an agent for preventing or treating a disease or a disorder, symptom of which is improved by cell functional regulation via thyroid hormone receptor, for example, hyperlipemia, obesity, hypothyroidism, hyperthyroidism, goiter, thyroid cancer, cardiac arrhythmia, congestive heart failure, diabetes, depression, osteoporosis, skin disorder, glaucoma, alopecia or the like. Since among the present compounds, some have high selectivity and affinity for TRβ of the thyroid hormone receptor, they are suitable to be used as a medicament as the thyroid hormone receptor ligand receptor having little side effect.

## Claims

**1.** A compound represented by the general formula (I):

[Chemical Formula 1]

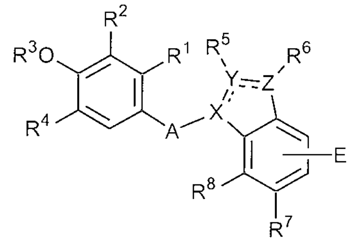

(I)

[wherein

[Chemical Formula 2] 
means a single bond or a double bond;
A means $-CH_2-$ or -CO-;
X, Y, and Z each independently means a nitrogen atom or a carbon atom (provided that one or two of X, Y, and Z mean a nitrogen atom and the rest means a carbon atom and, when Y and/or Z are a nitrogen atom, and form a double bond with one of the adjacent atoms, $R^5$ and/or $R^6$ are absent);
$R^1$ means a hydrogen atom or a C1-C6 alkyl group;
$R^2$ means a halogen atom, a C1-C6 alkyl group, a C2-C6 alkenyl group, a C3-C7 cycloalkyl group, a halo lower alkyl group, an alkanoyl group, an aryl group, a heteroaryl group, an aroyl group, an aralkyl group, a C1-C6 alkoxy group, an aryloxy group, an aralkyloxy group, $-(CH_2)_n-NR_9R^{10}$, $-CONR^9R^{10}$, $-NR^9COR^{11}$, $-S(O)_pR^{11}$, or $-SO_2NR^9R^{10}$, or means a 5- to 6-membered hydrocarbon ring which is formed by $R^1$ and $R^2$ together with the carbon atom to which they are bound (wherein n means an integer of 0 to 2, $R^9$ and $R^{10}$ each independently means a hydrogen atom, a C1-C6 alkyl group, a C2-C6 alkenyl group, a C3-C7 cycloalkyl group, an aryl group, a heteroaryl group, or an aralkyl group, or $R^9$ and $R^{10}$ mean a 5- to 6-membered heterocyclic ring which is formed by $R^9$ and $R^{10}$ together with the nitrogen atom to which they are bound, or alternatively, another nitrogen atom or oxygen atom, $R^{11}$ means a C1-C6 alkyl group, a C2-C6 alkenyl group, a C3-C7 cycloalkyl group, an aryl group, a heteroaryl group, or an aralkyl group, and p means an integer of 0 to 2);
$R^3$ means a hydrogen atom, a C1-C6 alkyl group, or an acyl group;
$R^4$ means a hydrogen atom or a C1-C6 alkyl group;
$R^5$ means a hydrogen atom, a C1-C6 alkyl group, a halo lower alkyl group, or a cyano group;
$R^6$ means a hydrogen atom or a C1-C6 alkyl group;
$R^7$ means a hydrogen atom, a halogen atom, or a C1-C6 alkyl group;
$R^8$ means a hydrogen atom, a halogen atom, or a C1-C6 alkyl group; and
E means any group selected from the group represented by the following formulae (a) to (f):

(a) $-NHCO-G-COR^{12}$,
(b) $-NH-G-COR^{12}$,

(c) -O-G-COR$^{12}$,
(d) -CONH-G-COR$^{12}$,
(e) -NHCO-G-tetrazolyl group, and
(f) -G-COR$^{12}$,

(wherein G means a single bond or a C1-C6 alkylene group, and $R^{12}$ means a hydroxy group or a C1-C6 alkoxy group)], or a prodrug thereof, or a pharmaceutically acceptable salt thereof.

**2.** The compound according to claim 1, wherein, in the general formula (I), $R^2$ is a halogen atom, a C1-C6 alkyl group, a C3-C7 cycloalkyl group, a halo lower alkyl group, an aryl group, a heteroaryl group, an aroyl group, an aralkyl group, a C1-C6 alkoxy group, or a 5- to 6-membered hydrocarbon ring which is formed by $R^1$ and $R^2$ together with the carbon atom to which they are bound, $R^5$ is a hydrogen atom, a C1-C3 alkyl group, or a halo lower alkyl group, and $R^7$ is a hydrogen atom or a halogen atom.

**3.** The compound according to claim 1, wherein A in the general formula (I) is $-CH_2-$.

**4.** The compound according to claim 1, wherein $R^1$, $R^3$, $R^4$, $R^6$, and $R^7$ in the general formula (I) are each a hydrogen atom.

**5.** The compound according to claim 1, wherein $R^2$ in the general formula (I) is an i-propyl group, a s-butyl group, a 4-fluorobenzyl group, or a 4-(fluorophenyl)hydroxymethyl group.

**6.** The compound according to claim 1, wherein $R^5$ in the general formula (I) is a methyl group or a trifluoromethyl group.

**7.** The compound according to claim 1, wherein $R^8$ in the general formula (I) is a methyl group.

**8.** The compound according to claim 1, wherein, in the general formula (I), E is -NHCO-G-COR$^{12}$, G is a single bond or $-CH_2-$, and $R^{12}$ is a hydroxy group or a C1-C3 alkoxy group.

**9.** The compound according to claim 1, wherein, in the general formula (I), one of X and Z is a nitrogen atom while the other is a carbon atom, and Y is a carbon atom.

**10.** The compound according to claim 1, which is represented by the general formula (II):

[Chemical Formula 3]

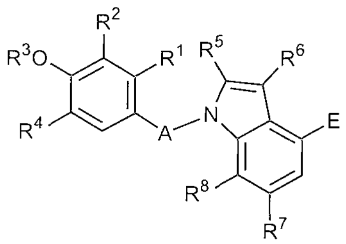

(I I)

[wherein all symbols are as defined in the general formula (I)].

**11.** The compound according to claim 10, wherein, in the general formula (II), A is $-CH_2-$, $R^1$, $R^3$, $R^4$, $R^6$, and $R^7$ are each a hydrogen atom, $R^2$ is an i-propyl group, a s-butyl group, a 4-fluorobenzyl group, or a 4-(fluorophenyl)hydroxybutyl group, $R^5$ is a methyl group or a trifluoromethyl group, $R^8$ is a methyl group, and E is -NHCO-G-COR$^{12}$ (wherein G is a single bond or $-CH_2-$, and $R^{12}$ is a hydroxy group or a C1-C3 alkoxy group).

**12.** A pharmaceutical composition comprising, as an active ingredient, a compound represented by the general formula (I) :

[Chemical Formula 4]

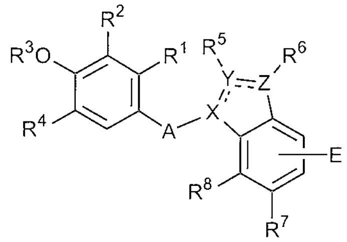

(I)

[wherein
[Chemical Formula 5]

means a single bond or a double bond;
A means $-CH_2-$ or -CO-;
X, Y, and Z each independently means a nitrogen atom or a carbon atom (provided that one or two of X, Y, and Z mean a nitrogen atom and the rest means a carbon atom and, when Y and/or Z are a nitrogen atom, and form a double bond with one of the adjacent atoms, $R^5$ and/or $R^6$ are absent);
$R^1$ means a hydrogen atom or a C1-C6 alkyl group;
$R^2$ means a halogen atom, a C1-C6 alkyl group, a C2-C6 alkenyl group, a C3-C7 cycloalkyl group, a halo lower alkyl group, an alkanoyl group, an aryl group, a heteroaryl group, an aroyl group, an aralkyl group, a C1-C6 alkoxy group, an aryloxy group, an aralkyloxy group, $-(CH_2)_n-NR^9R^{10}$, $-CONR^9R^{10}$, $-NR^9COR^{11}$, $-S(O)_pR^{11}$, or $-SO_2NR^9R^{10}$, or means a 5- to 6-membered hydrocarbon ring which is formed by $R^1$ and $R^2$ together with the carbon atom to which they are bound (wherein n means an integer of 0 to 2, $R^9$ and $R^{10}$ each independently means a hydrogen atom, a C1-C6 alkyl group, a C2-C6 alkenyl group, a C3-C7 cycloalkyl group, an aryl group, a heteroaryl group, or an aralkyl group, or $R^9$ and $R^{10}$ mean a 5- to 6-membered heterocyclic ring which is formed by $R^9$ and $R^{10}$ together with the nitrogen atom to which they are bound or alternatively, another nitrogen atom or oxygen atom, $R^{11}$ means a C1-C6 alkyl group, a C2-C6 alkenyl group, a C3-C7 cycloalkyl group, an aryl group, a heteroaryl group, or an aralkyl group, and p means an integer of 0 to 2);
$R^3$ means a hydrogen atom, a C1-C6 alkyl group, or an acyl group;
$R^4$ means a hydrogen atom or a C1-C6 alkyl group;
$R^5$ means a hydrogen atom, a C1-C6 alkyl group, a halo lower alkyl group, or a cyano group;
$R^6$ means a hydrogen atom or a C1-C6 alkyl group;
$R^7$ means a hydrogen atom, a halogen atom, or a C1-C6 alkyl group;
$R^8$ means a hydrogen atom, a halogen atom, or a C1-C6 alkyl group; and
E means any group selected from the group represented by the following formulae (a) to (f):

(a) $-NHCO-G-COR^{12}$,
(b) $-NH-G-COR^{12}$,
(c) $-O-G-COR^{12}$,
(d) $-CONH-G-COR^{12}$,
(e) -NHCO-G-tetrazolyl group, and
(f) $-G-COR^{12}$;
(wherein G means a single bond or a C1-C6 alkylene group, and $R^{12}$ means a hydroxy group or a C1-C6 alkoxy group)].

**13.** The pharmaceutical composition according to claim 12, wherein the compound as an active ingredient is such that, in the general formula (I), A is $-CH_2-$, $R^1$, $R^3$, $R^4$, $R^6$, and $R^7$ are each a hydrogen atom, $R^2$ is an i-propyl group, a s-butyl group, a 4-fluorobenzyl group, or a 4-(fluorophenyl)hydroxymethyl group, $R^5$ is a methyl group or a trifluor-

omethyl group, $R^8$ is a methyl group, and E is -NHCO-G-OCR$^{12}$ (wherein G is a single bond or -$CH_2$-, and $R^{12}$ is a hydroxy group or a C1-C3 alkoxy group.

**14.** The pharmaceutical composition according to claim 12 or claim 13, which is for use as a preventing or treating agent of a disease or a disorder, symptom of which is improved by cell functional regulation via a thyroid hormone receptor.

**15.** The pharmaceutical composition according to claim 14, wherein the disease or the disorder, symptom of which is improved by cell functional regulation via a thyroid hormone receptor, is hyperlipemia, obesity, hypothyroidism, hyperthyroidism, goiter, thyroid cancer, cardiac arrhythmia, congestive heart failure, diabetes, depression, osteoporosis, skin disorder, glaucoma, or alopecia.

**16.** Use of a compound represented by the following general formula (I) for the manufacture of a medicament as a thyroid hormone receptor ligand:

[Chemical Formula 6]

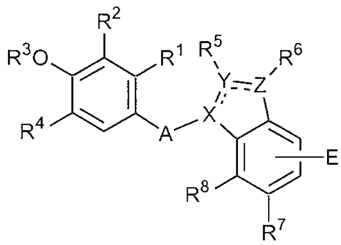

(I)

[wherein
[Chemical Formula 7]

 means a single bond or a double bond;
A means -$CH_2$- or -CO-;
X, Y, and Z each independently means a nitrogen atom or a carbon atom (provided that one or two of X, Y, and Z mean a nitrogen atom and the rest means a carbon atom and, when Y and/or Z are a nitrogen atom, and form a double bond with one of the adjacent atoms, $R^5$ and/or $R^6$ are absent);
$R^1$ means a hydrogen atom or a C1-C6 alkyl group;
$R^2$ means a halogen atom, a C1-C6 alkyl group, a C2-C6 alkenyl group, a C3-C7 cycloalkyl group, a halo lower alkyl group, an alkanoyl group, an aryl group, a heteroaryl group, an aroyl group, an aralkyl group, a C1-C6 alkoxy group, an aryloxy group, an aralkyloxy group, -$(CH_2)_n$-$NR^9R^{10}$, -$CONR^9R^{10}$, -$NR^9COR^{11}$, -$S(O)_pR^{11}$, or -$SO_2NR^9R^{10}$, or means a 5- to 6-membered hydrocarbon ring which is formed by $R^1$ and $R^2$ together with the carbon atom to which they are bound (wherein n means an integer of 0 to 2, $R^9$ and $R^{10}$ each independently means a hydrogen atom, a C1-C6 alkyl group, a C2-C6 alkenyl group, a C3-C7 cycloalkyl group, an aryl group, a heteroaryl group, or an aralkyl group, or $R^9$ and $R^{10}$ mean a 5- to 6-membered heterocyclic ring which is formed by $R^9$ and $R^{10}$ together with the nitrogen atom to which they are bound or alternatively, another nitrogen atom or oxygen atom, $R^{11}$ means a C1-C6 alkyl group, a C2-C6 alkenyl group, a C3-C7 cycloalkyl group, an aryl group, a heteroaryl group, or an aralkyl group, and p means an integer of 0 to 2);
$R^3$ means a hydrogen atom, a C1-C6 alkyl group, or an acyl group;
$R^4$ means a hydrogen atom or a C1-C6 alkyl group;
$R^5$ means a hydrogen atom, a C1-C6 alkyl group, a halo lower alkyl group, or a cyano group;
$R^6$ means a hydrogen atom or a C1-C6 alkyl group;
$R^7$ means a hydrogen atom, a halogen atom, or a C1-C6 alkyl group;
$R^8$ means a hydrogen atom, a halogen atom, or a C1-C6 alkyl group; and
E means any group selected from the group represented by the following formulae (a) to (f):

(a) -NHCO-G-COR$^{12}$,
(b) -NH-G-COR$^{12}$,

(c) -O-G-$COR^{12}$,
(d) -CONH-G-$COR^{12}$,
(e) -NHCO-G-tetrazolyl group, and
(f) -G-$COR^{12}$,

(wherein G means a single bond or a C1-C6 alkylene group, and $R^{12}$ means a hydroxy group or a C1-C6 alkoxy group)].

**17.** The use according to claim 16, wherein the compound defined by the general formula (I) is such that A is $-CH_2-$, $R^1$, $R^3$, $R^4$, $R^6$, and $R^7$ are each a hydrogen atom, $R^2$ is an i-propyl group, a s-butyl group, a 4-fluorobenzyl group, or a 4-(fluorophenyl)hydroxymethyl group, $R^5$ is a methyl group or a trifluoromethyl group, $R^8$ is a methyl group, and E is -NHCO-G-$COR^{12}$ (wherein G is a single bond or $-CH_2-$, and $R^{12}$ is a hydroxy group or a C1-C3 alkoxy group).

**18.** The use according to claim 16, wherein the medicament as a thyroid hormone receptor ligand is a preventing or treating agent of a disease or a disorder, symptom of which is improved by cell functional regulation via a thyroid hormone receptor.

**19.** The use according to claim 18, wherein the disease or the disorder, symptom of which is improved by cell functional regulation via a thyroid hormone receptor, is hyperlipemia, obesity, hypothyroidism, hyperthyroidism, goiter, thyroid cancer, cardiac arrhythmia, congestive heart failure, diabetes, depression, osteoporosis, skin disorder, glaucoma, or alopecia.

## INTERNATIONAL SEARCH REPORT

International application No.
PCT/JP2007/063612

A. CLASSIFICATION OF SUBJECT MATTER
*C07D209/08*(2006.01)i, *A61K31/404*(2006.01)i, *A61K31/416*(2006.01)i, *A61P3/04*(2006.01)i, *A61P3/06*(2006.01)i, *A61P3/10*(2006.01)i, *A61P5/14*(2006.01)i, *A61P5/16*(2006.01)i, *A61P9/04*(2006.01)i, *A61P9/06*(2006.01)i,
According to International Patent Classification (IPC) or to both national classification and IPC

B. FIELDS SEARCHED
Minimum documentation searched (classification system followed by classification symbols)
C07D209/08, A61K31/404, A61K31/416, A61P3/04, A61P3/06, A61P3/10, A61P5/14, A61P5/16, A61P9/04, A61P9/06, A61P17/00, A61P17/02, A61P17/14, A61P19/10, A61P27/06, A61P35/00, A61P43/00, C07D209/12, C07D231/56

Documentation searched other than minimum documentation to the extent that such documents are included in the fields searched

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | US 2005/0014812 A1 (FUJISAWA PHARMACEUTICAL CO., LTD.),<br>20 January, 2005 (20.01.05),<br>Full text; particularly, abstract; Claims 1, 3, 10; example 8<br>& WO 2004/108686 A2<br>(cited in the description of the present application as "patent document 11")<br>& AU 200390286 D0 | 1-7,9-10,12, 14-16,18-19 |
| X | WO 2006/057946 A2 (THRESHOLD PHARMACEUTICALS, INC.),<br>01 June, 2006 (01.06.06),<br>Full text; particularly, abstract; Claims 1 to 6, 42; page 66, 'compound 59'<br>& EP 1819338 A2 & AU 2005309761 A1<br>& CA 2587210 A1 | 1-2,4-7,9,12 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 September, 2007 (11.09.07) | 25 September, 2007 (25.09.07) |
| Name and mailing address of the ISA/<br>Japanese Patent Office | Authorized officer |
| Facsimile No. | Telephone No. |

**INTERNATIONAL SEARCH REPORT**

International application No.
PCT/JP2007/063612

C (Continuation). DOCUMENTS CONSIDERED TO BE RELEVANT

| Category* | Citation of document, with indication, where appropriate, of the relevant passages | Relevant to claim No. |
|---|---|---|
| X | WO 03/022814 A1 (ONO PHARMACEUTICAL CO., LTD.), 20 March, 2003 (20.03.03), Full text; particularly, abstract; Claims; (cited in the description of the present application as "patent document 13") & WO 01/66520 A1 (cited in the description of the present application as "patent document 12") & EP 1262475 A1 & EP 1424335 A1 & US 2003/176400 A1 & US 2004/180885 A1 & US 2005/004097 A1 & US 2006/194864 A1 & AU 4106801 A & BR 0109050 A & CA 2402174 A1 & CA 2459515 A1 & CN 1427824 A & HU 0301493 A2 & MX PA02008801 A & NO 20024281 A & NZ 521192 A & RU 2002123882 A & ZA 200207031 A | 1-2,4-7, 9-10,12 |
| X | WO 2004/078719 A1 (ONO PHARMACEUTICAL CO., LTD.), 16 September, 2004 (16.09.04), Full text; particularly, abstract; Claims; (cited in the description of the present application as "patent document 14") & EP 1600440 A1 & US 2006/089353 A1 | 1-2,4-7, 9-10,12 |
| A | JP 2002-538133 A (PFIZER PRODUCTS INC.), 12 November, 2002 (12.11.02), Full text; particularly, abstract; Claims & WO 00/51971 A1 (cited in the description of the present application as "patent document 1") & EP 1157001 A1 & US 6326398 B1 & US 2002/049226 A1 & US 2003/114521 A1 & JP 2005-162759 A & AP 200102259 D0 & AT 272609 T & AU 2457500 A & BG 105954 A & BR 0008701 A & CA 2363145 A1 & CN 1345303 A & CN 1515548 A & CZ 20013117 A3 & DE 60012700 D1 & DK 1157001 T3 & DZ 3020 A1 & EA 4229 B1 & EE 200100464 A & ES 2223454 T3 & HR 20010633 A2 & HU 0200248 A2 & ID 29889 A & IL 144765 D0 & IS 6054 A & MA 26723 A1 & NO 20014217 A & NZ 513449 A & OA 11839 A & PL 364802 A1 & PT 1157001 T & SK 12102001 A3 & TR 200102561 T2 & ZA 200106730 A | 1-19 |

Form PCT/ISA/210 (continuation of second sheet) (April 2005)

**INTERNATIONAL SEARCH REPORT**

International application No.
PCT/JP2007/063612

C (Continuation). DOCUMENTS CONSIDERED TO BE RELEVANT

| Category* | Citation of document, with indication, where appropriate, of the relevant passages | Relevant to claim No. |
|---|---|---|
| A | WO 01/070687 A1 (BAYER AG.), 27 September, 2001 (27.09.01), Full text; particularly, abstract; Claims; (cited in the description of the present application as "patent document 2") & EP 1268422 A1 & AU 6211301 A & CA 2403806 A & DE 10065434 A | 1-19 |
| A | JP 2004-517851 A (BAYER AG.), 17 June, 2004 (17.06.04), Full text; particularly, abstract; Claims & WO 02/51805 A1 (cited in the description of the present application as "patent document 3") & EP 1347959 A1 & US 2003/078288 A1 & US 2005/032874 A1 & CA 2433100 A1 & DE 10130830 A1 | 1-19 |
| A | WO 02/22586 A1 (BAYER AG.), 21 March, 2002 (21.03.02), Full text; particularly, abstract; Claims; (cited in the description of the present application as "patent document 6") & EP 1324987 A1 & US 2002/193610 A1 & AU 1048402 A & CA 2422353 A1 & DE 10046029 A1 | 1-19 |
| A | JP 2003-160559 A (PFIZER PRODUCTS INC.), 03 June, 2003 (03.06.03), Full text; particularly, abstract; Claims & EP 1297833 A1 (cited in the description of the present application as "patent document 4") & US 2003/078289 A1 & AT 313325 T & BR 0203877 A & CA 2404848 A1 & DE 60208132 D1 & ES 2253495 T3 & MX PA02009472 A | 1-19 |
| A | WO 2004/018421 A1 (KAROBIO AB.), 04 March, 2004 (04.03.04), Full text; particularly, abstract; Claims; (cited in the description of the present application as "patent document 5") & AU 2003251705 A1 & GB 219022 D0 | 1-19 |
| A | US 2004/0220415 A1 (BAYER AG.), 04 November, 2004 (04.11.04), Full text; particularly, astract; Claims & WO 02/079181 A1 (cited in the description of the present application as "patent document 7") & EP 1373239 A1 & CA 2442265 A1 & DE 10115408 A1 | 1-19 |

Form PCT/ISA/210 (continuation of second sheet) (April 2005)

**INTERNATIONAL SEARCH REPORT**

International application No.
PCT/JP2007/063612

C (Continuation). DOCUMENTS CONSIDERED TO BE RELEVANT

| Category* | Citation of document, with indication, where appropriate, of the relevant passages | Relevant to claim No. |
|---|---|---|
| A | JP 10-504297 T (KAROBIO AB.), 28 April, 1998 (28.04.98), Full text; particularly, astract; Claims & WO 96/05190 A1 (cited in the description of the present application as "patent document 8") & EP 0775129 A1 & US 5854282 A & AT 172460 T & AU 3345595 A & CA 2197185 A1 & DE 69505542 D1 & DK 775129 T3 & ES 2123287 T3 & GB 9416219 D0 | 1-19 |

Form PCT/ISA/210 (continuation of second sheet) (April 2005)

INTERNATIONAL SEARCH REPORT

International application No.
PCT/JP2007/063612

Continuation of A. CLASSIFICATION OF SUBJECT MATTER
(International Patent Classification (IPC))

*A61P17/00*(2006.01)i, *A61P17/02*(2006.01)i, *A61P17/14*(2006.01)i, *A61P19/10*(2006.01)i, *A61P25/24*(2006.01)i, *A61P27/06*(2006.01)i, *A61P35/00*(2006.01)i, *A61P43/00*(2006.01)i, *C07D209/12*(2006.01)i, *C07D231/56*(2006.01)i

(According to International Patent Classification (IPC) or to both national classification and IPC)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 00051971 A
- WO 01070687 A
- WO 02051805 A
- EP 1297833 A
- WO 2004018421 A
- WO 02022586 A
- WO 02079181 A
- WO 9605190 A
- EP 780386 A
- WO 9710219 A
- WO 2004108686 A
- WO 01066520 A
- WO 03022813 A
- WO 2004078719 A

### Non-patent literature cited in the description

- *Expert Opin. Ther. Patent,* 2004, vol. 14 (8), 1169-1183
- *Progress of Medicinal Chemistry,* 1980, vol. 17, 151-183
- Protecting Groups in Organic Synthesis. Wiley, 1999
- *J. Med. Chem.,* 2003, vol. 46 (9), 1580-1588
- *Tetrahedron Lett.,* 1987, vol. 28 (40), 4725-4728
- *J. Med. Chem.,* 1995, vol. 38 (4), 695-707
- *Tetrahedron,* 1990, vol. 46 (17), 6085-6112
- *Chem. Pharm. Bull.,* 2001, vol. 49 (1), 87-96
- *Bull. Chem. Soc. Jpn.,* 1995, vol. 68 (5), 1497-1507
- *Tetrahedron,* 2004, vol. 60 (2), 347-358
- *J. Chem. Soc. Perkin-I,* 2000, 1045-1075
- *Heterocycles,* 1994, vol. 38 (11), 2415-2422
- *Synthesis,* 1992, 1283-1286
- Comprehensive Heterocyclic Chemistry. Pergamon, 1984
- *Bioorg. Med. Chem. Lett.,* 2000, vol. 10 (10), 2607-2611
- Comprehensive Organic Transformations: A Guide to Functional Group Preparation. John Wiley & Sons Inc, 1999